(19) Europäisches Patentamt
European Patent Office
Office européen des brevets

(11) EP 4 385 499 A1

(12) EUROPEAN PATENT APPLICATION

(43) Date of publication:
19.06.2024 Bulletin 2024/25

(51) International Patent Classification (IPC):
A61K 9/19 (2006.01)   A61K 9/50 (2006.01)
A61K 41/00 (2020.01)

(21) Application number: 23020495.0

(22) Date of filing: 08.11.2023

(52) Cooperative Patent Classification (CPC):
A61K 9/19; A61K 9/5036; A61K 9/5094;
A61K 41/0052

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 15.11.2022  EP 22020559
07.12.2022  FR 2212910

(71) Applicant: **Nanobacterie**
**75116 Paris (FR)**

(72) Inventors:
• **Alphandery, Edouard**
**75116 Paris (FR)**
• **Chebbi, Imene**
**91140 Villebon Sur Yvette (FR)**

(54) **A METHOD TO ENABLE NANOPARTICLES STORED WITH A SPECIFIC TYPE OF ASSEMBLY TO MAINTAIN THIS TYPE OF ASSEMBLY UPON RECONSTITUTION**

(57) The invention relates to a composition comprising at least one nanoparticle or at least one chain of at least two nanoparticles, wherein each nanoparticle in the chain preferentially comprises an iron oxide mineral core preferentially surrounded by a coating, wherein the composition further comprises a cryo-protectant or protectant compound, wherein the volume occupied by the cryo-protectant or protectant compound in the composition is preferentially larger than the volume occupied by at least one chain in the composition, preferentially by a factor of at least 1, 2, 5, 10 or $10^3$, wherein the percentage in mass of cryo-protectant or protectant compound in the composition is preferentially comprised between 0.5 and 50 %, wherein the composition is preferentially isotonic.

EP 4 385 499 A1

## Description

### FIELD OF THE INVENTION

The field of the invention is that of nanoparticle or at least one constituent of the compositions, displaying a specific type of organization, such as an organization in chains, whose organization can be maintained over a long period of time, notably to store the chains, by lyophilizing these nanoparticle or at least one constituent of the compositions in the presence of a cryo-protectant or protectant compound, by maintaining these nanoparticle or at least one constituent of the compositions lyophilized or de-hydrated during a certain period of time, and by resuspending the at least one nanoparticle or at least one constituent of the composition or chains in a liquid such as water, preferentially before their use or administration to humans.

### TECHNICAL BACKGROUND

Some nanoparticle or at least one constituent of the compositions are known to arrange themselves in a specific way. For example, magnetosomes synthesized by magnetotactic bacteria which consist of iron oxide mineral cores covered with organic lipid bilayers form chains, (Alphandéry et al, Drug Discovery Today, V. 25, P. 1444, 2020). We were able to remove the lipid bilayer covering the mineral core of the magnetosomes, replace it with a synthetic coating, see reference above. However, the synthetic coating of the magnetosome mineral core such as carboxy-methyl-dextran or citric acid or a polymer can degrade in water over time. These nanoparticles can't easily be kept arranged in chains in suspension over a long period of time. Here, we propose a new product consisting of at least one nanoparticle or at least one constituent of the composition or chains of magnetosomes mixed with a cryo-protectant or protectant compound, which are lyophilized or de-hydrated to be stored in powder form over a long period of time. They are then preferentially resuspended in a liquid, where they preferentially reconstitute chains, before use.

### DESCRIPTION OF THE INVENTION

The invention relates to a composition or at least one constituent of the composition preferentially comprising at least one nanoparticle or at least one constituent of the composition or at least one chain of at least two nanoparticle or at least one constituent of the compositions, wherein preferentially each nanoparticle or at least one constituent of the composition in the chain or the at least one nanoparticle or at least one constituent of the composition preferentially comprises an iron oxide mineral core or an iron oxide core or a metallic core or a core preferentially composed of a metal oxide, preferentially surrounded by a coating, wherein preferentially the composition preferentially further comprises a cryo-protectant or protectant compound that is optionally mixed with water, wherein preferentially the dissociating energy between the coating and the core is preferentially larger than the dissociating energy between the cryoprotectant and the core, wherein the composition is preferentially in the form of a powder or a liquid suspension, wherein the coating preferentially maintains the chain arrangement above 0°C, wherein the cryo-protectant or protectant compound preferentially maintains the chain arrangement or composition or size or cohesion or at least one property or magnetic property, preferentially ferrimagnetic property of the at least one nanoparticle or at least one constituent of the composition or composition, preferentially below 0°C, or preferentially under the application of a temperature gradient or variation, or preferentially under a change in oxidation or reduction or oxidation state of the at least one nanoparticle or at least one constituent of the composition, wherein the chain arrangement or composition or size or cohesion or at least one property or magnetic property, preferentially ferrimagnetic property, of the at least one nanoparticle or at least one constituent of the composition or composition, is preferentially maintained at a temperature that is preferentially lower than 0°C, or preferentially under the application of a temperature gradient or variation, or preferentially under a change in oxidation or reduction or oxidation state of the at least one nanoparticle or at least one constituent of the composition, wherein the composition or at least one constituent of the composition is preferentially isotonic.

In some cases, the constituent of the composition is selected in the group consisting of: i) the nanoparticle, ii) the nanoparticle coating, iii) the nanoparticle core, iv) the cryo-protectant or protectant compound, v) the other compound, vi) at least one link or bond or force or interaction of or between at least one or two constituent(s) of the composition, vii) a solute, viii) a solvent, ix) an excipient, x) an active part or principle, xi) an inert part, xii) an organic or carbon or carbonaceous part, xiii) an inorganic or metallic part, xiv) a medical device or drug, xv) a pharmaceutical compound, xvi) an immunological compound, xvii) a metabolic compound, xviii) a chemotherapeutic compound, xix) a surgical compound, xx) a radio-sensitizer, xxi) a contrast agent, and xxii) a sonosensitizer of the composition.

In some cases, the constituent is comprised in the organic or inorganic part of the composition.

In some cases, the constituent is comprised in the inert or active part of the composition.

The invention also relates to a composition comprising at least one nanoparticle or at least one constituent of the composition or at least one chain of at least two nanoparticle,

wherein preferentially the at least one nanoparticle core or at least one constituent of the composition or at least one nanoparticle in the chain comprises a core, preferentially a mineral core, preferentially a metallic core, preferentially a crystallized core, most preferentially an iron oxide and/or mineral core, preferentially surrounded preferentially partly or fully by a coating,

wherein the composition preferentially further comprises a cryo-protectant or protectant compound, wherein preferentially the volume occupied by the cryo-protectant or protectant compound in the composition is larger than the volume occupied by the at least one nanoparticle or at least one constituent of the composition or the at least one chain in the composition, preferentially by a factor preferentially $\alpha$ of at least 1, 2, 5, 10 or $10^3$,

wherein preferentially the percentage in mass of cryo-protectant in the composition is comprised between 0.5 and 50 %.

The invention also relates to the composition according to the invention comprising at least one nanoparticle or at least one constituent of the composition or at least one chain of at least two nanoparticles, wherein preferentially at least one nanoparticle comprises an iron oxide mineral core surrounded by a coating,

wherein preferentially the composition further comprises a cryo-protectant or protectant compound, wherein preferentially the volume occupied by the cryo-protectant or protectant compound in the composition is larger than the volume occupied by at least one chain in the composition, by a factor of preferentially at least 1, 2, 5, 10 or $10^3$, wherein preferentially the percentage in mass of cryo-protectant or protectant compound in the composition is comprised between 0.5 and 50 %, wherein preferentially the composition is isotonic.

The invention also relates to the composition according to the invention, wherein the composition, comprises an organic part and/or an inorganic part, wherein the inorganic part preferentially comprises the core of the nanoparticle, wherein the organic part preferentially comprises the coating of the nanoparticle or at least one constituent of the composition different from the nanoparticle core and/or the cryo-protectant, and wherein the percentage in mass of the inorganic part is preferentially larger than the percentage in mass of the organic part.

In one embodiment of the invention, a protectant compound is a compound that maintains or prevents the variation of at least one property of the nanoparticle or at least one constituent of the composition or composition preferentially by more than $10^{20}$, $10^{10}$, $10^5$, 10, 5, 2, 1 0% or preferentially by a factor of more $10^{20}$, $10^{10}$, $10^5$, 10, 5, 2, 1 0, preferentially over time, preferentially over more than 1 second or 1 year, preferentially between the time $t_1$ and $t_2$, where the properties of the nanoparticle or at least one constituent of the composition or composition at times $t_1$ and $t_2$ are preferentially $P_1$ and $P_2$.

In some cases, $P_2/P_1$ and/or $t_2/t_1$ can be smaller than $10^{20}$, $10^{10}$, $10^5$, 10, 5, 2, 1 0.

In some other cases, $P_2/P_1$ and/or $t_2/t_1$ can be larger than 0, $10^{-10}$, 0.1, 0, 1, 10, 50, $10^3$ or $10^5$.

In some cases, the factor $\alpha$ is equal to $V_2/V_1$, where $V_2$ is the volume occupied by the cryoprotectant or protectant compound in the composition and $V_1$ is the volume occupied by the at the at least one nanoparticle or at least one constituent of the composition or the at least one chain in the composition. In some cases, $\alpha$ is larger than or equal to 1, 2, 5, 10, 100, $10^3$ or $10^5$.

In some other cases, $\alpha$ is smaller than or equal to $10^5$, $10^3$, 100, 10, 5, 2 or 1.

In some other cases, $\alpha$ is larger when the composition is partly or fully in a liquid state than when it is partly or fully in a solid or powder form.

In some cases, the percentage in mass of cryo-protectant or protectant compound or nanoparticle or of at least one constituent in or of the composition is larger than or equal to 0, $10^{-10}$, $10^{-1}$, 1, 5, 10, 50, 75, 99 or 100%.

In some other cases, the percentage in mass of cryo-protectant or protectant compound or nanoparticle or of at least one constituent in or of in the composition is smaller than or equal to 100, 99.99, 99, 85, 75, 50, 25, 20, 10, 5, 2, 1 or 0%.

In still some other cases, the percentage in mass of the nanoparticle or of at least one first constituent in the composition is larger preferentially by a factor $\beta$ than the percentage in mass of the cryo-protectant or protectant compound or of at least one second constituent in or of the composition in the composition, preferentially when the composition is in powder or solid form.

In some cases, the factor $\beta$ is equal to $PM_2/PM_1$, where $PM_2$ is the percentage in mass of the at least one nanoparticle or at least one constituent of the composition in the composition and $PM_1$ is the percentage in mass of the cryo-protectant or protectant compound in the composition.

In some cases, $\beta$ is larger than or equal to 1, 2, 5, 10, 100, $10^3$ or $10^5$.

In some other cases, $\beta$ is smaller than or equal to $10^5$, $10^3$, 100, 10, 5, 2 or 1.

In one embodiment of the invention, the composition or at least one constituent of the composition or nanoparticle, preferentially nanoparticle core, comprises a percentage in mass in at least one metal preferentially iron preferentially in terms of metallic composition that is larger than 0, 1, 5, 10, 50, 75, 90, 99 or 99.9%.

In one embodiment of the invention, the composition or at least one constituent of the composition or nanoparticle, preferentially nanoparticle coating, comprises a percentage in mass in at least one metal preferentially iron preferentially in terms of metallic composition that is smaller than 100, 99.9, 90, 75, 50, 25, 10, 5, 2 or 1%.

In one embodiment of the invention, the percentage(s) in mass of the nanoparticle or at least one constituent of the composition, preferentially in the dried composition, is(are) larger than or equal to 0, 1, 5, 10, 50, 75, 90, 99 or 99.9%.

In another embodiment of the invention, the percentage(s) in mass of the nanoparticle or at least one constituent of the composition, preferentially in the dried composition, is(are) smaller than or equal to 100, 99.9, 90, 75, 50, 25, 10, 5, 2 or 1%.

In one embodiment of the invention, the percentage(s) in mass of the inorganic and/or organic part(s) in the composition, preferentially in the dried composition, is(are) larger than or equal to 0, 1, 5, 10, 50, 75, 90, 99 or 99.9%.

In another embodiment of the invention, the percentage(s) in mass of the inorganic and/or organic part(s) in the composition, preferentially in the dried composition, is(are) smaller than or equal to 100, 99.9, 90, 75, 50, 25, 10, 5, 2 or 1%.

In one embodiment of the invention, the composition, the nanoparticle or at least one constituent of the composition(s), the core and/or coating of the nanoparticle, the cryoprotectant and/or the protectant compound or other compound has/have at least one of the following properties selected in the group consisting of:

(a) magnetic, diamagnetic, superparamagnetic, ferromagnetic, ferrimagnetic, and/or paramagnetic behavior(s) or property(ies), preferentially observed under the application of magnetic field of strength preferentially larger than $10^{-50}$, $10^{-40}$, $10^{-20}$, $10^{-10}$, $10^{-5}$, $10^{-2}$ or $10^{-1}$ T, preferentially observed at temperatures lower than $10^{10}$, $10^5$, $10^3$, $10^2$, 10 or 1 K, wherein in some cases, the core can have different magnetic property(ies) from the coating, for example, the core can be ferromagnetic or superparamagnetic while the coating can be diamagnetic or paramagnetic.

(b) a crystalline part or structure comprising at least 1, 2, 5, 10, 50, 100, $10^3$, $10^5$, $10^7$, $10^9$, $10^{20}$ or $10^{50}$ crystalline plane(s) or crystalline ordered structures, which can preferentially be observed or measured under electron microscopy, wherein in some cases, the core can have a different crystalline structure from the coating, for example, the core can comprise more than 1, 5, 10, $10^3$ or $10^5$ crystalline plane(s) or crystalline ordered structure(s) while the coating can have less than $10^5$, $10^3$, 10, 5 or 2 crystalline planes or crystalline ordered structures.

(c) a composition made of metal(s) or metal oxide(s), preferentially iron oxide, most preferentially maghemite and/or magnetite, wherein in some cases, the core comprises a different composition from the coating, for example, the core comprises more than 1, 5, 10, 25, 50, 75, 90, 95 or 99 percent or percent in mass of iron oxide while the coating comprises less than 99, 95, 90, 75, 50, 10, 5 or 1 percent or percent in mass of iron oxide, wherein this percentage can be the ratio between the quantity, volume, number of atoms, mass of iron oxide comprised in the core and/or coating divided by the total quantity, total volume, total number of atoms, total mass, of all chemical element(s) comprised in the core and/or coating.

(d) single domain, or be magnetically mono-domain,

(e) a magnetic microstructure, which can be characterized by the presence of magnetic field lines, which can be oriented in a preferential direction such as an axis of easy magnetization or a crystallographic direction of the core of the nanoparticle or at least one constituent of the composition(s) such as [111], where such a magnetic microstructure can under certain conditions be observable, in particular by electronic holography,

(f) a size comprised between 1 nm and $10^5$ $\mu$m, 1 nm and $10^3$ $\mu$m, 1 nm and 100 $\mu$m, 1 nm and 10 $\mu$m, 1 nm and 1 $\mu$m, 5 nm and 1 $\mu$m, 5 and 500 nm, 5 and 250 nm, 5 and 100 nm, 5 and 80 nm, 5 and 60 nm, 10 nm and 1 $\mu$m, 10 and 500 nm, 10 and 250 nm, 10 and 100 nm, 10 and 80 nm, 10 and 60 nm, 15 nm and 1 $\mu$m, 15 and 500 nm, 15 and 250 nm, 15 and 100 nm, 15 and 80 nm, 15 and 60 nm, 20 nm and 1 $\mu$m, 20 and 500 nm, 20 and 250 nm, 20 and 100 nm, 20 and 80 nm, or between 20 et 60 nm,

(g) a size in some cases larger than 0.1, 1, 2, 5, 10, 15, 20, 25, 30, 35 or 40 nm,

(h) a size in some other cases lower than $10^{10}$, $10^5$, $10^4$, 2000, 1000, 500, 400, 300, 200, 150, 120, 100, 95, 90, 80, 75, 70, 65, 60, 55, 50, 45, 40, 35, 30, 25, 20, 15, 10 or 5 nm,

(i) a zeta potential, charge, or surface charge comprised between $-10^{10}$ mV and $10^{10}$ mV, $-10^5$ mV and $10^5$ mV, $-10^4$ mV and $10^4$ mV, $-10^3$ mV, $-10^2$ mV and $10^2$ mV, -10 and 10 mV, preferentially at pH comprised between 0 and 14, 1 and 13, 2 and 12, 3 and 11, 4 and 10, 5 and 9, or between 6 and 8.

(j) a zeta potential, charge, or surface charge, which is in some cases larger than $-10^{50}$, $-10^{20}$, $-10^{10}$, $-10^5$, $-10^3$, -10, -5, -1, 0, 5, 10, 20, 50, or 100 mV, preferentially at pH larger than 0, 1, 2, 3, 4, 5, 6, 7, 8, 9, 10, 11, 12 or 13,

(k) a zeta potential, charge, or surface charge, which is in some other cases larger than $-10^{50}$, $-10^{20}$, $-10^{10}$, $-10^5$, $-10^3$, -10, -5, -1, 0, 5, 10, 20, 50, or 100 mV, preferentially at pH lower than 14, 13, 12, 11, 10, 9, 8, 7, 6, 5, 4, 3, 2, 1 or 0,

(l) a zeta potential, charge, or surface charge, which is in some other cases lower than $10^{50}$, $10^{20}$, $10^{10}$, $10^5$, $10^3$, 10, 5, 1, 0, -5, -10, -20, -50, or -100 mV, preferentially at pH larger than 0, 1, 2, 3, 4, 5, 6, 7, 8, 9, 10, 11, 12 or 13,

(m) a zeta potential, charge, or surface charge, which is in some other cases lower than $10^{50}$, $10^{20}$, $10^{10}$, $10^5$, $10^3$, 10, 5, 1, 0, -5, -10, -20, -50, or -100 mV, preferentially at pH lower than 14, 13, 12, 11, 10, 9, 8, 7, 6, 5, 4, 3, 2, 1 or 0.

(n) an isoelectric point comprised between 0 and 14, 1 and 13, 2 and 12, 3 and 11, 4 and 10, 5 and 9, or between 6 and 8,

(o) in some cases, an isoelectric point in some cases larger than 0, 1, 2, 3, 4, 5, 6, 7, 8, 9, 10, 11, 12 or 13, and/or

(p) in some other cases, an isoelectric point in some other cases lower than 14, 13, 12, 11, 10, 9, 8, 7, 6, 5, 4, 3, 2, 1 or 0,

(q) an isotonicity, preferentially in some cases at pH smaller or larger than 0, 1, 2, 3, 4, 5, 6, 7, 8, 9 10, 11, 12, 13 or 14, preferentially in some other cases at pH comprised between 0 &nd 14 or between 1 and 13 or between 3 and 10,

(r) an interaction strength between two constituents of the composition, such as the core and coating, that is sufficiently strong to form a complex comprising these two constituents,

(s) an interaction strength between two constituents of the composition, such as the nanoparticle or at least one constituent of the composition and cryo-protectant or protectant compound, that is sufficiently weak to prevent the formation of a complex comprising these two constituents

(t) an interaction or bond or interaction or force between two constituents of the composition that is of the type selected in the group consisting of : i) weak, ii) metallic, iii) ionic, iv) covalent, v) London or dispersion, vi) dipole-dipole, vii) hydrogen, viii) nonpolar or polar bond, ix) van der Waals, x) electrostatic, xi) charged, xii) magnetic, xiii) thermal, ix) atomic, x) molecular, xi) nanometric, xii) complexation, and xiii) solid, liquid, gaseous interaction or bond or interaction or force. and

(r) a solid, liquid or gaseous form or state.

In some cases, the coating and core form a complex.

In some other cases, the cryo-protectant or protectant compound and nanoparticle or at least one constituent of the composition different from the cryo-protectant or protectant compound don't form a complex or form a weaker or less stable complex than the complex formed by the coating and core. The invention also relates to a composition comprising at least one or two nanoparticle(s) or at least one constituent of the compositions, preferentially organized in a geometric figure or assembly, wherein each nanoparticle or at least one constituent of the composition in the geometric figure or assembly preferentially comprises a metallic or mineral or crystallized core or an iron oxide mineral core surrounded partly or fully by a coating,

wherein the composition preferentially further comprises a cryoprotectant or protectant compound preferentially mixed with water solid, liquid, or gas, wherein preferentially the dissociating energy between the coating and the core is preferentially larger than the dissociating energy between the cryoprotectant or protectant compound and the nanoparticle or at least one constituent of the composition different the cryoprotectant or protectant compound, wherein the cryo-protectant or protectant compound preferentially serves to maintain the geometric figure or assembly of the at least one nanoparticle or at least one constituent of the composition stable over a period of at least 1 second or one or six months.

In one embodiment, the protectant compound is selected in the group consisting of: i) a cryo-protectant, ii) a thermo-protectant, iii) an oxydo-protectant, iv) a chain-protectant, v) an activity-protectant and vi) a protectant of the composition or size or cohesion or at least one property or magnetic property of at least one nanoparticle or at least one constituent of the composition.

In some cases, an activity-protectant compound is a compound that protects the activity, preferentially a therapeutic, immunological, pharmacological, chemotherapeutical, metabolic, thermal, and/or vaccine-type activity of the nanoparticle or at least one constituent of the composition, i.e. preferentially without the activity-protectant compound the activity of the nanoparticle or at least one constituent of the composition is diminished or decreased or lost partly or fully preferentially over time or under thermal variation or under the application of a radiation on the composition or at least one constituent of the composition.

In some cases, the composition can comprise an active part and an inactive part or different parts with different types of activity, for example one part with a medical or thermal activity such as the part comprising the nanoparticle core and/or coating and another part with an activity consisting in preserving the at least one property of the nanoparticle or at least one constituent of the composition such as the part comprising the cryoprotectant or protectant compound.

In some cases, the composition is of high purity, preferentially in terms of metallic composition or of composition in iron.

In some cases, a high purity composition designates a composition that comprises more than 50, 90, 96, 99 or 99% of iron preferentially in terms of metallic composition.

In some cases, a cryo-protectant or protectant compound can be a compound that protects or maintains or avoids a change of at least one property of the composition or nanoparticle or at least one constituent of the composition, preferentially when the composition or nanoparticle or at least one constituent of the composition is cooled down, preferentially by at least or below 100, 10, 0, -1, -20, -50, -100, or -273 °C.

In some cases, a thermo-protectant or protectant compound can be a compound that protects or maintains or avoids a change of at least one property of the composition or nanoparticle or at least one constituent of the composition, preferentially when the composition or nanoparticle or at least one constituent of the composition is subject to a temperature gradient, preferentially by at least 0, 1, 2, 5, 10 or 100 °C.

In some cases, an oxydo-protectant or protectant compound can be a compound that protects or maintains or avoids a change of at least one property of the composition or nanoparticle or at least one constituent of the composition such as the oxidation state of the nanoparticle or at least one constituent of the composition or composition, preferentially when the composition or nanoparticle or at least one constituent of the composition is subject to oxidation or reduction.

In some cases, a chain-protectant or protectant compound can be a compound that protects or maintains or avoids a change or destruction or diminution or increase or variation in size of the chain of nanoparticle or at least one constituent of the composition, preferentially when the composition or nanoparticle or at least one constituent of the composition is subject to oxidation or reduction or subject to a temperature gradient or is cooled down or is exposed to a radiation.

In some cases, a size-protectant or protectant compound can be a compound that protects or maintains or avoids a change or destruction or diminution or increase or variation in size of the nanoparticle or at least one constituent of the composition, preferentially by more than 0, 1, 10, 100, $10^3$ or $10^5$ nm, preferentially when the composition or nanoparticle or at least one constituent of the composition is subject to oxidation or reduction or subject to a temperature gradient or is cooled down or is exposed to a radiation.

In some cases, a composition-protectant or protectant compound can be a compound that protects or maintains or avoids a change or destruction or diminution or increase or variation in composition of the nanoparticle or at least one constituent of the composition, preferentially by more than 1, 10, 99, 100, $10^3$, $10^5$ or $10^{10}$ atoms or percent of atoms, preferentially when the composition or nanoparticle or at least one constituent of the composition is subject to oxidation or reduction or subject to a temperature gradient or is cooled down or is exposed to a radiation.

In some cases, a cohesion-protectant or protectant compound can be a compound that protects or maintains or avoids a change or destruction or diminution or increase or variation in cohesion of the nanoparticle or at least one constituent of the composition or composition, preferentially when the composition or nanoparticle or at least one constituent of the composition is subject to oxidation or reduction or subject to a temperature gradient or is cooled down or is exposed to a radiation.

In some cases, the cohesion of the nanoparticle or at least one constituent of the composition or composition can be at least one interaction or at least one bond or interaction or force or link between at least two constituents of the composition, which preferentially maintain the cohesion or assembly or at least one property or the content of the composition or nanoparticle or at least one constituent of the composition.

In some cases, a magnetic property-protectant or protectant compound can be a compound that protects or maintains or avoids a change or destruction or diminution or increase or variation in magnetic property or coercivity or magnetization or saturating magnetization of the nanoparticle or at least one constituent of the composition or composition, preferentially by more than 1, 10, 99, 100, $10^3$, $10^5$ or $10^{10}$ Oe or mT or percent of this property, preferentially when the composition or nanoparticle or at least one constituent of the composition is subject to oxidation or reduction or subject to a temperature gradient or is cooled down or is exposed to a radiation.

In some cases, at least one magnetic property of the nanoparticle or at least one constituent of the composition or composition can be a diamagnetic, paramagnetic, superparamagnetic, ferromagnetic or ferrimagnetic property.

In some cases, the at least one property of the nanoparticle or at least one constituent of the composition or composition exists or is measured at a temperature lower than $10^{10}$, $10^5$, $10^3$, 100, 50, 10, 5, 2, 1, 0.1 or 0 K (Kelvin) or °C (Celsius degree).

In some cases, the at least one property of the nanoparticle or at least one constituent of the composition or composition exists or is measured at a temperature larger than 0, 0.1, 1, 5, 10, 50, 100, $10^3$, $10^5$ or $10^{10}$ K (Kelvin) or °C (Celsius degree).

In some cases, the nanoparticle or at least one constituent of the composition comprises a core and/or a coating, wherein the coating preferentially stabilizes the core and/or prevents the core to aggregate and/or leads to the chain arrangement of at least two nanoparticle or at least one constituent of the compositions.

In some other case, the nanoparticle or at least one constituent of the composition is amorphous and/or crystallized.

In still some other cases, the nanoparticle or at least one constituent of the composition can result from the assembly of atoms, entities, compounds that don't have a nanometer size, preferentially before assembly or taken individually, or are preferentially smaller than 100, 10, 1 or 0.1 nm, and preferentially display a nanometer size following their assembly, or are preferentially larger than 0.1, 1, 10 or 100 nm. In still some other cases, the nanoparticle or at least one constituent of the composition can result from the dis-assembly of atoms, entities, compounds that don't have a nanometer size, preferentially before dis-assembly, or are preferentially larger than 0.1, 1, 10 or 100 nm 1 μm, and preferentially display a nanometer size following their dis-assembly, or are preferentially smaller 1μm, 100, 10, 1 or 0.1 nm.

In still some other cases, assembly and/or dis-assembly occur(s) inside the body part or outside the body part.

In some cases, the iron oxide core is composed of at least one iron atom and at least one atom of oxygen. In some other cases, the core is composed of a metal oxide is composed of at least one metal atom and at least one oxygen atom.

In still some other cases, the core is a metallic core composed of at least one metal atom.

In still some other cases, the composition comprises at least one chemical element or a majority of such element or a percentage of mas of such element larger than or equal to 0, 1, 5, 10, 50, 90, or 99%.

In still some other cases, the composition comprises at least one chemical element or a minority of such element or a percentage of mas of such element smaller than or equal to 100, 99, 75, 50, 10, 5, 2, 1 or 0%.

In still some other cases, the chemical element is selected in the group consisting of: Actinium, Aluminum, Americium, Antimony, Argon, Arsenic, Astatine, Barium, Berkelium, Beryllium, Bismuth, Bohrium, Boron, Bromine, Cadmium, Calcium, Californium, Carbon, Cerium, Cesium, Chlorine, Chromium, Cobalt, Copernicium, Copper, Curium, Darmstadtium, Dubnium, Dysprosium, Einsteinium, Erbium, Europium, Fermium, Flerovium, Fluorine, Francium, Gadolinium, Gallium, Germanium, Gold, Hafnium, Hassium, Helium, Holmium, Hydrogen, Indium Iodine, Iridium, Iron, Krypton Lanthanum, Lawrencium, Lead, Lithium, Livermorium, Lutetium, Magnesium, Manganese, Meitnerium, Mendelevium, Mercury, Molybdenum, Moscovium, Neodymium, Neon, Neptunium, Nickel, Nihonium, Niobium, Nitrogen, Nobelium, Oganesson, Osmium, Oxygen, Palladium, Phosphorus, Platinum, Plutonium, Polonium, Potassium, Praseodymium, Promethium, Protactinium, Radium, Radon, Rhenium, Rhodium, Roentgenium, Rubidium, Ruthenium, Rutherfordium, Samarium, Scandium, Seaborgium, Selenium, Silicon, Silver, Sodium, Strontium, Sulfur, Tantalum, Technetium, Tellurium, Tennessine, Terbium, Thallium, Thorium, Thulium, Tin, Titanium, Tungsten, Uranium, Vanadium, Xenon, Ytterbium, Yttrium, Zinc, and Zirconium

In one embodiment of the invention, the composition comprises the at least one nanoparticle or at least one constituent of the composition with the cryoprotectant or protectant compound and optionally a liquid such as water or a gas or a solid or at least one substance that is different from the nanoparticle or at least one constituent of the composition and/or cryoprotectant or protectant compound. Such substance preferentially disperses or suspends the at least one nanoparticle or at least one constituent of the composition preferentially with the cryoprotectant or protectant compound. Such substance and/or cryoprotectant and/or protectant compound preferentially embeds and/or surrounds and/or act(s) as a matrix surrounding the at least one nanoparticle or at least one constituent of the composition and/or cryoprotectant or protectant compound. Such substance preferentially has a different function than the cryoprotectant or protectant compound. For example, the substance could enable the dispersion or re(dispersion), preferentially homogenously, of the at least one nanoparticle or at least one constituent of the composition, preferentially before administration of the composition to the body part, whereas the cryoprotectant or protectant compound could protect or maintain at least one property of the nanoparticle or at least one constituent of the composition, preferentially during storage of the composition preferentially in a lyophilized or dehydrated or powder form.

In another embodiment of the invention, the composition comprises the at least one nanoparticle or at least one constituent of the composition without the cryoprotectant or protectant compound and optionally a liquid such as water or a gas or a solid or at least one substance that is different from the nanoparticle or at least one constituent of the composition and/or cryoprotectant or protectant compound.

In another embodiment of the invention, the composition comprises an excipient, a solvent, an active principle, an inert compound, a metallic compound, at least one nanoparticle or at least one constituent of the composition, an ionic compound, at least one chemical element, a medical device, a drug, a pharmaceutical, immunological and/or metabolic compound, and/or a surfactant.

In another embodiment of the invention, the composition is a preparation or a suspension or a powder or a liquid, or a solid, or a gas, preferentially of or with the at least one nanoparticle or at least one constituent of the composition, optionally with the cryo-protectant or protectant compound.

In one embodiment of the invention, the cryoprotectant or protectant compound is or comprises at least one substance or compound or chemical function or molecule or atom, which protects or maintains or keeps at least one property of the composition or at least one constituent of the composition.

In one embodiment of the invention, the at least one property of the composition or of the at least one constituent of the composition is selected among: i) the chain arrangement or geometric figure or assembly of the at least two nanoparticle or at least one constituent of the composition(s) or of the at least one constituent of the composition, ii) the composition of the nanoparticle or at least one constituent of the composition or of the at least one constituent of the composition, preferentially by maintaining at least 1, 5, 10, 50, 75, 100, $10^3$ or $10^5$ atom(s) or metal(s) or % of atom(s) or metal(s) or % in mass of atom(s) or metal(s) preferentially in the composition or in the at least one constituent of the composition or nanoparticle or at least one constituent of the composition, iii) the size of the nanoparticle or at least one constituent of the composition or of the at least one constituent of the composition, preferentially by preventing a change in nanoparticle or at least one constituent of the composition size or in size of the at least one constituent of the composition, preferentially by more than 1, 5, 10, 100, $10^3$ or $10^5$ nm, iii) the oxidation state of the nanoparticle or at least one constituent of the composition or of the at least one constituent of the composition, iv) the at least one magnetic property, preferentially the ferrimagnetic property, of the nanoparticle or at least one constituent of the composition or of the at least one constituent of the composition, v) the at least one center of activity or of free radical capture or production of the nanoparticle or at least one constituent of the composition or of the at least one constituent of the composition, vi) the isotonicity or osmolality of the composition or of the at least one constituent of the composition, wherein preferentially the at least one property exists or is measured preferentially in some cases at or below the temperature of $10^5$, $10^3$, $10^2$, 50, 20, 10, 5, 2, 1, 0, -5, -10, -50, -77, -100, -200 or -273 °C, in some other cases at or above the temperature of $10^5$, $10^3$, $10^2$, 50, 20, 10, 5, 2, 1, 0, -5, -10, -50, -77, - 100, -200 or -273 °C.

In one embodiment, the center of activity or free radical production or capture is a compound that increases the quantity of free radicals produced or captured when: i) a radiation is applied on the center, preferentially designated as radio-sensitizer in this case, ii) light is applied on the center, preferentially designated as photosensitizer in this case, iii) thermal radiation or temperature increase or temperature decrease is applied on the center, preferentially designated as thermo-sensitizer in this case, iv) cryo-treatment or protection applied on the center, preferentially designated as cryo-sensitizer in this case, v) a magnetic field is applied on the center, preferentially designated as magneto-sensitizer in this case, vi) ultrasound is applied on the center, preferentially designated as sonosensitizer.

In one embodiment, the center of activity or free radical production or capture can be a center of physico-chemical disturbance, such as radiation, amplification, where the amplification can correspond to an effect of the physico-chemical disturbance applied on the body part that is more important in the presence than in the absence of the center, for example eradication or death of a tumor could be induced in the presence of the irradiated center while such effect would be absent or less pronounced in the absence of the center.

In one embodiment, the center of activity or free radical production or capture is a compound that amplifies or increases the physico-chemical disturbance or radiation or at least one parameter or property of the physico-chemical disturbance or radiation or at least one effect of a radiation or physico-chemical disturbance. When the center is exposed to radiation, light, thermal variation, cryo-treatment or cryo-protection, magnetic field, or ultrasound, it can be designated as radio-sensitizer, photosensitizer, thermo-sensitizer, cryo-sensitizer, magneto-sensitizer, or sonosensitizer, respectively.

In another embodiment of the invention, the at least one property of the composition is maintained, or kept or protected, preferentially by the cryo-protectant or protectant compound, when:

i) At least two nanoparticle or at least one constituent of the compositions remain arranged in chains or in a geometric figure or in or within an assembly,

ii) The nanoparticle or at least one constituent of the composition size is maintained or does not vary more than $10^5$, $10^3$, 50, 20, 10, 5, 2, 1 or 0%,

iii) The nanoparticle or at least one constituent of the composition composition is maintained or does not change or induce a change by or of more than 1, 5, 10, $10^3$, $10^5$ or $10^{20}$ atoms,

iv) The nanoparticle or at least one constituent of the composition magnetic property, preferentially ferrimagnetic one, is maintained or at least one magnetic property such as the nanoparticle or at least one constituent of the composition coercivity, nanoparticle or at least one constituent of the composition magnetization, or nanoparticle or at least one constituent of the composition saturating magnetization does not vary by more than: i) $10^5$, $10^3$, 50, 20, 10, 5, 2, 1 or 0%, ii) $10^5$, $10^3$, 50, 20, 10, 5, 2, 1 or 0 Oe, or iii) ii) $10^5$, $10^3$, 50, 20, 10, 5, 2, 1 or 0 emu or emu per gram or per mg of nanoparticle or at least one constituent of the composition.

v) The oxidation state of the nanoparticle or at least one constituent of the composition is maintained or does not change or induce a change by or of more than 1, 5, 10, $10^3$, $10^5$ or $10^{20}$ atoms, preferentially oxygen or of an atom that is oxidized or reduced,

vi) At least one center of activity or free radical production or capture remains comprised in the nanoparticle or at least one constituent of the composition or composition or remains active, i.e. preferentially able to produce or capture free radical preferentially under the application of a radiation, and/or

vii) The isotonicity, osmolality, and/or at least one other property of the nanoparticle or at least one constituent of the composition or composition does not change or vary by more than $10^{-3}$, 1, 5, 10, 25, 50, 75 or 100% where this percentage is preferentially equal to (P2-P1)/P1, preferentially measured in absolute terms or values, where P1 and P2 are values of isotonicity, osmolality, and/or at least one other property of the nanoparticle or at least one constituent of the composition or composition at a temperature T1 and T2 or at two different states of the nanoparticle or at least one constituent of the composition, respectively.

preferentially for or when the temperature of the composition or nanoparticle or at least one constituent of the composition is maintained at or decreased below or at the temperature of $10^5$, $10^3$, $10^2$, 50, 20, 10, 5, 2, 1, 0, -5, -10, -50, -77, -100, -200 or -273 °C.

In some cases, the percentage of variation of the at least one other property of the nanoparticle or at least one constituent of the composition or composition can be equal to (P2-P1)/P1, preferentially measured in absolute terms or values, where P1 and P2 are two different values of at least one other property of the nanoparticle or at least one constituent of the composition or composition, preferentially measured when the nanoparticle or at least one constituent of the composition or composition is at two different temperature or in two different conditions.

In some cases, the nanoparticle or at least one constituent of the composition or composition is at least one of the following conditions: i) liquid, ii) solid, and iii) gaseous.

In some cases, T1 and T2 are two different temperatures.

In some cases, T2 is smaller than T1, preferentially by at least $10^{-5}$, $10^{-3}$, 10 ', 0, 1, 2, 5, 10 or $10^3$ °C. In some cases, T1 and/or T2 is/are smaller than $10^5$, $10^3$, $10^2$, 50, 20, 10, 5, 2, 1, 0, -5, -10, -50, -77, - 100, -200 or -273 °C.

In some other cases, T1 and/or T2 is/are larger than -273, -200, -100, -77, -50, -10, -5, 0, 1, 2, 5, 10, 50, 100 or $10^3$ °C.

In one embodiment of the invention, the cryoprotectant or protectant compound is not used to protect the at least one nanoparticle or at least one constituent of the composition or at least one chain from the denaturation and/or destruction and/or loss of primary, secondary, tertiary, or quaternary structure of DNA, RNA, protein(s), lipid(s), enzyme(s), or at least one biological molecule.

In one embodiment of the invention, the cryoprotectant or protectant compound prevents a change, decrease, increase of at least one property, magnetic property, composition, cohesion, oxidation, or oxidation state, of the size or of at least one nanoparticle or at least one constituent of the composition or chain by more than 100, 50, 10 or 1%, where this percentage is preferentially equal to S2-S1/S1 or P2-P1/P1, where S1, P1, S2 and P2 are preferentially the sizes and properties of at least one nanoparticle or at least one constituent of the composition or chain or of at least one property, magnetic property, composition, cohesion, oxidation, or oxidation state, of at least one nanoparticle or at least one constituent of the composition before (S1, P1) and after (S2, P2).

In one embodiment of the invention, at least one perturbation or radiation is responsible for the change between (S1, P1) and (S2, P2), where the at least one perturbation is preferentially selected in the group consisting of:

i) The cooling down or temperature decrease or application of temperature gradient of or on the at least one nanoparticle or at least one constituent of the composition or chain preferentially by more than 0.1, 1, 50 or 100°C, preferentially from an initial temperature, preferentially above -273, -100, -50, 0, 5, or 10°C, preferentially down to a final temperature, preferentially lower than 100, 50, 0 or -50 °C,

ii) The exposure of the nanoparticle or at least one constituent of the composition to oxygen or to a larger or different or smaller amount of oxygen after or during the perturbation than before the perturbation, and

iii) The temperature increase of the nanoparticle or at least one constituent of the composition or chain preferentially by more than 0.1, 1, 5 or 10°C.

In some cases, the property of at least one nanoparticle or at least one constituent of the composition can be: i) the size, diameter, surface or volume, of the nanoparticle or at least one constituent of the composition or composition, ii) at least one magnetic property of the at least one nanoparticle or at least one constituent of the composition or composition, iii) the composition preferentially metallic and/or organic or carbonaceous of the at least one nanoparticle or at least one constituent of the composition or composition, iv) the cohesion of the nanoparticle or at least one constituent of the composition or composition, v) the percentage in mass of at least one metal in the at least one nanoparticle or at least one constituent of the composition or chain, vi) the chain arrangement of the at least one nanoparticle or at least one constituent of the composition, vii) the number of nanoparticle or at least one constituent of the compositions in the chain, viii) the surface charge of the at least one nanoparticle or at least one constituent of the composition or chain or composition, ix) the percentage of water or humidity or solid or liquid or gaseous material in the at least one nanoparticle or at least one constituent of the composition or chain or composition, x) the oxidation or redox state of the at least one nanoparticle or at least one constituent of the composition or composition, xi) the coercivity or remanent magnetization or magnetization or saturating magnetization of the at least one nanoparticle or at least one constituent of the composition or chain or composition, xiii) the core diameter or size or volume or surface area or ratio surface/volume of the at least one nanoparticle or at least one constituent of the composition, xiii) the coating thickness of the at least one nanoparticle or at least one constituent of the composition, and/or xiv) the isotonicity or osmolality of the at least one nanoparticle or at least one constituent of the composition or chain or composition.

In one embodiment of the invention, the composition can be cooled down, preferentially below or at 10, 5, 2, 1, 0, -5, -10, -50, -77, -90, -270, or -273 °C, or the composition below or at 10, 5, 2, 1, 0, -5, -10, - 50, -77, -90, -270, or -273 °C, can be reached or obtained preferentially for more than 1 second, 1 minute or 1 hour, preferentially without destroying at least one chain or geometric figure or property of at least one nanoparticle or at least one constituent of the composition, preferentially with at least one chain or geometric figure or property of at least one nanoparticle or at least one constituent of the composition that is maintained or exists. This effect is preferentially due to the presence of the cryo-protectant or protectant compound.

In one embodiment of the invention, the composition is lyophilized or desiccated, preferentially for more than 1 second, 1 minute or 1 hour, preferentially without destroying or changing or modifying or destroying at least one chain or geometric figure or property of at least one nanoparticle or at least one constituent of the composition, preferentially with at least one chain or geometric figure or at least one property of at least one nanoparticle or at least one constituent of the composition that is maintained or exists. This effect is preferentially due to the presence of the cryo-protectant or protectant compound.

In one embodiment of the invention, the composition is prepared or used by following at least one of the following steps:

- First, the composition is mixed in water or liquid or solid or gas or matrix or surfactant or solvent preferentially to add the cryoprotectant or protectant compound to the at least one nanoparticle or at least one constituent of the composition or chain in liquid or solid or gas or matrix or surfactant or solvent preferentially in suspension,
- Second, the composition is lyophilized or desiccated or dried or dehydrated preferentially to remove water from the composition or water is removed from the composition or the composition is dried or desiccated or at least one compound preferentially water is removed from the composition, and preferentially this second step is undertaken to keep the composition in a powder form for storage,
- Third, the composition is resuspended or mixed in water or liquid or solid or gas or matrix or surfactant or solvent, preferentially to be ready for use and/or administration to humans.

In one embodiment, the at least one step mentioned above or of at least one method according to the invention is repeated.

In one embodiment of the invention, the dissociation energy between a chemical group of a molecule of the coating or at least one first constituent of the composition and a chemical group of the core of the nanoparticle or at least one second constituent of the composition is larger than $10^{-5}$, 1, 0, 10 or 100 Kcal, KJ, or eV, preferentially per mol or per bond

In one embodiment of the invention, the dissociation energy between a chemical group of a molecule of the cryo-protectant or protectant compound or of at least one first constituent of the composition and a chemical group of the core and/or coating of the nanoparticle or at least one second constituent of the composition, is lower than $10^{20}$, $10^5$, $10^3$, 100, 10, 1 or 0 Kcal, KJ, or eV, preferentially per mol or per bond.

The invention relates to the composition according to invention, wherein the chemical affinity between the coating and the core of the nanoparticle is larger than: i) the chemical affinity between the cryoprotectant or protectant compound and the coating and/or ii) the chemical affinity between the cryoprotectant or protectant compound and the core of the nanoparticle.

In one embodiment of the invention, the coating is more strongly bound to or is in stronger interaction with the nanoparticle core than with the cryo-protectant or protectant compound.

In one embodiment of the invention, the coating can't be separated or isolated from the nanoparticle core or is inseparable or un-isolable from the nanoparticle core, preferentially using a magnet preferentially of strength smaller than 1 mT.

In another embodiment of the invention, the cryo-protectant or protectant compound can be separated or isolated from the nanoparticle core or is separable or isolable from the nanoparticle core, preferentially using a magnet or magnetic separation or centrifugation.

In some cases, this can be highlighted by the fact that: i) the coating and core of the nanoparticle can't be separated by a magnet of low intensity, *i.e.* preferentially of intensity smaller than 1 T or 1 mT, or of low magnetic field gradient, *i.e.* preferentially of magnetic field gradient smaller than 1 T or mT per cm preferentially of body part, whereas the cryoprotectant can preferentially be separated from the nanoparticle by such magnet, ii) the coating and core of the nanoparticle can't preferentially be separated by mixing the nanoparticle core and coating in water followed by centrifugation whereas the cryoprotectant can preferentially be preferentially more efficiently or easily separated from the nanoparticle by being mixed in water with the suspended nanoparticle followed by centrifugation.

In one embodiment, the coating comprises at least one compound or chemical function, which can establish interactions or weak interactions or weak bonds or covalent bonds with the core of the nanoparticle or at least one chemical function or atom or ion of the core part of the nanoparticle or at least one constituent of the composition, in particular iron oxide or any combination or ionic state of iron and/or oxygen. In some cases, such interactions or bonds maintain the nanoparticle or at least one constituent of the composition coating attached or linked or associated with or to the nanoparticle core, preferentially called associating or attaching or linking bonds or interactions.

In one embodiment of the invention, the nanoparticle or at least one constituent of the composition consists of at least one component selected in the group consisting of: i) a core, ii) a coating preferentially surrounding the core and iii) associating or linking or attaching bonds or interactions, preferentially between the core and coating or between at least one first constituent of the composition and at least one second constituent of the composition.

In one embodiment of the invention, the nanoparticle or at least one constituent of the composition consists of a core and a surrounding coating, which is preferentially associated or linked or attached to or with the core, preferentially in such a way or through sufficiently strong bonds or interactions that it does not detach or dissociates from the core or that it remains attached or bound to the core.

In some cases, the chain arrangement or geometric figure of the nanoparticle or at least one constituent of the compositions is maintained or exists or forms in the presence of the coating and/or or protectant compound.

In one embodiment of the invention, the composition comprises a cryo-protectant or protectant compound, which is associated or linked or bound with or to the nanoparticle core and/or coating or at least one constituent of the composition, preferentially in such a way or through sufficiently weak bonds or interactions that it detaches or dissociates from the nanoparticle core and/or coating or at least one constituent of the composition, preferentially when the cryo-protectant or protectant compound is washed away or removed from the nanoparticle or at least one constituent of the compositions with water or liquid or solid or gas or matrix or surfactant or solvent, by centrifugation or by using a magnet. In some cases, the cryo-protectant or protectant compound can be dissociated or detached or disassembled or unlinked from the nanoparticle core and/or coating or at least one constituent of the composition, preferentially in such a way that the chain arrangement or geometry or assembly of the nanoparticle or at least one constituent of the compositions or at least one property of the nanoparticle or at least one constituent of the composition is maintained.

In some cases, the cryo-protectant or protectant compound can be associated or attached or assembled or linked to or with the nanoparticle core or coating, or at least one constituent of the composition, preferentially in such a way that the chain arrangement or geometry or assembly or at least one property of the nanoparticle or at least one constituent of the compositions is maintained, preferentially when the composition is cooled down or a temperature gradient is applied on the composition or the composition is oxidized, preferentially below or at or by preferentially at least 100, 10, 5, 2, 1, 0, -10, -50, -77, -270, -273 °C, preferentially for more than 0.001, 0.1, 0, 1, 5, 10, $10^3$ or $10^5$ seconds, or when the composition is lyophilized or desiccated or when water or liquid or solid or gas or matrix or surfactant or solvent is removed from the composition partly or fully or when the percentage in mass of water in the composition is lower than 100, 50, 10, 5, 2, 1, 0, $10^{-3}$ or $10^{-5}$ % or when the composition is in powder form.

In some cases, the chain arrangement or geometric figure or assembly or at least one property of the nanoparticle or at least one constituent of the compositions can be observed by following at least one of the following step: i) removing the cryoprotectant or protectant compound or at least one constituent of the composition from the composition partly or fully, ii) suspending or resuspending the composition, preferentially lyophilized, preferentially in water or liquid or solid or gas or matrix or surfactant or solvent, iii) by depositing a droplet of the composition on top of a substrate, preferentially a carbon grid, iv) by waiting for water evaporation, and v) by observing the nanoparticle or at least one constituent of the composition arrangement under electron microscopy.

In some cases, preferentially in the absence of coating the chains or geometric figures do not form or exist while preferentially in the presence of coating the chains or geometric figures preferentially form or exist.

In some cases, when the temperature of the at least one nanoparticle or at least one constituent of the composition is lower than or equal to 100, 10, 5, 2, 1, 0, -10, -50, -77, -270 or -273 °C, preferentially for more than 0.001, 0.1, 0, 1, 5, 10, $10^3$ or $10^5$ seconds, or when the nanoparticle or at least one constituent of the composition is lyophilized or desiccated or dried or when water or liquid or solid or gas or matrix, or surfactant or solvent is removed from the composition or from the at least one nanoparticle or at least one constituent of the composition partly or fully or when the percentage in mass of water in the composition the at least one nanoparticle or at least one constituent of the composition is lower than 100, 50, 10, 5, 2, 1, 0, $10^{-3}$ or $10^{-5}$ %, the chain arrangement or the geometric figure preferentially does not exist or does not form or is destroyed preferentially in the absence of the cryo-protectant or thermo-protectant or oxydo-protectant or chain-protectant or protectant of the composition or size or cohesion or at least one magnetic property of at least one nanoparticle or at least one constituent of the composition, and/or the chain arrangement or the geometric figure or assembly preferentially exists or forms or is maintained or is not destroyed in the presence of the cryo-protectant or thermo-protectant or protectant compound.

In some cases, the at least one nanoparticle or at least one constituent of the composition and preferentially the cryoprotectant or protectant compound can be surrounded by or embedded in or mixed with water or liquid or solid or gas or matrix, or surfactant or solvent, which preferentially enables or favors or triggers the dispersion or suspension, preferentially within a homogenous manner, of the at least one nanoparticle or at least one constituent of the composition preferentially of or in the composition.

In one embodiment of the invention, the coating or at least one compound or chemical function comprised in the coating is or remains chemisorbed or physiosorbed or adsorbed or attached to or linked to or associated with or bound to the core of the nanoparticle or at least one constituent of the composition, preferentially in the presence of the cryo-protectant or protectant compound, preferentially surrounding or embedding or dispersing or suspending the nanoparticle or at least one constituent of the composition, preferably for a duration of more than 0.001, 0.1, 0, 1, 5, 10, $10^3$ or $10^5$ seconds, preferably for or when the composition is in powder form, preferentially for or when the percentage in mass of water or liquid or solid or gas or matrix, or surfactant or solvent in the composition is lower than 100, 50, 10, 5, 2, 1, 0, $10^{-3}$ or $10^{-5}$ %.

In one embodiment of the invention, the coating or at least one compound or chemical function comprised in the coating is not or does not remain chemisorbed or physiosorbed or adsorbed or attached to or linked to or associated with or bound to the core of the nanoparticle or at least one constituent of the composition, preferentially in the absence of the cryo-protectant or protectant compound, preferably surrounding or embedding or dispersing or suspending the nanoparticle or at least one constituent of the composition, preferably for a duration of more than 0.001, 0.1, 0, 1, 5, 10, $10^3$ or $10^5$ seconds, preferably for or when the composition is in powder form, preferably for or when the percentage in mass of water in the composition is lower than 100, 50, 10, 5, 2, 1, 0, $10^{-3}$ or $10^{-5}$ %. In one embodiment, the coating or at least one compound or chemical function comprised in the coating or at least one constituent of the composition is linked through bonds or interactions with or to $Fe^{2+}$ or $Fe^{3+}$ ions, hydroxyls $OH^-$, oxides 02, crystalline defects of the core, which may be in or at the surface of the core of the nanoparticle or at least one other constituent of the composition.

In one embodiment, the coating or at least one constituent of the composition comprises at least one compound, atom, ion, or chemical function such as an acid, carboxylic acid, phosphoric acid, or sulfonic acid function, wherein the compound, atom or ion contained in the coating is able to establish interactions or bonds with the core or with at least one atom of the core, a chemical function of the core, an ion of the core such as $Fe^{2+}$, $Fe^{3+}$, Hydroxyl $OH^-$, oxide $O_2^-$ or a crystalline defect of the core or at least one other constituent of the composition.

In one embodiment of the invention, the interactions or bonds exist or are maintained preferentially between at least two constituents of the composition when the composition is cooled down or exposed to a temperature or pressure gradient or oxidation or reduction, preferentially below or above or at or by preferentially at least $10^5$, 100, 10, 5, 2, 1, 0, $10^{-3}$, $10^{10}$, $10^{-20}$, -10, -50, -77, -270, -273 °C or bar, preferably for more than 0.001, 0.1, 0, 1, 5, 10, $10^3$ or $10^5$ seconds, or when the composition is lyophilized or desiccated or dried or when water or liquid or solid or gas or matrix, or surfactant or solvent is removed from the composition partly or fully or when the percentage in mass of water or liquid or solid or gas or matrix, or surfactant or solvent in the composition is lower than 100, 50, 10, 5, 2, 1, 0, $10^{-3}$ or $10^{-5}$ % or when the composition is in powder form, preferentially in the presence of the cryoprotectant or protectant compound. In some cases, these interactions or bonds keep the nanoparticle or at least one constituent of the compositions organized in chain or in geometric figure, i.e. that preferentially in the absence of these interactions or bonds, the nanoparticle or at least one constituent of the compositions are not organized in chain or geometric figure or these interactions maintain at least one property of the nanoparticle or at least one constituent of the composition or composition.

In some cases, a geometric figure is an assembly or aggregate of nanoparticle or at least one constituent of the compositions forming a geometric figure or assembly.

In some cases, a geometric figure or assembly is selected from the group consisting of: a Balbis, Concave polygon, Constructible polygon, Convex polygon, Cyclic polygon, Equiangular polygon, Equilateral polygon, Penrose tile, Polyform, Regular polygon, Simple polygon, Tangential polygon, Polygons with specific numbers of sides, Henagon, Digon, Triangle, Acute triangle, Equilateral triangle, Heptagonal triangle, Isosceles triangle, Obtuse triangle, Rational triangle, Right triangle, Kepler triangle, Scalene triangle, Quadrilateral, Cyclic quadrilateral, Kite, Parallelogram, Rhombus, Lozenge, Rhomboid, Rectangle, Square, Tangential quadrilateral, Trapezoid, Isosceles trapezoid, Pentagon, Hexagon, Lemoine hexagon, Heptagon, Octagon, Nonagon, Decagon, Hendecagon, Dodecagon, Tridecagon, Tetradecagon, Pentadecagon, Hexadecagon, Heptadecagon, Octadecagon, Enneadecagon, Icosagon, Swastika, Star polygon, Pentagram - star polygon, Hexagram, Star of David, Heptagram, Octagram, Star of Lakshmi, Decagram - star polygon, Annulus, Arbelos, Circle, Archimedes' twin circles, Bankoff circle, Circumcircle, Disc, Incircle and excircles of a triangle, Nine-point circle, Circular sector, Circular segment, Crescent, Indalo, Lens, Lune, Reuleaux polygon, Reuleaux triangle, Salinon, Semicircle, Tomahawk, Triquetra, Heart, Archimedean spiral, Astroid, Cardioid, Deltoid, Ellipse, Heart, Heartagon, Various lemniscates, Oval, Cartesian oval, Cassini oval, Oval of Booth, Ovoid, Superellipse, Taijitu, Tomoe, and/or Magatama.

In one embodiment of the invention, the nanoparticle or at least one constituent of the composition, the suspension, composition, or assembly of nanoparticles is stable, preferentially during a lapse of time, preferentially being its stability duration, which is preferentially larger than $10^{10}$, 5, 10, $10^{50}$ or $10^{100}$ minute(s), 1, 2, 3, 4, 5, 6, 10, 12, 24 or 36 months.

In some cases, the nanoparticle or at least one constituent of the compositions, the suspension of nanoparticle or composition, or assembly of nanoparticle can be stable at a concentration of nanoparticle or at least one constituent of the compositions larger than 1, 5, 10, 50, 100, 200, 500 or 1000 mg of nanoparticle or at least one constituent of the compositions per mL of solvent, water, matrix, or body part surrounding or comprising or embedding the or nanoparticle or at least one constituent of the compositions or composition.

In one embodiment of the invention, the body part is the body part of an individual, animal, or human. In an embodiment of the invention, the body part comprises more than or at least 1, 2, 5, 10, or 100 similar or different organism(s), apparatus, organ(s), tissue(s), cell(s), or biomolecule(s).

In some cases, the body part can be all or part of the head, neck, shoulder, arm, leg, knee, foot, hand, ankle, elbow, trunk, inferior members, or superior members.

In some other cases, the body part can be or belong to an organ, the musculoskeletal, muscular, digestive, respiratory, urinary, female reproductive, male reproductive, circulatory, cardiovascular, endocrine, circulatory, lymphatic, nervous (peripheral or not), ventricular, enteric nervous, sensory, or integumentary system, reproductive organ (internal or external), sensory organ, endocrine glands. The organ or body part can be human skeleton, joints, ligaments, tendons, mouth, teeth, tongue, salivary glands, parotid glands, submandibular glands, sublingual glands, pharynx, esophagus, stomach, small intestine, duodenum, jejunum, ileum, large intestine, liver, gallbladder, mesentery, pancreas, nasal cavity, pharynx, larynx, trachea, bronchi, lungs, diaphragm, kidneys, ureters, bladder, urethra, ovaries, fallopian tubes, uterus, vagina, vulva, clitoris, placenta, testes, epididymis, vas deferens, seminal vesicles, prostate, bulbourethral glands, penis, scrotum, pituitary gland, pineal gland, thyroid gland, parathyroid glands, adrenal glands, pancreas, heart, arteries, veins, capillaries, lymphatic vessel, lymph node, bone marrow, thymus, spleen, gut-associated lymphoid tissue, tonsils, brain, cerebrum, cerebral hemispheres, diencephalon, brainstem, midbrain, pons, medulla, oblongata, cerebellum, spinal cord, choroid plexus, nerves, cranial nerves, spinal nerves, ganglia, eye, cornea, iris, ciliary body, lens, retina, ear, outer ear, earlobe, eardrum, middle ear, ossicles, inner ear, cochlea, vestibule of the ear, semicircular canals, olfactory epithelium, tongue, taste buds, mammary glands, or skin.

In some cases, the body part or organ can belong to the blood circulation or circulatory system.

In some cases, the body part can be or comprise at least one tumor, cancer, virus, bacterium, or pathological cell.

In one embodiment of the invention, the body part is or comprises water, an excipient, a solution, a suspension, at least one chemical element, organic material, or gel, which can be synthetic or produced by a living organism.

Preferably, the body part of an individual, also designated as the body part, represents or is part of an individual or a whole individual, where the individual is preferentially a human, an animal, or an organism, preferentially a living or inactivated or dead organism, comprising at least one prokaryotic or eukaryotic cell.

In one embodiment of the invention, the body part is alive (or not), is any tissue, water, medium, substance, cell, organelle, organ protein, lipid, DNA, RNA, biological material, preferentially localized in a specific region of an individual, preferentially originating or extracted from such region.

In an embodiment of the invention, the body part comprises a pathological site, a healthy site, and/or a nanoparticle or at least one constituent of the composition or composition region.

In one embodiment of the invention, the body part is or comprises a pathological site or pathological cells.

In some cases, the pathological site can be defined as an unhealthy site, or a site that is in a different condition from a site of a healthy individual, or the site of an unhealthy individual. It can comprise pathological cells, such as tumor cells, bacteria, eukaryotic or prokaryotic cells, as well as viruses or other pathological material. Pathological cells can be cells that are: i) not arranged or working as they usual do in a healthy individual, ii) dividing more quickly than healthy cells, iii) healthy cells having undergone a transformation or modification, iv) dead, sometimes due to the presence of a virus or to other organisms, or v), in contact, in interaction, with foreign material not belonging to the individual, such as viruses, where viruses can possibly penetrate, colonize, or replicate in these cells. In some cases, pathological cells can be assimilated to viruses or to other organisms or entities that colonize cells or target cells or destroy cells or use cells or enter in interaction with cells, preferentially to enable their own reproduction, multiplication, survival, or death. In some cases, a pathological site can comprise healthy cells, preferentially with a lower number, activity or proliferation, than that of pathological cells. In one embodiment of the invention, the body part is or comprises a healthy site or healthy cells. In some cases, the healthy site can be defined as a site or region that comprises healthy cell(s), where a healthy cell can be defined as a cell that belongs to a healthy individual or to the body part of a healthy individual. In some cases, the healthy site can surround the pathological site when it is preferentially located at a distance of less than 1 or $10^{-9}$ m from the pathological site.

In some cases, the composition or at least one constituent of the composition or body part can be exposed to radiation.

Preferably, the radiation is selected from the group consisting of: i) a magnetic or electric or electromagnetic field or wave, a wave a particulate radiation, ii) laser light, iii) light produced by a lamp, iv) light emitted at a single wavelength, v) light emitted at multiple wavelengths, vi) a ionizing radiation, vii) microwave, viii) radiofrequencies, and ix) a sound, an ultrasound an infrasound, or an acoustic wave. In one embodiment of the invention, the radiation has at least one of the following properties:

i) it has a power or power density lower than 1000, 10 or 1 W (Watt), preferentially per cm, W per cm$^2$, or W per cm$^3$, preferentially of body part or composition, or preferentially per gram or milligram preferentially, preferentially of body part or composition,

ii1) it has an energy or energy density lower than 10$^5$, 10$^3$, 100 or 1 W.sec per cm, W.sec per cm$^2$, or W.sec per cm$^3$, preferentially of body part or composition,

ii2) it has an energy or energy density lower than 10$^5$, 10$^3$, 100 or 1 W.sec per gram or milligram, preferentially of body part or composition,

ii3) it has an energy or energy density lower than 10$^5$, 10$^3$, 100 or 1 (Joule), J, preferentially per cm, per cm$^2$, or per cm$^3$, preferentially of body part or composition, preferentially per gram of milligram, preferentially of composition or body part,

iii) it has a frequency lower than 10$^5$ 100, 10, 1, 10$^{-1}$ or 10$^{-3}$ MHz,

iv) it has a penetration depth in the body part lower than 10$^{10}$, 10$^5$, 10$^3$, 10, 1, 0, or 10$^{-5}$ cm,

v) it has a wavelength lower than 10$^{10}$, 10$^5$, 10$^3$, 100, 50, 10, 10, 5, 2, 1, 0.1 or 0 nm,

In another embodiment of the invention, the radiation has at least one of the following properties:

i) it has a power or power density larger than 10$^{-10}$, 10$^{-5}$, 10$^{-3}$, 10$^{-1}$, 0, 1, 5, 10, 100, 10$^3$ or 10$^5$ W (Watt), preferentially per cm, W per cm$^2$, or W per cm$^3$, preferentially of body part or composition, or preferentially per gram or milligram preferentially, preferentially of body part or composition,

ii1) it has an energy or energy density larger than 10$^{-10}$, 10$^{-5}$, 10$^{-3}$, 10 ', 0, 1, 5, 10, 100, 10$^3$ or 10$^5$ W.sec per cm, W.sec per cm$^2$, or W.sec per cm$^3$, preferentially of body part or composition,

ii2) it has an energy or energy density larger than 10$^{-10}$, 10$^{-5}$, 10$^{-3}$, 10 ', 0, 1, 5, 10, 100, 10$^3$ or 10$^5$ W.sec per gram or milligram, preferentially of body part or composition,

ii3) it has an energy or energy density larger than 10$^{-10}$, 10$^{-5}$, 10$^{-3}$, 10 ', 0, 1, 5, 10, 100, 10$^3$ or 10$^5$ (Joule), J, preferentially per cm, per cm$^2$, or per cm$^3$, preferentially of body part or composition, preferentially per gram of milligram, preferentially of composition or body part,

iii) it has a frequency lower than 10$^{-10}$, 10$^{-5}$, 10$^{-3}$, 10 ', 0, 1, 5, 10, 100, 10$^3$ or 10$^5$ MHz,

iv) it has a penetration depth in the body part lower than 10$^{-10}$, 10$^{-5}$, 10$^{-3}$, 10 ', 0, 1, 5, 10, 100, 10$^3$ or 10$^5$ cm,

v) it has a wavelength lower than 10$^{-10}$, 10$^{-5}$, 10$^{-3}$, 10$^{-1}$, 0, 1, 5, 10, 100, 10$^3$ or 10$^5$ nm,

In one embodiment of the invention, the nanoparticle or at least one constituent of the composition or composition is administered to or in the body part.

In another embodiment of the invention, the nanoparticle or at least one constituent of the composition or composition is administered at a distance of less than 1 or 10$^{-9}$ m away from the body part.

In still another embodiment of the invention, the nanoparticle or at least one constituent of the composition or composition is administered at a distance of more than 1 or 10$^{-9}$ m away from the body part.

In another embodiment of the invention, the nanoparticle or at least one constituent of the composition or composition is administered to or in the body part following at least one of the following administration routes: local, enteral, gastrointestinal, parenteral, topical, oral, inhalation, intramuscular, subcutaneous, intra-tumor, in an organ, in a vein, in arteries, in blood, or in tissue.

In some cases, the nanoparticle or at least one constituent of the compositions, the suspension of nanoparticle, the composition, or assembly of nanoparticles can be stable when at least one nanoparticle or at least one constituent of the composition is not degraded or does not lose partly or fully its coating or at least one constituent of the composition or can be administered to a body part or keeps or maintains its chain arrangement or geometric figure.

In some other cases, the nanoparticle or at least one constituent of the compositions, the suspension of nanoparticle, the composition, or assembly of nanoparticle can be stable when the optical density of the nanoparticle or at least one constituent of the compositions, the suspension of nanoparticle composition, or assembly of nanoparticle, preferentially mixed in water, preferentially measured at 480 nm or at another fixed wavelength, does not decrease by more than 1, 5, 10, 50, 75 or 90 % or by more than 10 $^{10}$, 10$^{-3}$, 10$^{-1}$, 0.5 or 0.7, preferentially within 1, 5, 10, 10$^3$, 10$^7$ or 10$^{20}$ seconds following homogenization or mixing or optical density measurement or absorption measurement of this suspension or composition. This percentage can be equal to $(OD_B - OD_A)/OD_B$ or $OD_A/OD_B$, where $OD_B$ is the optical density of the nanoparticle or at least one constituent of the compositions, the suspension of nanoparticles, or the composition, or assembly of nanoparticles, measured before the homogenization or mixing or optical density measurement or absorption measurement of the nanoparticles or at least one constituent of the composition, the suspension, composition, or assembly of nanoparticle and $OD_A$ is the optical density of the nanoparticle or at least one constituent of the compositions, the suspension of nanoparticle, composition, or assembly of nanoparticle, measured after the homogenization or mixing or optical density measurement or absorption measurement of the nanoparticle or at least one constituent of the compositions, the suspension of nanoparticle, the composition, or assembly of nanoparticle.

In some cases, the composition can be stable or considered stable when it is measured stable at a certain first time to and at a certain second time $t_1$, where $t_1$ follows to or is separated from to by a lapse of time $\Delta T$ of at least 1 second, 1 minute, 1 hour, 1 day, 1 month, 3 months, 6 months, 1, 2, 5, or 10 years. During $\Delta T$, the composition is preferentially being stored.

In some cases, the nanoparticle or at least one constituent of the composition can be suspended in a liquid or dispersed in a matrix or body part to yield a homogenous nanoparticle or at least one constituent of the composition dispersion or a highly stable nanoparticle or at least one constituent of the composition, composition, or suspension.

In one embodiment of the invention, the cryo-protectant or protectant compound serves to maintain the chain or geometric figure or at least one nanoparticle or at least one constituent of the composition property stable over a period of time, preferentially $\Delta T$, preferentially at least one or six months. In this case, the chain or geometric figure or at least one nanoparticle or at least one constituent of the composition property can preferentially be observed or measured, preferentially under electron microscopy measurements, at a first time to and at a second time $t_1$, where $t_1$ is preferentially separated from to by $\Delta T$.

In one embodiment of the invention, the cryo-protectant or protectant compound protects or maintains at least one nanoparticle or at least one constituent of the composition property or the arrangement of at least two nanoparticle or at least one constituent of the compositions in at least one chain or in at least one geometric figure.

In some cases, the protection or maintenance of the arrangement of at least two nanoparticles or at least one constituent of the compositions in at least one chain or in at least one geometric figure or of at least one property of the nanoparticle or of at least one constituent of the composition, occurs at a temperature preferentially of the composition that is preferentially lower than or equal to $10^3$, 500, 100, 50, 10, 5, 2, 1, 0, -5, -10, -20, -50 -100, -200, or -273 °C.

In some other cases, the protection or maintenance of the arrangement of at least two nanoparticle or at least one constituent of the compositions in at least one chain or in at least one geometric figure or of at least one property of the nanoparticle or at least one constituent of the composition occurs at a temperature preferentially of the composition that is preferentially larger than or equal to -273, -200, - 100, -50 -20, -10, -5, 0, 1, 2, 5, 10, 50, 100, 500, or $10^3$ °C.

In some cases, the protection or maintenance of the arrangement of at least two nanoparticle or at least one constituent of the compositions in at least one chain or in at least one geometric figure or of at least one property of the nanoparticle or at least one constituent of the composition occurs for a duration that is preferentially larger than $10^{-10}$, $10^{-5}$, $10^{-3}$, $10^{-1}$ 0, 1, 2, 5, 10, $10^2$, $10^3$ or $10^5$ minutes or seconds.

In some other cases, the protection or maintenance of the arrangement of at least two nanoparticle or at least one constituent of the compositions in at least one chain or in at least one geometric figure or of at least one property of the nanoparticle or at least one constituent of the composition occurs for a duration that is preferentially smaller than $10^{10}$, $10^5$, $10^3$, 10, 5, 2, 1, 0, $10^{-1}$, $10^{-3}$ or $10^{-5}$ minutes or seconds. The invention also relates to the composition according to the invention, wherein the composition comprises an organic part and/or an inorganic part, wherein the inorganic part preferentially comprises the core of the nanoparticle or at least one constituent of the composition, wherein the organic part preferentially comprises the coating of the nanoparticle or at least one constituent of the composition and/or the cryo-protectant or protectant compound, and wherein the percentage in mass of the inorganic part is preferentially larger, preferentially a factor $\xi$, than the percentage in mass of the organic part. In some cases, the organic part comprises more than 0, $10^{-10}$, 1, 5, 10, 35, 50, 75, 80, 90 or 100% in mass or volume of carbon or carbonaceous material.

In some other cases, the inorganic part comprises less than 100, 99, 80, 75, 50, 25, 10, 5, 2, 1 or 0% in mass or volume of carbon or carbonaceous material.

In still some other cases, the organic part comprises more carbon or carbonaceous material than the inorganic part.

In some cases, the factor $\xi$ is equal to I2/O1, where I2 is the percentage in mass of the inorganic part in the composition and O1 is the percentage in mass of the organic part in the composition, preferentially measured in the absence of or with minimal water or liquid or gas.

In some cases, $\xi$ is larger than or equal to 1, 2, 5, 10, 100, $10^3$ or $10^5$.

In some other cases, $\xi$ is smaller than or equal to $10^5$, $10^3$, 100, 10, 5, 2 or 1.

The invention also relates to the composition according to the invention, comprising i) and/or ii):

i) the at least one nanoparticle or at least one constituent of the composition comprising a) and/or b):

a) a core with at least one property selected in the group consisting of: i) a composition comprising at least one metal or metal oxide, preferentially iron oxide, most preferentially maghemite or magnetite, most preferentially essentially maghemite, preferentially with a percentage in mass of more than 0, 1, 50, 90, 99.6 % of iron or zinc or manganese in terms of metallic composition, ii) a size larger than 0, 1, 2, 5, 10, 20, 30 or 35 nm, and iii) a percentage in mass of organic material, preferentially of bacterial origin, preferentially non-denatured, that is lower than 100, 75, 50, 20, 5, 2 or 0%, iv) a crystallized structure partly or fully, and v) a structure comprising a first center of activity, and

b) a surrounding coating, preferentially with a thickness lower than 100, 10 or 5 nm or lower than the diameter of the core of the nanoparticle or at least one constituent of the composition, preferentially comprising a second center of activity,
and

ii) a cryo-protectant or protectant compound, preferentially sorbitol, preferentially surrounding the at least one nanoparticle or at least one constituent of the composition,
where the percentages in mass of the nanoparticle or at least one constituent of the composition core, nanoparticle or at least one constituent of the composition coating and/or cryo-protectant, preferentially in or of the composition, most preferentially comprised in the dried or de-hydrated composition, has(have) at least one property selected in the group consisting of:

    a) The percentage in mass of the nanoparticle or at least one constituent of the composition core is larger than the percentages in mass of the nanoparticle or at least one constituent of the composition coating and/or cryo-protectant or protectant compound, preferentially by a factor $a_1$,
    b) The percentage in mass of carbon or carbonaceous material of the nanoparticle or at least one constituent of the composition core is lower than the percentage(s) in mass of carbon or carbonaceous material of the nanoparticle or at least one constituent of the composition coating and/or cryo-protectant or protectant compound, preferentially by a factor $a_2$,
    c) The percentage in iron or metal mass of the nanoparticle or at least one constituent of the composition core is larger than the percentage(s) in iron or metal mass of the nanoparticle or at least one constituent of the composition coating and/or cryo-protectant, or protectant compound preferentially by a factor $a_3$,

wherein preferentially the strength of the bonds or interactions between the coating and core of the nanoparticle is stronger than the strength of the bond or interaction between the cryo-protectant or protectant compound and the coating and/or core of the nanoparticle or the strength of the bonds or interactions between the coating and core of the nanoparticle is preferentially strong while the strength of the bond or interaction between the cryo-protectant or protectant compound and the coating and/or core of the nanoparticle is preferentially weak,
and/or wherein preferentially the coating and core form a complex,
and/or wherein preferentially the cryo-protectant or protectant compound and nanoparticle preferentially don't form a complex.

In some cases, the percentages in mass of the nanoparticle or at least one constituent of the composition, and/or cryo-protectant or protectant compound in the composition is/are larger than or equal to 0, $10^{-10}$, 1, 5, 10, 25, 30, 50, 75, 80, 90, 99.99 or 100%.

In some other cases, the percentages in mass of the nanoparticle or at least one constituent of the composition, and/or cryo-protectant or protectant compound in the composition is/are smaller than or equal to 100, 99.99, 90, 80, 70, 75, 50, 25, 10, 1, 0.1 or 0%.

In some cases, $a_1$ = PM1/PM2, where PM1 is the percentage in mass of the nanoparticle core and PM2 is the percentages in mass of the nanoparticle coating and/or cryo-protectant.

In some other cases, $a_2$ = PM3/PM4, where PM3 is the percentage in mass of carbon or carbonaceous material of the nanoparticle core and PM4 is the percentage in mass of carbon or carbonaceous material of the nanoparticle coating and/or cryo-protectant.

In still some other cases, as = PM5/PM6, where PM5 is the percentage in iron or metal mass of the nanoparticle core and PM6 is the percentage in iron or metal mass of the nanoparticle coating and/or cryo-protectant.

In some cases, $a_1$, $a_2$, and/or $a_3$ is/are larger than or equal to 0, $10^{-10}$, 1, 5, 10, 25, 50, 100, $10^3$ or $10^5$.

In some other cases, $a_1$, $a_2$, and/or $a_3$ is/are smaller than or equal to $10^{40}$, $10^{10}$, 100, 50, 25, 10, 5, 2, 1 or 0.

In one embodiment of the invention, the strength of the bonds or interactions between the coating and core of the nanoparticle or between two constituents of the composition is strong when the core and coating of the nanoparticle or at least one constituent of the composition can be attracted or moved preferentially together by a magnet preferentially of strength stronger than $10^{-6}$, $10^{-3}$, $10^{-1}$ or 1 T. In some cases, such bonds or interactions can be designated as bonds or interactions that ensure the cohesion of the nanoparticle or at least one constituent of the composition preferentially by being resistant to or maintained in the presence of a force preferentially a magnetic force preferentially applied by a magnet preferentially of strength larger than $10^{-6}$, $10^{-3}$, $10^{-1}$ or 1 T.

In another embodiment of the invention, the strength of the bond or interactions between the cryo-protectant and the coating and/or core of the nanoparticle or between two constituents of the composition is weak when the cryo-protectant or protectant compound or at least a first constituent of the composition can't be attracted or moved preferentially together by a magnet preferentially of strength stronger than $10^{-6}$, $10^{-3}$, $10^{-1}$ or 1 T while the core and coating of the nanoparticle or at least one second constituent of the composition can be attracted or moved preferentially together by a magnet preferentially of strength stronger than $10^{-6}$, $10^{-3}$, $10^{-1}$ or 1 T.

In another embodiment of the invention, the strength of the bonds or interactions between the coating and core of the nanoparticle is stronger than the strength of the bond or interaction between the cryo-protectant or protectant compound and the coating and/or core of the nanoparticle, when the cryo-protectant or protectant compound can be less or less strongly attracted or moved by a magnet preferentially of strength stronger than $10^{-6}$, $10^{-3}$, $10^{-1}$ or 1 T than the nanoparticle or at least one constituent of the composition.

The invention also relates to the composition according to the invention, further comprising a compound, preferentially designated as the other compound, which has at least one property selected in the group consisting of:

A) It is a chemical element, preferentially as listed in the periodic table of Mendeleev,

B) It is selected in the group consisting of: i) Manganese, ii) Magnesium, iii), Potassium, iv) Calcium, v) Zinc, and vi) Sodium,

C) It originates from or is comprised in at least one chemical compound used to fabricate the at least one nanoparticle or at least one constituent of the composition,

D) It originates from or is comprised in at least one medium used to amplify or grow the living organism, preferentially a magnetotactic bacterium, which synthetizes the at least one nanoparticle or at least one constituent of the composition,

E) It is in ionic or charged form, preferentially in such a form that the interaction, preferentially electrostatic one, between the compound and the core or coating of the nanoparticle or at least one constituent of the compositions is favored,

F) It can be removed from the composition, for example by using a chelating agent or a solution containing a chelating agent that preferentially binds to such compound,

G) It has a concentration or percentage in mass in the composition that is lower than the concentration or percentage in mass of at least one constituent of the composition that is different from the other compound or that one of the following substances: i) the nanoparticle, ii) the nanoparticle core, iii) the nanoparticle coating, and iv) the cryo-protectant or protectant compound,

I) It is comprised either outside of the nanoparticle or at least one constituent of the composition core or at the surface of the nanoparticle core or at least one constituent of the composition,

J) It is not in a crystalline form or does not predominantly contribute to the crystalline form or type of the nanoparticle or at least one constituent of the composition.

The invention also relates to the composition according to the invention, wherein the composition or at least one constituent of the composition is in the form of: i) a powder, ii) a liquid, iii) a liquid suspension, iv) a solid, and/or v) one or a mixture of liquid, solid and/or gaseous state(s).

The invention also relates to the composition according to the invention, wherein the at least one chain of at least two nanoparticle or at least one constituent of the compositions has at least one property selected in the group consisting of:

A) the at least one chain exists during at least one step of fabrication or use of the at least two nanoparticles or at least one constituent of the composition, preferentially selected among: i) amplification of a nanoparticle-producing cell, ii) purification or isolation of nanoparticle or at least one constituent of the compositions preferentially from some material or chemical, organic or not, preferentially originating from the at least one nanoparticle producing-cell, iii) coating or formulation or mixture of the nanoparticle preferentially with at least one or two constituent(s) of the composition such as the cryo-protectant or protectant compound, and iv) administration or presence of the at least one chain in or with a body part or cell or matrix or medium or water or gel or material or polymer,

B) the at least one chain comprises at least two nanoparticles or at least one constituent of the compositions, wherein the at least one first direction such as a crystallographic first direction or a first direction perpendicular to a nanoparticle facet or edge or surface or crystallographic plane or a first direction parallel to a nanoparticle diameter of a first nanoparticle is aligned with at least one second direction such as a crystallographic second direction or a second direction perpendicular to a nanoparticle facet or edge or surface or crystallographic plane or a second direction parallel to a nanoparticle diameter of a second nanoparticle, wherein the alignment of the first and second directions is preferentially characterized by an angle between the first and second directions that is smaller than 180, 90, 45, 30, 20, 10, 5, 2, 1, 0.1 or 0 °, wherein such angle is preferentially measured or preferentially exists in at least one moment in time and/or one location in space preferentially during the life time of the chain,

C) the at least one chain comprises at least two nanoparticles or at least two constituents of the compositions, which are separated by a distance larger than $10^{-3}$, 0, 1, 5, 10, $10^2$, $10^3$, $10^5$ or $10^9$ nm, preferentially when the at least two nanoparticles or at least two constituents of the composition are disassembled or are not linked with each other by some binding material or interaction forces preferentially belonging to the composition but can preferentially be re-assembled preferentially by adding to or mixing with the at least two nanoparticles or at least two constituents of the compositions some binding material that preferentially re-assemble the at least two nanoparticles or at least two constituents of the composition,

D) the at least one chain comprises at least two nanoparticles or at least two constituents of the composition, which are separated by a distance smaller than $10^9$, $10^6$, $10^3$, 100, 50, 10, 5, 2, 1 or 0 nm, preferentially when the at least two nanoparticles or at least two constituents of the composition are assembled or are linked with each other by some binding material or interaction forces preferentially belonging to the composition,

and

E) the at least one chain is in the form of: i) a powder, ii) a liquid, iii) a liquid suspension, iv) a solid, and/or v) one or a mixture of liquid, solid and/or gaseous state(s).

The invention also relates to the composition according to the invention, wherein the nanoparticle or at least one constituent of the composition comprises at least one center of activity, preferentially of medical activity, preferentially selected in the group consisting of:

A) a thermal center that preferentially increases or decreases the heat or the cold, preferentially of the body part,

B) a medical center that preferentially increases or enhances the effect of a medical compound such as an immunotherapy, chemotherapy, hormonotherapy, radiotherapy, or surgical medical compound, and

C) a center of activity or free radical production or capture, which is preferentially characterized by at least one property selected in the group consisting of:

A) the nanoparticle core or at least one first constituent of the composition comprises a first center of activity or free radical production or capture $C_{1FRPC}$, wherein $C_{1FRPC}$ is preferentially selected in the group consisting of:

i) another metal than iron such as Zinc or Aluminum,
and

ii) another metal oxide than iron oxide such as Zinc Oxide or Aluminum Oxide.
and

B) the nanoparticle coating or at least one second constituent of the composition comprises a second center of activity or free radical production or capture $C_{2FRPC}$.

The invention also relates to the composition according to the invention, wherein at least one constituent of the composition, preferentially the nanoparticle core, is synthesized by a living organism or nanoparticle producing cell, preferentially a magnetotactic bacterium and/or at least another constituent of the composition, preferentially the nanoparticle coating, is not synthesized by a living organism.

The invention also relates to the composition according to the invention, wherein the center of activity is selected in the group consisting of:

A) a radio-sensitizer or amplificator of radiation, a radio-photosensitizer or amplificator of light radiation, an acoustic sensitizer or amplificator of acoustic radiation or wave, a sonosensitizer or amplificatory of acoustic wave, a particle radiation sensitizer or amplificator of particle radiation, where the particle comprises (or not) a mass, it is a thermal-sensitizer or amplificator of heat or cold or thermal treatment, an amplificator of the medical effect of a compound,

B) an attenuator of radiation, of light radiation, of acoustic radiation or wave, of particle radiation, where the particle comprises (or not) a mass, of heat or cold, of thermal treatment, and/or of the medical effect of a compound,

and

C) a compound, preferentially of meter or centimeter or millimeter or micrometer or nanometer or sub-nanometer or atomic size, preferentially selected in the group consisting of: 1) Acridine, such as Acridine Orange, acridine yellow, 2) ALA (5-Aminolevulinic acid), 3) Aluminum phthalocyanine tetrasulfonate (AlPcS4), 4) Aminolevulinic acid, delta- Aminolevulinic acid, 5) Antihistamines, 6) Azulene, 7) Bavteriochlorin, 8) TOOKAD or TOOKAD Soluble, 9) WST-11, 10) LUZ11, 11) BC19, 12) BC21, 13) porphyrin such as Benzoporphyrin derivative monoacid ring A (BPD-MA), 14) Chlorin such as Chlorin e6, m-tetrahydroxyphenylchlorin 15) Foscan, 16) Verteporfin, 17) benzoporphyrin derivative mono acid ring A, 18) Monoaspartyl chlorin(e6), 19) talaporfin sodium, 20) HPPH, 21) Transition metal compounds, 22) Chlorine e6 green porphyrin, 23) Chlorine e6 porphrin, 24) Coal Tar and Derivatives, 25) Contraceptives, Oral and Estrogens, 26) Curcumin, 27) Cyanine, 28) Cysview, 29) Dyes such as synthetic dyes, 30) Phenothiazinium salts, 31) Rose Bengal, 32) Squaraines, 33) BODIPY dyes, 34) Phenalenones, 35) benzophenoxazinium dyes, 36) Erythrosine, 37) Flavins, 38) Foscan, 39) Fotoscan, 40) Fullerenes such as cationic fullerenes, 41) Furocoumarins, 42) HAL (Hexaminolevulinate), 43) Hemoporfin, 44) 2-(1-Hexyloxyethyl)-2-devinyl pyropheophorbide (HPPH), 45) Hypericin, 46) Hypocrellin, 47) ICG (Indocyanine Green), 48) Levulan, 49) MAL -methyl aminolevulinate), 50) Meta-tetra(hydroxyphenyl)chlorin (m-THPC), 51) Metvix, 52) Methylene Blue, 53) Monoterpene, 54) Motexafin lutetium (Lu-Tex), 54) N-aspartyl chlorin e6 (NPe6), 55) Nanoparticle or at least one constituent of the composition or nanomaterial, 56) Natural products or compounds, 57) Non-Steroidal Anti-Inflammatory Drugs, 58) Palladium bacteriopheophorbide (WST09), 59) Phatalocyanin dyes, 60) Phenothiazines, 61) Photochlor, 62) Photofrin, 63) Photosens, 64) Phthalocyanine such as Liposomal ZnPC, 65) Chloroaluminium sulfonated phthalocyanine (CASP), 66) Silicon phthalocyanine (PC4), 67) RLP068, 68) Porfimer sodium, 69) Porfins, 69) Porphyrins, such as 5,10,15,20-Tetrakis(1-methylpyridinium-4-yl) porphyrin tosylate, 70) XF70, 71) Protoporphyrin, 72) ALA-induced protoporphyrin IX, 73) Psoralens, 74) Quantum dots, 75) Quinones, 76) Riboflavin, 77) Rose Bengal, 78) silicon or Silicon phthalocyanine (Pc4), 79) Sulfonamides, 80) Sulfonylureas, 81) Talaporfin or Talaporfin soudium, 82) Temoporfin, 82) Tetrahydropyrroles, 83) Tin ethyl etiopurpurin, 84) Titanium dioxide, 85) Toldudine blue O, 86) Transition metal compounds such as Ruthenium(II), polypyridyl complexes, ruthenium, rhodium, cyclometalated, Rh(II)-Rh(II) bridged dimer compounds, platinum(II), gold(III), 87) Verteporfin, 88) Vulnic based compound such as Aminovulinic, aminovulinic acid, 89) WST11, and 90) Xanthene, 91) ABS-FA, 92) Acrylonitrile Butadiene Styrene, 93) Styrene, 94) Folic acid, 95) AIMP NP, aminoacyl tRNA synthetase complex-interacting multifunctional protein, 96) Au Nanomaterial, 97) gold, 98) Au-MnO nanomaterial, 99) manganese oxide, 100) Antineoplastic drugs, 101) NSAIDs, 102) nonsteroidal anti-inflammatory drug, 103) Artemether, 104) 5-ALA (5-aminolevulinic acid), 105) Acridine, Acridine Orange, 106) Au-doped TiO2, 107) Carbon based nanomaterial, 108) carbon nanotube, 109) Chlorine, 110) Ce6, 111) PTX, Paclitaxel, 112) chemotherapeutic drug or compound, 113) infrared dye or IR783, 114) Curcumin, 115) Cyanine or Cu-Cyanine, 116) DHMS, 117) dimethylsulfure, 118) Docetaxel, 119) chemotherapeutic drug or compound, 119) DOX/Mn-TPPS@RBCS, 120) doxorubicin, 121) manganese, 122) blood cell, 123) red blood cell, cell, 124) polymer, 125) elastomer, 126) Erythosin or Erythosin B, 127) FA or FA-OI or FA-OI NP or folic acid, 128) F3-PLGA@MB/Gd NPs, 129) poly(lactic-co-glycolic acid), 130) gadolinium, 131) Fe-TiO2 or titanium oxide, 132) Fe-VS$_2$, 133) iron, 134) vanadium disulfide, 135) FMSNs-DOX, 136) silica, 137) HCQ, 138) hydrochloroquine, 139) HP, 140) hematoporphyrin, 141) HMME, 142) hematoporphyrin monomethyl ether, 143) HSYA or Hydroxysafflor yellow A, 144) Hypocrellin, Hypocrellin B, 145) IR780, 146) Levofloxacin, 147) LIP3 or Lithium phosphide, 148) Lithium, 149) Liposome or Liposomal nanomaterial, 150) Lomefoxacin, 151) MG@P NPs, 152) MnP or Manganese peroxidase, 153) MnTTP-HSAs, 154) HSA-wrapped metal-porphyrin complex, 155) albumin, 156) MnWOx, 157) MnWOx-PEG, 158), PEG, 159) metallic or bimetallic or multi-metallic compound preferentially oxide, 160) Mn (III)-HFs, 161) managense, 162) hemoporfin, 163) nano-compound or nanoroad or nanoflower or nanowire or quantum dot, 164) Noble or halogen or Hydrogen or alkali metals or Alkaline earth metals or Triels or Tetrels or Pnicto-gen or Chal-co-gens or metal or gas or liquid or solid preferentially nanomaterial, 165) oxygen indyocinanine preferentially nanoparticle or at least one constituent of the composition, 166) Phthalocyanines, 167) PIO or Pioglitazone, 168) Polymeric nanomaterial, 169) Porphyrin, 170) Pt-doped TiO$_2$, 171) R837, 172) Rose Bengal, 173) Sparfloxacin, 174) TAPP or 5,10,15,20-tetrakis (4-aminophenyl) porphyrin, 175) TiOz or titanium dioxide nanomaterial, 176) TCPP, isomer, or Tris(1-chloro-2-propyl) phosphate 177) TPI or Thermoplastic Polyimide or thermoplastic polymer, 178) TPZ or Tirapazamine, 179) Transition metal oxide, 180) nanoparticle or at least one constituent of the composition or Janus nanoparticle or at least one constituent of the composition, and 181) Xanthones, 182) AQ4N, 183) Apaziquone (E09), 184) Bromodeoxyuridine, 185) Carbogen, 186) Cetuximab, 187) Chemotherapeutic drug or compound, 188) Chlorpromazine, 189) C-reactive peptide, 190) Curcumin, 191) Diamide, 192) Diethylmaeate, 193) Dihydroartemisinin, 194) Docetaxel, 195) ECI301, 196) Etanidazole, 197) Fludarabine, 198) 5-Fluorouracil, 199) Fluorodeoxyuridine, 200) Gadolynium, 201) Gemcitabine, 202) HER-3 ADC, 203) HSP, 204) Hydrogen peroxide, 205) Hydroxyurea, 206) Hyperbaric oxygen, 207) Hyperthermia, 208) Hypoxic cell cytotoxic agent, 209) Irinotecan, 210) lanthanide-doped radiosensitizer-based metal-phenolic network, 211) Lidocaine, 212) Lododeoxyuridine, 213) Metronidazole, 214) misonidazole, 215) etanidazole, 216) nimorazole, 217) N-Ethylmalemide, 218) malmeide, 219) ethylmalmeide, 220) Nanomaterial such as those consisting of or composed of at least partly or fully gold, silver, bismuth, gadolinium, polysiloxane matrix and gadolinium chelates, hafnium, Tantalum, Zinc, Gadolinium, Germanium, Chromium, Praseodymium, Silicon, iron, platinum, cobalt, manganese, magnesium, iron, Titanium, carbon nanotube, quantum dot, nanoroad, Triflate, or metal oxide, 221) Nelfinavir, 222) Nicotinamide, 223) Nimotuzumab, 224) RNA, or miRNA, or miR-201, or miR-205, or miR-144-5p, or miR-146a-5p, or miR-150, or miR-99a, or miR-139-5p, or miR-320a, 225) Membrane active agent, 226) Mitomycin-C or Mitomycin, 227) Motexafin, 228) NBTXR3, 229) Oligonucleotide, 230) Paclitaxel, 231) Papaverine or Papaverine hydrochloride, 232) Paraxonase-2, 233) Pocaine, 234) Porfiromycin (POR), 235) Protein, 236) Peptide, 237) Radiosensitizing nucleosides or compounds, 238) Resveratrol, 239) RRx-001, 240) SiRNa, 241) Suppressors of sulfhydral groups, 242) SYM004, 243) Texaphyrins, 244) TH-302, and 245) Tirapazamine.

The invention also relates to the composition according to the invention combined with at least one nanoparticle or at least one nanoparticle-producing cell, preferentially a magnetotactic bacterium,

wherein the composition preferentially comprises a first cryo-protectant,
wherein the nanoparticle or at least one nanoparticle-producing cell preferentially comprises a second cryo-protectant,
wherein the first and second cryo-protectants are preferentially different compounds.
wherein preferentially the composition and nanoparticle-producing cells are used or fabricated or amplified separately or one after the other, i.e. the composition is preferentially used or fabricated after the nanoparticle-producing cells has been used to fabricate the nanoparticle or at least one constituent of the composition comprised in the composition.

In some cases, the first and second cryo-protectants can be the same compounds.

In some other cases, the first and second cryo-protectants can be different compounds.

In some other cases, the combination of the composition and nanoparticle or at least one nanoparticle-producing cell is or belongs to a system or combined system or combined product.

The invention also relates to the composition according to the invention, where the at least one nanoparticle or at least one constituent of the composition or the at least one chain is in a liquid suspension and the composition preferentially has at least one of the following properties:

i) it is isotonic to animal or human plasma or blood,
ii) the volume occupied by water or liquid or solid or gas or matrix, or surfactant or solvent or of at least one substance different from the nanoparticle or at least one constituent of the composition and/or cryoprotectant and/or protectant compound in the composition is larger than the volume occupied by the at least one chain in the composition, and
iii) the percentage in mass of water or liquid or solid or gas or matrix, or surfactant or solvent or of at least one substance different from the nanoparticle or at least one constituent of the composition and/or cryoprotectant and/or protectant compound in the composition is larger than the percentage in mass of the at least one chain or nanoparticle or at least one constituent of the composition in the composition.

In one embodiment of the invention, the at least one chain or nanoparticle or at least one constituent of the composition is in liquid suspension when it is mixed with a liquid. In some cases, the liquid can be water, an isotonic liquid, a liquid comprising an excipient, a surfactant, or an oil.

In some cases, the composition is isotonic, *i.e.* it preferentially has:

i) the same osmotic pressure as that of a body part such as a cell, a body fluid, plasma, blood, preferentially of a human or animal,
ii) the value of $abs(OP_{BP} - OP_{Comp})/OP_{BP}$ is lower than 100, 50, 10, 5, 2, 1 or $10^{-3}$%, where $OP_{BP}$ and $OP_{Comp}$ are the osmotic pressures of the body part and composition, respectively.

In another embodiment of the invention, $OP_{BP}$ and/or $OP_{Comp}$ is/are larger than $10^{-10}$, $10^{-5}$, $10^{-3}$, $10^{-1}$, 0, 1, 5, 10, 50 or 100 bar or atm.

In still another embodiment of the invention, $OP_{BP}$ and/or $OP_{Comp}$ is/are smaller than $10^{10}$, $10^5$, $10^3$, 10, 0, $10^{-1}$, $10^{-3}$, $10^{-5}$, or $10^{-10}$ bar or atm.

In one embodiment, the value of $abs(OP_{BP} - OP_{Comp})/OP_{BP}$ is kept low or the composition is maintained isotonic to avoid blood pressure increase, preferentially during or after administration of the composition to the body part.

In some cases, the composition can be hypertonic, *i.e.* that preferentially in this case, the composition causes at least one cell or body part to shrink.

In some other cases, the composition can be hypotonic, *i.e.* that preferentially in this case, the composition causes at least one cell or body part to swell.

In still some other cases, the composition can be isotonic, *i.e.* that preferentially in this case, the composition produces no change in cell or body part volume or less change in cell or body part volume than for hypertonic or hypotonic composition.

In one embodiment of the invention, the solutes or solute compounds in the composition, preferentially the nanoparticle or at least one constituent of the composition, chain of nanoparticle and/or cryo-protectant or protectant compound has(have) a concentration, preferentially designated as $C_{solute}$, preferentially designated as $C_{solute,outside}$ outside a cell or outside a body part, preferentially designated as $C_{solute,inside}$ inside a cell or inside a body part, which is such that $abs(C_{solute,outside} - C_{solute,inside}) / C_{solute,outside}$ is lower than or equal to 100, 50, 25, 10, 5, 2, 1, 0, $10^{-1}$, $10^{-3}$ or $10^{-5}$%.

In some other cases, $(C_{solute,outside} - C_{solute,inside}) / C_{solute,outside}$ is larger than or equal to $10^{-5}$, $10^{-3}$, $10^{-1}$, 0, 1, 2, 5, 10, 25, 50, 75 or 99%.

In one embodiment of the invention, the composition is isosmotic or the osmolarity or osmotic pressure of the composition, preferentially outside at least one cell or body part is the same as the osmolarity or osmotic pressure of an intracellular medium or body part or composition comprised inside at least one cell or body part.

In some cases, the body part can designate a liquid or liquid medium comprised in a body part such as the intracellular medium or plasma or blood.

In some cases, the composition is comprised in the body part, preferentially after or with administration to the body part.

In some other cases, the composition is comprised outside the body part, preferentially before or without administration to the body part.

In some cases, the composition can be hypoosmotic.

In some other cases, the composition can be hyperosmotic.

In one embodiment of the invention, the osmolality or osmolarity of the composition is larger than or equal to $10^{10}$, $10^{-5}$, $10^{-3}$, $10^{-1}$, 0, 1, 5, 10, 50, 100, 200, 250, 290, 300, 310, 350, 400, 500, $10^3$, $10^5$ or $10^{10}$ mOsm/kg or mOsm/L.

In another embodiment of the invention, the osmolality or osmolarity of the composition is lower than or equal to $10^{10}$, $10^5$, $10^3$, 500, 350, 310, 300, 250, 200, 100, 10, 5, 1, 0, $10^{-3}$, $10^{-5}$ or $10^{10}$ mOsm/kg or mOsm/L.

In another embodiment of the invention, the osmolality of the composition is equal to, close to, or does not differ by more than 1, 5, 10, 50, or 100% from the human plasma osmolality, preferentially comprised between 275 and 299 milli-osmoles per kilogram.

In another embodiment of the invention, the hydrostatic pressure or osmotic pressure or arterial pressure or systolic pressure or diastolic pressure or pressure of the composition or in the presence of the composition or following the administration of the composition or applied by the composition on the body part or the force pushing the composition or at least one compound or solute of the composition preferentially per unit surface of the body part, preferentially in some cases from outside the body part or at least one cell of the body part to inside the body part or inside at least one cell of the body part, preferentially in some other cases from inside the body part or inside at least one cell of the body part to outside the body part or outside at least one cell of the body part, is designated as $\xi$.

In some cases, $\xi$ can be lower than or equal to $10^5$, $10^3$, 500, 200, 180, 150, 140, 100, 50, 20, 10, 1, 0, $10^{-1}$, $10^{-3}$ or $10^{-5}$ mmHg or PSI or bar or mbar.

In some other cases, $\xi$ be larger than or equal to $10^{-5}$, $10^{-3}$, $10^{-1}$, 0, 1, 5, 10, 100, 120, 150, 500, $10^3$, $10^5$, or $10^{10}$ mmHg or PSI or bar or mbar.

In one embodiment of the invention, the volume occupied by water or a liquid and preferentially the cryoprotectant or protectant compound in the composition is larger than the volume occupied by the at least one chain or nanoparticle or at least one constituent of the composition in the composition. In this case, the at least one chain or nanoparticle or at least one constituent of the composition is preferentially suspended in a liquid, preferentially water, which comprises the cryo-protectant or protectant compound. In this case, the volume of liquid is preferentially maintained sufficiently large or larger than the volume occupied by the at least one chain or nanoparticle or at least one constituent of the composition in the composition to enable the suspension of the at least one chain or nanoparticle or at least one constituent of the composition in the composition or motion or Brownian motion of the at least one chain or nanoparticle or at least one constituent of the composition in the composition.

In some cases, the volume occupied by: i) water or liquid or solid or gas or matrix, or surfactant or solvent or of at least one substance different from the nanoparticle and/or cryoprotectant and/or protectant compound and ii) preferentially the cryoprotectant or protectant compound in the composition is larger than the volume occupied by the at least one chain or nanoparticle, preferentially by a factor $\alpha$ of preferentially at least 1.1, 2, 5, 10 or $10^3$. In this case, the composition is preferentially in liquid form. In some other cases, the volume occupied by: i) water or liquid or solid or gas or matrix, or surfactant or solvent or of at least one substance different from the nanoparticle and/or cryoprotectant and/or protectant compound and ii) preferentially the cryoprotectant or protectant compound in the composition is lower than the volume occupied by the at least one chain or nanoparticle in the composition, preferentially by a factor $\alpha$ of preferentially at least 1.1, 2, 5, 10 or $10^3$. In this case, the composition is preferentially in powder form.

In some cases, $\alpha$ can be equal to $V_{liq}/V_{chain}$, where $V_{liq}$ and $V_{chain}$ are the volumes occupied by the liquid or solid or gas or matrix, or surfactant or solvent or of at least one substance different from the nanoparticle and/or cryoprotectant and/or protectant compound, preferentially comprising or embedding or surrounding or dispersing or mixing the cryo-protectant or protectant compound and at least one chain or nanoparticle, respectively, where the two volumes can preferentially be measured by separating the at least one chain or nanoparticle from the liquid or solid or gas or matrix, or surfactant or solvent or at least one constituent of the composition different from the chain, nanoparticle, and/or cryoprotectant and/or protectant compound, using for example magnetic separation or using a magnet to attract the chains or nanoparticle and separate them from the liquid or solid or gas or matrix, or surfactant or solvent or of at least one substance different from the nanoparticle, chain and/or cryoprotectant and/or protectant compound.

In another embodiment of the invention, the mass of water or liquid and preferentially cryoprotectant or protectant compound in the composition is larger than the mass of at least one chain or nanoparticle or at least one constituent of the composition in the composition. In this case, the at least one chain or nanoparticle or at least one constituent of the composition is preferentially suspended in a liquid, preferentially water, or solid or gas or matrix, or surfactant or solvent or at least one substance different from the nanoparticle or at least one constituent of the composition and/or cryoprotectant and/or protectant compound, which comprises or embeds or surrounds or disperses or mixes or suspends the cryo-protectant or protectant compound. In this case, the mass of liquid or solid or gas or matrix, or surfactant or solvent or of at least one substance different from the nanoparticle or at least one constituent of the composition and/or cryoprotectant and/or protectant compound is preferentially maintained sufficiently large or larger than the mass of the at least one chain or nanoparticle or at least one constituent of the composition in the composition to enable the suspension of the at least one chain or nanoparticle or at least one constituent of the composition in the composition or motion or Brownian motion of the at least one chain or nanoparticle or at least one constituent of the composition in the composition.

In some cases, the mass of water or liquid or solid or gas or matrix, or surfactant or solvent or at least one substance different from the nanoparticle and/or cryoprotectant and/or protectant compound and preferentially cryoprotectant or protectant compound in the composition is larger than the mass of the at least one chain or nanoparticle in the composition, preferentially by a factor $\alpha$ preferentially of at least 1.1, 2, 5, 10 or $10^3$. In this case, the composition is preferentially in liquid or solid or gas form.

In some other cases, the mass of water or liquid or solid or gas or matrix, or surfactant or solvent or of at least one substance different from the nanoparticle and/or cryoprotectant and/or protectant compound and preferentially cryoprotectant or protectant compound in the composition is lower than the mass of the at least one chain or nanoparticle in the composition, preferentially by a factor $\alpha$ of preferentially at least 1.1, 2, 5, 10 or $10^3$. In this case, the composition is preferentially in powder form.

In some cases, $\alpha$ can be equal to $m_{liq}/m_{chain}$, where $m_{liq}$ and $m_{chain}$ are the masses of the liquid preferentially comprising the cryo-protectant or thermo-protectant or oxydo-protectant or chain-protectant or protectant of the composition or size or cohesion or at least one magnetic property of at least one nanoparticle and of at least one chain in the composition, respectively, where the two masses can preferentially be measured by separating the at least one chain or nanoparticle from the liquid or solid or gas or matrix, or surfactant or solvent or of at least one substance different from the nanoparticle and/or cryoprotectant and/or protectant compound, using for example magnetic separation or using a magnet to attract the chains or nanoparticle and separate them from the liquid or liquid or solid or gas or matrix, or surfactant or solvent or at least one substance different from the nanoparticle and/or cryoprotectant and/or protectant compound, where $m_{chain}$ and/or $m_{liq}$ can preferentially be measured by or following evaporation or removal of the liquid or solid or gas or matrix, or surfactant or solvent or of at least one substance different from the nanoparticle or at least one constituent of the composition and/or cryoprotectant and/or protectant compound from the composition or lyophilization or desiccation or water or solid or liquid or gas removal of or from the composition.

In one embodiment of the invention, the percentage in mass of water or liquid or solid or gas, preferentially comprising the cryo-protectant or protectant compound, preferentially not comprising the at least one chain or nanoparticle, in the composition, is larger than the percentage in mass of the at least one chain or nanoparticle in the composition.

In some cases, the percentage in mass of water or liquid or solid or gas preferentially comprising the cryo-protectant or protectant compound, preferentially not comprising the at least one chain or nanoparticle, in the composition is larger than the mass of the at least one chain or nanoparticle in the composition, preferentially by a factor $\alpha$ of preferentially at least 1.1, 2, 5, 10 or $10^3$. In this case, the composition is preferentially in liquid or solid or gaseous form.

In one embodiment of the invention, the percentage in mass of water or liquid or solid or gas or matrix, or surfactant or solvent or of at least one substance different from the nanoparticle and/or cryoprotectant and/or protectant compound, preferentially comprising the cryoprotectant or protectant compound, preferentially not comprising the at least one chain or nanoparticle in the composition, is smaller than the percentage in mass of the at least one chain or nanoparticle preferentially by a factor $\alpha$ preferentially of at least 1.1, 2, 5, 10 or $10^3$. In this case, the composition is preferentially in powder form.

In some cases, $\alpha$ can be equal to $P_{liq}/P_{chain}$, where $P_{liq}$ and $P_{chain}$ are the percentages in masses of the liquid or solid or gas or matrix, or surfactant or solvent or of at least one substance different from the nanoparticle and/or cryoprotectant and/or protectant compound, preferentially comprising the cryoprotectant or protectant compound and at least one chain or nanoparticle in the composition, respectively, where the two percentages in masses can preferentially be measured by separating the at least one chain or nanoparticle from the liquid or solid or gas or matrix, or surfactant or solvent or at least one substance different from the nanoparticle and/or cryoprotectant and/or protectant compound, using for example magnetic separation or using a magnet to attract the chains or nanoparticle or at least one constituent of the composition(s) and separate them from the liquid, where $P_{chain}$ and/or $P_{liq}$ can preferentially be measured by or following evaporation or removal of the liquid or solid or gas or matrix, or surfactant or solvent or at least one substance different from the nanoparticle and/or cryoprotectant and/or protectant compound from the composition or lyophilization or desiccation or water or liquid or solid or gas removal of or from the composition.

The invention also relates to the composition according to the invention, where the at least one chain or nanoparticle is in powder form, and the composition has preferentially at least one of the following properties:

    i) the volume occupied by water or liquid or solid or gas or matrix, or surfactant or solvent or of at least one substance different from the nanoparticle and/or cryoprotectant and/or protectant compound and preferentially the cryo-protectant or protectant compound in the composition is smaller than the volume occupied by the at least one chain or nanoparticle, and

ii) the percentage in mass of water or liquid or solid or gas or matrix, or surfactant or solvent or of at least one substance different from the nanoparticle and/or cryoprotectant and/or protectant compound and preferentially the cryo-protectant or protectant compound in the composition is smaller than the percentage in mass of the at least one chain or ,

wherein the composition is preferentially lyophilized, desiccated, dried, dehydrated or subjected to liquid, solid, or gas removal.

In some cases, the composition is lyophilized, desiccated, dried, dehydrated or subject to liquid, solid, or gas removal when water or liquid or solid or gas is removed, preferentially partly or fully, from the composition.

The invention also relates to the composition according to the composition, in which the composition is lyophilized or desiccated or dried or dehydrated or treated by lyophilization or desiccation or water or solid or liquid or solid or gas removal.

In one embodiment of the invention, the composition is dehydrated, or water or liquid or solid or gas is removed or absent partly or fully from the composition. In this case, the percentage in mass of water or liquid or gas or solid in the composition is preferentially larger before the composition is dehydrated or before water or liquid or solid or gas is removed from the composition than after the composition is dehydrated or after water or liquid or solid or gas is removed from the composition. In this case, the volume of water or liquid or solid or gas occupied in the composition is preferentially larger before the composition is dehydrated or before water or liquid or solid or gas is removed from the composition than after the composition is dehydrated or after water or liquid or solid or gas is removed from the composition. In this case, water or liquid or solid or gas is removed from the composition without removing the at least one chain or nanoparticle and preferentially also the cryo-protectant or protectant compound from the composition.

In one embodiment of the invention, the percentage in mass of water or solid or liquid or gas or solvent or surfactant or dispersing or suspending material in the composition is larger than $10^{-3}$, $10^{-1}$, 0, 1, 5, 10, 25, 50, 80 or 99%, preferentially before water or liquid or solid or gas or solvent or surfactant or dispersing or suspending material is or has been removed from the composition.

In another embodiment of the invention, the percentage in mass of water or liquid or solid or gas or solvent or surfactant or dispersing or suspending material in the composition is lower than or equal to 100, 99, 80, 50, 25, 10, 5, 2, 1 or 0%, preferentially after water or liquid or solid or gas or solvent or surfactant or dispersing or suspending material is or has been removed from the composition.

In another embodiment of the invention, the composition is lyophilized or dissected or subject to water, liquid, solid, gas removal partly or fully or the composition is first oxidized or reduced or cooled down or subject to a temperature or pressure gradient, preferentially below or at or above or by preferentially by at least $10^3$, 100, 50, 10, 0, -10, -50, -77, -200, -273 °C, and second water or liquid or solid or gas is removed from the composition partly or fully, preferentially without removing or deactivating or denaturing, or disassembling the at least one chain or nanoparticle or at least one constituent of the composition and/or cryo-protectant or protectant compound from the composition.

In another embodiment of the invention, the composition is lyophilized, dehydrated, dried and/or desiccated, and water or liquid or solid or gas is preferentially removed from the composition preferentially without removing the at least one chain or nanoparticle and/or cryo-protectant compound from the composition.

In one embodiment, the composition is comprised in a tablet or is a tablet form or powder form, preferentially after water or solid or gas has been removed partly or fully from the composition.

In one embodiment, the composition is stoked or stored or unused, preferentially for more than 0, 1, 10, $10^2$, $10^3$, $10^5$, or $10^{10}$ hour(s), day(s), month(s), year(s), preferentially without being used or administered to a body part, preferentially in powder form, preferentially without losing at least one its property or without losing its nanoparticle or at least one constituent of the composition coating or nanoparticle or at least one constituent of the composition core or protectant compound, partly or fully.

In one embodiment of the invention, the moisture or water or liquid or solid or gas content of the composition is lower than or equal to 100, 75, 50, 25, 10, 5, 4, 2, 1 or 0% w/w, preferentially weight of water or liquid or solid or gas for weight of composition or weight of water or liquid or solid or gas by weight of composition or mass of water or liquid or solid or gas for mass of composition or mass of water or liquid or solid or gas by mass of composition, preferentially after water or liquid or solid or gas has been removed from the composition partly or fully.

In another embodiment of the invention, the moisture or water or liquid or solid or gas content of the composition is larger than or equal to 0, 1, 2, 4, 5, 10, 25, 50, 75 or 100% w/w, preferentially weight of water or liquid or solid or gas for weight of composition or weight of water or liquid or solid or gas by weight of composition or mass of water or liquid or solid or gas for mass of composition or mass of water or liquid or solid or gas by mass of composition, preferentially before water or liquid or solid or gas has been removed from the composition.

The invention also relates to the composition according to the invention, where the at least one chain or nanoparticle or at least one constituent of the composition and preferentially the cryo-protectant or protectant compound, which is/are preferentially in powder form, is/are suspended or resuspended or (re)mixed or (re)dispersed in a liquid or water or solid or gas or treated by suspension or resuspension or (re)mixture or (re)dispersion in a liquid or water or solid or gas, preferentially more than 1, 2, 5 or 10 times. In this case, liquid or water or solid or gas is preferentially removed from the composition before the composition is suspended or resuspended or (re)mixed or (re)dispersed in a liquid or water.

The invention also relates to the composition according to the invention, wherein the coating or at least one constituent of the composition is selected in the group of compounds consisting of: 1) citric acid, 2) oleic acid, 3) polymethacrylic acid, 4) poly(ethyleneoxide)-b-poly(methacrylic) acid, 5) polyacrylic acid (PAA), 6) polylactic acid, 7) poly(ethylene oxide)-blockpoly(glutamic) acid, 8) Phosphonic acid 9) Albumin, 10) Alendronate, 11) Alginate, 12) Au, $Al_2O_3$, 13) Aluminium, 14) Aluminium hydroxide, 15) Arabinogalactan, 16) Bentonite, 17) Carboxymethylcellulose, 18) Cellulose, 19) Chitosan, 20) Cholseterol, 21) Citrate, 22) Dextran, 23) Dimercaptosuccinic acid, 24) Dopamine, 25) DOPC or Dioleoylphosphatidylcholine or phospholipd, 26) DTAP or Di(tert.-amyl)peroxide, 27) DVB or Divinylbenzène, 28) Ethylcellulose, 29) Erythrocyte, 30) at least one fatty acids, 31) Ferrite, 32) Folic acid, 33) Gelatin, 34) serum albumin, preferentially human, 35) Liposome, 36) MIPS or Inositol-3-phosphate synthase, 37) MnO or manganese oxide, 38) $Mn_3O_4$, 39) Oleic acid, 40) at least one polymer or enantiomer, 41) PEI or Polyetherimide, 42) PEG or Polyethylene glycol, 43) Poly(ethylene oxide) or PEO, 44) PGA or Polyglycolic acid, 45) PLA or poly(lactide acid), 46) PLGA or PLG or poly(lacticco-glycolic acid), 47) Phosphatidylcholine, 48) Phosphorylcholine, 49) Pluronic, 50) Polyacrylamide, 51) Polyacrylic acid or PAA, 52) Polyaniline, 53) Polyethylene glycol with/without terminal carboxyl groups, 54) Peptide or Polypeptide, 55) Poly(vinyl alcohol) or PVA, 56) Ploy(N-isopropylacrylamide) or PIA, 57) Poly(vinylpyrrolidone) or PVP, 58) Poly(oligoethylene oxide) or POO, 59) Poly(N,N-dimethyl ethylamino acrylate, 60) Poly(imine), 61) Poly(acrylic acid), 62) Poly-D-L lactide, 63) Polyalkylcyanoacrylate, 64) Polymer such as PAMAM or Poly(amidoamine) or PDMAEMA or poly(2-(dimethylamino)ethyl methacrylate) or PPEGMA or Poly (ethylene glycol) methyl ether methacrylate, 65) Poly NIPAAM or Poly (N-isopropylacrylamide) or temperature-responsive polymer or temperature-sensitive polymer 66) Polyacrylic acid, 67) Polydipyrrole or dicarbazole, 68) Poly-L-lysine, 69) Polymethylmethaacrylate, 70) Polymersome, 71) Polystyrene, 72) PVA or Polyvinyl Alcohol, 73) PVP or Polyvinylpyrrolidone, 74) Silica, preferentially amorphous or mesoporous, 75) Silane, 76) $SiO_2$, 77) Sodium Oleate, 78) Starch, 79) styrene, preferentially styrene-divinylbenzene, 80) TaOx, 81) ZrOz, 82) at least one metal or semi-metal, 83) at least one metal oxide or semi-metal oxide, 84) at least one alkali metal, 85) at least one alkaline earth metal, 86) at least one transition metal, 87) at least one post-transition metal, 88) at least one metalloid, 89) at least one lanthanide, 90) at least one actinide, 91) at least one non-metal, 92) at least one halogen, 93) at least one noble gas, i) Polysacharides such as Agarose, alginate, carregeenan, chitosan, dextran, haparin, Gum Arabic, Pullulan and Starch, ii) Acids such as citric acids, oleic acids, polymethacrylic acid, poly(ethyleneoxide)-b-poly(methacrylic acid, polyacrylic or PAA acid, polylactic acid, poly(ethylene oxide)-blockpoly(glutamic acid, Phosphonic acid, Dimercaptosuccinic acid, fatty acid, folic acid, oleic acid, poly(lactide acid or PLA, PAA or Polyacrylic acid, compounds comprising at least one carboxylic function, iii) Polymers such as Dextran, Poly(ethylene oxide), Poly(vinyl alcohol), Ploy(N-isopropylacrylamide), Poly(vinylpyrrolidone), Poly(oligoethylene oxide), Poly(N,N-dimethyl ethylamino acrylate), Poly(imine), Poly(acrylic acid), iv) Carboxylates, v) inorganic compounds such as SiOz, $Al_2O_3$, ZrOz, ferrites, MnO, $Mn_3O_4$, Au, Bentonite, Carbon such as inactivated, activated, graphitized carbon, vi) at least one metals, vii) organic compounds such as MIP, Cellulose, DV8, Ppy, Chitosan, Polyacrylamide, alginate, PEI, surfactants, viii) compounds comprising Phosphate, ix) compounds comprising Silica, x) compounds comprising Gold, xi) compounds comprising Dextran based, xii) compounds comprising PEG, xiii) compounds comprising PVA, xiv) compounds comprising Alginate, xv) compounds comprising Chitosan, xvi) compounds comprising the chemical function Alcohol, xvii) compounds comprising the chemical function Amide, xviii) compounds comprising the chemical function Aldehyde, and 94) any derivative or combination of any of these compounds.

The invention also relates to the composition according to the invention, wherein the coating or at least one constituent of the composition has at least one chemical group selected in the group of i) OH , ii) $NH_2$, iii) COOH, iv) Thiol, v) Phosphate, and a basic or acid derivative of at least one of these compounds.

The invention also relates to the composition according to the invention, wherein the coating or coating material or at least one constituent of the composition comprises a chemical function, preferentially selected in the group consisting of i) OH-, ii) NH2, iii) COOH, iv) Thiol, v) Phosphate, and a basic or acid derivative of at least one of these functions, which has an interaction or forms a chemical bond with an atom or chemical group located at the surface of the core, preferentially a hydroxyl group (Fe-OH), where the interaction or bond is preferentially selected in the group consisting of: i) an electrostatic interaction or bond, *i.e.* preferentially due to the difference in charge between the coating and the surface or core of the core, ii) a hydrophobic interaction or bond, iii) a Chelating interaction or bond, iv) a metallic interaction or bond, v) a Covalent interaction or bond.

In one embodiment of the invention, the cryoprotectant or protectant compound interacts or forms with the core or coating of the nanoparticle or at least one constituent of the composition, hydrogen or van der walls or London bonds or interaction, preferentially weak interactions, preferentially when water or liquid or gas or solid molecules are displaced or cooled down or removed or detached or moved away preferentially from the coating, core, coating surface, core surface, partly or fully.

In one embodiment of the invention, as the cryoprotectant or protectant compound replaces the water or liquid or gas or solid molecules, preferentially when the composition is cooled down or subject to a temperature and/or pressure gradient or oxidized or reduced, the coating and/or core of the nanoparticle or at least one constituent of the composition preferentially retains its structure and function and/or at least two nanoparticle or at least one constituent of the compositions remain arranged in chain or at least one nanoparticle or at least one constituent of the composition maintains at least one property.

In one embodiment, the composition comprising the cryoprotectant or protectant compound does not comprise ice or melted material or dissociated nanoparticle or at least one constituent of the composition, preferentially at least one piece or a majority of at least one of these materials, while the composition not comprising the cryoprotectant or protectant compound comprises ice or melted material or dissociated nanoparticle or at least one constituent of the composition, preferentially at least one piece or a majority of at least one of these materials, or the composition comprising the cryoprotectant or protectant compound comprises more ice or melted material or dissociated nanoparticle or at least one constituent of the composition, preferentially at least one piece or a majority of at least one of these materials, than the composition not comprising the cryoprotectant or protectant compound, preferentially when the composition is cooled down, exposed to temperature, pressure gradient, lyophilized, dried, desiccated, subject to solid, liquid, and/or gas removal, partly or fully.

In one embodiment of the invention, the composition comprising the cryoprotectant or protectant compound has a lower melting or phase-transition, preferentially from one phase to another phase selected among liquid, solid, gas, point than the composition not comprising the cryoprotectant or protectant compound.

In one embodiment of the invention, the cryoprotectant or protectant compound is a nonpermeable cryoprotectant or protectant compound.

In some cases, the cryoprotectant or protectant compound can be selected among: a sugar, trehalose, sucrose, starch, hydroxyethyl starch, polyvinyl pyrrolidone, and/or polyethylene oxide.

In some cases, the cryo-protectant or protectant compound can be nonpermeable cryoprotectant or protectant compound, *i.e.* preferentially it does not enter cells or coatings and thus preferentially stays extracellular or outside the coating when the composition is cooled down.

In some cases, the composition is cooled or subject to temperature and/or pressure gradient or to oxidation or to reduction during or for cryopreservation or preservation of the composition.

In some cases, a nonpermeable cryoprotectant or protectant compound is used to protect cells during the cooling of the composition or exposure of the composition to a temperature and/or pressure gradient or to oxidation or to reduction.

Preferentially, when a cooling or slow cooling or slow freezing rate or slow temperature and/or pressure gradient is applied to the composition or the composition is cooled down or subject to a temperature and/or pressure gradient(s) or to oxidation or to reduction, preferentially slowly, there is sufficient time for water or liquid or solid or gas comprised in the nanoparticle or at least one constituent of the composition preferentially in the coating of the nanoparticle or at least one constituent of the composition to move out of the nanoparticle or at least one constituent of the composition or coating, preferentially under the osmotic pressure, preferentially resulting in a reduction or change in the volume or size of the coating or nanoparticle or at least one constituent of the composition. In the absence of a cryoprotectant or protectant compound, such behavior may or can destroy or damage the coating or nanoparticle or at least one constituent of the composition and/or result in the destruction of the nanoparticle or at least one constituent of the composition chain arrangement or in the change of at least one property of the nanoparticle or at least one constituent of the composition.

In one embodiment, the cryoprotectant or protectant compound is permeable. Preferentially, a permeable cryoprotectant or protectant compound can enter a cell or the coating and preferentially preserve the cell or coating or nanoparticle or at least one constituent of the composition, preferentially from the osmotic pressure or damage or destruction.

In some cases, the cryo-protectant or protectant compound can be dimethyl sulfoxide (DMSO), glycerol, ethylene glycol, or propylene glycol.

In some cases, the cryoprotectant or protectant compound can be used to induce the vitrification of intracellular environment or of the coating or of the inner part or surface part of the coating or nanoparticle or at least one constituent of the composition or core, preferentially before ice crystal formation, preferentially in the composition, preferentially preventing excessive loss volume of cell or coating or nanoparticle or at least one constituent of the composition or core.

In one embodiment of the invention, the cryoprotectant or protectant compound is selected in the group consisting of: i) natural product, ii) soybean flour product, iii) carbohydrate, iv) lipid, v) saccharide, vi) zwitterionic molecule, vii) l-carnitine, and viii) antifreeze compounds such as antifreeze proteins.

The invention also relates to the composition according to the invention, wherein the coating or at least one of its chemical functions or atoms forms a first type of interaction or chemical bond with an atom or chemical function of the core, wherein the cryoprotectant or protectant compound or at least one of its chemical functions or atoms forms a second type of interaction or chemical bond with the coating or at least one of its chemical functions or atoms,

> wherein the first type of interaction or chemical bond is preferentially different from the second type of interaction or chemical bond,
> wherein the first type of interaction or chemical bond is preferentially selected in the group consisting of: i) an electrostatic interaction or bond, *i.e.* preferentially due to the difference in charge between the coating and the surface or core, ii) a hydrophobic interaction or bond, iii) a Chelating interaction or bond, iv) a metallic interaction or bond, and v) a Covalent interaction or bond,
> wherein the second type of interaction or chemical bond is preferentially a hydrogen, London or Van Der Walls interaction or bond.

In some cases, the coating or coating material can comprise a chemical function, preferentially selected in the group consisting of i) OH⁻, ii) $NH_2$, iii) COOH, iv) Thiol, v) Phosphate, and a basic or acid derivative of at least one of these functions.

In some other cases, the core can comprise a chemical function, preferentially located at the surface of the core of the nanoparticle or at least one constituent of the composition, preferentially a hydroxyl group (Fe-OH).

The invention also relates to the composition according to the composition, wherein the cryoprotectant or protectant compound or at least one constituent of the composition is selected in the group consisting of: 1) Acetamide, 2) Acetate, 3) Albumin, 4) Amino acids, 5) Ammonium acetate, 6) Arginine, 7) Alcohols containing at least one or two hydroxyl groups, 8) Bridger, 9) Choline magnesium chloride sodium bromide, 10) Diethyl glycol, 11) Dimethylacetamide, 12) Dimethyl sulfoxide (DMSO), 13) Disaccharide, 14) Erythritol, 15) Ethanol, 16) Ethylene glycol, 17) Formamide, 18) Fructose, 19) Glucose, 20) Glycerol, 21) Glycerol 3-phosphate, 22) Glycol such as Diethyl glycol or Triethylene glycol, 23) Glycine, 24) Lactose, 25) L-tyrosine, 26) lysine hydrochloride, 27) Mannitol, 28) MDP (2-Methyl-2,4-pentanediol), 29) Phenylalanine, 30) Planic, 31) Polymers, 32) Polyethylene glycol such as PEG4000, polyethylene glycol succinate, folate modified distearoylphosphatidyl ethanolamine-polyethylene glycol, 33) Polyethyleneimine (PEI), 34) Polyvinylpyrrolidone (PVP), 35) Proline, 36) Propylene glycol, 37) Protein, 38) Pyridine (Pyridine-N-Oxide), 39) Ribose, 40) Sarcosine, 41) Serine, 42) Serum albumin, 43) Sodium bromide, 44) Sodium chloride, 45) Sodium dodecyl sulfonate, 46) Sodium glutamate, 47) Sodium iodide, 48) Sodium sulfate, 49) Sorbitol, 50) Starch (hydroxyethyl starch), 51) Sugar, 52) Sucrose, 53) The cell bank series, 54) Trehalose, 55) Triethylene glycol, 56) Trimethylamine, 57) Tween 80, 58) Tryptophan, 59) Valine, 60) Xylose, and 61) a combination or derivative of any of these compounds.

The invention also relates to the composition according to the invention, wherein the composition preferentially comprises an inert part and/or an active part, wherein the inner part preferentially does not comprise at least one center of activity or the active part preferentially comprises at least one center of activity or the active part preferentially comprises more or a larger number of centers of activity than the inner part, wherein the inner part preferentially ensures the cohesion of the composition or preferentially comprises links or bonds or forces or atoms or ions or nanoparticle or at least one constituent of the compositions that maintain the at least one or two constituent(s) of the composition assembled together within one volume, wherein the center of activity is preferentially comprised in the nanoparticle or at least one constituent of the composition or at least one constituent of the composition, wherein the center of activity preferentially increases or decreases or amplifies or attenuates the production of heat or cold by the nanoparticle or at least one constituent of the composition or at least one constituent of the composition, the medical activity of the nanoparticle or at least one constituent of the composition or at least one constituent of the composition, and/or the radiation or strength or power or wavelength or intensity or frequency of the radiation applied on the nanoparticle or at least one constituent of the composition or at least one constituent of the composition,

> where the center of activity is preferentially selected in the group consisting of:
>
> > A) a thermal center that preferentially increases the thermal activity of the nanoparticle or at least one constituent of the composition or at least one constituent of the composition, preferentially heat or the cold, preferentially by at least 0.1 °C, preferentially locally around the nanoparticle or at least one constituent of the composition or at least one constituent of the composition or at a distance from the nanoparticle or at least one constituent of the composition or at least one constituent of the composition of preferentially less than 1 μm,
> > B) a thermal center that preferentially decreases the thermal activity of the nanoparticle or at least one constituent of the composition or at least one constituent of the composition, preferentially heat or the cold, preferentially by at least 0.1 °C, preferentially locally around the nanoparticle or at least one constituent of the composition or at least one constituent of the composition or at a distance from the nanoparticle or at least one constituent of the composition or at least one constituent of the composition preferentially of less than 1 $\mu$m,

C) a medical center that preferentially increases or enhances the effect or activity or strength of a medical compound, preferentially selected in the group consisting of: an enhancer of an immunotherapy, chemotherapy, hormonotherapy, radiotherapy or radio-enhancer, contrast agent, sonosensitizer, and surgical medical compound,

D) a medical center that preferentially decreases the effect or activity or strength of a medical compound, preferentially selected in the group consisting of: an attenuator of an immunotherapy, chemotherapy, hormonotherapy, radiotherapy or radio-enhancer, contrast agent, sonosensitizer, and surgical medical compound,

E) a center of radiation amplification that preferentially increases the strength of a radiation applied on the nanoparticle or at least one constituent of the composition or at least one constituent of the composition, preferentially by at least $10^{-5}$ or 0.1 Gray or Watt or Candela, preferentially locally around the nanoparticle or at least one constituent of the composition or at least one constituent of the composition or at a distance from the nanoparticle or at least one constituent of the composition or at least one constituent of the composition preferentially of less than 1 $\mu$m,

F) a center of radiation attenuation that preferentially decreases the strength of a radiation applied on the nanoparticle or at least one constituent of the composition or at least one constituent of the composition, preferentially by at least $10^{-5}$ or 0.1 Gray or Watt or Candela, preferentially locally around the nanoparticle or at least one constituent of the composition or at least one constituent of the composition or at a distance from the nanoparticle or at least one constituent of the composition or at least one constituent of the composition of preferentially less than 1 $\mu$m, and

G) a center of activity or free radical production or capture,

wherein the at least one center of activity is preferentially characterized by at least one property selected in the group consisting of:

I) the nanoparticle or at least one constituent of the composition core or at least a first constituent of the composition preferentially comprises a first center of activity, $C_{A1}$, preferentially being a first center of activity or free radical production or capture $C_{1FRPC}$, wherein $C_{A1}$ or $C_{1FRPC}$ is preferentially selected in the group consisting of:

i) another metal than iron such as Zinc or Aluminum,
ii) another metal oxide than iron oxide such as Zinc Oxide or Aluminum Oxide,
iii) a compound that is essentially or in majority or at least partly inorganic or metallic,

and

II) the nanoparticle or at least one constituent of the composition coating or second constituent of the composition preferentially comprises a second center of activity, $C_{A2}$, preferentially being a second center of activity or free radical production or capture $C_{2FRPC}$, preferentially being a compound that is essentially or in majority or at least partly organic or non-metallic,

III) the cryo-protectant or third constituent of the composition preferentially comprises a third center of activity, $C_{A3}$, preferentially being a center of activity that preferentially protects or maintains or prevents the diminution or increases the activity of $C_{A1}$ and/or $C_{A2}$,

wherein $C_{A1}$, $C_{A2}$, and/or $C_{A3}$ has(have) at least one property selected in the group consisting of:

i) It(they) is(are) preferentially over time or under storage or under use of the composition or under administration of the composition to the body part.
ii) $C_{A1}$, $C_{A2}$, and/or $C_{A3}$ is(are) preferentially different compounds,
iii) $C_{A1}$, $C_{A2}$, and/or $C_{A3}$ activity can preferentially be measured by comparing the activity of a body part or medium comprising the composition with that of a body part or medium not comprising the composition, where the body part or medium comprising and not comprising the composition are exposed to similar or the same radiation or thermal variation,
iv) $C_{A1}$, $C_{A2}$, and/or $C_{A3}$ is(are) preferentially separated by a distance of at least 0.1, 1, 5, 10, 100 or $10^3$ nm,
v) $C_{A1}$, $C_{A2}$, and/or $C_{A3}$ is(are) preferentially different from at least one whole constituent of the composition, *i.e.* preferentially the at least one whole constituent preferentially comprises at least one other substance that is different from a center of activity,
vi) $C_{A1}$, $C_{A2}$, and/or $C_{A3}$ occupies(y) preferentially less than 100 or 99 or 50% of the volume or location of the at least one constituent,
vii) $C_{A1}$, $C_{A2}$, and/or $C_{A3}$ preferentially has(have) a percentage in mass of less than 100 or 99 or 50% relatively to the mass of the at least one constituent,

viii) $C_{A1}$, $C_{A2}$, and/or $C_{A3}$ preferentially release or diffuse, preferentially in an outward direction relatively to at least one constituent of the composition, or expel or activate at least one atom, electron, free radical, ion, metal, DNA, RNA, protein, lipid, enzyme, biological or non-biological material, organic or non-organic material, an immune entity from or of or in or at the surface or outside the nanoparticle or at least one constituent of the composition or at least one constituent of the composition, preferentially against or to fight against a disease or preferentially against or to deactivate or kill at least one pathological cell or preferentially to activate a first type of immune entity that deactivates or kills at least one pathological cell or disease or preferentially to deactivate a second type of immune entity that protects at least one pathological cell or disease or tumor or tumor environment, and ix) $C_{A1}$, $C_{A2}$, and/or $C_{A3}$ preferentially capture or diffuse, preferentially in an inward direction relatively to at least one constituent of the composition, at least one atom, electron, free radical, ion, metal, DNA, RNA, protein, lipid, enzyme, biological or non-biological material, organic or non-organic material, an immune entity from or of or in or at the surface or inside the nanoparticle or at least one constituent of the composition or at least one constituent of the composition, preferentially against or to fight against a disease or preferentially against or to deactivate or kill at least one pathological cell or preferentially to activate a first type of immune entity that deactivates or kills at least one pathological cell or disease or preferentially to deactivate a second type of immune entity that protects at least one pathological cell or disease or tumor or tumor environment,

wherein the at least one constituent of the composition is preferentially selected in the group consisting of: i) the nanoparticle coating, ii) the nanoparticle core, iii) the cryo-protectant or protectant compound, iv) the other compound, v) a link or bond or interaction between at least two constituents of the composition, and vi) water or liquid or solid or matrix or gas or surfactant or solvent preferentially embedding or surrounding at least one constituent of the composition.

wherein the immune entity(ies), preferentially the first and/or second immune entity(ies), is/are preferentially selected in the group consisting of: i) DNA preferentially different types of DNA, ii) RNA preferentially different types of RNA, iii) an antigen, ii) an antibody, iii) an immune cell, preferentially of or belonging to the innate and/or adaptative immune system(s), iv) an antigen presenting cell (APC), v) a basophil, vi) a dendritic cell, vii) an eosinophil, viii) a granulocyte, ix) a killing cell, x) a natural killer, xi) a leukocyte, xii) a lymphocyte, xiii) a macrophage, preferentially of M1 and/or M2 type(s), xiii) a mast cell, xiv) a neutrophil, xv) a phagocyte, xvi) a B cell, xvii) a T cell, xviii) a CD8 or CD8$^+$ or CD4 or CD4$^+$ or Treg or MAIT or Ty6 T lymphocyte or cell, xix) a helper cell preferentially of Thl or Th2 type, and xx) a gamma delta T cell.

In one embodiment of the invention, the composition is desiccated or lyophilized or dehydrated or dried or subject to liquid, gas, and/or solid removal. In this case, the percentage in mass of water or liquid, gas, and/or solid in the composition is preferentially smaller than 100, 75, 50, 75, 20, 10, 5, 2, 1, 0, $10^{-1}$, $10^{-3}$ or $10^{-5}$%. In this case, the quantity of water or liquid, gas, and/or solid in the composition is preferentially smaller than 100, 50, 10, 5, 2, 1, 10 ', $10^{-3}$, or $10^{-5}$ grams of water or liquid, gas, and/or solid in the composition per gram of composition.

In some other cases, the composition can be non-desiccated, non-dehydrated, or non-dehydrated or dried or not subjected to liquid, gas, and/or solid removal. In this case, the percentage in mass of water or liquid, gas, and/or solid in the composition is preferentially larger than $10^{-5}$, $10^{-3}$, $10^{-1}$, 0, 1, 5, 10, 25, 50, 75 or 100%. In this case, the quantity of water or liquid, gas, and/or solid in the composition is preferentially larger than $10^{-10}$, $10^{-5}$, $10^{-1}$, 0, 1, 5, 10 or 50, 10 grams of water or liquid, gas, and/or solid in the composition per gram of composition.

The invention also relates to the composition according to the invention, which is lyophilized or desiccated or dehydrated or subject to liquid, gas, and/or solid removal, wherein the percentage in mass of cryoprotectant or protectant compound is preferentially larger than the percentage in mass of coating in the lyophilized or desiccated or dehydrated composition or composition subjected to liquid, gas, and/or solid removal, wherein the lyophilized or desiccated or dehydrated composition or composition subjected to liquid, gas, and/or solid removal is preferentially more stable than non-lyophilized or non-desiccated or non-dehydrated composition or composition not subjected to liquid, gas, and/or solid removal.

The invention also relates to the composition, where the percentage in mass of the cryo-protectant or protectant compound is comprised between 0.5 and 50%.

In some cases, the percentage in mass of a substance comprised in the composition, e.g. the cryo-protectant or protectant compound, nanoparticle or at least one constituent of the composition core, nanoparticle coating, chain, liquid or water, is equal to or proportional to the mass of this substance divided by the mass of all the substances comprised in the composition.

In some cases, the percentage in mass of the cryo-protectant or protectant compound, nanoparticle coating, nanoparticle core, at least one chain or nanoparticle or at least one constituent of the composition, liquid or water, gas, solid, in the composition is larger than $10^{-5}$, $10^{-3}$, $10^{-1}$, 0, 1, 5, 10, 25, 50, 75, 90 or 99%.

In some other cases, the percentage in mass of the cryo-protectant or protectant compound, nanoparticle coating, nanoparticle core or at least one constituent of the composition, at least one chain, liquid or water, gas, solid, in the composition is lower than 100, 75, 50, 25, 10, 5, 2, 1, 0, $10^{-3}$ or $10^{-5}$%.

In one embodiment of the invention, the percentage in mass of cryo-protectant or protectant compound in the composition according to the invention is larger than the percentage in mass of nanoparticle coating, nanoparticle core, at least one chain, solid, gas, liquid and/or water, in the composition.

In another embodiment of the invention, the percentage in mass of cryo-protectant or protectant compound in the composition according to the invention is smaller than the percentage in mass of nanoparticle coating, nanoparticle core, at least one chain, solid, gas, liquid and/or water, in the composition.

The invention also relates to the composition according to the invention, wherein the nanoparticle core or at least a first constituent of the composition comprises a first center of activity or free radical production or capture $C_{1FRPC}$,

wherein $C_{1FRPC}$ is preferentially selected in the group consisting of:

    i) another metal than iron such as Zinc or Aluminum, and
    ii) another metal oxide than iron oxide such as Zinc Oxide or Aluminum Oxide,

In some cases, $C_{1FRPC}$ can comprises at least one atom that is preferentially linked to at least one oxygen or iron atom of the core preferentially through at least one metallic bound.

The invention also relates to the composition according to the invention, wherein the coating or at least a second constituent of the composition comprises a second center of activity or free radical production or capture $C_{2FRPC}$.

The invention also relates to the composition according to the invention, wherein $C_{2FRPC}$ is linked to at least one atom of the coating or second constituent of the composition, preferentially through at least one bond, weak or strong bond, preferentially covalent bond.

In one embodiment of the invention, the center a free radical production or capture is at least one atom, molecule, chemical function, ionized or not, charged or not, which produces or captures at least one free radical.

In one embodiment of the invention, a free radical is at least one atom, molecule, ion or chemical function that has: i) at least one unpaired valence electron, ii) a capacity to dimerize, iii) a short lifetime, and/or iv) two unpaired electrons.

In one embodiment of the invention, a free radical is at least one atom, molecule, ion or chemical function that is: i) a chemically reactive species, ii) a hydroxyl radical (HO·), iii) a triplet oxygen, iv) a triplet carbene, and/or v) (:CH2).

In one embodiment of the invention, radicals are generated or produced when the composition is excited in at least one of the following ways: i) by being exposed to redox reactions, ii) by being subjected to radiation, preferentially ionizing or electromagnetic radiation, iii) by being heated, iv) by being exposed to electrical discharges, v) by undergoing electrolysis, vi) by being exposed to pH variation.

In one embodiment of the invention, the center a free radical production is at least one atom, molecule, chemical function, ionized or not, charged or not, which produces at least one free radical, preferentially in such a way that the quantity or concentration of free radicals present when the composition comprises the center of activity or free radical production is more important than the quantity or concentration of free radicals present when the composition does not comprise the center of activity or free radical production, where the comparison is preferentially being made using the same or similar conditions of excitation for the composition comprising the center of activity or free radical production than for the composition not comprising the center of activity or free radical production.

In one embodiment of the invention, the center a free radical capture is at least one atom, molecule, chemical function, ionized or not, charged or not, which captures at least one free radical, preferentially in such a way that the quantity or concentration of free radicals present when the composition comprises the center of activity or free radical capture is less important than the quantity or concentration of free radicals present when the composition does not comprise the center of activity or free radical capture, where the comparison is preferentially being made using the same or similar conditions of excitation for the composition comprising the center of activity or free radical capture than for the composition not comprising the center of activity or free radical capture.

The invention also relates to the composition according to the invention, wherein $C_{1FRPC}$ and/or $C_{2FRPC}$ is/are at least one anti-oxidizing compound.

The invention also relates to the composition according to the invention, wherein $C_{1FRPC}$ and/or $C_{2FRPC}$ is/are at least one oxidizing compound.

In one embodiment of the invention, the core of the nanoparticle or at least one constituent of the composition in the composition according to the invention has at least one property selected in the group consisting of: a) it is ferrimagnetic, b) it is composed of maghemite or magnetite or of an intermediate composition between maghemite and magnetite, c) it has a size comprised between 0.1 and 100 nm, and d) it comprises at least one crystallographic plane.

In another embodiment of the invention, the coating of the nanoparticle or at least one constituent of the composition in the composition according to the invention has at least one property selected in the group consisting of: a) it has a thickness lower than 10 μm, b) it comprises a number of crystallographic planes per unit surface that is lower than the number of crystallographic planes per unit surface of the core, c) it has a non-neutral charge, d) it is amorphous and e) it is organic, and f) it has a thickness that is lower than 10 nm or than the diameter of the core.

In another embodiment of the invention, the cryoprotectant or protectant compound in the composition according to the invention is comprised in a matrix or volume embedding the core and/or coating of the nanoparticle or at least one constituent of the composition.

The invention also relates to the composition according to the invention, wherein the core or nanoparticle core or at least one constituent of the composition is synthesized by a living organism or nanoparticle-producing cells, preferentially a magnetotactic bacterium and/or the coating is not synthesized by a living organism or nanoparticle-producing cell.

In one embodiment of the invention, the nanoparticle or at least one constituent of the compositions are synthesized biologically or by a living organism, designated as synthetizing living organism or nanoparticle-producing cell, which preferentially consists or comprises at least 1, 2, 5, 10, $10^3$, $10^6$ or $10^9$ eukaryotic cell(s), prokaryotic cell(s), or part of these cells.

In some cases, part of eukaryotic or prokaryotic cell(s) can be biological material originating or produced by these cells such as RNA, DNA, organelle, nucleolus, nucleus, ribosome, vesicle, rough endoplasmic reticulum, Golgi apparatus, cytoskeleton, smooth endoplasmic reticulum, mitochondrion, vacuole, cytosol, lysosome, centrosome, cell membrane. In some cases, a biological synthesis can be defined as a synthesis involving a majority of steps, or more than 1, 2, 5 or 10 steps, or more than 1, 2, 5, 25, 50, 75 or 90% of steps, which involve chemical reactions occurring with the involvement of at least 1, 2, 10, $10^3$, $10^6$ or $10^9$ living organisms, or parts of living organisms such as DNA, RNA, proteins, enzymes, lipids.

In some cases, the synthetizing living organism or nanoparticle-producing cell can be magnetotactic bacteria, other types bacteria than magnetotactic bacteria or enzymes of certain bacteria, preferentially synthetizing nanoparticle or at least one constituent of the compositions extra-cellularly, such as Mycobacterium paratuberculosis, Shewanella oneidensi, Geothrix fermentans, ants, fungi, or various plants.

In still another embodiment of the invention, the nanoparticles or at least one constituent of the compositions is/are synthesized or produced or crystallized or assembled or transformed into a nanoparticle(s) or at least one constituent of the composition by a compartment, organelle, or other biological material, such as protein, lipid, enzyme, DNA, or RNA, which is preferentially produced by or originates from an eukaryotic or prokaryotic cell.

In another embodiment of the invention, the nanoparticle or at least one constituent of the compositions are synthesized by or in at least one eukaryotic cell, prokaryotic cell, or part of this cell.

In another embodiment of the invention, the nanoparticle or at least one constituent of the compositions are synthesized by or in: i) the matrix or medium or environment located outside of at least one eukaryotic cell, prokaryotic cell, or part of this cell, or ii) the extracellular matrix.

In one embodiment of the invention, the nanoparticle or at least one constituent of the compositions are synthesized by a living organism when at least 1, 2, 5, 10 or 100 step(s) of their production, such as crystallization of iron oxide, stabilization of the iron oxide mineral, organization of the nanoparticle or at least one constituent of the compositions, for example in chains or aggregates, involves or is due to a living organism.

The invention also relates to the nanoparticle or at least one constituent of the compositions for use, wherein the nanoparticle or at least one constituent of the compositions are magnetosomes synthesized by, originating from, extracted from, or isolated from magnetotactic bacteria.

In one embodiment of the invention, the magnetosome is synthesized by, produced by, originates from, extracted from, isolated from magnetotactic bacteria.

In one embodiment of the invention, magnetotactic bacteria are selected from the group consisting of: *Magnetospirillum magneticum* strain AMB-1, magnetotactic coccus strain MC-1, three facultative anaerobic vibrios strains MV-1, MV-2 and MV-4, the *Magnetospirillum magnetotacticum* strain MS-1, the *Magnetospirillum gryphiswaldense* strain MSR-1, a facultative anaerobic magnetotactic spirillum, *Magnetospirillum magneticum* strain MGT-1, and an obligate anaerobe, and *Desulfovibrio magneticus* RS-1.

In one embodiment of the invention, a magnetotactic bacterium is defined as a bacterium able to synthesize magnetosomes, wherein these magnetosomes are preferentially characterized by at least one of the following properties: i) they are produced intracellularly, ii) they are magnetic, iii) they comprise a mineral, iv) their core is preferentially composed of a metallic oxide such as iron oxide, v) their core is surrounded by biological material such as lipids, proteins, endotoxins, which can preferentially be removed, vi) they are arranged in chains, vii) they produce heat under the application of an alternating magnetic field.

In one embodiment of the invention, the magnetosomes possess one or several property(ies) in common with the nanoparticle or at least one constituent of the compositions such as at least one magnetic, size, composition, chain arrangement, charge, core, mineral, coating, or crystallinity property.

In one embodiment of the invention, magnetosomes comprise the mineral part synthesized by magnetotactic bacteria, i.e. preferentially the crystallized iron oxide produced by these bacteria. In this case, magnetosomes or magnetosome mineral parts preferentially do not comprise proteins, lipids, endotoxins, or biological materials comprising carbon or do not comprise more or comprise less than 0.1, 1, 10, 30, 50 or 75% or percent in mass of carbon, which is/are produced by these bacteria.

The invention also relates to the composition according to the invention, wherein $C_{1FRPC}$ and/or $C_{2FRPC}$ is/are is/are photosensitizer(s), preferentially selected in the group consisting of: 1) Acridine, such as Acridine Orange, acridine yellow, 2) ALA (5-Aminolevulinic acid), 3) Aluminum phthalocyanine tetrasulfonate (AlPcS4), 4) Aminolevulinic acid, delta- Aminolevulinic acid, 5) Antihistamines, 6) Azulene, 7) Bavteriochlorin, 8) TOOKAD or TOOKAD Soluble, 9) WST-11, 10) LUZ11, 11) BC19, 12) BC21, 13) porphyrin such as Benzoporphyrin derivative monoacid ring A (BPD-MA), 14) Chlorin such as Chlorin e6, m-tetrahydroxyphenylchlorin 15) Foscan, 16) Verteporfin, 17) benzoporphyrin derivative mono acid ring A, 18) Monoaspartyl chlorin(e6), 19) talaporfin sodium, 20) HPPH, 21) Transition metal compounds, 22) Chlorine e6 green porphrin, 23) Chlorine e6 porphrin, 24) Coal Tar and Derivatives, 25) Contraceptives, Oral and Estrogens, 26) Curcumin, 27) Cyanine, 28) Cysview, 29) Dyes such as synthetic dyes, 30) Phenothiazinium salts, 31) Rose Bengal, 32) Squaraines, 33) BODIPY dyes, 34) Phenalenones, 35) benzophenoxazinium dyes, 36) Erythrosine, 37) Flavins, 38) Foscan, 39) Fotoscan, 40) Fullerenes such as cationic fullerenes, 41) Furocoumarins, 42) HAL (Hexaminolevulinate), 43) Hemoporfin, 44) 2-(1-Hexyloxyethyl)-2-devinyl pyropheophorbide (HPPH), 45) Hypericin, 46) Hypocrellin, 47) ICG (Indocyanine Green), 48) Levulan, 49) MAL -methyl aminolevulinate), 50) Meta-tetra(hydroxyphenyl)chlorin (m-THPC), 51) Metvix, 52) Methylene Blue, 53) Monoterpene, 54) Motexafin lutetium (Lu-Tex), 54) N-aspartyl chlorin e6 (NPe6), 55) Nanoparticle or at least one constituent of the composition or nanomaterial, 56) Natural products or compounds, 57) Non-Steroidal Anti-Inflammatory Drugs, 58) Palladium bacteriopheophorbide (WST09), 59) Phatalocyanin dyes, 60) Phenothiazines, 61) Photochlor, 62) Photofrin, 63) Photosens, 64) Phthalocyanine such as Liposomal ZnPC, 65) Chloroaluminium sulfonated phthalocyanine (CASP), 66)

Silicon phthalocyanine (PC4), 67) RLP068, 68) Porfimer sodium, 69) Porfins, 69) Porphyrins, such as 5,10,15,20-Tetrakis(1-methylpyridinium-4-yl) porphyrin tosylate, 70) XF70, 71) Protoporphyrin, 72) ALA-induced protoporphyrin IX, 73) Psoralens, 74) Quantum dots, 75) Quinones, 76) Riboflavin, 77) Rose Bengal, 78) silicon or Silicon phthalocyanine (Pc4), 79) Sulfonamides, 80) Sulfonylureas, 81) Talaporfin or Talaporfin soudium, 82) Temoporfin, 82) Tetrahydropyrroles, 83) Tin ethyl etiopurpurin, 84) Titanium dioxide, 85) Toldudine blue O, 86) Transition metal compounds such as Ruthenium(II), polypyridyl complexes, ruthenium, rhodium, cyclometalated, Rh(II)-Rh(II) bridged dimer compounds, platinum(II), gold(III), 87) Verteporfin, 88) Vulnic based compound such as Aminovulinic, aminovulinic acid, 89) WST11, and 90) Xanthene.

The invention also relates to the composition according to the invention, wherein $C_{1FRPC}$ and/or $C_{2FRPC}$ is/are is/are sonosensitizer(s), preferentially selected in the group consisting of: 1) ABS-FA, 2) Acrylonitrile Butadiene Styrene, 3) Styrene, 4) Folic acid, 5) AIMP NP, aminoacyl tRNA synthetase complex-interacting multifunctional protein, 6) Au Nanomaterial, 7) gold, 8) Au-MnO nanomaterial, 9) manganese oxide, 10) Antineoplastic drugs, 11) NSAIDs, 12) nonsteroidal anti-inflammatory drug, 13) Artemether, 14) 5-ALA (5-aminolevulinic acid), 15) Acridine, Acridine Orange, 16) Au-doped TiO2, 17) Carbon based nanomaterial, 18) carbon nanotube, 19) Chlorine, 20) Ce6, 21) PTX, Paclitaxel, 22) chemotherapeutic drug or compound, 23) infrared dye or IR783, 24) Curcumin, 25) Cyanine or Cu-Cyanine, 26) DHMS, 27) dimethylsulfure, 28) Docetaxel, 29) chemotherapeutic drug or compound, 30) DOX/Mn-TPPS@RBCS, 31) doxorubicin, 32) manganese, 33) blood cell, 34) red blood cell, cell, 35) polymer, 36) elastomer, 37) Erythosin or Erythosin B, 38) FA or FA-OI or FA-OI NP or folic acid, 39) F3-PLGA@MB/Gd NPs, 40) poly(lactic-co-glycolic acid), 41) gadolinium, 42) Fe-TiO2 or titanium oxide, 43) Fe-VS2, 44) iron, 45) vanadium disulfide, 46) FMSNs-DOX, 47) silica, 48) HCQ, 49) hydrochloroquine, 50) HP, 51) hematoporphyrin, 52) HMME, 53) hematoporphyrin monomethyl ether, 54) HSYA or Hydroxysafflor yellow A, 55) Hypocrellin, Hypocrellin B, 56) IR780, 57) Levofloxacin, 58) LIP3 or Lithium phosphide, 59) Lithium, 60) Liposome or Liposomal nanomaterial, 61) Lomefoxacin,, 62) MG@P NPs, 63) MnP or Manganese peroxidase, 64) MnTTP-HSAs, 65) HSA-wrapped metal-porphyrin complex, 66) albumin, 67) Mn-WOx, 68) MnWOx-PEG, 69), PEG, 70) bimetallic oxide, 71) Mn (III)-HFs, 72) managense, hemoporfin, 73) Nanoroads, 74) Noble metal nanomaterial, 75) OI NP or oxygen indyocyanine, 76) Phthalocyanines, 77) PIO or Pioglitazone, 78) Polymeric nanomaterial, 79) Porphyrin, 80) Pt-doped TiO2, 81) R837, 82) Rose Bengal, 83) Sparfloxacin,, 84) TAPP or 5,10,15,20-tetrakis (4-aminophenyl) porphyrin, 85) TiO2 or titanium dioxide nanomaterial, 86) TCPP, isomer, or Tris(1-chloro-2-propyl) phosphate 87) TPI or Thermoplastic Polyimide or thermoplastic polymer, 88) TPZ or Tirapazamine, 89) Transition metal oxide, 90) nanoparticle or at least one constituent of the composition or Janus nanoparticle or at least one constituent of the composition, and 91) Xanthones.

The invention also relates to the composition according to the invention, wherein $C_{1FRPC}$ and/or $C_{2FRPC}$ is/are radio-sensitizer, preferentially selected in the group consisting of: 1) AMG102, 2) AQ4N, 3) Apaziquone (E09), 4) Bromodeoxyuridine, 5) Carbogen, 6) Cetuximab, 7) Chemotherapeutic drug or compound, 8) Chlorpromazine, 9) C-reactive peptide, 10) Curcumin, 11) Diamide, 12) Diethylmaeate,, 13) Dihydroartemisinin, 14) Docetaxel, 15) ECI301, 16) Etanidazole, 17) Fludarabine, 18) 5-Fluorouracil, 19) Fluorodeoxyuridine, 20) Gadolynium, 21) Gemcitabine, 22) HER-3 ADC, 23) HSP, 24) Hydrogen peroxide, 25) Hydroxyurea, 26) Hyperbaric oxygen, 27) Hyperthermia, 28) Hypoxic cell cytotoxic agent, 29) Irinotecan, 30) lanthanide-doped radiosensitizer-based metal-phenolic network, 31) Lidocaine, 32) Lododeoxyuridine, 33) Metronidazole, 34) misonidazole, 35) etanidazole, 36) nimorazole, 37) N-Ethylmalemide, 38) malmeide, 39) ethylmalmeide, 40) Nanomaterial such as those consisting of or composed of at least partly or fully gold , silver, bismuth, gadolinium, polysiloxane matrix and gadolinium chelates, hafnium, Tantalum, Zinc, Gadolinium, Germanium, Chromium, Praseodymium, Silicon, iron, platinum, cobalt, manganese, magnesium, iron, Titanium, carbon nanotube, quantum dot, nanoroad, Triflate, or metal oxide, 41) Nelfinavir, 42) Nicotinamide, 43) Nimotuzumab, 44) RNA, or miRNA, or miR-201, or miR-205, or miR-144-5p, or miR-146a-5p, or miR-150, or miR-99a, or miR-139-5p, or miR-320a, 45) Membrane active agent, 46) Mitomycin-C or Mitomycin, 47) Motexafin, 48) NBTXR3, 49) Oligonucleotide, 50) Paclitaxel, 51) Papaverine or Papaverine hydrochloride, 52) Paraxonase-2, 53) Pocaine, 54) Porfiromycin (POR), 55) Protein, 56) Peptide, 57) Radiosensitizing nucleosides or compounds, 58) Resveratrol, 59) RRx-001, 60) SiRNa, 61) Suppressors of sulfhydral groups, 62) SYM004, 63) Texaphyrins, 64) TH-302, and 65) Tirapazamine. In one embodiment of the invention, $C_{1FRPC}$ and $C_{2FRPC}$ act synergistically, *i.e.* that the photo-sensitizing or sono-sensitizing or radio-sensitizing strength of the nanoparticle or at least one constituent of the composition comprising $C_{1FRPC}$ and $C_{2FRPC}$ is larger than the photo-sensitizing or sono-sensitizing or radio-sensitizing strength of the nanoparticle or at least one constituent of the composition comprising only $C_{1FRPC}$ or $C_{2FRPC}$.

In some cases, the photo-sensitizing or sono-sensitizing or radio-sensitizing strength is the concentration or quantity of radical species, preferentially free radical species, produced or captured by the at least one photo-sensitizer, radio-sensitizer, sono-sensitizer, center of activity or free radical capture or production, or composition, or is or is proportional to the strength or intensity of the acoustic wave, ultrasound, radiation, light, electromagnetic radiation, preferentially applied on the photo-sensitizer, radio-sensitizer, sono-sensitizer, center of activity or free radical capture or production or composition.

In one embodiment of the invention, $C_{A1}$, $C_{A2}$, $C_{A3}$, $C_{1FRPC}$ and/or $C_{2FRPC}$ act anti-synergistically, *i.e.* that the photo-sensitizing or sono-sensitizing or radio-sensitizing or activity strength of the nanoparticle or at least one constituent of the composition comprising at least two compounds selected among $C_{A1}$, $C_{A2}$, $C_{A3}$, $C_{1FRPC}$ and $C_{2FRPC}$ is lower than the photo-sensitizing or sono-sensitizing or radio-sensitizing strength of the nanoparticle or at least one constituent of the composition comprising only $C_{A1}$, $C_{A2}$, $C_{A3}$, $C_{1FRPC}$ or $C_{2FRPC}$.

In one embodiment of the invention, the composition is introduced or administered to or in the body part or is used for such purpose of being introduced or administered to or in the body part, preferentially of an animal or human.

Preferentially, following its introduction or administration, the nanoparticle or at least one constituent of the composition firstly comprises at least two or three centers of activities selected in the group consisting of: $C_{A1}$, $C_{A2}$, $C_{A3}$, $C_{1FRPC}$ and $C_{2FRPC}$ during a time $t_1$ and secondly comprises at least one or two centers of activities, preferentially comprised in the nanoparticle core, preferentially selected in the group consisting of $C_{A1}$, $C_{1FRPC}$ during a time $t_2$, where $t_2$ follows $t_1$.

In some cases, $t_1$ and/or $t_2$ can be longer than $10^{-10}$, $10^{-5}$, $10^{-3}$, $10^{-1}$, 0, 1, 5, 10 or $10^3$ second(s) or minute(s).

In some other cases $t_1$ and/or $t_2$ can be shorter than $10^{10}$, $10^5$, $10^3$, 10, 0, 1, 5, $10^{-1}$ or $10^{-3}$ second(s) or minute(s).

In some cases, $t_2$ can be separated from $t_1$ by more than $10^{-10}$, $10^{-5}$, $10^{-3}$, $10^{-1}$, 0, 1, 5, 10 or $10^3$ second(s) or minute(s).

In some other cases, $t_2$ can be separated from $t_1$ by less than $10^{10}$, $10^5$, $10^3$, 10, 0, 1, 5, $10^{-1}$ or $10^{-3}$ second(s) or minute(s).

The invention also relates to the composition according to the invention, where the composition is or comprises: i) a medical device, ii) a drug, iii) a pharmaceutical product or preparation, iv) a medical product or preparation, iv) a biological product or preparation, vi) an adjuvant, vii) an excipient, viii) an active principle, ix) a vaccine or vaccine component, vi) a product, vii) a suspension, and/or viii) a lyophilized suspension or composition or preparation.

In one embodiment, the composition according to the invention is used for/in: i) the treatment of an infection, ii) the treatment of a viral disease, iii) the treatment of a disease, preferentially a cancerous disease, iv) radiotherapy, v) photodynamic therapy, and/or vi) sonodynamic therapy.

In some cases, the disease can be due to the malfunction of an organ or body part preferentially of an individual.

In some cases, the treatment can be the therapy and/or the diagnosis of a disease or a cosmetic treatment.

In some cases, the treatment can induce the death, destruction, denaturation, or inactivation of at least 1 biological material, such as cell, preferentially pathological cell, RNA, DNA, protein, lipid, or enzyme, where the death of cell can occur through apoptosis or necrosis.

The invention also relates to nanoparticle or at least one constituent of the compositions for use in the treatment or diagnosis of a the disease preferentially selected from the group consisting of: a disease associated with a proliferation of cells that is different from the cellular proliferation in a healthy individual, a disease associated with the presence of pathological cells in the body part, a disease associated with the presence of a pathological site in an individual or body part, a disease or disorder or malfunction of the body part, a disease associated with the presence of radio-resistant or acoustic-resistant cells, an infectious disease, an auto-immune disease, a neuropathology, a cancer, a tumor, a disease comprising or due to at least one cancer or tumor cell, a cutaneous condition, an endocrine disease, an eye disease or disorder, an intestinal disease, a communication disorder, a genetic disorder, a neurological disorder, a voice disorder, a vulvovaginal disorder, a liver disorder, a heart disorder, a heating disorder, a mood disorder, anemia, preferentially iron anemia, and a personality disorder.

In some cases, the disease or disorder can be the disease or disorder of or belonging to the individual or body part, or the disease or disorder from which the individual is suffering.

In one embodiment of the invention, the cancer or tumor selected from the group consisting of: the cancer of an organ, cancer of blood, cancer of a system of a living organism, adrenal cancer, anal cancer, bile duct cancer, bladder cancer, bone cancer, brain cancer, breast cancer, cervical cancer, colon/rectum cancer, endometrial cancer, esophagus cancer, eye cancer, gallbladder cancer, heart cancer, kidney cancer, laryngeal and hypopharyngeal cancer, leukemia, liver cancer, lung cancer, nasal cavity and paranasal sinus cancer, nasopharyngeal cancer, neuroblastoma, non-Hodgkin lymphoma, oral cavity and oropharyngeal cancer, osteosarcoma cancer, ovarian cancer, pancreatic cancer, pancreatic penile cancer, prostate cancer, retinoblastoma, rhabdomyosarcoma, salivary gland cancer, sarcoma, skin cancer, small intestine cancer, stomach cancer, testicular cancer, thymus cancer, thyroid cancer, uterine cancer, uterine sarcoma cancer, vaginal cancer, vulvar cancer, waldenstrom macroglobulinemia wilms tumor, castleman disease ewing family of tumor, gastrointestinal carcinoid tumor, gastrointestinal stromal tumor, myelodysplastic syndrome pituitary tumor, and a cancerous disease such as gestational trophoblastic disease, Hodgkin disease, Kaposi sarcoma, malignant mesothelioma, and multiple myeloma.

The invention also relates to a method for the treatment of anemia or anemia disease or to nanoparticle or at least one constituent of the compositions, in particular magnetosomes, for use in the treatment of anemia disease, preferentially iron anemia disease, wherein magnetosomes are administered to the body part of an individual, preferentially to reduce or stop anemia.

The invention also relates to a method for the treatment of an anemia disease, wherein this disease is selected from the group consisting of: Iron or metal deficiency anemia, Vitamin deficiency anemia, Anemia of chronic disease, Aplastic anemia, Anemia associated with bone marrow disease, Hemolytic anemia, Sickle cell anemia, Thalassaemia, Pernicious anaemia, Fanconi anaemia, Sideroblastic Anemia, Congenital Dyserythropoietic Anemia (CDA), Diamond Blackfan Anemia, and Megaloblastic Anemia. In some cases, anemia is a decrease in the total amount of red blood cells (RBCs) or hemoglobin in the blood, or a lowered ability of the blood to carry oxygen.

The invention also relates to a method of fabrication of the composition according to the invention, which comprises at least one of the following steps:

- **Step 1** of amplifying magnetotactic bacteria preferentially in a in at least one medium, preferentially selected among a pre-growth, growth and/or fed-batch medium(a), preferentially comprising:

  1) the compounds necessary for the growth of magnetotactic bacteria and/or the production of magnetosomes, which are preferentially selected in the group consisting of:

  - a source of carbon, which is preferentially selected from the group consisting of: at least one compound comprising at least one atom of carbon, lactic acid, Na lactate, acetate, glycolate, glucose, pyruvate, succinate, carbon dioxide, glycerol and combinations thereof, at a concentration preferentially comprised between 1 nM and 2 Mol/L;
  - a source of iron preferentially selected from the group consisting of: at least one compound comprising at least one atom of iron, iron citrate, iron quinate, iron chloride, iron sulfate, $FeCl_3$, and combinations thereof, at a concentration preferentially comprised between 1 nM and $2.10^{-3}$ Mol/L;
  - a source of nitrogen preferentially selected from the group consisting of: at least one compound comprising at least one atom of nitrogen, nitrate salt, nitrogen gas, ammonium, ammonia, ammonium salt, urea, an amino acid, ammonia gas, and combinations thereof, at a concentration preferentially comprised between 1 nM and 4 Mol/L;
  - a source of oxygen preferentially selected from the group consisting of: at least one compound comprising at least one atom of oxygen, oxygen or air or compressed air, preferentially in the form of a gas, the source of oxygen being in some cases bubbled or introduced to the growth medium, at a gas rate that is preferentially comprised between 5 mL of gas per minute and 50000 mL of gas per minute;
  - a source of phosphate preferentially comprising or consisting of at least one compound comprising at least one atom of phosphate, at a concentration preferentially comprised between 1 nM and $2.10^{-1}$ Mol/L;
- a source of potassium preferentially comprising or consisting of at least one compound comprising at least one atom of potassium, at a concentration preferentially comprised between 1 nM and $2.10^{-1}$ Mol/L;
- a source of sulfur or sulfate preferentially comprising or consisting of at least one compound comprising at least one atom of sulfur or sulfate, at a concentration preferentially comprised between 1 nM and $4.10^{-1}$ Mol/L;
- a source of manganese preferentially comprising or consisting of at least one compound comprising at least one atom of manganese, at a concentration preferentially comprised between 1 nM and $4.10^{-1}$ Mol/L;
- a source of vitamin preferentially selected from the group consisting of: at least one compound comprising at least one vitamin, Biotin, Calcium, pantothenate, Folic acid, Inositol, Nicotinic acid, p-Aminobenzoic acid, Pyridoxine HCl, Riboflavin, Thiamine, Thiamine HCL and derivatives thereof and combinations thereof, at a concentration preferentially comprised between 1 nM and $10^{-4}$ Mol/L, and
- a source of calcium preferentially comprising or consisting of at least one compound comprising at least one atom of calcium, at a concentration preferentially comprised between 1 nM and $10^{-1}$ Mol/L.

2) at least one compound necessary for doping the magnetosomes with $C_{A1}$ or $C_{1FRPC}$ or another metal than iron, preferentially zinc or aluminum, for example a source of zinc, preferentially zinc sulfate or zinc citrate or zinc chlorate or zinc quinate.

- **Step 2** of extracting magnetosomes from magnetotactic bacteria and/or of purifying the extracted magnetosomes preferentially by heating them to yield magnetosome minerals preferentially comprising a percentage in mass of organic material coming from magnetotactic bacteria that is lower than 100, 50 or 1%,
- **Step 3** of coating the magnetosome minerals preferentially with a coating material preferentially comprising the compound $C_{A2}$ or $C_{2FRPC}$ by mixing the magnetosome minerals with the coating material, where the mixing is preferentially realized in at least one of the following conditions:

    i) under sonication,
    ii) under the application of radiation,
    iii) under temperature variation,
    iv) under pH changes,

    v) under oxidoreduction potential adjustment,

preferentially using a ratio between the quantity or mass of magnetosome minerals and the quantity or mas of coating material, preferentially of compound D, that is preferentially adjusted or varied or larger than 1,
- **Step 4** of adding at least one cryoprotectant to the coated magnetosome minerals preferentially obtained at the end of step 3,
- **Step 5** of lyophilizing or dehydrating or drying or desiccating the composition obtained at the end of step 4,
- **Step 6** of re-suspending the lyophilized composition preferentially obtained from step 5, preferentially in water or in another liquid.

The invention also relates to the method according to the invention, wherein the concentration of the source of zinc, preferentially zinc citrate or zinc sulfate, is comprised between 1 and 100 $\mu$M, preferentially 2 and 50 $\mu$M, most preferentially 5 and 20 $\mu$M.

The invention also relates to the method according to the invention, wherein the nanoparticle or at least one constituent of the compositions fabricated according to the method comprises a quantity of metal other than iron, preferentially zinc or aluminum, which is incorporated in or comprised in the metallic core that is: i) comprised between 0.1 and 100 mg of metal other than iron comprised in the core per gram of iron comprised in the core, ii) preferentially comprised between 0.5 and 10 mg of metal other than iron comprised in the core per gram of iron comprised in the core, iii) most preferentially comprised between 1 and 5 mg of metal other than iron comprised in the core per gram of iron comprised in the core.

In one embodiment of this invention, the nanoparticle or at least one constituent of the composition is or belongs to or is comprised in the group of nanoparticle or at least one constituent of the compositions selected from: a nanosphere, a nano-capsule, a dendrimer, a carbon nanotube, a lipid/solid nanoparticle or at least one constituent of the composition, a lipid or protein or DNA or RNA based nanoparticle or at least one constituent of the composition, a nanoparticle or at least one constituent of the composition with an inner aqueous environment surrounded by a layer, preferentially a stabilizing layer, most preferentially a phospholipid layer, a multilayer nanoparticle or at least one constituent of the composition, a polymeric nanoparticle or at least one constituent of the composition, a quantum dot, a metallic nanoparticle or at least one constituent of the composition, a polymeric micelle or nanoparticle or at least one constituent of the composition, a carbon based nano-structure, a nanobubble, a nanosome, a pharmacyte, a niosome, a nanopore, a microbivore, a liposome, a virus, preferentially recombinant, a herbal nanoparticle or at least one constituent of the composition, an antibody, and a vesicle.

In another embodiment of this invention, the nanoparticle or at least one constituent of the composition is not or does not belong to or is not comprised in at least one nanoparticle or at least one constituent of the composition belonging to the group of: a nanosphere, a nanocapsule, a dendrimer, a carbon nanotube, a lipid/solid nanoparticle or at least one constituent of the composition, a lipid or protein or DNA or RNA based nanoparticle or at least one constituent of the composition, a nanoparticle or at least one constituent of the composition with an inner aqueous environment surrounded by a layer, preferentially a stabilizing layer, most preferentially a phospholipid layer, a multilayer nanoparticle or at least one constituent of the composition, a polymeric nanoparticle or at least one constituent of the composition, a quantum dot, a metallic nanoparticle or at least one constituent of the composition, a polymeric micelle or nanoparticle or at least one constituent of the composition, a carbon based nano-structure, a nanobubble, a nanosome, a pharmacyte, a niosome, a nanopore, a microbivore, a liposome, a virus, preferentially recombinant, a herbal nanoparticle or at least one constituent of the composition, an antibody, and a vesicle.

In some cases, the nanoparticle or at least one constituent of the composition can be in a liquid, gaseous, or solid state, preferentially before, during or after its presence or administration in the body part.

In some other cases, the nanoparticle or at least one constituent of the composition can't be in one or two of the liquid, gaseous, or solid states, preferentially before, during or after its presence or administration in the body part.

In still some other cases, the nanoparticle or at least one constituent of the compositions can be assimilated to or be comprised in a ferrofluid, a chemical or biological ferrofluid, wherein chemical and biological ferrofluids are fluids containing iron, preferentially forming nanoparticle or at least one constituent of the compositions, which are fabricated through a chemical or biological synthesis, respectively.

In still some other cases, the ferrofluid or nanoparticle or at least one constituent of the composition assembly can comprise the nanoparticle or at least one constituent of the compositions and an excipient, a solvent, a matrix, a gel, which preferentially enables the administration of the nanoparticle or at least one constituent of the compositions to the individual or body part.

In still some other cases, the nanoparticle or at least one constituent of the composition can comprise synthetic material and/or biological material and/or inorganic material and/or organic material.

In one embodiment of the invention, (the) nanoparticle or at least one constituent of the composition(s) is/are or designate: i) a suspension of nanoparticle or at least one constituent of the compositions, ii) a composition comprising nanoparticle or at least one constituent of the compositions, iii) an assembly of nanoparticle or at least one constituent of the compositions, iv) a nanoparticle or at least one constituent of the composition region, v) the mineral part or core of the nanoparticle or at least one constituent of the composition, vi) the organic part of the nanoparticle or at least one constituent of the composition, vii) the inorganic part of the nanoparticle or at least one constituent of the composition, viii) or the coating of the nanoparticle or at least one constituent of the composition.

In one embodiment of the invention, nanoparticle or at least one constituent of the composition(s) or the nanoparticle or at least one constituent of the composition(s) represent(s) or is or are an assembly or suspension or composition of more or comprising more than $10^{-100}$, $10^{-50}$, $10^{-10}$, $10^{-5}$, 10 ', 1, 10, $10^2$, $10^3$, $10^5$, $10^{10}$, $10^{20}$ or $10^{50}$ nanoparticle or at least one constituent of the composition(s) or mg of nanoparticle or at least one constituent of the composition(s) or mg of iron comprised in nanoparticle or at least one constituent of the composition(s) or mg of nanoparticle or at least one constituent of the composition(s) per $cm^3$ or mg of nanoparticle or at least one constituent of the composition(s) per $cm^3$ of body part or mg of iron comprised in nanoparticle or at least one constituent of the composition(s) per $cm^3$ or mg of iron comprised in nanoparticle or at least one constituent of the composition(s) per $cm^3$ of body part. In some cases, an assembly or suspension or composition comprising a large number of nanoparticles or at least one constituent of the compositions can be used to induce or produce a temperature increase, radical or reactive species, or the dissociation of a compound from the nanoparticle or at least one constituent of the compositions.

In another embodiment of the invention, nanoparticles or at least one constituent of the composition(s) or the nanoparticles or at least one constituent of the composition(s) represent(s) or is or are an assembly or suspension or composition of less or comprising less than $10^{100}$, $10^{50}$, $10^{20}$, $10^{10}$, $10^5$, $10^2$, 10, 1, 5, 2, 1, $10^{-1}$, $10^{-5}$, $10^{-10}$ or $10^{-50}$ nanoparticle or at least one constituent of the composition(s) or mg of nanoparticle or at least one constituent of the composition(s) or mg of iron comprised in nanoparticle or at least one constituent of the composition(s) or mg of nanoparticle or at least one constituent of the composition(s) per $cm^3$ or mg of nanoparticle or at least one constituent of the composition(s) per $cm^3$ of body part or mg of iron comprised in nanoparticle or at least one constituent of the composition(s) per $cm^3$ or mg of iron comprised in nanoparticle or at least one constituent of the composition(s) per $cm^3$ of body part. In some cases, an assembly or suspension or composition of nanoparticle or at least one constituent of the compositions comprising a low number of nanoparticle or at least one constituent of the composition(s) can be used to prevent toxicity.

In one embodiment of the invention, the nanoparticle or at least one constituent of the composition(s) or assembly of nanoparticle or at least one constituent of the compositions can represent or be the region, also designated as nanoparticle region or region of at least one constituent of the composition, volume, surface, length, which comprises the nanoparticle or at least one constituent of the compositions or where nanoparticle or at least one constituent of the compositions are located.

In some cases, the volume of the region occupied by the nanoparticle or at least one constituent of the compositions in the body part is designated as nanoparticle region or region of at least one constituent of the composition.

In some cases, the nanoparticle or at least one constituent of the composition region can be the volume occupied by an assembly of nanoparticles or of at least one constituent of the compositions in the body part, where the nanoparticle or the at least one constituent of the compositions are preferentially separated by less than $10^9$, $10^6$, $10^3$ or 10 nm.

In some cases, the nanoparticle assembly or assembly of at least one constituent of the composition is a more general term than nanoparticle or at least one constituent of the composition region, which could designate any type of nanoparticle or at least one constituent of the composition assembly, before, during, or after nanoparticle administration or administration of at least one constituent of the composition to or in the body part.

In some cases, the separating distance between the nanoparticle or at least one constituent of the compositions within the nanoparticle assembly or assembly of at least one constituent of the composition or nanoparticle region or region of at least one constituent of the composition can correspond to the average or maximum distance separating the nanoparticle(s) or at least one constituent of the compositions within this assembly.

In some cases, the distribution in separating distances between nanoparticle(s) or at least one constituent of the compositions can highlight the presence of a minority of nanoparticle(s) or at least one constituent of the compositions, *i.e.* preferentially less than 50, 10, 1, $10^{-2}$ or $10^{-5}$ % of the total number of nanoparticle(s) or at least one constituent of the compositions in the individual, with either small separating distances, *i.e* separating distances preferentially lower than $10^9$, $10^6$, $10^3$ or 10 nm, or with large separating distances, *i.e.* separating distances preferentially larger than $10^9$, $10^6$, $10^3$ or 10 nm. In this case, the presence of this minority of nanoparticle(s) or at least one constituent of the composition is preferentially not taken into consideration to estimate the average or maximum separating distance between the nanoparticle or at least one constituent of the compositions.

The invention also relates to nanoparticle or at least one constituent of the compositions for use according to the invention, wherein the nanoparticle(s) or at least one constituent of the compositions is/are crystallized, metallic, or magnetic.

In an embodiment of the invention, the nanoparticle or at least one constituent of the compositions are crystallized. In this case, they preferentially possess more than or at least 1, 2, 10, $10^2$, $10^3$, $10^6$ or $10^9$ crystallographic plane(s) or regular atomic arrangement(s), preferentially observable by electron microscopy.

In one embodiment of the invention, the nanoparticle or at least one constituent of the compositions are metallic. In this case, they comprise at least 1, 10, $10^3$, $10^5$ or $10^9$ metallic atom(s) or contain at least 1, 10, 50, 75 or 90% of metallic atoms, where this percentage can be the ratio between the number or mass of metallic atoms in the nanoparticle or at least one constituent of the composition divided by the total number or mass of all atoms in the nanoparticle or at least one constituent of the composition. The nanoparticle or at least one constituent of the compositions, preferentially metal oxide nanoparticle or at least one constituent of the compositions, can also contain at least 1, 10, $10^3$, $10^5$ or $10^9$ oxygen atom(s), or contain at least 1, 10, 50, 75 or 90% of oxygen atoms, where this percentage can be the ratio between the number or mass of oxygen atoms in the nanoparticle or at least one constituent of the compositions divided by the total number or mass of all atoms in the nanoparticle or at least one constituent of the compositions.

In another embodiment of the invention, the metal or metal atom is selected in the list consisting of: Lithium, Beryllium, Sodium, Magnesium, Aluminum, Potassium, Calcium, Scandium, Titanium, Vanadium, Chromium, Manganese, Iron, Cobalt, Nickel, Copper, Zinc, Gallium, Rubidium, Strontium, Yttrium, Zirconium, Niobium, Molybdenum, Technetium, Ruthenium, Rhodium, Palladium, Silver, Cadmium, Indium, Tin, Cesium, Barium, Lanthanum, Cerium, Praseodymium, Neodymium, Promethium, Samarium, Europium, Gadolinium, Terbium, Dysprosium, Holmium, Erbium, Thulium, Ytterbium, Lutetium, Hafnium, Tantalum, Tungsten, Rhenium, Osmium, Iridium, Platinum, Gold, Mercury, Thallium, Lead, Bismuth, Polonium, Francium, Radium, Actinium, Thorium, Protactinium, Uranium, Neptunium, Plutonium, Americium, Curium, Berkelium, Californium, Einsteinium, Fermium, Mendelevium, Nobelium, Lawrencium, Rutherfordium, Dubnium, Seaborgium, Bohrium, Hassium, Meitnerium, Darmstadtium, Roentgenium, Copernicium, Nihonium, Flerovium, Moscovium, and Livermorium or Livermorium atom.

In another embodiment of the invention, the nanoparticle or at least one constituent of the composition contains less than 1, 10, $10^3$, $10^5$ or $10^9$ metallic atom(s) or contains less than 1, 10, 50, 75 or 90% of metallic atoms, where this percentage can be the ratio between the number or mass of metallic atoms in the nanoparticle or at least one constituent of the composition divided by the total number or mass of all atoms in the nanoparticle or at least one constituent of the composition. It can also contain less than 1, 10, $10^3$, $10^5$ or $10^9$ oxygen atom(s), or contain less than 1, 10, 50, 75 or 90% of oxygen atoms, where this percentage can be the ratio between the number or mass of oxygen atoms in the nanoparticle or at least one constituent of the composition divided by the total number or mass of all atoms in the nanoparticle or at least one constituent of the composition.

In one embodiment of the invention, the nanoparticle or at least one constituent of the composition is magnetic when it has a magnetic behavior or property, where the magnetic behavior or property is preferentially selected from the group consisting of a diamagnetic, superparamagnetic, paramagnetic, ferromagnetic, and ferrimagnetic behavior or property.

In some cases, the behavior or property (magnetic or not) of the at least one nanoparticle or at least one constituent of the composition can be observed or exists at a temperature, which is lower than: i) $10^5$, $10^3$, 500, 350, 200, 100, 50, 20, 10, 1, 0.5 or 1 K (Kelvin), ii) the Curie temperature, or iii) the blocking temperature.

In some other cases, the behavior or property (magnetic or not) of the at least one nanoparticle or at least one constituent of the composition can be observed or exists at a temperature, which is larger than: i) 0.5, 1, 10, 20, 50, 100, 200, 350, 500, $10^3$ or $10^5$ K, ii) the Curie temperature, or iii) the blocking temperature.

In still some other cases, the magnetic behavior or property can be observed or exists at a temperature, which is between $10^{-20}$ and $10^{20}$ K, or between 0.1 and 1000 K.

In one embodiment of the invention, the nanoparticle or at least one constituent of the compositions have or are characterized by at least one property selected in the group consisting of: i) the presence of a core, preferentially magnetic, preferentially mineral, preferentially composed of a metal or at least one metallic atom or metal oxide such as iron oxide, most preferentially maghemite or magnetite, or an intermediate composition between maghemite and magnetite, ii) the presence of a coating that preferentially surrounds the core preferentially partly or fully and preferentially prevents nanoparticle or at least one constituent of the composition aggregation, preferentially enabling nanoparticle or at least one constituent of the composition administration in an organism or in the body part or stabilizing the nanoparticle or at least one constituent of the composition core, where coating thickness may preferably lie between 0.1 nm and 10 $\mu$m, between 0.1 nm and 1 $\mu$m, between 0.1 nm and 100 nm, between 0.1 nm and 10 nm, or between 1 nm and 5 nm, iii) magnetic properties leading to or being a diamagnetic, paramagnetic, superparamagnetic, ferromagnetic, or ferrimagnetic behavior, iv) a coercivity larger than 0.01, 0.1, 1, 10, 100, $10^3$, $10^4$, $10^5$, $10^9$ or $10^{20}$ Oe, v) a ratio between remanent and saturating magnetization larger than 0.01, 0.1, 0.2, 0.3, 0.4, 0.5, 0.75, 0.9 or 0.99, vi) a saturating magnetization larger than 0.1, 1, 5, 10 or 50 emu/g, vii) magnetic properties such as coercivity, remanent and saturating magnetization, preferentially measured or observed at a temperature larger than 0.1 K, 1 K, 10 K, 20 K, 50 K, 100 K, 200 K, 300 K, 350 K or 3000 K, viii) a crystallinity, *i.e.* nanoparticle or at least one constituent of the compositions preferentially possessing at least 1, 2, 5, 10 or 100 crystalline plane(s), preferentially observable or measured by electron microscopy, ix) the presence of a single domain, x) a size that is larger than 0.1, 0.5, 1.5, 10, 15, 20, 25, 30, 50, 60, 70, 80, 100, 120, 150 or 200 nm, xi) a size lying between 0.1 nm and 10 $\mu$m, between 0.1 nm and 1 $\mu$m, between 0.1 nm and 100 nm, between 1 nm and 100 nm, or between 5 nm and 80 nm, xii) a non-pyrogenicity or apyrogenicity, which preferentially means that nanoparticle or at least one constituent of the compositions possess an endotoxin concentration lower than $10^{20}$, 10000, 1000, 100, 50, 10, 5, 2 or 1 EU (endotoxin unit) per mg of nanoparticle or at least one constituent of the composition or per mg of iron comprised in nanoparticle or at least one constituent of the compositions, or which means that nanoparticle or at least one constituent of the compositions do not trigger fever or an increase in whole body temperature larger than 100, 50, 6.6, 5, 3, 2 or 1 °C following their administration to a living organism or body part, xiii) a synthesis by a synthetizing living organism, preferentially by bacteria, xiv) a chemical synthesis, xv) the presence of less than 50, 25, 15, 10, 5, 2 or 1% of organic or carbon material originating from the synthetizing living organism, xv), the presence of more than 99, 95, 80, 70, 60, 50 or 25% of mineral material originating from the synthetizing living organism, and xvi) a specific absorption rate (SAR) that is larger than 1, 10, 1000 or $10^4$ Watt per gram of nanoparticle or at least one constituent of the composition, preferentially measured under the application of an alternating magnetic field of strength preferentially larger than 0.1, 1, 10 or 100 mT, and/or frequency larger than 1, 10, 100 or 1000 KHz, alternatively preferentially measured under the application of the acoustic wave, alternatively under the application of a radiation such as an electromagnetic acoustic, or light radiation.

In another embodiment of the invention, the nanoparticle or at least one constituent of the compositions have or are characterized by at least one property selected in the group consisting of: i) a coercivity lower than 0.01, 0.1, 1, 10, 100, $10^3$, $10^4$, $10^5$, $10^9$ or $10^{20}$ Oe, ii) a ratio between remanent and saturating magnetization lower than 0.01, 0.1, 0.2, 0.3, 0.4, 0.5, 0.75, 0.9 or 0.99, iii) a saturating magnetization lower than 0.1, 1, 5, 10, 50, 200, 1000 or 5000 emu/g, iv) magnetic properties preferentially measured or observed at a temperature lower than 0.1 K, 1 K, 10 K, 20 K, 50 K, 100 K, 200 K, 300 K, 350 K or 3000 K, v) a size that is lower than 0.1, 0.5, 1.5, 10, 15, 20, 25, 30, 50, 60, 70, 80, 100, 120, 150 or 200 nm, vi) the presence of more than 50, 25, 15, 10, 5, 2 or 1% of organic or carbon material originating from the synthetizing living organism, vii) the presence of less than 99, 95, 80, 70, 60, 50 or 25% of mineral material originating from the synthetizing living organism, and xi), a specific absorption rate (SAR) that is lower than 1, 10, 1000 or $10^4$ Watt per gram of nanoparticle or at least one constituent of the composition, preferentially measured under the application of an alternating magnetic field of strength preferentially lower than 0.1, 1, 10, or 100, 200, 500, $10^3$ or $10^5$ mT, and/or of frequency preferentially lower than 1, 10, 100, $10^3$, $10^5$ or $10^9$ KHz, alternatively preferentially measured under the application of the acoustic wave, alternatively under the application of a radiation such as an electromagnetic acoustic, or light radiation.

In some cases, the mineral can be the part of the nanoparticle or at least one constituent of the composition or magnetosome that does not comprise organic material or comprises a low percentage in mass of organic material, preferentially less than 100, 99, 50, 20, 10, 5, 1, $10^{-1}$ or $10^{-2}$ percent or percent in mass of organic material. The mineral is preferentially the core of the nanoparticle or at least one constituent of the composition.

In some other cases, the mineral can comprise a percentage in mass of organic material larger than 0, $10^{-50}$, $10^{-10}$, $10^{-2}$, $10^{-1}$ or 1 percent or percent in in mass of organic material. This can be the case when the purification step unsuccessfully removes the organic material or when organic material is added to the mineral after the purification step.

In some cases, the nanoparticle or at least one constituent of the composition can be surrounded by a coating or at least one constituent of the composition. The coating or at least one constituent of the composition can be made of a synthetic, organic, or inorganic material or of a substance comprising a function selected in the group consisting of carboxylic acids, phosphoric acids, sulfonic acids, esters, amides, ketones, alcohols, phenols, thiols, amines, ether, sulfides, acid anhydrides, acyl halides, amidines, amides, nitriles, hydroperoxides, imines, aldehydes, and peroxides. In some cases, the coating or at least one constituent of the composition can be made of carboxy-methyl-dextran, citric acid, phosphatidylcholine (DOPC), or oleic acid. In some cases, the coating or at least one constituent of the composition can enable the dispersion of the nanoparticle or at least one constituent of the compositions in a matrix or solvent such as water, preferentially without aggregation or sedimentation of the nanoparticle or at least one constituent of the compositions. In some cases, the coating or at least one constituent of the composition can enable internalization of the nanoparticle or at least one constituent of the compositions in cells. In some other cases, the coating can enable: i) to bind two or more nanoparticle or at least two constituents of the composition together preferentially in a chain, ii) to prevent nanoparticle or at least one constituent of the composition aggregation and/or, iii) to obtain uniform nanoparticle or at least one constituent of the composition distribution.

In one embodiment, at least one binding material binds at least two nanoparticle or at least two constituents of the compositions preferentially in chain or geometric figure or assembly.

In some cases, the binding materials can be the same material as the coating and/or have at least one property in common with the coating or at least one constituent of the composition.

In some cases, the binding material can be a different material from the coating, and/or comprise water or a gel or tissue or cellular or cellular component such as cytoplasm and/or have at least one property different from the coating or at least one constituent of the composition.

In one embodiment of the invention, the nanoparticle or at least one constituent of the compositions are non-pyrogenic. Preferentially, non-pyrogenic nanoparticle or at least one constituent of the compositions i) comprise less than $10^{100}$, $10^{50}$, $10^{20}$, $10^8$, $10^5$, $10^3$, or 10 EU (endotoxin unit) or EU per $cm^3$ of body part or EU per mg of nanoparticle or at least one constituent of the composition or EU per $cm^3$ of body part per mg of nanoparticle or at least one constituent of the composition, or ii) induce a temperature increase of the individual or body part of less than $10^5$, $10^3$, $10^2$, 50, 10, 5, 4, 3, 2 or 1°C, preferentially above physiological temperature, preferentially before, after or without the application of the acoustic wave or radiation on the nanoparticle or at least one constituent of the composition.

In one embodiment of this invention, the nanoparticle or at least one constituent of the composition is composed of or comprises a chemical element of the families selected from the group consisting of: metals (alkali metal, alkaline earth metal, transition metals), semimetal, non-metal (halogens element, noble gas), chalcogen elements, lanthanide, and actinide.

In another embodiment of the invention, the nanoparticle or at least one constituent of the composition is composed of or comprises a chemical element selected from the group consisting of: hydrogen, lithium, sodium, potassium, rubidium, caesium, francium, beryllium, magnesium, calcium, strontium, barium, radium, scandium, yttrium, lanthanide, actinide, titanium, zirconium, hafnium, rutherfordium, vanadium, niobium, tantalum, dubnium, chromium, molybdenum, tungsten, seaborgium, manganese, technetium, rhenium, bohrium, iron, ruthenium, osmium, hessium, cobalt, rhodium, iridium, meitherium, nickel, palladium, platinum, darmstadtium, copper, silver, gold, roentgenium, zinc, cadmium, mercury, copernicum, boron, aluminium, gallium, indium, thallium, ununtrium, carbon, silicon, germanium, tin, lead, fleovium, nitrogen, phosphorus, arsenic, antimony, bismuth, ununpentium, oxygen, sulphur, selenium, tellurium, polonium, livermorium, fluorine, chlorine, bromine, iodine, astatine, ununseptium, helium, neon, argon, krypton, xenon, radon, ununoctium, lanthanum, cerium, praseodymium, neodymium, promethium, samarium, europium, gadolinium, terbium, dysprosium, holmium, erbium, thulium, ytterbium, lutetium, actinium, thorium, proctactinium, uranium, neptunium, plutonium, americium, curium, berkelium, californium, einsteinium, fermium, mendelevium, nobelium, and lawrencium.

In some cases, the nanoparticle or at least one constituent of the composition can also be composed of or comprise an alloy, a mixture, or an oxide of this(these) chemical element(s).

In some cases, the nanoparticle or at least one constituent of the composition can be composed of more than $10^{-50}$, $10^{-20}$, $10^{-10}$, $10^{-5}$, $10^{-2}$, 1, 5, 10, 50, 75, 80, 90, 95 or 99% of one or several of this(these) element(s), where this percentage can represent the mass or number of this(these) chemical elements comprised in the nanoparticle or at least one constituent of the composition divided by the total number or total mass of all chemical elements comprised in the nanoparticle or at least one constituent of the composition or by the total mass of the nanoparticle or at least one constituent of the composition.

In some other cases, the nanoparticle or at least one constituent of the can be composed of or comprise less than $10^{50}$, $10^{-20}$, $10^{-10}$, $10^{-5}$, $10^{-2}$, 1, 5, 10, 50, 75, 80, 90, 95 or 9% of one or several of this(these) chemical element(s).

In still some other cases, this(these) chemical element(s) is(are) comprised inside the nanoparticle or at least one constituent of the composition , or at the surface of the nanoparticle or at least one constituent of the composition, or in the mineral or core of the nanoparticle or at least one constituent of the composition, or in the coating of the nanoparticle or at least one constituent of the composition.

In one embodiment of this invention, the nanoparticle or at least one constituent of the composition or is not composed of or does not comprise at least one chemical element belonging to the family selected from the group consisting of: metals (alkali metal, alkaline earth metal, transition metals), semimetal, non- metal (halogens element, noble gas), chalcogen elements, lanthanide, actinide.

In another embodiment of the invention, the nanoparticle or at least one constituent of the composition is devoid of or does not comprise at least one chemical element selected from the group consisting of: hydrogen, lithium, sodium, potassium, rubidium, caesium, francium, beryllium, magnesium, calcium, strontium, barium, radium, scandium, yttrium, lanthanide, actinide, titanium, zirconium, hafnium, rutherfordium, vanadium, niobium, tantalum, dubnium, chromium, molybdenum, tungsten, seaborgium, manganese, technetium, rhenium, bohrium, iron, ruthenium, osmium, hessium, cobalt, rhodium, iridium, meitherium, nickel, palladium, platinum, darmstadtium, copper, silver, gold, roentgenium, zinc, cadmium, mercury, copernicum, boron, aluminium, gallium, indium, thallium, ununtrium, carbon, silicon, germanium, tin, lead, fleovium, nitrogen, phosphorus, arsenic, antimony, bismuth, ununpentium, oxygen, sulphur, selenium, tellurium, polonium, livermorium, fluorine, chlorine, bromine, iodine, astatine, ununseptium, helium, neon, argon, krypton, xenon, radon, ununoctium, lanthanum, cerium, praseodymium, neodymium, promethium, samarium, europium, gadolinium, terbium, dysprosium, holmium, erbium, thulium, ytterbium, lutetium, actinium, thorium, proctactinium, uranium, neptunium, plutonium, americium, curium, berkelium, californium, einsteinium, fermium, mendelevium, nobelium, and lawrencium.

In another embodiment of the invention, the nanoparticle or at least one constituent of the composition is not composed of or does not comprise an alloy, a mixture, or an oxide of this(these) chemical element(s).

In one embodiment of the invention, the nanoparticle or at least one constituent of the composition is defined as a particle with a size in one dimension, which is larger than 10 ', 1, 2, 5, 10, 20, 50, 70, 100, 200 or 500 nm. A nanoparticle or at least one constituent of the composition with a large size can have a larger coercivity and/or a larger remanent magnetization and/or can preferentially more strongly or more efficiently absorb the energy or power of the acoustic wave than a nanoparticle or at least one constituent of the composition with a small size. In some cases, the amount of energy or power absorbed by the nanoparticle or at least one constituent of the composition is increased by a factor of more than 1.001, 1.01, 1.1, 1.2, 1.5, 2, 5, 10, $10^3$, $10^5$ or $10^7$ by increasing the size of the nanoparticle or at least one constituent of the composition by a factor of more than 1.001, 1.01, 1.1, 1.2, 1.5, 2, 5, 10, $10^3$, $10^5$ or $10^7$.

In another embodiment of the invention, the nanoparticle or at least one constituent of the composition is defined as a particle with a size in one dimension, which is lower than $10^4$, $10^3$, $10^2$, 10, 1 or $10^{-1}$ nm. In some cases, a nanoparticle or at least one constituent of the composition with a small size can more easily be administered, for example intravenously, or can enable the avoidance of some toxicity effects, such as embolism.

In still another embodiment of the invention, the size of the nanoparticle or at least one constituent of the composition lies between $10^{-2}$ and $10^{20}$ nm, $10^{-2}$ and $10^4$ nm, between $10^{-1}$ and $10^3$ nm, or between 1 and $10^2$ nm. This can be the case when the nanoparticle or at least one constituent of the composition or nanoparticle assembly or assembly of at least one constituent of the composition possesses a well-defined, preferentially narrow, distribution in sizes.

In still another embodiment of the invention, the size distribution of the nanoparticle or at least one constituent of the composition is lower than $10^{-10}$, $10^5$, 1000, 100, 75, 50, 25, 10, 5, 2 or 1 nm. A narrow size distribution of nanoparticles or of at least one constituent of the composition may be desired to prevent aggregation, to favor elimination, or to favor an organization preferentially in chains of the nanoparticles or of at least one constituent of the composition.

In still another embodiment of the invention, the distribution in size of the nanoparticle or of at least one constituent of the composition is larger than 1000, 100, 75, 50, 25, 10, 5, 2 or 1 nm. A large size distribution of the nanoparticle or of at least one constituent of the composition may in some cases enable nanoparticle or at least one constituent of the composition to be eliminated more rapidly.

In another embodiment of the invention, the surface charge of the nanoparticle or at least one constituent of the composition, is larger than -200, -100, -50, -10, -5, 0.1, 1, 2, 5, 10, 50 or 100 mV, preferentially at a pH lower than 0.1, 1, 2, 3, 4, 5, 6, 7, 8, 9, 10, 11, 12, 13 or 14. Preferentially, a nanoparticle or at least one constituent of the composition can have a large surface charge preferentially at low pH preferentially when it is surrounded by a coating or material that enables to reach such charge without being destroyed.

In another embodiment of the invention, the nanoparticle or at least one constituent of the composition has a surface charge, which is lower than -200, -100, -50, -10, -5, 0.1, 1, 2, 5, 10, 50 or 100 mV, preferentially at a pH larger than 0.1, 1, 2, 3, 4, 5, 6, 7, 8, 9, 10, 11, 12, 13 or 14. A nanoparticle or at least one constituent of the composition can have a low surface charge preferentially at high pH preferentially when it is surrounded by a coating or material that enables to reach such charge without being destroyed.

In still another embodiment of the invention, the nanoparticle or at least one constituent of the composition has a surface charge comprised between +200 and -200 mV, +100 and -100 mV, +50 and -50 mV, +40 et-40mV, +20 and -20, +10 and -10 mV, or between +5 and -5 mV, preferentially at a pH lower than 0.1, 1, 2, 3, 4, 5, 6, 7, 8, 9, 10, 11, 12, 13 or 14.

In still another embodiment of the invention, the nanoparticle or at least one constituent of the composition has a surface charge comprised between +200 and -200 mV, +100 and -100 mV, +50 and -50 mV, +40 et-40mV, +20 and -20, +10 and -10 mV, or between +5 and -5 mV, preferentially at a pH larger than 0.1, 1, 2, 3, 4, 5, 6, 7, 8, 9, 10, 11, 12, 13 or 14.

In another embodiment of the invention, the nanoparticle or at least one constituent of the composition has a weight or a mass, preferentially expressed in unit such as gram (g), kilogram (kg), or milligram (mg). A gram of nanoparticle or at least one constituent of the composition can be a gram of metal such as iron comprised in the nanoparticle or at least one constituent of the composition. The mass or weight of the nanoparticle or at least one constituent of the composition can correspond to the mass or weight of one nanoparticle or at least one constituent of the composition or to the mass or weight of an assembly of nanoparticles or of at least one constituent of the composition.

In an embodiment, the mass of the nanoparticles or of at least one constituent of the composition is larger than $10^{-20}$, $10^{-10}$, $10^{-5}$, $10^{-2}$, 1, 10, $10^3$, $10^9$ or $10^{20}$ gram. In some cases, a large mass or quantity of the nanoparticle or at least one constituent of the composition may be desired to increase the quantity of acoustic wave or radiation energy absorbed by the nanoparticle or at least one constituent of the composition.

In an embodiment, the mass of the nanoparticle or at least one constituent of the composition is lower than $10^{-20}$, $10^{-10}$, $10^{-5}$, $10^{-2}$, 1, 10, $10^3$, $10^9$ or $10^{20}$ gram. In some cases, a low mass or quantity of nanoparticle or at least one constituent of the composition may be desired to prevent or minimize toxicity of nanoparticle or at least one constituent of the composition.

In one embodiment of the invention, the nanoparticle or at least one constituent of the composition is arranged in chains or assembly comprising more than 0, 1, 2, 3, 4, 5, 6, 7, 8, 9, 10, 15, 20, 25, 30, 35 or 40 nanoparticle(s) or constituent(s) of the compositions.

In one embodiment of the invention, the nanoparticle or at least one constituent of the composition is arranged in chains or assembly comprising less than 100, 50, 10, 5, 2, 1 or 0 nanoparticle(s) or constituent(s) of the compositions.

In another embodiment of the invention, the nanoparticle or at least one constituent of the composition is arranged in chains or assembly, which have: i) a length smaller than $2.10^{10}$, $2.10^5$, $2.10^{-3}$ or $2.10^2$ nm, or ii) a number of nanoparticle or at least one constituent of the compositions in each chain or assembly smaller than 2, 5, 10, $10^2$ or $10^3$. In some cases, short chains or assemblies of nanoparticle or of at least one constituent of the compositions may be desired or obtained, for example to favor internalization of nanoparticles or at least one constituent of the composition preferentially in cells or preferentially after partial or total destruction of long chains.

In another embodiment of the invention, the nanoparticles or at least one constituent of the composition are arranged in chains or assemblies, which have: i) a length longer than $10^{-1}$, 1, 5, 10, $2.10^2$, $2.10^{-3}$ or $2.10^5$ nm or ii) a number of nanoparticle or at least one constituent of the compositions in each chain or assembly larger than 2, 5, 10, $10^2$ or $10^3$. In some cases, long chains of nanoparticle or of at least one constituent of the compositions may be desired or obtained to increase the quantity of heat or compounds dissociated from the nanoparticle or at least one constituent of the composition under the application of an acoustic wave or radiation or to prevent aggregation of nanoparticle or at least one constituent of the composition or enable uniform distribution of nanoparticle or at least one constituent of the composition. In still another embodiment of the invention, the nanoparticles or at least one constituent of the composition are arranged in chains or assemblies, which have: i) a length between $10^{-1}$ and $10^{10}$ nm, or between 1 and $10^5$ nm, or ii) a number of nanoparticle or of at least one constituent of the composition in each chain or assembly between 2 and $10^5$, 2 and $10^3$, 2 and $10^2$, or 2 and 50.

In still another embodiment of the invention, the nanoparticles or at least one constituent of the composition is(are) arranged in chains or assemblies when they are bound or linked to each other or when the crystallographic directions of two adjacent nanoparticles or of at least two constituents of the composition in the chain or assemblies are aligned, wherein such alignment is preferentially characterized by an angle between two crystallographic directions belonging to two adjacent nanoparticle or at least two adjacent constituents of the compositions or constituents in the chains or assemblies of less than 90, 80, 70, 60, 50, 20, 10, 3, or 2 ° (degree).

Preferentially when the nanoparticle or at least one constituent of the compositions is(are) biologically synthesized, the nanoparticle or at least one constituent of the compositions can be arranged in chains or assemblies: i) inside the organism that synthesizes them, also designated as nanoparticle or at least one constituent of the composition-producing cell or synthetizing living organism, or ii) outside this organism. Preferentially, nanoparticle or at least one constituent of the compositions are arranged in chains or assemblies after or before their extraction or isolation from this organism.

In one embodiment of the invention, the nanoparticle or at least one constituent of the composition is(are) not arranged in chains or assemblies.

In another embodiment of the invention, the nanoparticle or at least one constituent of the compositions is(are) synthesized chemically or are not synthesized by a living organism when less than 1, 2, 5, 10 or 100 step(s) of their production, such as crystallization of iron oxide, stabilization of the iron oxide mineral, organization of the nanoparticle or at least one constituent of the compositions, involves or is due to a living organism. In some cases, a chemical synthesis can be defined as a synthesis involving a majority of steps, or more than 1, 2, 5 or 10 steps, or more than 1, 2, 5, 25, 50, 75 or 90% of steps, which involve chemical reactions preferentially occurring without the involvement of living organisms, or parts of living organisms such as DNA, RNA, proteins, enzymes, lipids.

In another embodiment of the invention, a chemical synthesis can be used to produce a chemical substance or compound that mimics, copies, or reproduces the compartment, organelle, or other biological material, wherein this chemical synthesis or chemical substance can be used or can result in the production of the nanoparticle or at least one constituent of the compositions. In some cases, the compartment, organelle, or other biological material, can be a lysosome, an endosome, a vesicle, preferentially biological material that has the capacity or the function either to dissolve or transform crystallized iron into free iron or to transform free iron into crystalized iron. In some cases, this transformation is partial and preferentially results in the destruction or formation of partly crystallized assembly of iron atoms or ions, or preferentially results in a mixture of crystallized iron and non-crystallized iron. In some cases, crystallized iron can be defined as an assembly of iron atoms or ions that leads to the presence of crystallographic planes, preferentially observable using a technique such as transmission or scanning electron microscopy as a characterization method, and free iron can preferentially be defined as one of several iron atoms or ions that do not lead to the presence of crystallographic planes, preferentially highlighted by the absence of diffraction patterns, using for example transmission or scanning electron microscopy as a characterization method.

The invention also relates to nanoparticle or at least one constituent of the compositions for use, wherein nanoparticles or at least one constituent of the composition are or are assimilated to chemical analogues of magnetosomes, such as iron oxide nanoparticle or at least one constituent of the compositions designated as Sigma nanoparticle or at least one constituent of the compositions (ref: 637106-25G), SPION20 (nanomag®-D-spio 20, Ref: 79-02-201), SPION50 (synomag-D50, Ref: 104-000-501), SPION100 (nanomag®-D-spio 100, Ref: 79-00-102) or nanoparticle or at least one constituent of the compositions or at least one constituent of the composition synthesized using a similar method as for these nanoparticle or at least one constituent of the compositions or at least one constituent of the composition but yielding improved or additional properties such as an arrangement in chains.

In some cases, chemical analogues of magnetosomes or at least one constituent of the composition can be synthesized chemically and/or are not synthesized by magnetotactic bacteria.

In some cases, chemical analogues of magnetosomes or at least one constituent of the composition possess at least 1, 2, 3, 4, 5, 6, 7, 8, 9, or 10 common property(ies) with the magnetosomes, where these common properties are preferentially a ferrimagnetic behavior, preferentially a coercivity larger that $10^{50}$, $10^{-10}$, $10^{-2}$, 1, 5, 10 or 100 Oe at a temperature preferentially larger than 0, 5, 10, 50, 100, 200, 300, 500 or 1000 K, a large size, preferentially a size larger than 1, 5, 10, 20, 50 or 70 nm, and/or a chain arrangement, preferentially an arrangement of more than 1, 2, 5 or 10 nanoparticle or at least one constituent of the compositions in chain.

In one embodiment of the invention, the nanoparticle or at least one constituent of the compositions or magnetosomes are purified to remove more than 10, 50 or 90 percent or percent in mass of endotoxins and/or other biological material such as proteins or lipids preferentially originating from the synthetizing living organism or magnetotactic bacteria. In some other cases, the nanoparticle or at least one constituent of the compositions or magnetosomes are purified to remove less than 100, 99.9, 99, 95 or 90 percent or percent in mass of endotoxins and/or other biological material. This purification step preferentially yields purified nanoparticle or at least one constituent of the compositions or magnetosomes. In some cases, this percentage can be equal to $[Q_{BP}-Q_{AP}]/Q_{BP}$ or $Q_{AP}/Q_{BP}$, where $Q_{BP}$ and $Q_{AP}$ are the quantities of endotoxins, biological material, proteins, or lipids before and after the purification step, respectively.

In some cases, the purification step can consist in using a method or detergent(s) such as NaOH and/or KOH, which is/are preferentially mixed with the synthetizing living organism or magnetotactic bacteria or bacterial debris, preferentially to remove organic material or separate the organic material from the inorganic material comprised in the nanoparticle or at least one constituent of the compositions or magnetosomes and preferentially then be able to harvest the nanoparticle or at least one constituent of the composition or magnetosome mineral, preferentially comprised in the nanoparticle or at least one constituent of the compositions or magnetosomes.

In some cases, the purified nanoparticle or at least one constituent of the compositions or magnetosomes are or comprise preferentially partly or fully at least one mineral.

In an embodiment of the invention, the nanoparticle or at least one constituent of the compositions according to the invention are drugs, medical devices, cosmetic products, biological products, products used for research purposes, or products used to determine the properties of biological samples.

The invention relates to a method for storing the composition according to the invention, which comprises at least one of the following step(s):

- **Step 1:** Choosing or preparing the composition or at least one constituent of the composition in the form of a liquid suspension,
- **Step 2:** Lyophilizing, desiccating, dehydrating the composition or at least one constituent of the composition, or removing water from the composition or at least one constituent of the composition,
- **Step3:** Storing the composition or at least one constituent of the composition in a powder form, preferentially during more than 1 second, 3 months or 1 year.
  and
- **Step 4:** Suspending or Resuspending the composition or at least one constituent of the composition, preferentially in water.

In one embodiment of the invention, the bonds between: i) the nanoparticle core or nanoparticle coating and ii) the cryoprotectant or protectant compounds are weak bonds, preferentially Van der Walls bonds, preferentially non-covalent or non-metallic bonds.

In one embodiment of the invention, the bonds between: i) the nanoparticle core or nanoparticle coating and ii) the cryoprotectant or protectant compounds are weaker or weaker in strength than the bonds between the coating and the core of the nanoparticle.

In one embodiment of the invention, there exists partly or fully a complexation between the core and the coating of the nanoparticle, preferentially within part or the whole lifespan of the composition, preferentially during lyophilization or storage of the composition, preferentially when the composition is in liquid, gas, or solid form, or in one of these sates in a dominant manner.

In another embodiment of the invention, there does not exist partly or fully a complexation between the coating or core of the nanoparticle and the cryoprotectant or protectant compound, preferentially within part or the whole lifespan of the composition, preferentially during lyophilization or storage of the composition, preferentially when the composition is in liquid, gas, or solid form, or in one of these sates in a dominant manner.

In one embodiment of the invention, the cryoscopic constant of the composition or of at least one constituent of the composition or the Ebullioscopic constant of the composition or of at least one constituent of the composition or the depression of freezing point of the solvent or of the composition or of at least one constituent of the composition is smaller than $10^{-10}$, $10^5$, $10^3$, 100, 50, 20, 10, 5, 2, 1, 0 kg $\times$ K / mol.

In one embodiment of the invention, the cryoscopic constant of the composition or of at least one compound of the composition, or the Ebullioscopic constant of the composition or of at least one constituent of the composition or the depression of freezing point of the solvent or of the composition or of at least one constituent of the composition is larger than $10^{-10}$, $10^{-5}$, $10^{-3}$, $10^{-1}$, 0, 1, 5, 10, 50 or 100 kg $\times$ K / mol.

The invention also relates to a method for storage or preservation of at least one property of at least one nanoparticle or at least one constituent of the composition such as the chain or assembly arrangement, preferentially by following at least one of the following step(s):

1. Mixing at least one nanoparticle or at least one constituent of the composition with a cryoprotectant or protectant compound;
2. Lyophilizing or desiccating or dehydrating or applying a temperature or pressure gradient or temperature decrease or oxidation or reduction or inducing a change of state to the composition or at least one constituent of the composition preferentially issued from step 1;
3. Storing or keeping the composition or at least one constituent of the composition preferentially issued from step 2, preferentially for a lapse of time of more than 1 second, 1 day, 1 month or 1 year;
4. (re)suspending or (re)dispersing the composition or at least one constituent of the composition preferentially issued from step 3, preferentially in a liquid such as water or in a solid or in a gas sate, preferentially in such a way that the nanoparticle or at least one constituent of the composition has maintained at least one of its property such as its chain or assembly arrangement, preferentially in such a way that the at least one nanoparticle or at least one constituent of the composition can be injected to a body part;

wherein the change of state of the state of the composition or at least one constituent of the composition is preferentially selected in the group consisting of: i) liquid to solid, ii) liquid to gas, iii) solid to liquid, iv) solid to gas, v) gas to liquid, and vi) gas to solid.

In one embodiment of the invention, the cryoprotectant or protectant compound occupies a larger volume than the coating or core or nanoparticle or at least one other constituent of the composition, preferentially when the composition is in a liquid state or is mixed in a liquid.

In one embodiment of the invention, the distance between center of the nanoparticle or at least one constituent of the composition and the external surface of the nanoparticle or at least one constituent of the composition or coating or the thickness of the coating preferentially without the cryoprotectant or protectant compound is smaller than the thickness or diameter of the volume comprising the cryoprotectant or protectant compound.

In one embodiment of the invention, the volume occupied by the coating or nanoparticle or at least one constituent of the composition preferentially without the cryoprotectant or protectant compound is smaller than the volume occupied by the cryoprotectant or protectant compound.

In one embodiment of the invention, the coating material has at least one different property from the cryoprotectant or protectant compound, preferentially selected among the group consisting of: i) the coating material is not located in the same region or location than the cryoprotectant or protectant compound in the composition, ii) the coating material interacts or complexes more strongly with the nanoparticle core than the cryoprotectant or protectant compound in the composition, and iii) the coating material has a different composition or structure or magnetic property or crystallinity or is different than/from the cryoprotectant or protectant compound or has at least 1, 5, 10, $10^3$ or $10^5$ atom(s) that differ from that(those) of the cryoprotectant or protectant compound.

In one embodiment, the function of the cryoprotectant or protectant compound in the composition is to replace water molecule or maintain the size, composition, chain arrangement, or magnetic property of the at least one nanoparticle or at least one constituent of the composition, preferentially during cooling or application of a temperature/pressure gradient to the composition, preferentially to avoid damages caused by ice on the coating or nanoparticle or at least one constituent of the composition.

In one embodiment, the composition is or is maintained at a temperature larger than -273, -50, -1, 0, 2, 5, 10, 100, $10^3$ or $10^5$ °C, preferentially for more than $10^{-10}$, 0, 1, 5, 10, $10^3$ or $10^{10}$ second(s).

In another embodiment, the composition is or is maintained at a temperature smaller than $10^5$, $10^3$, 100, 10, 5, 2, 1, 0, -10, -50, -100, -250°C, preferentially for more than $10^{-10}$, 0, 1, 5, 10, $10^3$ or $10^{10}$ second(s). In one embodiment, the composition is or is maintained under a pressure larger than $10^{-50}$, $10^{-10}$, $10^{-5}$, $10^{-1}$, 0, 1, 5, 10, $10^3$, $10^5$, $10^{10}$ or $10^{20}$ bar, preferentially for more than $10^{-10}$, 0, 1, 5, 10, $10^3$ or $10^{10}$ second(s).

In another embodiment, the composition is or is maintained under a pressure smaller than $10^{50}$, $10^{-10}$, 10, 0, $10^{-3}$, $10^{-5}$, $10^{-10}$ or $10^{-20}$ bar, preferentially for more than $10^{-10}$, 0, 1, 5, 10, $10^3$ or $10^{10}$ second(s).

In one embodiment of the invention, the presence of the cryoprotectant or protectant compound in the composition enables to store the composition, preferentially in a powder form, preferentially for more than $10^{-10}$, 0, 1, 5, 10, $10^3$ or $10^{10}$ second(s), preferentially in such a way that the composition maintains at least one of its properties.

In one embodiment of the invention, the presence of a nanoparticle core or at least one constituent of the composition, preferentially a metallic one, enhances the effect of the cryoprotectant or protectant compound in the composition, preferentially by facilitating the interaction between the coating and the cryoprotectant or protectant compound, preferentially due to a stabilization of the coating on the core. In one embodiment of the invention, the composition of the at least one constituent of the composition is a mixture of amorphous and crystalline structure, wherein the nanoparticle core or at least one constituent of the composition is preferentially essentially or dominantly crystalline, wherein the nanoparticle coating or at least one other constituent of the composition, cryoprotectant and/or protectant compound is/are preferentially essentially or dominantly amorphous.

In one embodiment of the invention, the cryoprotectant and/or protectant compound protects and/or preserves and/or maintains at least one property of at least one constituent of the composition.

In one embodiment of the invention, at least one constituent of the composition, preferentially amorphous, needs the presence of the cryoprotectant and/or protectant compound to have at least one of its properties protected and/or preserved and/or maintained preferentially following exposure of the composition to a temperature and/or pressure gradient(s) and/or radiation, i.e. preferentially without the cryoprotectant and/or protectant compound the at least one property of the or at least one constituent of the composition would preferentially be damaged or changed.

In one embodiment of the invention, the at least one constituent of the composition needs the presence of the cryoprotectant and/or protectant compound to have at least one of its properties protected and/or preserved and/or maintained preferentially following exposure of the composition to a temperature and/or pressure gradient(s) and/or radiation, i.e. preferentially without the cryoprotectant and/or protectant compound the at least one property of the at least one constituent of the composition would preferentially be damaged or changed.

The invention also relates to a method for the cryo-preservation, size-preservation, composition-preservation, cohesion-preservation, magnetic property-preservation, or preservation of at least one property of the composition or nanoparticle or at least one constituent of the composition preferentially by following at least one of the following steps:

1. Mixing at least one nanoparticle or at least one constituent of the with a cryoprotectant or protectant compound;
2. Lyophilizing or desiccating or dehydrating or applying a temperature or pressure gradient or temperature decrease or oxidation or reduction or radiation inducing a change of state to the composition or at least one constituent of the composition preferentially issued from step 1;

3. Storing or keeping the composition or at least one constituent of the composition preferentially issued from step 2, preferentially for a lapse of time of more than 1 second, 1 day, 1 month or 1 year;

4. (re)suspending or (re)dispersing the composition or at least one constituent of the composition preferentially issued from step 3, preferentially in a liquid such as water or in a solid or in a gas sate, preferentially by or under sonication or radiation application, preferentially under sterile conditions, ore preferentially prior to sterilization of the composition, preferentially in such a way that the nanoparticle or at least one constituent of the composition has maintained at least one of its property such as its chain or assembly arrangement, preferentially in such a way that the at least one nanoparticle or at least one constituent of the composition can be injected to a body part;

In some cases, the preservation of at least one property of the composition or nanoparticle or at least one constituent of the composition can be the variation by less than $10^5$, $10^3$, $10^2$, 90, 75, 60, 50, 25, 10, 5, 2, 1 or 0% of this property, where this variation is preferentially equal to (P2-P1)/P1, where P1 and P2 are preferentially this property before and after the composition or nanoparticle or at least one constituent of the composition have been subjected to a temperature and/or pressure gradient or cooled down or exposed to radiation or oxidized or reduced.

The invention also relates to a composition or kit or ensemble or system or assembly comprising:

i) A first component or constituent consisting of or comprising at least one living organism or cell or bacterium or nanoparticle or at least one constituent of the nanoparticle-producing cell or magnetotactic bacterium and a first cryo-protectant or protectant compound, preferentially used to preserve the activity of the living organism such as its ability to divide or produce nanoparticle or at least one constituent of the composition(s) preferentially under storage; and/or

ii) A second component or constituent consisting of or comprising at least one nanoparticle or at least one constituent of the composition and a second cryo-protectant or protectant compound, preferentially used to preserve at least one property of at least one nanoparticle or at least one constituent of the composition, preferentially under storage;

wherein the first and second cryo-protectants or protectant compounds are preferentially different or have at least one different function.

In some cases, the first and second components or constituents are sold or used separately or at different times or under different conditions.

In some cases, the first component or constituent is necessary or used or serves as starting material for the production or fabrication of the second component.

In some other cases, the first and second components or constituents are sold or used together or at similar times or under similar conditions.

The invention also relates to a method for the cryo-preservation, size-preservation, composition-preservation, cohesion-preservation, magnetic property-preservation, or preservation of at least one property of the composition or nanoparticle or at least one constituent of the composition preferentially by following at least one of the following steps:

1. Storing or amplifying a living organism, preferentially a nanoparticle-producing cell or magnetotactic bacterium in the presence of a first cryo-protectant or protectant compound;

2. Mixing at least one nanoparticle or at least one constituent of the composition with a second cryo-protectant or protectant compound;

3. Lyophilizing or desiccating or dehydrating or applying a temperature or pressure gradient or temperature decrease or oxidation or reduction or radiation or inducing a change of state to the composition or at least one constituent of the composition preferentially issued from step 2;

4. Storing or keeping the composition or at least one constituent of the composition preferentially issued from step 2, preferentially for a lapse of time of more than 1 second, 1 day, 1 month or 1 year;

5. (re)suspending or (re)dispersing the composition or at least one constituent of the composition preferentially issued from step 4, preferentially in a liquid such as water or in a solid or in a gas sate, preferentially by or under sonication or radiation application, preferentially under sterile conditions, or preferentially prior to sterilization of the composition, preferentially in such a way that the nanoparticle or at least one constituent of the composition has maintained at least one of its property such as its chain or assembly arrangement, preferentially in such a way that the at least one nanoparticle or at least one constituent of the composition can be injected to a body part;

In some cases, the first cryo-protectant or protectant compound can be used to protect a living organism or can be DMSO or Ethylene glycol or Glycerol or 2-Methyl-2,4-pentanediol (MPD) or Propylene or glycol or Sucrose or Trehalose.

The invention also relates to a method for enabling nanoparticles or at least one constituent of the composition to be stored preferentially in powder form preferentially with a specific type of assembly or geometric figure preferentially a chain assembly preferentially to maintain this type of assembly upon reconstitution preferentially in liquid such as water, where this method preferentially comprises at least one of the following steps of:

- first step of mixing the at least one nanoparticle or at least one constituent of the composition with at least tone protectant compound,
- second step of removing water or at least one compound that is different from the at least one nanoparticle or at least one constituent of the composition and/or from the at least one protectant compound, preferentially by lyophilizing the composition or at least one constituent of the composition preferentially originating from step 1,
- third step of re-suspending the composition or at least one constituent of the composition preferentially originating from step 2, preferentially in a liquid, solid, or gas, most preferentially in water.

The invention also relates to a method of fabrication or cryo-preservation of the composition or at least one constituent of the composition according to the invention and optionally at least one nanoparticle-producing cell,
which comprises at least one of the following steps:

- **Step 1** of storing or cryo-preserving the at least one nanoparticle-producing, preferentially in a medium comprising a first cryo-protectant or protectant compound,
- **Step 2** of amplifying magnetotactic bacteria or nanoparticle-producing cells preferentially in at least one medium, comprising:

   1) the compounds necessary for the growth of magnetotactic bacteria or at least one nanoparticle-producing cell, which are preferentially selected in the group consisting of:

   - a source of carbon preferentially selected from the group consisting of: at least one compound comprising at least one atom of carbon, lactic acid, Na lactate, lactic acid, acetate, glycolate, glucose, pyruvate, succinate, carbon dioxide, glycerol and combinations thereof, at a concentration preferentially comprised between 1 nM and 2 Mol/L;
   - a source of iron preferentially selected from the group consisting of: at least one compound comprising at least one atom of iron, iron citrate, iron quinate, iron chloride, iron sulfate, $FeCl_3$, and combinations thereof, at a concentration preferentially comprised between 1 nM and $2.10^{-3}$ Mol/L;

   - a source of nitrogen preferentially selected from the group consisting of: at least one compound comprising at least one atom of nitrogen, nitrate salt, nitrogen gas, ammonium, ammonia, ammonium salt, urea, an amino acid, ammonia gas, and combinations thereof, at a concentration preferentially comprised between 1 nM and 4 Mol/L;
   - a source of oxygen preferentially selected from the group consisting of: at least one compound comprising at least one atom of oxygen, oxygen or air or compressed air, preferentially in the form of a gas, the source of oxygen being in some cases bubbled or introduced to the growth medium, at a gas rate that is preferentially comprised between 5 mL of gas per minute and 50000 mL of gas per minute;
   - a source of phosphate preferentially consisting of at least one compound comprising at least one atom of phosphate, at a concentration preferentially comprised between 1 nM and $2.10^{-1}$ Mol/L;
   - a source of potassium preferentially consisting of at least one compound comprising at least one atom of potassium, at a concentration preferentially comprised between 1 nM and $2.10^{-1}$ Mol/L;
   - a source of sulfur or sulfate preferentially consisting of at least one compound comprising at least one atom of sulfur or sulfate, at a concentration preferentially comprised between 1 nM and $4.10^{-1}$ Mol/L;
   - a source of manganese preferentially consisting of or comprising at least one compound comprising at least one atom of manganese, at a concentration preferentially comprised between 1 nM and $4.10^{-1}$ Mol/L;
   - a source of vitamin preferentially selected from the group consisting of: at least one compound comprising at least one vitamin, Biotin, Calcium, pantothenate, Folic acid, Inositol, Nicotinic acid, p-Aminobenzoic acid, Pyridoxine HCl, Riboflavin, Thiamine, Thiamine HCL and derivatives thereof and combinations thereof, at a concentration preferentially comprised between 1 nM and $10^{-4}$ Mol/L, and
   - a source of calcium preferentially consisting of or comprising at least one compound comprising at least one atom of calcium, at a concentration preferentially comprised between 1 nM and $10^{-1}$ Mol/L.

   2) at least one compound preferentially necessary for doping the magnetosomes with $C_{1FRPC}$ or a center of activity or another metal than iron, preferentially zinc or aluminum, for example a source of zinc, preferentially zinc sulfate or zinc citrate or zinc chlorate or zinc quinate.

- **Step 3** of extracting or isolating magnetosomes or nanoparticles from magnetotactic bacteria or nanoparticle-producing cells;
- **Step 4** of purifying the extracted or isolated magnetosomes or nanoparticles preferentially by heating them preferentially to yield magnetosome minerals or nanoparticle comprising a percentage in mass of organic material preferentially originating from magnetotactic bacteria or from the nanoparticle-producing cell that is lower than 100, 50, 20, 10, 5, 2 or 1%,
- **Step 5** of coating the magnetosome minerals or nanoparticles with a coating material, preferentially comprising the compound $C_{2FRPC}$ or a center of activity, preferentially by mixing the magnetosome minerals or nanoparticles with the coating material, where the mixing is preferentially realized in at least one of the following conditions:

  under sonication,
  under the application of radiation,
  under temperature variation,
  under pH changes,
  under oxidoreduction potential adjustment,
  preferentially using a ratio between the quantity or mass of magnetosome minerals and the quantity or mas of coating material, preferentially of compound D, that is adjusted or varied or larger than 1,

- **Step 6** of adding at least one cryoprotectant or protectant compound, preferentially the second cryoprotectant or protectant compound, to the coated magnetosome minerals or nanoparticles preferentially obtained at the end of step 5,
- **Step 7** of lyophilizing or dehydrating or drying or desiccating the composition preferentially obtained at the end of step 6,
- **Step 8** of re-suspending the lyophilized or dehydrated composition preferentially obtained of step 7, preferentially in water,

wherein the first and second cryo-protectants or protectant compounds when they are present are compounds that are either the same or different compounds.

The invention also relates to a method for storing the composition or preserving over time at least one property of the nanoparticle or at least one constituent of the composition, or preserving the geometric arrangement or assembly, preferentially chain arrangement of the at least one nanoparticle or at least one constituent of the composition, which comprises at least one of the following steps:

- **Step 1:** Choosing or preparing the composition or at least one constituent of the composition preferentially in the form of a liquid suspension,

- **Step 2:** Lyophilizing, desiccating, dehydrating the composition or at least one constituent of the composition, or removing water or liquid or ion or atom, preferentially totally or partly or essentially different from iron, from the composition or at least one constituent of the composition,
- **Step 3:** Storing the composition or at least one constituent of the composition preferentially essentially or totally or partly in a powder or solid form, preferentially during or for more than 1 second or 3 months, and
- **Step 4:** Suspending or resuspending the composition or at least one constituent of the composition, preferentially in water in water or liquid or gas or solid, preferentially under isotonic conditions.

The invention also relates to a method for the fabrication, the cryo-preservation, size-preservation, composition-preservation, cohesion-preservation, magnetic property-preservation, or preservation of the composition or of at least one property of the composition or at least one constituent of the composition according to the invention, by following at least one of the following steps:

- **Step 1:** Mixing at least one nanoparticle or at least one constituent of the composition with a cryoprotectant or protectant compound,
- **Step 2:** Lyophilizing or desiccating or dehydrating or applying a temperature or pressure gradient or temperature decrease or oxidation or reduction or radiation inducing a change of state to the composition or at least one constituent of the composition preferentially originating from step 1,
- **Step 3:** Storing or keeping the composition or at least one constituent of the composition preferentially originating from step 2, preferentially for a lapse of time of more than 1 second, 1 day, 1 month or 1 year,
- **Step 4:** (re)suspending or (re)dispersing the composition or at least one constituent of the composition preferentially originating from step 3, preferentially in a liquid such as water or in a solid or in a gas sate, preferentially by or under sonication or radiation application, preferentially under sterile conditions, or preferentially prior to sterilization of the composition, preferentially in such a way that the nanoparticle or at least one constituent of the composition maintains at least one of its property such as its chain arrangement, preferentially in such a way that the at least one nanoparticle or at least one constituent of the composition can be injected to a body part or that the composition is isotonic.

The invention also relates to a method for removing at least one compound from the composition or at least one constituent of the composition according to the invention, preferentially the other compound, by following at least one of the following steps:

- **Step 1:** Mixing the composition or at least one constituent of the composition with at least one chelating agent and/or introducing at least one chelating agent in the composition or at least one constituent of the composition,
- **Step 2:** Positioning a magnet near the composition or at least one constituent of the composition to attract the magnetic nanoparticle or at least one constituent of the composition in the region where the magnet is located,
- **Step 3:** Removing the part of the composition or at least one constituent of the composition that has not been attracted by the magnet or that is not magnetic,
- **Step 4:** Re-suspending the magnetic nanoparticle or at least one constituent of the composition or the part of the composition that is magnetic in a liquid, solid or gas, preferentially in a presence of a cryoprotectant or protectant compound,
- **Step 5:** Lyophilizing or desiccating or dehydrating or applying a temperature or pressure gradient or temperature decrease or oxidation or reduction or radiation inducing a change of state to the composition or at least one constituent of the composition, preferentially originating from step 4,
- **Step 6:** Storing or keeping the composition or at least one constituent of the composition preferentially originating from step 5, preferentially for a lapse of time of more than 1 second, 1 day, 1 month or 1 year,
- **Step 7:** (re)suspending or (re)dispersing the composition or at least one constituent of the composition preferentially originating from step 6, preferentially in a liquid such as water or in a solid or in a gas sate, preferentially by or under sonication or radiation application, preferentially under sterile conditions, or preferentially prior to sterilization of the composition or at least one constituent of the composition, preferentially in such a way that the nanoparticle or at least one constituent of the composition maintains at least one of its property such as its chain arrangement, preferentially in such a way that the at least one nanoparticle or at least one constituent of the composition can be injected to a body part or is isotonic,

    wherein the composition or at least one constituent of the composition preferentially comprises a magnetic part and/or a non-magnetic part;
    wherein the magnetic part of the composition is preferentially a part of the composition that can be attracted or isolated or moved or modified partly or fully by a magnet, preferentially of strength larger than the strength of the earth magnetic field or 0, $10^{-6}$, $10^{-3}$ or $10^{-1}$ T,
    wherein the magnetic part of the composition is preferentially a part of the composition that can be attracted or isolated or moved or modified partly or fully by a magnet, preferentially of strength larger than the strength of the earth magnetic field or $10^{-6}$, $10^{-3}$ or $10^{-1}$, T, more importantly than the non-magnetic part.

The invention also relates to a method for the fabrication, storage, preservation, preservation of the geometric arrangement or assembly, preferentially chain arrangement of the at least one nanoparticle or at least one constituent of the composition, cryo-preservation, size-preservation, composition-preservation, cohesion-preservation, magnetic property-preservation, or preservation of the composition or of at least one property of the composition or at least one constituent of the composition according to the invention, which comprises at least one of the following steps:

- **Step 1:** Choosing or preparing the composition or at least one constituent of the composition, preferentially by mixing at least one nanoparticle or at least one constituent of the composition with a cryoprotectant or protectant compound,
- **Step 2:** Lyophilizing or desiccating or dehydrating or removing water or liquid or ion or atom, preferentially totally or partly or essentially different from iron, from the composition or at least one constituent of the composition, applying a temperature or pressure gradient or temperature decrease or oxidation or reduction or radiation inducing a change of state to the composition or at least one constituent of the composition preferentially originating from step 1,
- **Step 3:** Storing or keeping the composition or at least one constituent of the composition preferentially essentially or totally or partly in a powder or solid form, preferentially originating from step 2, preferentially for a lapse of time of more than 1 second, 1 day, 1 month or 1 year,
- **Step 4:** (re)suspending or (re)dispersing the composition or at least one constituent of the composition preferentially originating from step 3, preferentially in a liquid such as water or in a solid or in a gas sate, preferentially by or under sonication or radiation application, preferentially under sterile conditions, or preferentially prior to sterilization of the composition or at least one constituent of the composition, preferentially in such a way that the nanoparticle or at least one constituent of the composition maintains at least one of its property such as its chain arrangement, preferentially in such a way that the at least one nanoparticle or at least one constituent of the composition can be injected to a body part or that the composition is isotonic.

In one embodiment, the at least one method or composition or at least one constituent of the composition according to the invention is used for the treatment or detection of a disease or for the preparation or storage or activation or preservation of the composition or at least one constituent of the composition or for the administration of the composition or at least one constituent of the composition to a body part. In some cases, the at least one constituent of the composition can be or have at least one property in common with the composition, or the composition can be the at least one constituent of the composition. In some other cases, the at least one constituent of the composition is different from or has at least one different property from the composition, or the composition is different from the at least one constituent of the composition.

In one embodiment of the invention, the at least one constituent of the composition, preferentially the nanoparticle coating, consists of or comprises at least one compound selected in the group consisting of: i) at least one acid such as citric acid, oleic acid, polymethacrylic acid, poly(ethyleneoxide)-b-poly(methacrylic acid) acid, polyacrylic (PAA) acid, polylactic acid, poly(ethylene oxide)-blockpoly(glutamic acid), Phosphonic acid, ii) Albumin, iii) a bisphosphonate, iv) Alendronate, v) Alginate, vi) a metal, vii) Au, viii) Al203, ix) Alginate, x) Aluminium, xi) Aluminium hydroxide, xii) Arabinogalactan, xiii) Bentonite, xiv) cellulose, xv) Carboxymethylcellulose, xvi) Chitosan, xvii) Cholseterol, xviii) Citrate, xix) Dextran, xx) Dimercaptosuccinic acid, xxi) Dopamine, xxii) DOPC, xxiii) DTAP, xxiv) DVB, xxv) Ethylcellulose, xxvi) Erythrocyte, xxvii) Fatty acid, xxviii) Ferrite, xxix) Folic acid, xxx) Gelatin Human, xxxi) serum albumin, xxxii) Liposome, xxxiii) MIPS, xxxiv) MnO, xxxv) Mn3O4, xxxvi) Oleic acid, xxxvii) PEI, xxxviii) PEG, xxxix) PEO-PGA, xl) PLA (poly(lactide acid), xli) PLGA, xlii) Phosphatidylcholine, xliii) Phosphorylcholine, xliv) Pluronic, xlv) Polyacrylamide, xlvi) Polyacrylic acid, xlvii) PAA, xlviii) Polyaniline Polyethylene glycol preferentially with terminal carboxyl groups , xlix) peptides, L) polypeptides, Li) Poly(ethylene oxide), Lii) Poly(vinyl alcohol), Liii) Ploy(N-isopropylacrylamide), 54) Poly(vinylpyrrolidone), 55) Poly(oligoethylene oxide), 56) Poly(N,N-dimethyl ethylamino acrylate), 57) Poly(imine), 58) Poly(acrylic acid), 59) Poly-D-L lactide, 60) Polyalkylcyanoacrylate, 61) Polymer, 62) PAMAM or PDMAEMA or PPEGMA or PolyNIPAAM, 63) Polyacrylic acid, 64) Polydipyrrole/dicarbazole, 65) Poly-L-lysine, 66) Polymethylmethaacrylate, 67) Polymersomes, 68) Polysaccharide, 69) Agarose or alginate or carrageenan or chitosan or dextran or heparin or Gum Arabic or Pullulan or Starch, 70) Polystyrene, 71) PVA, 72) PVP Silica, 73) an amorphous or mesoporous compound, 74) Silane, 75) SiO2, 76) Sodium Oleate, 77) Starch, 78) Styrène, 79) TaOx, 80), ZrO2, 81) Polysacharides, 82) Agarose or alginate or carrageenan or chitosan or dextran or carboxy-methyl-dextran or heparin or Gum Arabic or Pullulan or Starch, 82) Acide, 83) polymethacrylic acid or poly(ethyleneoxide)-b-poly(methacrylic acid) or polyacrylic acid (PAA) or polylactic acid or poly(ethylene oxide)-blockpoly(glutamic acid) or Phosphonic acid or Dimercaptosuccinic acid or Fatty acids or folic acid or PLA (poly(lactide acid) or Polyacrylic acid PAA 84) Polymer, 85) Dextran or Poly(ethylene oxide) or Poly(vinyl alcohol) or Ploy(N-isopropylacrylamide) or Poly(vinylpyrrolidone) or Poly(oligoethylene oxide) or Poly(N,N-dimethyl ethylamino acrylate) or Poly(imine) or Poly(acrylic acid), 86) Carboxylate, 87) inorganic compound, 88) SiO2 or Al2O3 or ZrO2 or ferrite or MnO or Mn3O4 or Au or Bentonite or Carbon preferentially activated, graphitized, 89) organic metals, 90) MIPs or Celulose or DV8 or Ppy or Chitosan or Polyacrylamide or alginate or PEI or surfactants or Phosphates or Silica or Gold or Dextran or PEG or Alginate or Chitosan, 90) or compound with a chemical function such as Alcohol for example PVA (poly(vinyl alcohol), amide, for example Poly(N-isopropylacrylamide), aldehydes, 91) a compound with a type of interactions with avec hydroxyl groups at the surface of the iron oxide (Fe-OH), 92) a compound with electrostatic interactions, preferentially with a difference in charge between at least two constituents of the composition, 93) a compound with a hydrophobic, chelating, covalent, interaction or bond, 93) a compound with a type of functional group that can preferentially be attached at the surface of a metal or iron oxide such as -OH, 94) PEG or Dextran or Polyvinylalcool or pluronic or Dopamine or Amine or Cysteine or phosphonic acid or carboxylic acid or trimethoxy or silane, 95) a compound with a -NH2 group, 96) Chitosan or polyethylenimine or Ploy(L-lysine) or PEG with terminal amine groups or Ethylenamine, 97) a compound with a -COOH group, 98) Polyacrylic acid, 99) carboxymethylcellulose, 100) PEG with terminal carboxyl groups, 101) alginate, 102) Polymethacrylic acid, 103) citrate, and 104) iminodiacetic acid or nitrilotriacetic acid or ethylendiamin or tetraacetic acid or diethylenetrianine pentaacetic acid or folic acid or L-cysteine or an amino acid or Thiol or Dimercaptosuccinic acid or a Phosphate compound or pyridoxal phosphate or adenosine diphosphate or nicotinamide adenine dinucleotide phosphate.

The invention also relates to a method for activating the composition or at least one center of activity of the composition, according to the invention, preferentially by applying a radiation or physico-chemical disturbance on the composition preferentially for a sufficiently long time, preferentially for more than $10^{-3}$, 1, 0, 1, 10, or $10^3$ second(s), wherein the activation of the composition or of at least one constituent of the composition preferentially comprises at least one at least one of the following event(s) or step(s) of:

i) releasing or diffusing or triggering the release or diffusion, preferentially in an outward direction relatively to at least one constituent of the composition, (of) at least one atom, electron, free radical, ion, metal, DNA, RNA, protein, lipid, enzyme, biological or non-biological material, organic or non-organic material, an immune entity from or of or in or at the surface or inside the nanoparticle or at least one constituent of the composition or at least one constituent of the composition, preferentially against or to fight against a disease or preferentially against or to deactivate or kill at least one pathological cell or preferentially to activate a first type of immune entity that deactivates or kills at least one pathological cell or disease or preferentially to deactivate a second type of immune entity that protects at least one pathological cell or disease or tumor or tumor environment,

ii) capturing or diffusing or triggering the release or diffusion, preferentially in an inward direction relatively to at least one constituent of the composition, (of) at least one atom, electron, free radical, ion, metal, DNA, RNA, protein, lipid, enzyme, biological or non-biological material, organic or non-organic material, an immune entity from or of or in or at the surface or inside the nanoparticle or at least one constituent of the composition or at least one constituent of the composition, preferentially against or to fight against a disease or preferentially against or to deactivate or kill at least one pathological cell or preferentially to activate a first type of immune entity that deactivates or kills at least one pathological cell or disease or preferentially to deactivate a second type of immune entity that protects at least one pathological cell or disease or tumor or tumor environment, and

iii) activating or triggering the activation (of) at least one atom, electron, free radical, ion, metal, DNA, RNA, protein, lipid, enzyme, biological or non-biological material, organic or non-organic material, an immune entity from or of or in or at the surface or inside the nanoparticle or at least one constituent of the composition or at least one constituent of the composition, preferentially against or to fight against a disease or preferentially against or to deactivate or kill at least one pathological cell or preferentially to activate a first type of immune entity that deactivates or kills at least one pathological cell or disease or preferentially to deactivate a second type of immune entity that protects at least one pathological cell or disease or tumor or tumor environment,

wherein the at least one constituent of the composition is preferentially selected in the group consisting of: i) the nanoparticle coating, ii) the nanoparticle core, iii) the cryo-protectant or protectant compound, and iv) the other compound,

wherein the immune entity, preferentially the first and/or second immune entity(ies), is preferentially selected in the group consisting of: i) DNA preferentially different types of DNA, ii) RNA preferentially different types of RNA, iii) an antigen, ii) an antibody, iii) an immune cell, preferentially of or belonging to the innate and/or adaptative immune system(s), iv) an antigen presenting cell (APC), v) a basophil, vi) a dendritic cell, vii) an eosinophil, viii) a granulocyte, ix) a killing cell, x) a natural killer, xi) a leukocyte, xii) a lymphocyte, xiii) a macrophage, preferentially of M1 and/or M2 type(s), xiii) a mast cell, xiv) a neutrophil, xv) a phagocyte, xvi) a B cell, xvii) a T cell, xviii) a CD8 or CD8+ or CD4 or CD4+ or Treg or MAIT or $T\gamma\delta$ T lymphocyte or cell, xix) a helper cell preferentially of Th1 or Th2 type, and xx) a gamma delta T cell,

wherein the radiation is preferentially selected from the group consisting of: i) a magnetic or electric or electromagnetic field or wave, a wave a particulate radiation, ii) laser light, iii) light produced by a lamp, iv) light emitted at a single wavelength, v) light emitted at multiple wavelengths, vi) a ionizing radiation, vii) microwave, viii) radiofrequencies, and ix) a sound, an ultrasound an infrasound, or an acoustic wave. wherein preferentially the physico-chemical disturbance is or is caused by or induces at least one action selected from the group consisting of:

i) a variation of an environment of at least one constituent of the composition, where the environment of the at least one constituent of the composition is preferentially a liquid, solid, or gaseous medium or at least one substance surrounding or including the at least one constituent of the composition,

ii) a variation of the environment of the at least one constituent of the composition preferentially selected from the group consisting of: a pH variation of this environment that is preferentially between $10^{-3}$ and 10 pH units, a variation in temperature of this environment that is preferentially between $10^{-13}$ and $10^3$ °C, a variation in redox potential of this environment that is preferentially between 0.001 and 100 V, a variation in viscosity of this environment that is preferentially between $10^{-9}$ and $10^{20}$ Pa.s, and a variation in the concentration of at least one substance of the environment that is preferentially between $10^{-13}$ and $10^{10}$ mole per liter, micromole per liter, nanomole per liter, mole per milliliter, micromole per milliliter, nano-mole per milliliter, mole per cubic meter, mole per cubic decimeter, mole per cubic centimeter or mole per cubic millimeter,

iii) a modification of at least one condition of the at least one constituent of the composition selected from the group consisting of a pH variation of the at least one constituent of the composition preferentially between $10^{-3}$ and 10 pH units, a temperature variation preferentially between $10^{-13}$ and $10^3$ ° C, a variation in standard potential preferentially between 0.001 V and 100 V, an increase or decrease in charge of the at least one constituent of the composition preferentially between 0.001 and 100 Volt, a variation preferentially between 1 and $10^{10}$ atom(s) in the number of atoms comprised in the at least one constituent of the composition,

iv) a variation of the concentration of at least one substance of an environment of the at least one

constituent of the composition preferentially larger than $10^{-13}$ mole per liter, micromole per liter, nano-mole per liter, mole per milliliter, micromole per milliliter, nan-mole per milliliter, mole per cubic meter, mole per cubic decimeter, mole per cubic centimeter, or mole per cubic millimeter,

v) a variation in chemical composition of at least one substance of an environment of the at least one constituent of the composition preferentially of less than $10^{10}$ mole per liter, micromole per liter, nano-mole per liter, mole per milliliter, micromole per milliliter, nanomole per milliliter, mole per cubic meter, mole per cubic decimeter, mole per cubic centimeter, or mole per cubic millimeter,

vi) a modification of at least one substance in an environment of the at least one constituent of the composition that is preferentially selected from the group consisting of a chemical modification, a structural modification, an appearance of at least one substance in the environment, a disappearance of at least one substance from the environment, and combinations thereof, and

vii) a variation of chemical composition preferentially of less than $10^{10}$ substances in an environment of the at least one constituent of the composition, where the variation is preferentially selected from the group consisting of a chemical modification, a structural modification, an appearance of at least one substance in the environment or disappearance of at least one substance from the environment, and combinations thereof.

In one embodiment of the invention, the chelating agent or the at least one constituent of the composition is preferentially selected in the group consisting of: (i) chelating agents which have one or more carboxyl groups, (ii) chelating agents which have one or more hydroxyl groups, (iii) chelating agents which have one or more amino and/or carboxyl and/or ketone groups, (iv) chelating agents which have one or more phosphonate and/or phosphonic acid groups, (V) chelating agents which have one or more bisphosphonate and/or trisphosphonate and/or tetra phosphonate groups, (vi) chelating agents which have one or more Sulfonate and/or Sulfonic acid groups, and (vii) chelating agents of monodentate or polydentate type or poly chelating agent that may comprise one or a plurality of functional groups, for example among carboxyl, hydroxyl or amino groups, (viii) of polysaccharide type. (ix) the chelating agents with one or more carboxyl groups or 9) ALA (alpha-lipoic acid) or 10) calcein or 11) carboxyfluorescein or deferasirox or dipicolinic acid or DTPA (diethylenetriaminepen taacetic acid) or EDTA (ethylenediaminetetraacetic acid) or folic acid or vitamin B9 or lactic acid or rhodamine B or carboxymethyl dextran or oxalic acid or citric acid or a compound comprising one or more citric and/or citrate functional groups or phenolic acid or 12) a chelating agent comprising one or more acetate and/or acetic functional groups or BAPTA (aminophenoxyethanetetraacetic acid) or CDTA (cyclohexane-1,2-diaminetetraacetic acid) or EDDHMA (ethylenediaminedi(ohydroxy-p-methylphenyl)acetic acid) or CaNa-EDTA or EDTCA (ethylenediaminetetraacetic acid with Cetavlon(R) or ammonium-type surfactant or EDDA or ethylenediamine-N,N'-diacetic acid) or EDDHA or ethylenediamine-N,N'-bis(2-hydroxyphenylacetic acid or EGTA or ethylene glycol bis(B-aminoethyl ether)-N,N,N',N'-tetraacetic acid or HEDTA (N-(2-hydroxyethyl)ethylenediaminetriacetic acid or HEEDTA or hydroxy-2-ethylenediaminetriacetic acid or NTA or nitrile triacetate or 13) a molecule comprising one or more hydroxyl functional groups, such as catechol or derivatives thereof, or else deferiprone or 14) a molecule comprising one or more amino functional groups, such as dopamine and/or deferoxamine or 15) a molecule comprising one or more aminocarboxylic and/or ketone functional groups, such as doxorubicin, caffeine, D-penicil lamine, pyrroloquinoline and HEIDA (hydroxyethylimino N,N-diethanoic acid) or 16) a molecule comprising at least one phosphonate or phosphonic functional group, such as AEPN (2-aminoethylphosphonic acid) or AMP (aminotris(methylenephosphonate)) or ATMP (aminotris(methylenephosphonic acid)), CEPA (2-carboxy ethylphosphonic acid) or DMMP (dimethyl methylphosphonate) or DTPMP (diethylenetriaminepenta(methylenephos phonic acid)) or EDTMP (ethylenediaminetetra (methylenephosphonic acid)) or HEDP (1-hydroxyethylidene 1,1-diphosphonic acid) or HDTMP (hexamethylenediaminetetra(methylenephosphonic acid)) or HPAA (2-hydroxyphosphonocarboxylic acid) or PBTC (phosphonobutanetricarboxylic acid), PMIDA (N-(phosphor nomethyl)iminodiacetic acid) or TDTMP (tetramethylenediamine-tetra(methylenephosphonic acid)) or ADP (adenosine diphosphoric acid) or 1-12-4-(dipyrromethene boron difluoride)butanoyl)aminododecanoyl-2-hydroxy-sn-glycero-3-phosphate or a sodium salt of L-O-phosphatidic acid or a sodium salt of 1-palmitoyl-2-(dipyrromethene boron difluoride)undecanoylsn-glycero-3-phospho-L-serine or 17) a molecule containing at least one bisphosphonate, trisphosphonate or tetraphosphonate functional group, such as 1-hydroxymethylene-bis-phosphonic acid, propanetriphosphonic acid, (nitrilotris(methylene))trisphosphonic acid or (phosphinylidynetris(methylene))trisphosphonic, or 18) a molecule comprising one or more Sulfonate or Sulfonic acid functional groups, or else a dimercapto group, such as BPDS (bathophenanthroline disulfonate or 4,7-di(4-phenylsulfonate)-1,10-phenanthroline), DMPS (dimercaptopropane Sulfonate or 2,3-dimercapto-1-propanesulfonic acid), Sulforhodamine 101 or DMSA (dimercaptosuccinic acid) or 19) polydentate ligands, i.e. chelating agents having more than one atom capable of binding to a metal atom, such as hemoglobin, chlorophyll, porphyrins and organic compounds containing pyrrole rings or 20) polymeric compounds, in particular polysaccharide compounds or 21) rhodamine B, ascorbic acid, citric acid, folic acid, erythrosine, hemoglobin, a low-molecular-weight dextran, anthranilic acid, calcein, alendronate, 3-cyclohexy lamino-1-propanesulfonic acid (CAPS) or EDTA.

The invention also relates to the composition according to the invention, wherein each nanoparticle or at least one constituent of the composition preferentially comprises a metallic core preferentially an iron oxide mineral core preferentially surrounded by a coating, wherein the composition further preferentially comprises a protectant compound selected in the group consisting of:

iv) a cryo-protectant or a compound that preferentially protects or maintains at least one property of the composition when it is cooled down, preferentially below 0°C,

v) a thermo-protectant or a compound that preferentially protects or maintains at least one property of the composition when it is exposed to a temperature gradient, preferentially of more than 0.1, 1 or 10°C,

vi) an oxydo-reduction-protectant or a compound that preferentially protects or maintains at least one property of the composition when it is exposed to a reduction or an oxidation, preferentially resulting in a different oxidation state of the nanoparticle or at least one constituent of the composition,

vii) a pressure-protectant or a compound that preferentially protects or maintains at least one property of the composition when it is exposed to a pressure or pressure variation, preferentially a pressure larger than $10^{-10}$, $10^{-3}$, 1, 0, 1, $10^3$ or $10^5$ bar or atm or mbar or Pa,

viii) a pH-protectant or a compound that preferentially protects or maintains at least one property of the composition when it is exposed to a pH variation, preferentially a pH variation of more than 0, 1, 3, 5, 10 or 14 pH unit(s),

ix) a radiation-protectant or a compound that preferentially protects or maintains at least one property of the composition when it is exposed to a radiation, preferentially a radiation selected in the group consisting of: : i) a magnetic or electric or electromagnetic field or wave, a wave a particulate radiation, ii) laser light, iii) light produced by a lamp, iv) light emitted at a single wavelength, v) light emitted at multiple wavelengths, vi) a ionizing radiation, vii) microwave, viii) radiofrequencies, and ix) a sound, an ultrasound an infrasound, or an acoustic wave.

wherein the at least one property of the composition is preferentially selected in the group consisting of: i) a chain arrangement of at least two nanoparticle or at least one constituent of the compositions, ii) an activity, iii) the size, iv) the cohesion, v) the magnetic property, vi) the composition of at least one constituent of the composition,

wherein preferentially the chain arrangement of the two nanoparticle or at least one constituent of the compositions is maintained when at least two nanoparticle or at least one constituent of the compositions are or remain arranged in changed in the composition,

wherein preferentially the activity of at least one constituent of the composition is maintained when it does not decrease or disappear,

wherein preferentially the size of at least one constituent of the composition is maintained is maintained when it does not vary by more than 50% or by more than 0.1, 1, 10 or $10^6$ nm,

wherein preferentially the cohesion of at least one constituent of the composition is maintained when at least 1, 2, or 3 or all constituents of the composition remain in the composition,

wherein preferentially the magnetic property of at least one constituent of the composition is maintained when the coercivity, magnetization, remanent magnetization, saturation magnetization does not vary by more than 50% or by more than 100 mT or $10^3$ Oe or Oe per mg of constituent(s) or when at least one constituent of the composition does not change from one magnetic state to another magnetic state,

wherein preferentially the composition of at least one constituent of the composition is maintained when less than 1, 5, 10, $10^5$ or $10^{10}$ atom(s) preferentially per constituent or 50% of constituents remain in the composition,

wherein preferentially the activity of the composition is preferentially selected in the group consisting of: a therapeutic, immunological, pharmacological, chemotherapeutical, metabolic, thermal, vaccine, hyperthermia, cryo-thermal, ablative, prophylactic, and diagnosis activity of at least one constituent of the composition,

wherein preferentially the magnetic state is selected in the group consisting of: a diamagnetic, paramagnetic, superparamagnetic, ferromagnetic, and ferrimagnetic state.

wherein preferentially the core comprises a first center activity, $C_{A1}$,

and/or

wherein preferentially the coating comprises a second center of activity, $C_{A2}$,

wherein $C_{A1}$ and $C_{A2}$ are preferentially different compounds,

wherein $C_{A1}$ and $C_{A2}$ are preferentially selected in the group consisting of:

A) a radio-sensitizer or amplifier of radiation, a radio-photosensitizer or amplifier of light radiation, an acoustic sensitizer or amplifier of acoustic radiation or wave, a sonosensitizer or amplificatory of acoustic wave, a particle radiation sensitizer or amplifier of particle radiation, where the particle comprises (or not) a mass, it is a thermal-sensitizer or amplifier of heat or cold or thermal treatment, an amplifier of the medical effect of a compound,

and

B) an attenuator of radiation, of light radiation, of acoustic radiation or wave, of particle radiation, where the particle comprises (or not) a mass, of heat or cold, of thermal treatment, and/or of the medical effect of a compound,

wherein preferentially the volume occupied by the protectant compound in the composition is larger than the volume occupied by at least one chain in the composition, by a factor of at least 1, 2, 5, 10 or $10^3$,

wherein preferentially the percentage in mass of protectant compound in the composition is comprised between 0.5 and 50 %,

wherein preferentially the distance separating $C_{A1}$ and $C_{A2}$ in the composition is preferentially larger than 1 nm,

wherein preferentially the composition is in the form of a powder or a liquid suspension,

wherein preferentially the composition is isotonic,

The invention also relates to the composition according to the invention, wherein an amplifier of radiation is preferentially at least one atom that is the center of production of radical species, which is preferentially activated or produces radical species when it is exposed to radiation.

The invention also relates to the composition according to the invention, wherein an attenuator of radiation is preferentially at least one atom that is the center of capture of radical species, which is preferentially activated or captures radical species when it is exposed to radiation.

The invention also relates to nanoparticle or at least one constituent of the composition or composition for use in a method, preferentially for increasing the production of free radicals or for increasing the production of free radicals or amplifying radiation during a sonodynamic, photodynamic or radiation therapy or exposure of a body part to a radiation, preferentially comprising the steps of:

1) preferentially introducing or reintroducing in a body part to be treated at least one nanoparticle or at least one constituent of the composition or at least one constituent of the composition preferentially comprising:

- preferentially a first center of activity, $C_{A1}$, such as a first center of activity or free radical production or radiation amplification, $C_{1FRP}$, comprised in its core,

and

- preferentially a second center of activity, $C_{A2}$, such as a second center of activity or free radical production or radiation amplification, $C_{2FRP}$, comprised in its coating,

2) preferentially applying an external radiation on the body part comprising the nanoparticle or at least one constituent of the composition(s) preferentially during a time $t_1$;

3) preferentially not applying the external radiation on the body part comprising the nanoparticle or at least one constituent of the composition(s) during a time $t_2$ or applying a radiation of lower intensity, energy, power or power density during the time $t_2$ than during the time $t_1$;

4) preferentially re-applying the external radiation on the body part comprising the nanoparticle or at least one constituent of the composition(s) during a time $t_3$ or applying an external radiation of larger intensity, energy, power or power density during $t_3$ than during $t_2$;

wherein $C_{A1}$ and $C_{A2}$ are preferentially different compounds,

wherein $C_{A1}$ and $C_{A2}$ are preferentially selected in the group consisting of:

C) a radio-sensitizer or amplifier of radiation, a photosensitizer or amplifier of light radiation, an acoustic sensitizer or amplifier of acoustic radiation or wave, a sonosensitizer or amplificatory of acoustic wave or ultrasound, a particle radiation sensitizer or amplifier of particle radiation, where the particle comprises (or not) a mass, it is a thermal-sensitizer or amplifier of heat or cold or thermal treatment, an amplifier of the medical effect of a compound, and

D) an attenuator of radiation, of light radiation, of acoustic radiation or wave, of particle radiation, where the particle comprises (or not) a mass, of heat or cold, of thermal treatment, and/or of the medical effect of a compound,

wherein optionally the nanoparticle or at least one constituent of the composition is comprised in a composition,

wherein optionally the composition comprises the nanoparticle or at least one constituent of the composition and a protectant compound,

wherein the distance separating $C_{A1}$ and $C_{A2}$ in the composition is preferentially larger than 0.1 or 1 nm,

The invention also relates to the nanoparticle or at least one constituent of the compositions or at least one constituent of the composition for use in the method of claim 1 or **2**, wherein $C_{A1}$ and/or $C_{A2}$ is/are comprised in the nanoparticle or at least one constituent of the composition(s) at a concentration ranging from 1 to $10^{10}$ $C_{A1}$ and/or $C_{A2}$ per nanoparticle or at least one constituent of the composition, wherein $C_{A1}$ and/or $C_{A2}$ is/are preferentially atom(s), ion(s), nanoparticle or at least one constituent of the composition(s), nanoelement(s), or assembly(ies) of atom(s), ion(s), nanoparticle or at least one constituent of the composition(s), nanoelement(s), or assembly(ies), wherein $C_{A1}$ and/or $C_{A2}$ preferentially has(have) a size lower than the size of at least one nanoparticle or at least one constituent of the composition, of its core, of its coating or of 100, 10 or 1 nm.

The invention also relates to the nanoparticle or at least one constituent of the compositions or at least one constituent of the composition for use in the method according to claim 1 or 2, wherein at least two steps among steps 1), 2), 3) and 4) follow each other, *i.e.* that preferentially: i) step 2 follows step 1), step 2) follows step 3), step 2) follows step 4), step 3) follows step 1), step 3) follows step 2), step 3) follows step 4), step 4) follows step 1), step 4) follows step 2), step 4) follows step 3), step 1) follows step 2), step 1) follows step 3), and/or step 1) follows step 4).

In one embodiment of the invention, the at least one method of the invention is used for at least one of the following purposes: i) decreasing the production of free radicals, ii) reducing radiation, iii) reducing the effect of radiation, iv) increasing the destruction of the body part, preferentially pathological or tumor cells or a tumor or cancer or virus or a pathological part of the body part, v) destroying or inactivating at least 1, 1.1, 2, 5 or 10 times more pathological or tumor cells or virus preferentially when the pathological cell or pathological body part is exposed to radiation in the presence of the composition than when the pathological cell or pathological body part is exposed to radiation in the absence of the composition, vi) reducing side effects of radiation or preserving the body part, preferentially non-pathological or healthy cells or healthy body part, preferentially surrounding the pathological body part, vii) preserving or maintaining activated or alive at least 1, 1.1, 2, 5 or 10 times more healthy cells preferentially when the healthy cells are exposed to radiation or subjected to an indirect such as an immune reaction that occurs after the application of radiation on the body part in the presence of the composition than when the healthy cells are exposed to radiation or subjected to an indirect such as an immune reaction that occurs after the application of radiation on the body part in the presence of the composition in the absence of the composition, viii) sonodynamic therapy, ix) photodynamic therapy, x) radiation therapy, xi) a diagnosis, and x) a treatment.

In one embodiment of the invention, at least one method according to the invention comprises at least 1, 2, 3 or 4 step(s), which is repeated at least 1, 2, 3, 4, 5 or 10 time(s).

In another embodiment of the invention, at least one method according to the invention comprises at least 1, 2, 3 or 4 step(s), which is repeated less than 10, 5, 2 or 1 time(s).

In some cases, $C_{A1}$ can be $C_{1FRP}$ or have at least one property in common with $C_{1FRP}$.

In some other cases, $C_{A2}$ can be $C_{2FRP}$ or have at least one property in common with $C_{2FRP}$.

In some cases, the constituent of the composition can be selected in the group consisting of: i) the constituent of the composition, ii) the other constituent of the composition, iii) the first constituent of the composition, and v) the second constituent of the composition.

The description is followed by the following non-limiting example(s)

**EXAMPLE 1:** *Magnetosome minerals coated with citric acid and carboxy-methyl-dextran lyophilized in the presence of cryoprotectants for long term storage and sustained anti-tumor activity.*

We report a method to formulate natural iron oxide nanoparticle or at least one constituent of the compositions, called magnetosomes, by amplifying magnetotactic bacteria in non-toxic growth media, by extracting these nanoparticle or at least one constituent of the compositions from magnetotactic bacteria under alkaline lysis, by purifying them by heat above 400 °C. This results in pure non-pyrogenic magnetosome minerals, M-uncoated, which are further coated with biocompatible citric acid or carboxy-methyl-dextran compounds, to yield stable M-CA and M-CMD. The last steps of the formulation consist in adding sorbitol, preferentially 5% sorbitol, to M-CMD and a mixture of sucrose and PEG 4000, preferentially 3.75% sucrose and 1.25 % PEG 4000, to M-CA, in lyophilizing these mixtures, resulting in NP powders of (M-CMD)ε and (M-CA)ε that display a long term stability, preferentially over at least 6 months, having preserved their pre-lyophilization properties, *i.e.* their stability in suspension, their chain arrangement, their carbon content, as well as their surface charge and surface chemical groups, in addition, we have established that (M-CMD)ε and (M-CA)ε are isotonic, preferentially with osmolalities comprised between 275 and 290 mosm/kg $H_2O$ upon NP reconstitution in water, fully biocompatible, *i.e.* sterile, non-pyrogenic, non-cytotoxic towards 3T3, L929, and V79 healthy cells up to a NP concentration of 1 mg/ml, and efficient in destroying prostate PC3-luc tumor cells when they are heated up to a maximum temperature of 46°c during 30 minutes in the presence of these cells under the application of low intensity ultrasound or alternating magnetic field. These results demonstrate that (M-CMD)ε and (M-CA)ε display a long-term storage capacity, a full biocompatibility, as well as a potential to destroy tumor cells under hyperthermia.

Among the different types of nanoparticle or at least one constituent of the compositions (NP), those made of iron oxide (IONP) display one of the highest level of biocompatibility, hence enabling their use for medical applications, in particular cancer treatment. Chemically synthesized IONP often suffer from a series of drawbacks such the use of toxic compounds in their fabrication process, a crystallinity, nonuniform shapes, and/or small sizes with low magnetization. To overcome these disadvantages, a biological synthesis route has been developed, in which IONP, called magnetosomes, are produced intracellularly by gram-negative magnetotactic bacteria (MTB). MTB have adjusted magnetosome properties over millions of years through a Darwinian process to yield an optimized system of magnetic guidance also called magnetotactisme in which the magnetosome magnetic moment aligns parallel to the earth magnetic field, enabling MTB to orientate in this direction, therefore easing their search for an optimal living environment. Magnetosomes thus consist of magnetite ($Fe_3O_4$) well crystalized cuboctahedric nanocrystals surrounded by a stabilizing phospholipid bilayer originating from MTB. They display at individual level a size of 35 to 120 nm, leading to ferrimagnetic properties. At the larger scale of magnetosome assembly, they are observed to for chains, which prevent their agglomeration. While these features led to superior antitumor efficacy for magnetosomes than for their chemical counterparts, such results were obtained with magnetosomes in non-finalized formulations.

Part of our work consists in improving the magnetosome fabrication process to render these NP injectable to humans. For that, toxic CMR or animal-based compounds are first removed from MTB growth media, second, the natural organic magnetosome membrane that contains lipopolysaccharides known as endotoxins is removed and further treatments are undertaken to yield magnetosome minerals designated as M-uncoated, which are non-pyrogenic but prone to agglomeration/sedimentation due to strong magnetic dipole interactions. Third, M-uncoated are covered with biocompatible compounds trough coordinate bonds between a coating material, i.e. citric acid (CA) or carboxy-methyl dextran (CMD), and iron cations at mineral surface, to yield stabilized M-CA or M-CMD. The further steps, which are the topic of this example, consist in adding cryoprotectants to M-CA and M-CMD and lyophilizing the obtained mixtures to transform them in a NP powder, designated as (M-CA)ε and (M-CMD)ε, which can be stored for a long period of time without losing its properties.

To improve long-term storage stability and avoid disassembling the coating from the magnetosome minerals, coated magnetosome minerals should better be stored in powder than liquid suspension. To eliminate water from M-CA and M-CMD, we have lyophilized these NP in the presence of various cryoprotectants, whose cryoprotecting action possibly relies on the creation of an amorphous glassy matrix that immobilizes nanoparticle or at least one constituent of the compositions during freezing and therefore avoids that crystallized ice produced during this step damages the coating. Since the efficacy of cryoprotection presumably depends on the type and concentration of cryoprotectant used in a formulation, we have tested various cryoprotectants, i.e. Glucose, Mannitol, PEG 4000, Sorbitol, Sucrose, Trehalose, at different concentrations, which we have added to M-CA and M-CMD. We have then determined the optimized conditions enabling to obtain stable and isotonic (M-CA)ε and (M-CMD)ε, which can be stored in powder form and resuspended in water, while maintaining their physico-chemical properties, i.e. their coating thickness/composition or magnetosome chain arrangement, their biocompatibility, i.e. their sterility, non-pyrogenicity, non-cytotoxicity towards healthy cells, as well as their faculty to destroy PC3-luc tumor cells under mile hyperthermia conditions of 41-46 °c triggered by the application of low intensity ultrasounds or alternating magnetic field.

## MATERIALS ET METHODS:

**Materials:** *Magnetospirillum gryphiswaldense* strain MSR-1 (DSM 6361) was purchased from Deutsche sammlung von mikro-organismen und zellkulturen (brunswick, germany). Pc3-luc tumor cell line derived from PC3 human prostate adenocarcinoma was purchased from Perkinelmer. Mouse fibroblast 3T3 cell line was purchased from American type culture collection (ATCC® CCL-163™). Hamster Chinese lung male V79-4 cell line was purchased from ATCC® (CCL-93). Mouse fibroblast L929 cell line was purchased from ATCC® (CCL-1). Potassium hydroxide (KOH) and citric acid were purchased in pharmaceutical grade from Merck. Carboxymethyl dextran (CMD) was purchased from TDB labs. Sucrose, glucose, trehalose, mannitol, sorbitol and polyethylene glycol 4000 (peg 4000) were purchased in pharmaceutical grade from Merck. Dextran T1 was purchased from pharmacosmos. Tryptic soy broth (TSB) and fluid thioglycolate medium (FTM) were purchased from Merck. Phosphate-buffered saline (PBS), sodium hydroxide (NOH), hydrochloric acid (HCl), nitric acid ($HNO_3$), and Triton X-100 were purchased from Thermo fisher scientific. Dulbecco's modified eagle's medium (DMEM) with/without phenol red, fetal bovine serum (FBS), penicillin-streptomycin, HEPES buffer solution, 0.25% trypsin - EDTA solution, and trypan blue solution were purchased from Gibco. Resazurin dye was purchased from Invitrogen.

**Culture of MSR-1 magnetotactic bacteria:** Briefly, MSR-1 bacteria were first amplified in two steps of pre-culture (PC) with the iron-deficient pre-growth medium, then cultivated in the last step with the appropriate conditions to promote the synthesis of magnetosomes. During the PC 1, 300 μl of MSR-1 cryo-stored were incubated in 50 ml of pre-growth medium at 29.5°c for 6 days in static state followed by 1 day under agitation at 110 rpm. Then, the pc2 was performed by transferring all the cells in a 15 1 bioreactor (Applikon) containing 6 1 of pre-growth medium and agitated at 200 rpm and 29.5°c for 3 days. during the last step of culture, the PC 2 cells were transferred in a 40 L bioreactor (Applikon) filled with 26 1 of growth medium. The culture conditions such as oxygen concentration, temperature and agitation speed were respectively maintained between 0.1 and 1%, at 29.5°c and under 200 rpm during the entire fermentation, the pH was kept at 6.9 by automatically adding an acidic medium (pH = 3), called the *"fed-batch"* solution. The bacterial growth was followed at every 24 h by taking a sample of 5 ml from the culture to determine the optical density at 565 nm ($OD_{565}$) using a UV-visible spectrophotometer. MSR-1 magnetotactic bacteria (MTB) were cultivated for a total of 19 days, using bacterial growth media composed of pharmaceutical grade chemicals, which are free of toxic compounds and heavy metals other than iron. The pre-growth medium (PGM), growth medium (GM) and fed-batch medium (FBM) comprise: i) sodium lactate (preferentially 2.6 g/L in PGM, 1.3 g/L in GM, 100 g/L in FBM), ii) ammonium chloride (preferentially 0.4 g/L in PGM, 0.2 g/L in GM, 4.8 g/L in FBM), iii) magnesium sulfate heptahydrate (preferentially 0.1 g/L in PGM, 0.03 g/L in GM, 2.4 g/L in FBM), dipotassium phosphate (preferentially 0.5 g/L in PGM, 0.07 g/L in GM, 6 g/L in FBM), iron (III) chloride hexahydrate (preferentially 0 g/L in PGM, 0 gL/L in GM, 2 g/L in FBM), mineral elixir (preferentially 0.5 mL in PGM, 0.08 mL in FBM, 7 mL in FBM), vitamin elixir (preferentially 0.1 mL in PGM, 0.07 mL in GM, 1 mL in FBM). The mineral elixir comprises 1 g/L of iron (II) sulfate heptahydrate and 30 g/L of calcium chloride. The vitamin mixture comprises 0.002 g/L biotin, 0.4 g/L calcium penthotenate, 0.002 g/L folic acid, 2 g/L inositol, 0.4 g/L nicotinic acid, 0.2 g/L p-aminobezoic acid, 0.4 g/L pyridoxine HCl, 0.2 g/L riboflavin, 0.4 g/L Thiamine HCl. The 10 first days include 2 steps of pre-culture used to amplify the bacteria in the pre-growth medium devoid of the iron source. The third step lasts about 9 days. it consists in promoting the intracellular synthesis of magnetosomes by cultivating MSR-1 bacteria in their optimized microaerophilic conditions and by gradually adding iron ion sources via fed-batch solution. At the end of the bacterial culture, 32 1 of bacterial culture were concentrated to reduce the liquid volume using a tangential filter column. After that, MSR-1 concentrate (about 5 1 in total) was stored at -80°c for further steps of magnetosome production.

**Extraction and purification of magnetosomes:** Briefly, the concentrated MSR-1 cells were diluted at an $OD_{565}$ value of 20 and then lysed in 2 m KOH at 80°c for 1 h under mechanical stirring at 150 rpm. Using magnetic selection, magnetosome chains (MgC) were collected and washed 2 times with 1 × phosphate-buffered saline (PBS) followed by 3 times washing with deionized water. After being concentrated in 50 ml-conical tubes, Mgc were frozen at -80°c for 24 h and next dried at -50°c, 0.003 mbar for 24 h using a lyophilizer (labconco, free zone 70020 2.5 1) to transform them into powder. Subsequently, 100 mg of this powder were heated at 6°c/min in a muffle furnace using a heating program comprised of several heating steps of temperatures comprised between 50°C and 420 °C (maintained for 2 h), for purifying the organic material of post-extracted magnetosomes and yielding magnetosome minerals or "uncoated magnetosomes" (M-uncoated).

**Coating of M-Uncoated with citric acid (CA) and carboxymethyl-dextran (CMD):** The coating process was carried out under aseptic conditions, using a biosafety hood and pyrogen-free materials. The solutions of CA and CMD were prepared in pyrogen-free water at 25 mg/ml and 150 mg/ml, respectively. These solutions were then filtered with a 0.22 μm polyethersulfone filter for sterilization. After that, 200 ml of each solution (CA or CMD) were added to the 1 1-glass beaker containing 2 g of M-uncoated (corresponding to 1 g of Fe) and 300 ml of pyrogen-free water. Each suspension was then sonicated for 1 min in pulse mode with a pulse length of 0.1 s and pulse interval of 0.1 s, at 20 W and ambient temperature, using a probe sonicator (PS) (branson, digital sonifier s-250d) with 25 mm-diameter tips. following the short time sonication, the pH values were adjusted at 6 and 4.5 for CA and CMD, respectively, using 1 M NaOH or 1 M Hcl. Subsequently, these two suspensions were resonicated for 1 h using the PS (Branson, digital sonifier s-250d) at the same parameters as described earlier to prepare magnetosome minerals coated with CA and CMD, called M-CA and M-CMD respectively. After the sonication, the suspensions of M-CA and M-CMD were centrifuged at 10°c and 3380 g for 45 min to remove excess of coating agents in the supernatant. each coated magnetosome minerals were then re-suspended in 10 ml of water to obtain a final concentration of 100 mg/ml of iron and stored at +4°c.

**Selection of cryoprotectants:** Under aseptic conditions, 0.5 ml of M-CMD suspension at 100 mg/ml of iron was mixed with 0.5 ml of various cryoprotectant solutions, such as glucose, mannitol, sorbitol, sucrose, and trehalose, to obtain the formulated samples of 1 ml at 50 mg/ml of iron containing 5% or 10% w/v cryoprotectant. each sample was immediately frozen in nitrogen liquid for 15 min after 30 s of vortexing, then lyophilized at -50°c (shelf temperature), 0.003 mbar during 20 h for primary desiccation (labconco, free zone 70020 2.5 1). Afterward, the secondary desiccation during 6 h was triggered by increasing the temperature to 40°c (shelf temperature) with a heating rate of 0.3°c/min while maintaining the pressure at 0.003 mbar. Likewise, M-CA suspension at 100 mg/ml of iron tested with different cryoprotectant solutions to obtain the formulated samples of 1 ml at 50 mg/ml of iron containing 5% or 10% w/v cryoprotectant for glucose, mannitol, sorbitol, trehalose, and sucrose; 0.5%, 2.5%, 5%, 7.5%, 10% and 15% w/v cryoprotectant for peg 4000 and dextran t1; and 2.5%, 3.75%, 5%, 6.25% and 7.5% w/v cryoprotectant for the combinations of sucrose - peg 4000 and sucrose - Dextran T1 with the mass ratios of sucrose to PEG or Dextran of 1:1, 1:2, 1:3, 2:1, 2:2, 2:3, 3:1, 3:2 and 3:3. After lyophilization, some formulated magnetosome samples were resuspended in 1 ml of sterile water for further characterizations. The selected formulations of M-CA and M-CMD with cryoprotectants were called (M-CA)ε and (M-CMD)ε, respectively.

**Transmission electron microscopy (TEM):** 1 ml of MSR-1 bacteria at the end of the culture was diluted at an $OD_{565nm}$ value of 1 and then rinsed 2 times with deionized water by centrifugation at 2400 g for 10 min. The different magnetosome suspensions were diluted at 50 μg/ml of iron. Afterward, 7 μl of each sample were deposited on a carbon-coated copper grid (300 mesh from oxford instruments). The grid was then left dry at ambient temperature for at least 3 h before being observed under a transmission electron microscope (Jeol JEM-2100) operated at 200 kv. Using the image j software, nanoparticle or at least one constituent of the composition size was estimated by measuring the diameter of around 400 mineral cores randomly selected from magnetosome chains inside the MSR-1 bacteria.

**Colloidal stability by colorimetric dosage:** 1 ml of magnetosome suspension was vortexed for 30 s then a sample of 50 μl was immediately taken, called "the sample $t_0$". the suspension was then rested on the bench for 2 hours and another sample of 50 μl was subsequently taken from the superior part of the liquid, called "the sample $t_{2h}$". To transform into iron ions, each sample was dissolved overnight in 950 μl of 37% v/v HCl at ambient temperature. Then 20 μl were withdrawn and mixed with 50 μl of 20% v/v $H_2O_2$ for 15 min to oxidize $Fe^{2+}$ to $Fe^{3+}$ ions. After that, the solution was added with 880 μl of ultrapure water and then mixed with 50 μl of 2 m KSCN to form the complex between $fe^{3+}$ ions and thiocyanate ions, whose absorption at 476 nm was measured in a spectrophotometer. The concentration in iron was consequently determined using a calibration range. the stability rate of magnetosome suspension was calculated as the ratio of the concentration in iron of the sample $t_{2h}$ to that of the sample $t_0$.

**Osmolality measurement:** 30 μl of magnetosome suspension at 50 mg/ml of iron were introduced into a micro-sample tube and then placed on the plate of the osmometer (advanced osmometer, model 2020) to determine the osmolality of each sample.

**CHNS elemental analysis:** 5 mg of magnetosome powder were packed in an aluminum capsule and introduced into CHNS elemental analyzer (thermofisher, flash 2000) to determine the rate of carbon and nitrogen from organic materials in each preparation.

**Fourier transform infrared spectroscopy (FT-IR):** 2 mg of magnetosome powder was deposited on a germanium crystal plate, connected to an FT-IR spectrometer. The tip was lowered until contact with the crystal and thus the powder sample. FT-IR spectra were then recorded in a scanning range of 590 - 4000 $cm^{-1}$ with a resolution of 4 $cm^{-1}$.

**Zeta-potential measurement:** Each magnetosome suspension was diluted at 50 μg/ml in iron with ultrapure water. Then it was divided into five samples of 2 ml for preparation at different pH varied from 2 to 10 at 25°c using HCl and NaOH solutions. 1.5 ml of each sample was subsequently introduced in a 4.5 ml-disposable cuvette and measured in a zeta potential analyze.

**Residual moisture analysis:** 10 mg of lyophilized powder were placed in an aluminum oxide crucible and introduced into thermogravimetric analysis (TGA) instrument. Then under the flow of nitrogen gas, the sample was heated from 30°c to 120°c at 6°c/min and maintained at 120°c for 1 h to determine the weight loss of lyophilized powder.

**Sterility test:** Under aseptic conditions, 20 mg of each magnetosome powder were incubated in two conditions, 10 ml of tryptic soy broth (TSB) at 25°c and 10 ml of fluid thioglycolate medium (FTM) at 35°c for 14 days. the culture media without NP were considered negative controls, on the last day, 1 ml was taken from each sample and placed against the magnet for 10 min to only recuperate the medium. The turbidity was then measured with 900 μl of such medium at 600 nm. And the remaining 100 μl were incubated on a solid Luria-Bertani agar plate at 25°c or 35°c for 3 days to possibly detect contaminant colonies. The other tests, *i.e.* growth promotion test and method suitability test, were also carried out in parallel with the sterility test of magnetosomes to verify the robustness of this test.

**Endotoxin quantification by the limulus amebocyte lysate (LAL) assay:** Endotoxin quantification was carried out on different magnetosome powders using a "pierce chromogenic endotoxin quant" kit under aseptic conditions. All materials contacting NP samples were sterile and pyrogen-free. Before the assay, each magnetosome powder was prepared at 40 μg/ml in iron in endotoxin-free water and then heated at 70°c for 15 min to denature any residual proteins interfering with the assay. After that, 25 μl from such suspension was introduced into a 96 well-plate pre-equilibrated at 37°C for at least 10 min. Keeping the plate at 37°C, 25 μl of the reconstituted amebocyte lysate reagent were added to each well and incubated for 12 min, followed by the incubation with 50 μl of the reconstituted chromogenic substrate for 6 min. Ultimately, 25 μl of 25% acetic acid was added to each well to stop the reaction. The optical density of such mixture was subsequently measured at 405 nm in a microplate spectrophotometer. The concentration of endotoxin was determined by the standard curve of *e. coli* endotoxin which was established at the same time as the samples.

**Growth of various cell lines: 3T3, L929, V-79, PC3-luc:** A 2 ml-cryotube of different cell lines (3T3, L929, V-79, or PC3-Luc) was thawed in a water bath at 37°c for 10 min. Each of these cell lines was then introduced into a 75 $cm^2$-culture flask containing 10 ml of suitable culture medium, *i.e.* DMEM supplemented with 10% v/v of fbs, 1% v/v of penicillin-streptomycin mixture used for 3T3, V-79, and PC3-Luc, while DMEM supplemented with 10% v/v of hs, 1% v/v of penicillin-streptomycin mixture, and 1% v/v of HEPES buffer for L929.

After being sed in the flask, the cells were maintained at 37°c in a 5% $CO_2$ incubator. The culture medium was renovelled twice per week for each flask, when the cells reached about 80% confluence, the cell passage was carried out. For that, all the liquid medium was first removed from the flask, and 1 ml of 0.25% trypsin - EDTA was added to collect all the adherent cells in suspension, then only 0.5 ml of cell suspension was retained to be mixed with 10 ml of fresh medium in a novel 75 $cm^2$-culture flask and re-incubated at 37°c in the 5% $CO_2$ incubator. Following 3 passages, each cell line was ready for further experiment.

**Cytotoxicity test.** 3T3, L929, and V-79 cell lines were each seeded on a sterile 96-well plate ($10^4$ cells/well) and then incubated overnight at 37°c in a 5% $CO_2$ incubator for cell adhesion. After that, all the medium was delicately removed from the plate. 100 μl of the suspension of lyophilized (M-CA)ε or (M-CMD)ε, prepared at various concentrations in the suitable culture medium without phenol red for each cell line, were then added to each well. In 3T3, V-79, L929 cell plate, 0.001, 0.1, 0.25, and 1 mg/ml in iron of magnetosome suspensions were added. After adding the nanoparticle or at least one constituent of the composition suspensions, the plates were re-incubated for 24 h at 37°c in a 5% $CO_2$ incubator. To determine the cell viability, 10 μl of resazurin reagent were added to each well and homogenized under agitation at 120 rpm for 10 min. The plates were next incubated at 37°C in the 5% $CO_2$ incubator for approximately 4 h. Once the blue color in the wells of untreated cells turned to pink color, all the liquid in each well was transferred to a 1.5-ml Eppendorf tube. The tube was then centrifuged at 14100 g for 10 min to remove all the nanoparticle or at least one constituent of the compositions which could interfere with the assay. Subsequently, 100 μl of the supernatant was transferred into a novel 96 well-plate to be measured at excitation and emission wavelengths of 530 and 590 nm, respectively, using a fluorescence reader. The cell viability was calculated using the formula:

$$\text{Cell viability (\%)} = \left(\frac{Ft-Fb}{Fc-Fb}\right) \times 100\%$$

where, $Ft$ is the fluorescence intensity of treated wells, $Fb$ is the fluorescence intensity of blank measured in the empty wells containing 100 μl of medium mixed with 10 μl of resazurin, and $Fc$ is the fluorescence intensity of control wells measured in the untreated cells incubated in 100 μl of medium mixed with 10 μl of resazurin.

**Hyperthermia treatments under AMF (alternating magnetic field) and LIU (low intensity ultrasound) applications.** PC3-luc cells were seeded on a sterile 96-well plate ($3 \times 10^4$ cells/well) and incubated overnight at 37°c in a 5% $CO_2$ incubator for cell adhesion. Then all the medium was removed from each well and the adherent cells were rinsed 2 times with DMEM without phenol red (white DMEM). 100 μl of (M-CA)ε or (M-CMD)ε suspension prepared at 1 mg/ml of iron in white DMEM supplemented with 10% v/v of FBS, 1% v/v of penicillin-streptomycin and 1% v/v of HEPES buffer were added to each well. Subsequently, all the wells were re-incubated for 3 h at 37 °c in a 5% $CO_2$ incubator. After that, the hyperthermia experiment was divided into three conditions in which there were for each condition 3 wells containing only Pc3-luc cells, 3 other wells containing cells incubated with (M-CA)ε, and 3 other wells of cells with (M-CMD)ε. In the first condition, the wells were not exposed to any hyperthermia source. in the second condition, 3 wells per time were placed in a polystyrene holder positioned at the center of a copper coil, in which an AMF of 42 mt and 195 KHz was applied for 30 min with an easy heat equipment to heat the cells, in the third condition, each of the 3 wells was soaked in an adaptor filled with degassed water to make the connection with an ultrasound plane transducer (3.6 cm in diameter) and thus to be exposed for 30 min to ultrasound intensity lying between 0.3 and 1 w/$cm^2$, at 1 MHz and in continuous mode, produced by ultrasound generator (Primo Therasonic 460). To limit the evaporation of culture medium, each well was covered by a cap when exposed to a hyperthermia source. The temperature was monitored over time using a flexible thermocouple probe (physitemp, it-18) inserted into each well and Dasylab software, the SAR of the sample was calculated using the following equation: $SAR\ (W/g_{Fe}) = (C_{medium}/C_{Fe}) \times (\Delta t/\delta t)$ where, $C_{medium} = 4.2$ j.g$^{-1}$.k$^{-1}$ is the specific heat capacity of water, $C_{fe}$, which is measured in g of iron per g of water, represents the iron concentration in the magnetosome sample, and $\Delta t/\delta t$ (measured in °c/s) is the slope of temperature variation with time of the magnetosome sample after subtracting that of the control samples. Following the hyperthermia treatments, all the wells were incubated for 24 h at 37°c in a 5% $CO_2$ incubator. After that, the cell viability in each well was determined using resazurin reagent. **Quantification in the iron of magnetosomes internalized by cells.** After taking all the supernatant for the cell viability assay, PC3-luc cells adhered at the bottom of the wells were delicately rinsed 3 times with 300 μl of 1x pbs. Each well was then incubated with 50 μl of 0.25% trypsin - EDTA for 1 min at 37 °c and 5% $CO_2$, followed by the introduction of 150 μl of complete DMEM. After that, all the liquid in each well (200 μl) was transferred into a 1.5 ml-eppendorf tube. After a few seconds of vortexing, a sample of 20 μl was taken from the Eppendorf tube and mixed with 20 μl of trypan blue to determine cell concentration using a Malassez hemocytometer. 180 μl of the remaining cell suspension were centrifuged at 14100 g for 20 min to remove all the supernatant. The cell pellet was next dissolved overnight in 20 μl of 37% w/v HCl and 145 μl of 70% w/v $HNO_3$ at ambient temperature. Finally, the solution was diluted with ultrapure water in a total volume of 5 ml and analyzed in an inductively coupled plasma-mass spectrometer (agilent, 7900 icp-ms) to determine the magnetosome iron content internalized into PC3-luc cells.

**Statistical analyzes.** The data were analyzed using graphpad prism version 8.0. All measurements were performed in triplicate (n = 3) and data were reported as the mean $\pm$ standard deviation (sd). Statistical comparisons were carried out using one-way anova and the differences were considered significant when * p < 0.05, ** p < 0.01, and *** p < 0.001.

**RESULTS AND DISCUSSIONS.**

**Magnetosome formulation: summary of the previously published first steps and presentation of the additional/complementary steps proposed in this study.** This study presents additional aspects concerning the implementation of a magnetosome-based formulation for injection in humans. The first steps of this formulation, are summarized, step 1 consists in cultivating magnetotactic bacteria, leading to magnetotactic bacteria of spirilla shape containing chains of magnetosomes in their cytoplasm with magnetosome mean sizes of 36.6 (standard deviation of 6.4 nm). Step 2 involves magnetosome extraction from magnetotactic bacteria issued from step 1, using KOH bacterial lysis and magnetic separation of magnetosomes from bacterial debris, leading to extracted magnetosomes arranged in chains (Mg-Ch), where each magnetosome is surrounded by a bacterial inflammatory membrane, which seems too immunogenic in the absence of specific treatment for injection to human. Step 3 comprises the purification of Mg-Ch through combustion to remove/denature bacterial organic material from Mg-Ch, leading to non-pyrogenic magnetosome minerals (M-uncoated) with a low percentage of carbon of 0.2%, which tend to aggregate due to their bare surface. M-uncoated are coated with two biocompatible compounds, *i.e.* citric acid (CA) or carboxy-methyl dextran (CMD), yielding non-pyrogenic coated magnetosome minerals (M-CA and M-CMD) reconstituted in chains.

The objective of this example is to add some further steps in the preparation of coated magnetosomes to be able to store M-CA and M-CMD over a long period of time, while preventing coating degradation. To achieve this aim, M-CA and M-CMD have been kept in the form of an anhydrous powder, which can be reconstituted in a NP suspension at any time, depending on need. We have then verified that reconstituted M-CA and M-CMD, designated as $(M\text{-}CA)\varepsilon$, $(M\text{-}CMD)\varepsilon$, $(M\text{-}CA)_{fw}$, $(M\text{-}CMD)_{fw}$ for lyophilized M-CA and M-CMD before and after NP resuspension in water, respectively, have preserved their physicochemical properties and anti-tumor activity.

**Formulation of M-CA and M-CMD in the presence of cryo-protectant or thermo-protectant or oxydo-protectant or chain-protectant or protectant of the composition or size or cohesion or at least one magnetic property of at least one nanoparticle or at least one constituent of the composition for long term storage.** Here, we determine the conditions of fabrication of an injectable magnetosome formulation with a long-term stability. The latter is evaluated by measuring the percentage of absorption stability, measured at 480 nm over time, of a suspension of magnetosomes, preferentially of 50 mg of magnetosomes during 2 hours following its homogenization. The percentages of 100% and 0% correspond to stable and unstable suspensions, respectively. we chose to carry out stability measurements with 50 mg of magnetosomes, since we anticipate to administer this amount of magnetosomes in a human prostate tumor of 1-2 cm diameter based on our pre-clinical mouse efficacy data extrapolated to humans. furthermore, a lapse of time of 2 hours was selected for stability assessment, which seems to be long enough for a nurse or doctor to be able to administer a stable magnetosome suspension in tumors. Given that the purified magnetosome minerals devoid of active bacterial organic matter (M-uncoated) were unstable, *i.e.* their percentage of stability is 0%, they were coated with CA and CMD, leading to M-CA and M-CMD, which are perfectly stable, *i.e.* their percentage of stability is 100%, which is larger to that of M-uncoated and M-gC. When M-CA and M-CMD are lyophilized in the absence of a specific compound that can preserve their coating, they form NP powders designated as $(M\text{-}CA)_{fd}$ and $(M\text{-}CMD)_{fd}$ characterized by the presence of agglomerated magnetosomes. When $(M\text{-}CA)_{fd}$ and $(M\text{-}CMD)_{fd}$ are resuspended in water following the lyophilization step, they rapidly sediment leading to a percentage of stability lower than 5%. To enable the reconstitution of M-CA and M-CMD in stable NP water suspension following lyophilization, *i.e.* without damaging M-CA/M-CMD coating, M-CA and M-CMD at the foreseen therapeutic dose of 50 mg/ml were mixed with various cryo-protectant or thermo-protectant or oxydo-protectant or chain-protectant or protectant of the composition or size or cohesion or at least one magnetic property of at least one nanoparticle or at least one constituent of the compositions, *i.e.* glucose, mannitol, sorbitol, sucrose, or trehalose, used at percentages in mass of 5% and 10%. after being lyophilized, such formulations were resuspended in water to determine their colloidal stability. Considering M-CMD first, only 5% and 10% sorbitol succeed in maintaining 100% colloidal NP stability. Furthermore, the osmolality of the M-CMD suspension increases with the percentage of sorbitol added to the M-CMD suspension, from 0 mosm/kg $H_2O$ in the absence of cryoprotectant to 280 and 560 mosm/kg $H_2O$ for 5 % and 10 % of sorbitol, respectively. M-CMD formulated with 5% sorbitol leads to an osmolality value lying within the range of plasma osmolality (275 - 290 mosm/kg $H_2O$), which is acceptable for human injection. Turning now to M-CA, M-ca mixed with 10% of glucose, sorbitol or sucrose, leads to 100% colloidal stability. However, such formulations are hypertonic, *i.e.* their osmolality is larger than 300 mosm/kg $H_2O$. To solve this issue, we selected M-CA mixed with 10% sucrose that leads to an osmolality of 330 mosm/kg $H_2O$, which is the closest value to the plasma osmolality, and we added to such mixture either PEG 4000 or Dextran T1, to decrease the osmolality of the formulation to the plasma osmolality. Whereas the use of Dextran t1 enables stabilizing the formulation, it yields an osmolality that is lower than the plasma osmolality. Hence, Dextran T1 was abandoned. By contrast, by combining sucrose with PEG 4000, a 100% stable formulation was reached while the sole addition of 7.5% sucrose or 15% PEG 4000 led to a stability at about 90 or 50%, respectively. This result suggests a synergistic effect between sucrose and PEG 4000, which appear to be mutually supportive, *i.e.* PEG 4000 could effectively protect NP during freezing by encapsulating NP in a glassy matrix, and amorphous sucrose could serve as a *"water replacement"* reservoir allowing the formation of hydrogen bonds with citric acid on NP surface, and consequently preserving the coating integrity against the stress induced by dehydration. The optimal combination of sucrose and PEG 4000 corresponds to 1.25% of PEG 4000, *i.e.* the lowest concentration of PEG 4000 to prevent the risk of molecular mobility and degradation of the formulation during storage, which could be a consequence of a too large PEG concentration, and the largest percentage in mass of sucrose, *i.e.* 3.75%, to yield stable formulated M-CA in suspension. The M-CA formulation with 1.25% of PEG 400 and 3.75% of sucrose displays a osmolality of 225 mOsm/Kg $H_2O$, which can be adjusted to be isotonic upon reconstitution.

**M-CA/M-CMD preserve their physicochemical properties following their formulation.** To examine if magnetosomes formulated with 5% sorbitol for M-CMD, or 1.25% of PEG 400 and 3.75% of sucrose for M-CA, which are designated as (M-CMD)$_f$ and (M-CA)$_\varepsilon$ before removal of cryoprotectant and as (M-CMD)$_{wf}$ and (M-CA)$_{wf}$ after removal of cryoprotectant, have maintained their physicochemical properties, we have compared the percentages of carbon and nitrogen as well as the FT-IR spectra and surface charges of formulated magnetosomes with those of magnetosomes harvested at the various steps preceding formulation, *i.e.* M-CA, M-CMD, M-gC, and M-uncoated. Concerning the percentages in mass of carbon and nitrogen in magnetosomes at the various formulation steps, they first decrease from carbon and nitrogen percentages of %C=22.7% and %N=2.2% in M-gC down to %C=012% and %N=0.01% in M-uncoated, corresponding to the removal of the magnetosome membrane in M-gC to yield M-uncoated. Then, they increase a first time from M-uncoated to M-CA (%C =1.61%, %N=0.01%) and M-CMD (%C=3.80%, %N=0.01%), which is due to the addition of a coating of CA and CMD to the surface of M-uncoated, and a second time from M-CA and M-CMD to (M-CA)$_f$ (%C=18.60% and %N=0.01%) and (M-CMD)$_f$ (%C=18.56% and %N=0.01%), as the cryoprotectant is added to the formulated magnetosomes. In the final step, these percentages decrease again following the washing of the cryoprotectant from the formulated magnetosomes, *i.e.* down to %C=1.6% and %N=0.01% in (M-CA)$_{fw}$ and %C=3.83%, %N=0.01% in (M-CMD)$_{fw}$. The FT-IR spectra of the magnetosomes at the various steps of their formulation further confirm the CHNS trends described above. Indeed, Mg-C FT-IR spectrum displays vibration bands of P-O (at 1037 cm$^{-1}$), C-O (at 1410 cm$^{-1}$), N-H (at 1542 and 1641 cm$^{-1}$), and O-H (at 3276 cm$^{-1}$), corresponding to functional groups present in the phospholipid magnetosome membrane. Concerning M-uncoated, its FT-IR spectrum does not indicate the presence of peaks above 1100 cm$^{-1}$, which is consistent with the removal of most organic material in this sample. The two peaks at 612 and 693 cm$^{-1}$ are attributed to Fe-O stretching vibrations, which come from the iron oxide comprised in the magnetosome mineral core, and are therefore present in all FT-IR spectra of the different types of magnetosomes. The coating of M-uncoated with CA and CMD leads to a series of peaks in the range of 1000 - 1300 cm$^{-1}$ and 1630 - 1750 cm$^{-1}$, which correspond to the stretching vibrations of c-o and c=o, respectively, attributed to the carboxylate groups of CA and CMD in M-CA and M-CMD. Further on in the formulation process, lyophilized magnetosomes (M-CA)$_f$ and (M-CMD)$_f$ display intense bands of alcohol and alkane groups, *e.g.* the stretching vibration bands of C-O, O-H and C-H in the range of 970 - 1250 cm$^{-1}$, 3200 - 3550 cm$^{-1}$ and 2850 - 3000 cm$^{-1}$, respectively, corresponding to sucrose and PEG 4000 in (M-CA)$_\varepsilon$, and sorbitol in (M-CMD)$_\varepsilon$. Finally, following a washing step, (M-CA)$_{fw}$ and (M-CMD)$_{fw}$ display FT-IR spectra, which are very close to those M-CA and M-CMD, indicating that the washing procedure has easily separated the cryoprotectant from the coated magnetosome, due to the absence of a strong binding between cryoprotectant molecules and magnetosome coating agents. CHNS and FT-IR measurements suggest that the surface of formulated magnetosomes remains unchanged compared with that of coated magnetosomes before formulation. To confirm this deduction, we have measured the surface charges of the formulated magnetosomes that we have compared with those of non-formulated magnetosomes. (M-CA)$_\varepsilon$ and (M-CMD)$_\varepsilon$ display very similar surface charges as those of M-CA and M-CMD when the pH of the suspensions containing these NP is varied between 2 and 10. Such behavior indicates that the cryoprotectant efficiently maintains magnetosome surface charges in (M-CA)$_\varepsilon$ and (M-CMD)$_\varepsilon$, possibly by efficiently protecting the coating layers in M-CA and M-CMD against degradation/removal during freeze-drying, and by preventing the cryoprotectant to be strongly bound to NP. Furthermore, under conditions of use for human injection, *i.e.* at the therapeutic dose of 50 mg/ml in iron and at pH of ~6.5, (M-CA)$_\varepsilon$ and (M-CMD)$_\varepsilon$ display surface charges that are below -30 mv, creating the conditions of strong repulsive electrostatic forces in (M-CA)$_\varepsilon$ / (M-CMD)$_f$ suspensions, which can prevent NP agglomeration caused by magnetic dipole interactions and stabilize these NP in suspension. Electron microscopy measurements conducted on formulated magnetosomes washed of their cryoprotectant, *i.e.* on (M-CA)$_{fw}$ and (M-CMD)$_{fw}$, show that formulated magnetosomes preserve a chain arrangement after the freeze-drying step, a property complementary to that of their surface charge, ensuring their stability and preventing their aggregation. Finally, the long-term stability of formulated magnetosomes, which is sought for here, is ensured when the amount of water remaining in the freeze-dried product after the freeze-drying process, called residual moisture (RM), is sufficiently small, *i.e.* typically below 3% for a pharmaceutical product. The RM content of (M-CA)$\varepsilon$ and (M-CMD)$_f$ were measured by introducing these NP in a TGA , by heating them at 120°c during 1 h with a heating rate of 6°c/min, and by measuring the weight loss of these NP attributed to water evaporation. The RM contents of (M-CA)$_f$and (M-CMD)$_f$ lie within the rage of (1.8-2.4)%, which are pharmaceutically acceptable. Furthermore, due to this efficient lyophilization process, (M-CA)$\varepsilon$ and (M-CMD)$\varepsilon$ could be resuspended in water, preferentially after 6 months, where they maintain their stability after a storage time of half a year.

**Sterility/non-pyrogenicity of formulated magnetosomes.** Compared to unformulated magnetosomes, formulated magnetosomes must not only preserve their physicochemical properties, but also their biocompatibility. To study this last aspect, we first examined the sterility of the formulated magnetosomes by introducing (M-CA)$\varepsilon$ and (M-CMD)$_f$ during 14 days in solutions of tryptic soy broth (TSB) and fluid thioglycolate medium (FTM) at 30°c and 37°c, respectively. indeed, these conditions are known to amplify bacterial contaminants when they are initially present in a tested sample, and therefore to enable their detection. To determine the presence (or absence) of bacteria in (M-CA)$\varepsilon$ and (M-CMD)$\varepsilon$, the optical density of these NP in suspension was measured at 600 nm (OD$_{600}$) at the end of the incubation time. The value of OD$_{600}$ reflects the turbidity of these suspensions, which is associated with the presence of potential bacterial contaminant. (M-CA)$\varepsilon$ and (M-CMD)$_f$ display a low value of OD$_{600}$ < 0.1, which is comparable to OD$_{600}$ < 0.1 of M-uncoated and of the sterile TSB and FTM media before incubation (NC), indicating the absence of bacterial contaminants in (M-CA)$\varepsilon$ and (M-CMD)$_f$. This result is further confirms the absence of colonies in agar plates inoculated with (M-CA)$\varepsilon$ and (M-CMD)$_f$. To complement the assessment of the sterility and non-pyrogenicity of (M-CA)$\varepsilon$ and (M-CMD)$\varepsilon$, the endotoxin concentration of these NP was measured. (M-CA)$\varepsilon$ and (M-CMD)$_f$ appear to contain a very low endotoxin concentration of 2-5 EU/mg of iron, comparable to the values measured for M-uncoated. Taken altogether, these results highlight the efficacy of the magnetosome purification step in removing bacterial contaminants from M-gC, whose initial presence before a specific treatment is revealed by the high M-gC OD$_{600}$ value of 1-2.5 as well as a large M-gC endotoxin concentration of 50 EU/mg in iron. Such behavior can be attributed on the one hand to a double step depyrogenation process, *i.e.* first by mixing M-gC with KOH at 80 °c and second by heating M-gC above 400°C, and on the second hand to formulation steps carried out under ascetic conditions, *i.e.* the coating of magnetosome minerals with CA or CMD, the addition of cryoprotectant to coated magnetosomes M-CA and M-CMD, the lyophilization of the obtained mixture, are carried out under sterile hood.

**Non-cytotoxicity of formulated magnetosomes.** To examine the cytotoxicity of the lyophilized formulated magnetosomes, 100 $\mu$l of (M-CA)$_f$ and (M-CMD)$_f$ suspensions of concentrations varied between 0.001 and 1 mg of NP in iron per ml were brought into contact with various mammalian cell lines, *i.e.* 3T3, L929, and V-79 cells, during 24 hours at 37°c. Following this treatment, the cell viability was assessed by measuring cellular metabolic activity with the resazurin assay. All cell lines display a viability larger than 70% for all tested concentration of (M-CA)$_{fw}$ and (M-CMD)$_{fw}$, revealing that these NP are non-cytotoxic under the tested conditions, and that the presence of a cryoprotectant and of a lyophilization step in the magnetosome formulation does not result in added cytotoxicity.

**Efficient hyperthermia treatment using formulated magnetosome minerals excited under alternating magnetic field and ultrasonic sources.** We study if (M-CA)$_f$ and (M-CMD)$_f$ maintain the therapeutic activity observed in non-fully formulated magnetosomes M-CA and M-CMD. More specifically, we examine if (M-CA)$_f$ and (M-CMD)$_f$ efficiently destroy prostate tumor cells when 100 $\mu$l of (M-CA)$_f$ or (M-CMD)f at a concentration of 1 mg of np in iron per ml of water suspension are incubated with PC3-luc cells during 3 h, and the resulting mixture is exposed during 30 minutes to an AMF of 42 mt and 195 KHz or to a low intensity ultrasound of 0.3-1 w/cm$^2$ and 1 MHz. Such conditions of excitation lead to a temperature increase of these mixtures from ambient temperature of 22-25°c to moderate hyperthermia temperature of 46°c, where the temperature rise is significantly faster with the ultrasonic than with the magnetic excitation, leading to the temperature being maintained at 46°c for almost 30 minutes and slightly less than 10 minutes with the ultrasound and magnetic field, respectively. The specific absorption rates (SAR) of (M-CA)$_f$ and (M-CMD)$_f$ are measured under exposure of these NP to AMF/LIU after subtraction of the initial temperature increase due to AMF/LIU in the absence of NP. While (M-CA)$\varepsilon$ and (M-CMD)f display a large SAR value of 229 $\pm$ 16 W/g$_{Fe}$ under AMF excitation, they produce a close to zero SAR value under LIU, highlighting the different nature of the heating with the two excitation sources, *i.e.* a heating originating from NP excitation by the AMF or absorption of ultrasound energy by cells, using AMF or LIU application, respectively. Moreover, the heating properties of the two types of formulated magnetosomes, *i.e.* (M-CA)$\varepsilon$ and (M-CMD)f are similar. This indicates that the differences between the two coatings in nature/composition, coating thicknesses, carbon contents, do not significantly affect (M-CA)$\varepsilon$ and (M-CMD)$\varepsilon$ heating properties. We may conclude that certain heating contributions such as the Brownian one in the induction mechanism, which can depend on the coating properties, are not dominantly involved in the observed heating properties. To further assess the efficacy of the heat treatment in destroying tumor cells, Pc3-luc cells treated as described above were re-incubated overnight at 37°C under 5% $CO_2$. Their viability was subsequently measured. In the absence of a heat treatment, PC3-Luc cells incubated with (M-CA)$_f$ and (M-CMD)$_f$ display a viability of ~80%, indicating the absence of cytotoxicity of (M-CA)$_f$ and (M-CMD)$_f$ towards these cells. This behavior agrees with that observed with healthy mammalian cells. By contrast, in the presence of M-CA/M-CMD and a heating session, the cell viability decreases by 30-40% and 60-85% following AMF and LIU application, respectively, compared with the two controlled conditions in which the cells are either only incubated with (M-CA)$_f$ / (M-CMD)$_f$ without heating or only exposed to AMF / LIU without NP exposure. These results suggest that LIU leads to more efficient cellular destruction than AMF, possibly due to a temperature maintained at 46°c for a longer period of time with LIU than with AMF. Furthermore, it seems that although both types of formulated magnetosomes form an efficient pair with LIU to destroy prostate tumor cells, M-CMD outperforms M-CA, a behavior that we are currently trying to understand. Given that NP internalization in tumor cells is often correlated with efficient cellular destruction, we estimated the amount of (M-CA)$_f$ and (M-CMD)$_f$, which is internalized in PC3-luc cells following a similar treatment as that used to measure cell viability. Following treatments, we have destroyed and dissolved the tumor cells, and measured their iron content by ICP-MS. The quantities of NP internalized in PC3-luc tumor cells, $Q_i$, is larger for (M-CMD)$_f$ than for (M-CA)$_f$ in all tested conditions, *i.e.* with/without AMF/LIU treatments. $Q_i$ are estimated as 17-20 and 30 pg of NP per cell following AMF/LIU treatment for (M-CA)$\varepsilon$ and (M-CMD)$\varepsilon$, respectively. Compared to (M-CMD)$\varepsilon$, (M-CA)$\varepsilon$ displays a lower internalization in PC3-luc cells by a factor of 1.5-2. This behavior may be attributed to the more negative surface charge at physiological pH for (M-CMD)f than for (M-CA)$_f$, possibly resulting in a better affinity of (M-CMD)$_f$ for the cationic sites of the plasma cellular membrane and subsequently to the higher cellular internalization possibly via pinocytosis for (M-CMD)$_f$ than for (M-CA)$_f$. Considering now the potential impact of NP internalization on cell viability, it can be observed that the increase in NP internalization between M-CMD and M-CA is either correlated with an enhanced cellular mortality following LIU treatment or uncorrelated with a change in cellular mortality under AMF application. Such behaviors could be explained by ultrasounds acting synergistically with internalized NP to destroy tumor cells, *i.e.* ultrasounds could destroy/inactivate the cell membrane while NP could act perturbatively inside the cells from their intracellular location. By contrast, whereas AMF is expected to produce local heating at NP site, it is not supposed to act perturbatively against the cellular membrane. Thus, it is possible that in the presence of internalized NP, ultrasounds act more perturbatively than AMF, resulting in more efficient cellular destruction.

**Example 2: *Set-up of a stable Magnetospirillum gryphiswaldense MSR1 cellular bank to produce pharmaceutical grade magnetosomes, (Nguyen et al, Applied Microbiology and Biotechnology (2023) 107:1159-1176).***

We report the first successful fabrication of a pharmaceutical cellular bank (PCB) containing magnetotactic bacteria (MTB), which belong to the *Magnetospirillum gryphiswaldense* MSR1 species. To produce such PCB, we amplified MTB in a minimal growth medium essentially devoid of other heavy metals than iron and CMR products. The PCB enabled to acclimate MTB to such reduced conditions and then to produce highly pure magnetosomes composed of more than 99.9% of iron. The qualification of the bank as a PCB relies first on a preserved identity of the MTB compared with the original strain, second on genetic bacterial stability observed over 100 generations or under cryo-preservation for 16 months, third on a high level of purity highlighted by an absence of contaminating microorganisms in the PCB. Furthermore, the PCB was prepared under high-cell load conditions ($9.10^8$ cells/mL), allowing large-scale bacterial amplification and magnetosome production. In the future, the PCB could therefore be considered for commercial as well as research orientated applications in nanomedicine. We describe for the first-time conditions for setting-up an effective pharmaceutical cellular bank preserving over time the ability of certain specific cells, i.e. MTB, to produce nano-minerals, i.e. magnetosomes, within a pharmaceutical setting.

Magnetotactic bacteria (MTB) belong to a polyphyletic group of bacteria with the distinctive feature of producing intracellular magnetic nanoparticle or at least one constituent of the compositions called magnetosomes (Amann et al. 2007 DOI 10.1007/7171_037.; Blakemore, 1982, Ann. Rev Microbiol 1981. 36:117-38.; Komeili, 2012, DOI: 10.1111/j.1574-6976.2011.00315.x). MTB are ubiquitous in aquatic environments and are preferentially found in sediments and water columns in the oxic-anoxic transition zone and the anoxic region (Flies et al., 2005, doi:10.1016/j.femsec.2004.11.006; Lefèvre and Bazylinski, 2013, http://dx.doi.org/10.1128/MMBR.00021-13; Schüler, 1999, J. Molec. Microbiol. Biotechnol. (1999) 1(1): 79-86.). These habitats have a specific chemical stratification that favors magnetosome biosynthesis in terms of iron availability and oxygen levels, since biomineralization occurs in microaerobic or anaerobic conditions (Faivre and Schüler, 2008, DOI: 10.1021/cr078258w; Lefevre, C.T., Menguy, N., Abreu, F., Lins, U., Posfai, M., Prozorov, T., Pignol, D., Frankel, R.B., Bazylinski, D.A., 2011. A Cultured Greigite-Producing Magnetotactic Bacterium in a Novel Group of Sulfate-Reducing Bacteria. Science 334, 1720-1723. https://doi.org/10.1126/science.1212596). Magnetosomes are subcellular organelles consisting of an iron oxide or sulfide crystal enveloped in a phospholipid bilayer membrane associated with specific proteins (Abreu et al., 2008, DOI: 10.2436/20.1501.01.46; Grünberg et al., 2004, DOI: 10.1128/AEM.70.2.1040-1050.2004). They are almost always organized in a unique chain that allows passive alignment of the bacteria along the Earth magnetic field lines (Klumpp and Faivre, 2012, doi:10.1371/journal.pone.0033562).

Magnetite magnetosomes as biogenic iron oxide nanoparticle or at least one constituent of the compositions have several benefits over their chemical counterparts, which require drastic synthesis conditions such as organic solvents, high temperature and pressure and additional steps to stabilize and vectorize the particles (Laurent et al., 2008, Chem. Rev. 2008, 108, 2064-2110). These advantages derive from their highly regulated synthesis thanks to a specific set of genes, resulting in a high crystallinity and a narrow size distribution and a ferrimagnetic stable monodomain. These 30 magnetosome-specific genes are organized in 4 operons clustered in a magnetosome island (MAI) (Proksch et al., 1995, ; DOI: 10.1063/1.113508, Ullrich et al., 2005, doi:10.1128/JB.187.21.7176-7184.2005). Their unique properties in addition to their biocompatibility (Araujo et al., 2015, doi:10.3390/md13010389; Carvallo et al., 2023, https://doi.org/10.1016/j.jmmm.2023.170726; Dasdag, 2014, DOI: 10.4172/2161-0398.1000141) make magnetosomes attractive nanostructure candidates for biomedical and biotechnological applications such as contrast agents in MRI imaging (Lisy et al., 2007, Investigative Radiology • Volume 42, Number 4, April 2007), diagnostic and detection tools or targeted drug delivery systems with functionalized membranes (Lang and Schüler, 2006, http://dx.doi.org/10.1088/0953-8984/18/38/S19; Wacker et al., 2007, doi:10.1016/j.bbrc.2007.03.156). Another promising application is cancer treatment using local moderate hyperthermia induced after intra-tumoral injection of magnetosomes and application of an external energy source such as laser (Plan Sangnier et al., 2018, https://doi.org/10.1016/j.jconre1.2018.04.036) or alternative magnetic field (Le Fèvre et al., 2017, doi: 10.7150/thno.18927).

However, the commercial use of magnetosomes first requires scaling up magnetosome production. Despite facing certain hurdles (Basit et al., 2020, https://doi.org/10.1186/s12934-020-01455-5), this objective was achieved with several MTB such as Magnetospirillum gryphiswaldense MSR1, Magnetospirillum magneticum AMB1, Magnetospirillum ME1 and Magnetovibrio blakemorei MV1, which were shown to produce more than 10 mg of magnetosomes per liter of medium under specific optimized conditions (Alphandéry, 2020, https://doi.org/10.1016/j.drudis.2020.06.010). Large-scale batch culture of magnetotactic bacteria was also reported for the strain MG-T1 in a 1000L growth medium, yielding the production of 2.6 g of magnetosomes after 4 days (Matsunaga et al., 1990, IEEE transactions on magnetics, vol. 26, no. 5, september 1990). The highest yield of magnetosome production was achieved with *Magnetospirillum gryphiswaldense* strain MSR1 using a pH-stat fed-batch strategy, resulting in 356.52 mg of dried magnetosome per liter of growth medium (Zhang et al., 2011, Applied and environmental microbiology, sept. 2011, p. 5851-5856). The second challenge lies in being able to produce pure magnetosomes. This is not a natural outcome of MTB production. Indeed, under amplification in a non-reduced or enriched non-pharmaceutical grade medium in other metals than iron, these microorganisms have been shown to yield the incorporation in magnetosomes of certain other heavy metals than iron such as manganese, zinc, cobalt and copper (Muñoz et al., 2020, https://doi.org/10.1038/s41598-020-68183-z; Tanaka et al., 2012, J. Mater. Chem., 2012, 22, 11919). Such medium should probably be avoided for pharmaceutical applications. On the one hand, it leads to a reduction in magnetosome iron content relatively to other heavy metals and to some variations in magnetosome crystalline structure, mean size, coercivity, anisotropy, heating properties (Alphandery et al., 2011, ACS Nano 2011, 5, 8, 6279-6296; Marcano et al., 2020, J. Phys. Chem. C 2020, 124, 22827-22838, 2018, J. Phys. Chem. C 2018, 122, 7541-7550), depending on the exact content of such medium. In the other hand, it contains carcinogenic, mutagenic and reprotoxic chemicals, heavy metals other than iron and products of unknown composition (Riese et al., 2020, Microb Cell Fact (2020) 19:206; Zhang et al., 2011, Applied and environmental microbiology, sept. 2011, p. 5851-5856), which are not recommended by pharmaceutical standards above certain threshold values as specified in ICH-Q3D guidelines (EMA, 2018a). In our case, we have therefore developed a minimal pharmaceutically acceptable growth medium to amplify *Magnetospirillum gryphiswaldense* MSR1 and produce highly pure magnetosomes (Berny et al., 2020, https://doi.org/10.3389/fbioe.2020.00016; Nguyen et al., 2023, Applied Microbiology and Biotechnology (2023) 107:1159-1176), hence abiding by such international recommendations.

As an extension of our previous work (Berny et al., 2020, https://doi.org/10.3389/fbioe.2020.00016; Nguyen et al., 2023, Applied Microbiology and Biotechnology (2023) 107:1159-1176), we report here the development of a stable pharmaceutical cell bank (PCB) of MSR1 magnetotactic bacteria, which can be used to produce highly pure magnetosomes "on demand". Such cell bank appears essential to reduce the costs and delays induced by launching MTB growth from a commercial cell bank (CCB), to provide a genetically stable and pure starting inoculum for reproducible production processes (Harel and Harel, 2013, Cell & Tissue Transplantation & Therapy 2013:5 1-7; Sood et al., 2011, Inoculum Preparation. Elsevier, pp. 151-164. https://doi.org/10.1016/B978-0-08-088504-9.00090-8), and to yield purer magnetosomes with the PCB than with the CCB. Here, we follow the rules of the cell banking system, which stipulate that a cell bank should first consist of cell banks of one or two levels (EMA, 2018bICH Q5D Derivation and characterisation of cell substrates used for production biotechnological/biological products - Scientific guideline [WWW Document]. European Medicines Agency. URL https://www.ema.europa.eu/en/ich-q5d-derivation-characterisation-cell-substrates-used-production-biotechnological-biological (accessed 6.1.23); Seth, 2015, Recent advances in optimal cell banking of mammalian cells for biopharmaceutical production. Pharmaceutical Bioprocessing 3, 35-43. https://doi.org/10.4155/pbp.14.46), second comply with pharmaceutical standards, e.g. in terms of pharmaceutical types of medium used for bacterial amplification (EMA, 2018c, EMA, 2018c. ICH Q7 Good manufacturing practice for active pharmaceutical ingredients - Scientific guideline [WWW Document]. European Medicines Agency. URL https://www.ema.europa.eu/en/ich-q7-good-manufacturing-practice-active-pharmaceutical-ingredients-scientific-guideline (accessed 6.1.23), 2018a, EMA, 2018a. ICH Q3D Elemental impurities - Scientific guideline [WWW Document]. European Medicines Agency. URL https://www.ema.europa.eu/en/ich-q3d-elemental-impurities-scientific-guideline (accessed 6.1.23).), third be identical to the original MTB strain, pure, and stable according to ICH-Q5D criteria (EMA, 2018b EMA, 2018b. ICH Q5D Derivation and characterisation of cell substrates used for production biotechnological/biological products - Scientific guideline [WWW Document]. European Medicines Agency. URL https://www.ema.europa.eu/en/ich-q5d-derivation-characterisation-cell-substrates-used-production-biotechnological-biological (accessed 6.1.23).; Sobolewska-Ruta and Zaleski, 2019, Sobolewska-Ruta, Advancements of Microbiology 58, 87-100. https://doi.org/10.21307/PM-2019.58.1.087), fourth be preservable over time while maintaining its stability (Kim et al., 2009, Method for the Industrial Use of <small>D</small>-Amino Acid Oxidase-Overexpressing <I>Escherichia coli</I>. Bioscience, Biotechnology, and Biochemistry 73, 299-303. https://doi.org/10.1271/bbb.80507; Stanbury et al., 2017, , Principles of Fermentation Technology (Third Edition). Butterworth-Heinemann, Oxford, pp. 335-399. https://doi.org/10.1016/B978-0-08-099953-1.00006-5). To add even more value to our PCB, we demonstrate that it can be prepared under conditions of high cell density and cryopreserved as such. This allows to launch a large scale MTB cultivation, simply by starting with an inoculum of the PCB, hence fulfilling the desired main function of the PCB (Müller et al., 2022, Seed Train Intensification Using an Ultra-High Cell Density Cell Banking Process. Processes 10, 911. https://doi.org/10.3390/pr10050911).

**MATERIALS AND METHODS:**

Strain: *Magnetospirillum gryphiswaldense* strain MSR1 (DSM6361) was purchased from Deutsche Sammlung von Mikroorganismen und Zellkulturen (Brunswick, Germany) and stored in aliquots of 1 mL at -80 °C as cryo-stored and labeled as Control Cell Bank (CCB).

**Media composition for cell bank culture and highly pure magnetosome production.** Composition of the non-reduced growth medium used to prepare the commercial cell bank (CCB): Per liter, the growth medium of the commercial cell bank used by DSMZ to cultivate Magnetospirillum gryphiswaldense MSR1 magnetotactic bacteria ("DSMZ - Medium 380," n.d.) consisted of 0.68 g of KH2PO4, 0.12 g of NaNO3, 0.37 g of L(+)-tartaric acid, 0.37 g of succinic acid, 0.05 g of Na-acetate, 0.1 g of yeast extract, 5 mL of modified Wolin mineral solution, 2 mL of Fe(III) quinate solution (0.01 M), 0.5 mL of sodium resazurin (0.1% w/v), 0.05 g of Na-thioglycolate and 1 mL of a vitamin solution containing 7 different vitamins (composition presented below). The pH of such medium was adjusted to 6.75 by addition of NaOH 1M. Per liter, the modified Wolin mineral solution consisted of 1.5 g of nitrilotriacetic acid, 3 g of $MgSO_4,7H_2O$, 0.5 g of $MnSO_4,H_2O$, 1 g of NaCl, 0.1 g of $FeSO_4.7H_2O$, 0.18 g of $CoSO_4.7H_2O$, 0.1 g of $CaCl_2.2H_2O$, 0.18 g of $ZnSO_4.7H_2O$, 0.01 g of $CuSO_4.5H_2O$, 0.02 g of $AlK(SO_4)2.12H_2O$, 0.01 g of $H_3BO_3$, 0.01 g of $Na_2MoO_4.2H_2O$, 0.03 g of $NiCl_2.6H_2O$, 0.3 mg of $Na_2SeO_3.5H_2O$ and 0.4 mg of $Na_2WO_4.2H_2O$. The pH of the modified Wolin mineral solution was firstly adjusted to 6.5 after nitrilotriacetic acid dissolution by adding first KOH 1M. Secondly, pH was adjusted to pH 7.0 after dissolution of all remaining components by adding KOH 1M. Per liter, the 7 vitamins solution consisted of 0.1 g of vitamin B12, 0.08 g of p-aminobenzoic acid, 0.02 g of D-(+)-biotin, 0.2 g of nicotinic acid, 0.1 g of Ca-pantothenate, 0.3 g of pyridoxine hydrochloride and 0.2 g of thiamine-HCl.$_2H_2O$. Per liter, the Fe(III) quinate solution 0.01 M consisted of 4.5 g of $FeCl_3.6H_2O$ and 1.9 g of quinic acid.

**Composition of the minimal growth medium used to prepare the pharmaceutical cell bank (PCB):** Per liter, the pharmaceutical cell bank growth medium used to grow Magnetospirillum gryphiswaldense MSR1 magnetotactic bacteria consisted of 2.6 g of Na-lactate, 0.4 g of $NH_4Cl$, 0.1 g of $MgSO_4,7H_2O$, 0.5 g of $K_2HPO_4$, 5 mL of ferric citrate solution, 0.1 mL of a vitamin solution containing 9 different vitamins and 0.5 mL of a mineral elixir solution. Per liter, the ferric citrate solution consisted of 5.259 g of ferric citrate. Per liter, the vitamin solution containing 9 different vitamins consisted of 0.002 g of biotin, 0.4 g of Ca-pantothenate, 0.002 g of folic acid, 2 g of inositol, 0.4 g of nicotinic acid, 0.2 g of p-aminobenzoic acid, 0.4 g of pyridoxine HCl, 0.2 g of riboflavin and 0.4 g of thiamine HCl. Per liter, the mineral elixir solution consisted of 39.74 g of $CaCl_2,2H_2O$ and 1 g of $FeSO_4,7H_2O$. All chemicals were purchased in pharmaceutical grade from Merck (Darmstadt, Germany).

**Composition of the minimal growth medium**

**used to store the PCB.** The medium used to store the PCB consisted of the minimal growth medium describe in the above section supplemented with 5 % (w/v) of dimethylsulfoxyde (DMSO).

**Composition of the minimal pre-growth medium used for the pre-growth steps in 7.5 L bioreactor culture.** The pre-growth minimal medium composition is the same as the PCB minimal medium composition except that it does not contain iron citrate. Per liter, the pre-growth minimal medium consisted of 2.6 g of Na-lactate, 0.4 g of $NH_4Cl$, 0.1 g of $MgSO_4,7H_2O$, 0.5 g of $K_2HPO_4$, 0.1 mL of a vitamin solution containing 9 different vitamins and 0.5 mL of a mineral elixir solution. Per liter, the vitamin solution containing 9 different vitamins consisted of 0.002 g of biotin, 0.4 g of Ca-pantothenate, 0.002 g of folic acid, 2 g of inositol, 0.4 g of nicotinic acid, 0.2 g of p-aminobenzoic acid, 0.4 g of pyridoxine HCl, 0.2 g of riboflavin and 0.4 g of thiamine HCl. Per liter, the mineral elixir solution consisted of 39.74 g of $CaCl_2,2H_2O$ and 1 g of $FeSO_4,7H_2O$. All chemicals were purchased in pharmaceutical grade from Merck (Darmstadt, Germany).

**Composition of the minimal growth medium used for the growth step in 7.5 L bioreactor culture.** Per liter, the growth minimal medium consisted of 1.3 g of Na-lactate, 0.223 g of $NH_4Cl$, 0.027 g of $MgSO_4.7H_2O$, 0.067 g of $K_2HPO_4$, 0.067 mL of 9 vitamins solution and 0.08 mL of mineral elixir solution. Per liter, the vitamin solution containing 9 different vitamins consisted of 0.002 g of biotin, 0.4 g of Ca-pantothenate, 0.002 g of folic acid, 2 g of inositol, 0.4 g of nicotinic acid, 0.2 g of p-aminobenzoic acid, 0.4 g of pyridoxine HCl, 0.2 g of riboflavin and 0.4 g of thiamine HCl. Per liter, the mineral elixir solution consisted of 39.74 g of $CaCl_2.2H_2O$ and 1 g of $FeSO_4.7H_2O$. All chemicals were purchased in pharmaceutical grade from Merck (Darmstadt, Germany).

**Composition of the minimal feed medium used for the growth step in 7.5 L bioreactor culture.** Per liter, the feed minimal medium consisted of 100 g of lactic acid, 4.77 g of $NH_3$, 2.4 g of $MgSO_4.7H_2O$, 6 g of $K_2HPO_4$, 2 g of $FeCl_3.6H_2O$, 1 mL of 9 vitamins solution and 7 mL of mineral elixir solution. Per liter, the vitamin solution containing 9 different vitamins consisted of 0.002 g of biotin, 0.4 g of Ca-pantothenate, 0.002 g of folic acid, 2 g of inositol, 0.4 g of nicotinic acid, 0.2 g of p-aminobenzoic acid, 0.4 g of pyridoxine HCl, 0.2 g of riboflavin and 0.4 g of thiamine HCl. Per liter, the mineral elixir solution consisted of 39.74 g of $CaCl_2.2H_2O$ and 1 g of $FeSO_4.7H_2O$. All chemicals were purchased in pharmaceutical grade from Merck (Darmstadt, Germany).

**Conditions of cultivation of magnetotactic bacteria and magnetosome preparation. First culture condition: High cell load pharmaceutical cell bank production in 3L bioreactor batch culture.** The PCB was produced in batch culture in a 3L bioreactor filled with 0.5 L of PCB minimal growth medium. Cultures were performed in triplicate. Before inoculation with 5 mL of CCB cryo-stock, the medium was sparged with a gas mixture of $O_2/N_2$ (2/98 %) for 20 minutes and the temperature was pre-adjusted at 29.5°C. Batch cultivation was conducted for 6 days at 29.5°C with a fixed agitation of 110 rpm. The $pO_2$ concentration and pH were monitored throughout cultivation to maintain microaerobic conditions. The pO2 concentration was maintained below 2 % vol. by bubbling $O_2/N_2$ (2/98 %) gas if necessary.

During the first phase of culture, the pH was stable at around 7 and the oxygen partial pressure was maintained at around 20 mbar (~2% vol.) to promote cell growth without compromising magnetosome synthesis by sparging the culture every 24 hours (Heyen and Schüler, 2003, Growth and magnetosome formation by microaerophilic Magnetospirillum strains in an oxygen-controlled fermentor. Appl Microbiol Biotechnol 61, 536-544. https://doi.org/10.1007/s00253-002-1219-x) and prevent oxygen accumulation in the large headspace volume (1/5 v/v) that could occur while stirring the growth medium due to gas-liquid interface transfer under low bacterial oxygen uptake (Seidel et al., 2021, Oxygen Mass Transfer in Biopharmaceutical Processes: Numerical and Experimental Approaches. Chemie Ingenieur Technik 93, 42-61. https://doi.org/10.1002/cite.202000179). During the second phase of cultivation, the oxygen partial pressure decreased to 0.25 % sat. from 118 h to the end of culture, indicating that the biomass had reached a point where the oxygen uptake rate became higher than the oxygen transfer rate. Concomitantly, the pH increased rapidly, indicating a rapid biomass growth rate. After 142 hours of cultivation, a slowing of the pH increase indicated the end of the exponential growth phase. Previous test cultures for cell bank production with biomass sampling confirmed the relationship between lactate uptake, pH evolution and growth. The final $OD_{565}$ reached 0.279 with a generation time of 19.93 h. Using the Petroff hemocytometer, a correspondence factor of 9.108 cells/mL for an $OD_{565}$ of 1 gives a final bacterial concentration of 2.5.108 cells/mL. After cultivation, MSR1 cells were centrifuged at 4000 rpm for 45 min (GR4i Jouan Centrifuge, Thermo). Pellet was resuspended in fresh PCB storage minimal medium supplemented with DMSO to reach a concentration of 9.108 cells/mL, i.e. ~ $OD_{565}$ of 1 according to a high cell density cryopreservation strategy. Cryotubes were labeled as PCB and frozen at -80°C prior their used for PCB characterization experiments and as starting inoculum for production of highly pure magnetosomes during 7.5L fed-batch cultures experiments.

**Second culture condition: Subculturing experiment in 150 mL bottle.** 1 mL of PCB were subcultured 24 times in 50 mL of PCB minimal medium in 150 mL bottles at 29.5°C and 110 rpm (Thermo Scientific, MaxQ 2000) for 3 to 5 days. After each subculture, the cell bank medium was discarded and replaced by fresh medium to reach a concentration of 9.107 cells/mL (~ $OD_{565}$ of 0.1). This experiment was performed in sextuplet and used to characterize PCB stability over several bacterial generations. **Third culture condition: MTB cultivation on agar plates.** A volume of 1 mL of CCB and PCB bacterial suspensions were deposited on top of LB agar plates, which were then incubated at 29.5 and 37°C for 3 days. LB agar was prepared using 20 g of LB broth and 15 g of agar per liter and served to detect bacterial contaminants that if present should develop as observable colonies of microorganisms. This experiment was used to characterize the purity of the PCB.

**Fourth culture condition: Fed-batch culture in 7.5L bioreactor using minimal medium to produce highly pure magnetosomes starting from the PCB.** During the first pre-growth step (preculture 1, PC1), 450 $\mu$L of unfrozen CCB, of unfrozen PCB or of 16 months stored PCB were grown in 250 mL of pre-growth minimal medium in 500 mL square bottles and incubated for 8 days at 29.5 °C. On the last day, PC1 were agitated in an orbital shaker (Thermo Scientific, MaxQ 2000) operating at 110 rpm.

During the second pre-growth step (preculture 2, PC2), 500 mL of PC1 were transferred in a 3L bioreactor (Applikon) filled with 1.5 L of pre-growth minimal medium and incubated for 2 days at 29.5 °C under agitation at 110 rpm. These pre-growth steps were carried out in the absence of iron and in the presence of an initial quantity of oxygen until depletion to microaerobic conditions. During the pH-stat fed-batch culture inspired from (Zhang et al., 2011, Semicontinuous Culture of Magnetospirillum gryphiswaldense MSR-1 Cells in an Autofermentor by Nutrient-Balanced and Isosmotic Feeding Strategies. Appl Environ Microbiol 77, 5851-5856. https://doi.org/10.1128/AEM.05962-11), a volume of PC2 was transferred in a 7.5L automatized bioreactor (Applikon) containing 4L of minimal growth medium to achieve an initial OD565 of 0.11 $\pm$ 0.01. The pOz was kept below 0.1 % vol. by controlling airflow and stirring rates between 20 to 107 mL/min and 150 to 250 rpm respectively after initial oxygen depletion during the first 12 hours of culture. The temperature was maintained at 29.5°C and pH was kept at 6.84 by addition of an acidic feed medium (pH = 2.99 $\pm$ 0.02). All these parameters were adjusted and monitored using an ez-Control controller (Applikon) and BioXpert software (Applikon). This growth step was carried out under microaerobic conditions in presence of iron. After cultivation ended at 140 h, the bacterial culture was washed and concentrated using a tangential filter column (MiniKros Sampler, PES 0.2 $\mu$m) to a volume of 1 L and stored at -80°C before magnetosome extraction. Cultures were performed in duplicate. Measurement of cell density ($OD_{565}$), dry cell weight and magnetic response were performed on bacterial samples harvested at 0, 72, 96, 120 and 140 h of cultivation. This experiment was used to characterize PCB stability over long term storage at -80°C and to compare PCB and CCB performances. In addition, the purity of magnetosomes produced with the PCB was investigated.

**Magnetosomes extraction.** Bacterial concentrates of 7.5L PCB fed-batch cultures were thawed and diluted with deionized water to reach a final OD565 of 20. Cells were lysed in KOH 2 M agitated at 150 rpm with a stirring blade for 1 h at 80°C. Lysate was then placed against a Neodymium magnet overnight to separate magnetosomes from the supernatant containing cell debris. The liquid phase was gently removed using a vacuum pump. Magnetosomes were washed 2 times with 10X phosphate-buffered saline (PBS) and then 3 times with deionized water by magnetic selection against a Neodymium magnet. Subsequently, magnetosomes were collected in 50 ml conical tubes and centrifuged at 4000 g for 45 min at 6°C (Eppendorf, Centrifuge 5810 R) and the supernatant was disposed of. Conical tubes were kept at -80°C for 48 h. Extracted frozen magnetosomes were lyophilized at -50°C and 0.003 mbar for 48 h using a freeze drier (Labconco, Free Zone® 70020 2.5 L). Freeze-dried magnetosomes were ground to obtain a raw powder and finally weighed to determine the yield in dry weight of magnetosomes for each culture.

**Cell bank characterization. Measurement of cell density ($OD_{565}$), dry cell weight, cell numeration and magnetic response.** Optical density at 565 nm ($OD_{565}$) of a suspension containing MTB mixed in 1 mL was measured using an UV-visible spectrophotometer (Secomam, UviLine 9400). To determine dry cell weight (DCW), 20 mL of bacterial sample from MSR-1 culture were centrifuged at 4000 rpm for 35 min at 4°C (Eppendorf, Centrifuge 5810 R). Pellets were kept at -80°C for 48 h and lyophilized at -50°C and 0.003 mbar for 48h using a freeze drier (Labconco, Free Zone® 70020 2.5 L). Dry pellets were weighed to determine DCW. Bacterial samples, after dilution to an $OD_{565}$ of 0.5, were observed under an optical microscope (Zeiss, Primo Vert) to determine bacterial morphology, cell concentration using a Petroff hemocytometer and magnetic response as described by Berny et al. (2020).

**Flow cytometry.** After thawing, CCB and PCB samples were centrifugated at 12 000 g for 10 min (Eppendorf, miniSpin plus). A first sample of CCB and PCB was resuspended in phosphate-buffered saline (PBS) while a second was resuspended in ethanol 70% (v/v) and heated at 50°C for 1 h. Pellets were subsequently recovered and resuspended in phosphate-buffered saline (PBS). Bacteria were stained with propidium iodide (PI) at a concentration of 100 ng/L and analyzed using a CytoFLEX (Beckman Coulter). Diluted samples were excited using a 488 nm laser and fluorescence for forward scatter (FSC) and side scatter (SSC) signals was measured, which reflect the distribution in bacterial size and granulometry, respectively. Cell viability was assessed using PI after excitation at 488 nm and fluorescence was detected using a 610/20 BP filter (FL8).

**Transmission electron microscopy.** Bacterial sample was diluted to an $OD_{565}$ of 0.5 and centrifugated at 12 000 g for 10 min (Eppendorf, miniSpin plus). The cell pellet was washed 3 times with deionized water and kept at -80°C. After thawing, 5 $\mu$L of the bacterial suspension was deposited on a carbon-coated copper grid (300 mesh grid, Oxford Instruments) and dried for 3 h at room temperature. Grids were subsequently observed using a transmission electron microscope JEOL JEM-2100 operated at 200 kV. Magnetosome number per bacterium and magnetosome core size were measured on 100 bacteria and 250 magnetosomes respectively using ImageJ software.

**PCR methods For MSR1 strain identification,** 5 mL of PCB were centrifuged at 4000 rpm for 30 min at 4°C (Eppendorf, Centrifuge 5810 R) and suspended in 1 mL of deionized water. The suspension was heated at 95°C for 20 min to obtain the DNA extract. PCR mix consists of 50 $\mu$L of PCR buffer, dNTP 200 $\mu$M, primers 0.2 $\mu$M, MgCl2 1.5 mM and 0.2 $\mu$L of TaQ pol (Platinium Taq DNA Polymerase, Invitrogen). Selected P209 and P326 primer couples adapted from Guo et al. (2011) were designed based on the MSR1 16S ribosomal sequence (accession number: CP027526.1). P209 primers couple consist of a 23 dNTP forward primer with a Tm of 68°C (5' GTACCGTCATCATCATCGTCCCC 3') and a 20 dNTP reverse primer with a Tm of 66.4°C (5'GTGAGGTAACGGCTCACCAA 3'). P326 primers couple consist of a 20 dNTP forward primer with a Tm of 66.5°C (5' GCGATTCCGACTTCATGCAC 3') and a 20 dNTP reverse primer with a Tm of 65°C (5' TGGTGACTTGTCTTCGGACG 3'). Amplification was performed using a thermocycler GeneAmp® PCR System 9700 (Applied Biosystems) by mixing 8.7 $\mu$L of PCR mix, 5 $\mu$L of PCB DNA extract and 40.3 $\mu$L of sterile deionized water with the following heating sequences: 1 min-94°C, 1 min-68.4°C and 2 min-72°C for 35 cycles. Gel migration was performed at 120 V for 55 min on agarose gel 2% composed of 100 mL TAE buffer 1X, 2 g agarose and 10 $\mu$E SYBR Safe (Invitrogen). Gel revelation was performed using a BIO-RAD Gel Doc™ EZ imager. Negative and positive controls consist of sterile deionized water and CCB DNA (DSMZ 6361) respectively.

**Genomic variant analysis** A variant analysis was performed between genomes from CCB and PCB. CCB DNA was purchased from DSMZ while PCB DNA was extracted using a MasterPure Gram Positive DNA Purification Kit (Biosearch Technologies, LGC). DNA sequencing and variant analysis were performed by the Next Generation Sequencing (NGS) Core Facility. Samples were prepared in accordance with the TruSeq genomic protocol using the sequencing kit NextSeq 500/550 High Output Kit v2 (Illumina). Sequencing was performed using a NextSeq NB552053 system (Illumina) with 50-34 sequencing cycles (paired-end). Collected data were sorted using bcl2fastq2-2.18.12 and adapter trimming was performed with Cutadapt 3.2. Quality control was carried out using FastQC v0.11.5. Mapping was performed using BWA 0.7.17-r1188 software with Magnetospirillum gryphiswaldense MSR1 genome as reference (accession number: CP027526.1). Genomic variations were detected using Freebayes v1.1.0 against the reference genome presented above. For each PCB sample, genomic variations already present in CCB genome sequencing were removed from the variations found. To assess the presence of a genetic variations in most bacteria, a filter on the allelic frequency of variations with a minimum coverage of 10 reads was applied with a level of 100 and 80 %.

**Total intracellular iron assay** Total intracellular iron concentration was determined using an iron destructive colorimetric assay. Bacterial samples at 0, 72, 96, 120 and 140 h of cultivation were first concentrated or diluted to an $OD_{565}$ of 2 in a final volume of 2 mL with deionized water. Pellets were recovered after centrifugation at 12 000 g for 10 min (Eppendorf, miniSpin plus), washed 3 times with deionized water and kept at -80°C. After thawing, cells were resuspended in 375 $\mu$L of HCl 12 N and placed at 50°C overnight in a heating block (Type OBT2, Grant Instruments). A volume of 125 $\mu$L of $HNO_3$ 11 N was added to the cell lysate. After 24 hours at room temperature, samples were mixed with 500 $\mu$L of deionized water. 50 $\mu$L of the sample was mixed with 50 $\mu$L of $H_2O_2$ 20% (v/v), vortexed. After 15 min, 850 $\mu$L of deionized water were added and then 50 $\mu$L of KSCN 2 M. Absorbance was measured at 476 nm using a spectrophotometer (Secomam, UviLine 9400). Iron concentrations were determined in accordance with a standard calibration curve (0.41-3.31 mg.L$^{-1}$).

**Magnetosomes purity assessment by inductively coupled plasma mass spectrometry (ICP-MS) measurement** 2 to 5 mg of extracted magnetosomes were dissolved for 48h at room temperature with a mix of 70 $\mu$L of HCl 37% (w/v) and 572 $\mu$L of $HNO_3$ 70% (w/v). Samples were then diluted in deionized water to a final volume of 10 mL. Concentrations of elements classified in the ICH-Q3D guidelines, i.e. As, Cd, Hg, Pb, Co, Ni, V, Ag, Au, Ir, Os, Pd, Pt, Rh, Ru, Se, Tl, Ba, Cr, Cu, Li, Mo, Sb, Sn, Al, B, Ca, Fe, K, Mg, Mn, Na, W and Zn, were measured using an inductively coupled plasma-mass spectrometer (Agilent 7900 Quadrupole ICP-MS).

## RESULTS

Here, we present the conditions of preparation of a pharmaceutical cell bank (PCB) which can be used first to store MSR1 magnetotactic bacteria while preserving the properties of these bacteria and second to launch runs of production of pharmaceutical grade magnetosomes. In particular, we demonstrate that the identity, purity and the essential aspects of stability of this strain are preserved during storage. Preserved identity, purity, and stability of MSR1 magnetotactic bacteria prepared in a minimal growth medium to yield a pharmaceutically acceptable cell bank. The CCB (commercial cell bank) was prepared by amplifying MSR1 magnetotactic bacteria in a complete growth medium ("DSMZ - Medium 380," n.d.) which contains potentially toxic heavy metals and CMR compounds, making the CCB difficult to consider as starting material for bacterial amplifications within a pharmaceutical setting. We therefore prepared a PCB (pharmaceutical cell bank) starting from the CCB, by amplifying MSR1 magnetotactic bacteria in a growth medium devoid of toxic compounds (Berny et al., 2020, A Method for Producing Highly Pure Magnetosomes in Large Quantity for Medical Applications Using Magnetospirillum gryphiswaldense MSR-1 Magnetotactic Bacteria Amplified in Minimal Growth Media. Front. Bioeng. Biotechnol. 8, 16. https://doi.org/10.3389/fbioe.2020.00016; Nguyen et al., 2023, Non-pyrogenic highly pure magnetosomes for efficient hyperthermia treatment of prostate cancer. Appl Microbiol Biotechnol 107, 1159-1176. https://doi.org/10.1007/s00253-022-12247-9). When we undertook such modifications, which led to the transition from the CCB to PCB, we observed that the identity of the MSR1 bacterial strain was preserved, i.e. bacteria contained in both banks displayed the same characteristics and produced the same desired end-product, i.e. magnetosomes. The preserved identity of magnetotactic bacteria in the PCB is highlighted by the following observations. First, bacteria display a similar average cell length in the two banks, i.e. $1.20 \pm 0.25$ nm for CCB and $1.21 \pm 0.25$ nm for PCB, with no significative difference in cell size distribution, i.e. the Mann-Whitney test carried out on such data led to a p value of 0.5823. Second, the bacterial morphology appears to be unchanged between both banks, which is revealed by similar cytometry spectra for CCB and PCB bacteria, and the observation of a spirillum morphology in the TEM images of typical magnetotactic bacteria belonging to both banks. Third, the number of magnetosomes per cell, $N_{mag}$, and the magnetosome mean size, $S_{mag}$, evolved from $N_{mag} = 21.19 \pm 5.55$ and $S_{mag} = 39.99 \pm 9.59$ nm for CCB, to $N_{mag} = 14.44 \pm 6.28$ and $S_{mag} = 37.73 \pm 6.71$ nm for PCB, indicating that despite a decrease in the number of magnetosomes per cell between CCB and PCB, the size of the magnetosomes remained similar in the two banks. This further supports the idea of a preserved strain identity. Fourth, comparison of the bacterial genomes originating from the two banks, which is carried out through PCR amplification of two representative fragments of sizes 209 and 236 pb from MSR1 16S ribosomal sequence, revealed the same bands after gel migration, which is indicative of a similar genomic identity for the two banks. The absence of contaminant in the PCB was demonstrated by growing PCB bacteria in LB agar plates containing a growth medium enabling bacterial growth with a broad-spectrum. No colony was observed after 48 h of incubation at a temperature of 29.5°C and 37°C, revealing the purity of the PCB.

We then studied the stability of the PCB, which is an essential aspect to be able to use this bank in the long term. First, we studied if the genetic region encoding the proteins responsible for magnetosome production was stable. For that, the genomes of three different preparations of PCB (PCB1, PCB2, PCB3) were compared with the well-known genome of MSR1 (CP027526.1). Although 27, 38 and 82 genomic variations were detected for PCB1, PCB2 and PCB3, they were not considered as significantly affecting the bacterial genomic stability. Indeed, on the one hand no genomic variation was found to occur in PCB with a 100% frequency. On the other hand, genomic mutations occurring with frequencies of 94% and 85% are associated first with single nucleotide polymorphism in the gene ptsI 2 encoding a phosphoenolpyruvate protein phosphotransferase resulting in a missense variation, and second with a variation in the gene flhF which is involved in flagellar synthesis. Therefore, the integrity of the genomic region involved in magnetosome mineralization, i.e. the MAI, was fully preserved. The results regarding PCB identity, purity and genomic stability assessment compared to CCB are summarized in Table 1. After establishing the genomic stability of the PCB, we examined if the PCB remained stable after a thermal treatment, which could ensure its long-term preservation. The PCB was stored at -80°C for 4 months, and after storage, bacterial viability was determined by staining the cells with iodide propidium (IP) and determining cell size, granularity and fluorescence using a cytometer technique. 78.4% of cells were observed to be alive, which represents a sufficiently large number to enable the launch of a run of bacterial growth leading to magnetosome production. In addition, all cells (dead and alive) displayed a similar size and granularity, revealing that the integrity of the strain was not affected by their thermal treatment.

Here, we also show that the PCB creates the conditions for the acclimation of MSR1 magnetotactic bacteria to a minimal growth medium, further enabling bacterial amplification in such medium. For that, we have cultivated unfrozen MSR1 magnetotactic bacteria issued from the PCB and CCB. At first sight, the growth of the two types of bacteria, which was carried out in 7.5L bioreactor using a pH-stat fed-batch method, displayed similar trends, i.e. i) similar bacterial optical density measured at 565 nm (OD565), biomass (DCW), generation time (GT), total quantity of iron internalized in bacteria (TQIIB), volume of feed injected in the culture (VFIC), following 140 hours of growth, for unfrozen PCB (OD565 = $2.83 \pm 0.22$, DCW = $5.28 \pm 0.39$ g, GT = $17.96 \pm 1.55$ h, TQIIB = $8.10 \pm 1.64$ mg/L and VFIC = $212 \pm 26$ mL) and unfrozen CCB (OD565 = $3.90 \pm 0.28$, DCW = $6.76 \pm 0.25$ g, GT = $18.26 \pm 0.26$ h, TQIIB = $9.53 \pm 0.73$ mg/L and VFIC = $235 \pm 6$ mL) and ii) similar increases of the biomass and quantity of iron internalized in bacteria during bacterial growth. However, interestingly, magnetosome production at 140 hours was significantly larger for the PCB than for the CCB, i.e. the magnetosome volumetric yield (MVY), total intracellular iron as magnetosomes (TIIM), and number of magnetosomes per cells (NMC), display higher values for PCB (MVY = $4.98 \pm 1.13$ mg/L, TIIM = $44.38 \pm 1.14$ %, NMC = $24.15 \pm 13.18$) than for CCB (MVY = $1.45 \pm 0.13$ mg/L, TIIM = $9.26 \pm 0.49$ %, NMC = $12.11 \pm 4.79$), without leading to a significant change in magnetosome sizes, i.e. SM = $36.94 \pm 6.07$ for PCB and SM = $35.16 \pm 6.94$ for CCB. The magnetosome size, which is essential to yield magnetosome ferrimagnetic properties and chain arrangement, is preserved in the PCB. Furthermore, the acclimatation of the MSR1 strain to the minimal growth medium that the PCB enables to achieve, leads to a higher conversion rate of the internalized iron into magnetosomes and to 3.4 times larger magnetosome production in the PCB than in the CCB. In other words, without being acclimated to the minimal growth medium, the MSR-1 strain leads to a less efficient transformation rate of internalized iron into magnetosomes.

The effect of bacterial storage after 16 months at -80°C on the stability of the MSR-1 strain is also examined by comparing the parameters of bacterial growth and magnetosome production between the unfrozen and frozen MSR1 strains originating from PCB. These parameters appear to be very similar for both conditions, indicating that the capacity of MSR-1 bacteria to grow and produce magnetosomes under our well-established conditions involving pre-growth and growth steps are preserved during storage.

Finally, we determined whether the stability of the MSR1 strain in PCB is maintained over many bacterial generations of 100, which is largely sufficient to allow magnetosome production using our three-step bacterial amplification method involving typically 16 to 17 generations. After having cultivated bacteria belonging to the PCB over 100 generations in the presence of frequent exposure to high oxygen concentration, we observed that the essential parameters characterizing the bacteria and magnetosomes remained unchanged, i.e. the bacterial cell length is 1-1.5 $\mu$m, a maximum magnetic response is observed in all bacteria, the number of magnetosomes per cell remains between $8.90 \pm 4.02$ and $19.78 \pm 6.60$, that is to say there are enough magnetosomes to ensure polar magnetotaxis and the magnetosome mean size that has slightly decreased over 100 generations remains sufficiently large (> 20 nm) to preserve the most essential magnetosome magnetic feature, i.e. its ferrimagnetic property. Therefore, despite frequent exposures of MSR1 bacteria to high oxygen concentrations during growth, magnetotactic bacteria originating from PCB maintain a persistent capacity of being able to produce magnetosomes over 100 generations of MTB amplifications. It allows us to consider the use of PCB for bacterial amplification and associated magnetosome mass-production using large fermentation volumes requiring an important number of bacterial generations.

Highly pure magnetosomes originating from PCB. We then studied the purity level of magnetosomes produced with PCB in a reduced growth medium. Magnetosomes issued from PCB possess an iron purity level, defined as $\Sigma$Mi/MFe, where Mi and MFe are the masses of all heavy metals comprised in magnetosomes and of iron, respectively, which is $\Sigma$Mi/MFe = 99.935%Fe and ~ 93.6%Fe of iron using the medium of Zhang et al. (2011), https://doi.org/10.1128/AEM.05962-11. This clearly indicates a large improvement in magnetosome purity achieved with the PCB compared with magnetosomes produced in non-reduced growth media (Berny et al., 2020, https://doi.org/10.3389/fbioe.2020.00016).

Furthermore, considering the less biocompatible metallic compounds in class I according to ICH-Q3D standard, their concentration in magnetosomes is very low, i.e. the average concentration of As, Cd, Hg and Pb was $4.72 \pm 0.39 \ 10^{-5}$ g/gFe. Considering parenteral administration of magnetosomes for cancer treatment, the recommended cumulative maximal concentration of class I metals is 25 $\mu$g/day (EMA, 2018a). When we apply this rule to magnetosomes, it results in a maximum daily injectable therapeutic dose of 530 mg in iron of magnetosomes. For a typical dose of 50 mg in iron of magnetosomes, (side note) that we plan to administer in a localized prostate tumor of Gleason 7 of typical diameter 1 to 2 cm, the average quantity class I metals contained in magnetosomes in average is $2.36 \pm 0.20 \ \mu$g, which is more than 10 times below the maximum cumulative daily injectable dose of these metals of 25 $\mu$g.

## DISCUSSION

Here, we have determined conditions for producing a MSR1 pharmaceutical cell bank, i.e. a cell bank containing magnetotactic bacteria amplified under pharmaceutically acceptable conditions with preserved identity, purity and stability compared with the commercial cell bank (CCB), leading to the production of highly pure magnetosomes, i.e. essentially devoid of other heavy toxic metals than iron listed in the ICH-Q3D standards

To achieve this aim, we used a series of optimal growth conditions during the growth. First, during the growth step, we bubbled oxygen in the growth media at 20 mbar, i.e. ~ 2 % vol., to favor MSR1 growth up while avoiding exceeding these values above for which magnetosome production would be prevented (Heyen and Schüler, 2003; Riese et al., 2020). Second, we fixed the iron concentration at 100 $\mu$M to maximize biomass and magnetosome production (Schüler and Baeuerlein, 1996, Iron-limited growth and kinetics of iron uptake in Magnetospirillum gryphiswaldense. Arch Microbiol 166, 301-307. https://doi.org/10.1007/s002030050387). Third, we recovered cells in an optimal physiologic state at the end of the growth phase at 142 hours, i.e. after the MTB growth phase would stop amplifying. Fourth, we removed from the standard MSR1 magnetotactic bacterium growth medium the compounds that are considered as potentially toxic according to ICH-Q3D standards (As, Cd, Hg, Pb, Co, Ni, V, Ag, Au, Ir, Os, Pd, Pt, Rh, Ru, Se, Tl, Ba, Cr, Cu, Li, Mo, Sb, and Sn) to end up with a minimal growth medium essentially containing Cl, Mg, K, Fe and Ca that we successfully used to amplify MSR1 magnetotactic bacteria.

Interestingly, the method that we employed enabled to avoid mutations in the MAI locus responsible for magnetosome formation in MSR1 (Uebe and Schüler, 2016, Magnetosome biogenesis in magnetotactic bacteria. Nat Rev Microbiol 14, 621-637. https://doi.org/10.1038/nrmicro.2016.99), hence enabling to overcome the difficulties associated with the frequent mutations of MSR1 associated with oxidative stress (Popa et al., 2009, Effect of oxidative stress on the growth of magnetic particles in Magnetospirillum magneticum. Int Microbiol 12, 49-57; Ullrich et al., 2005, Genomic Region of Magnetospirillum gryphiswaldense Comprises a Magnetosome Island Which Undergoes Frequent Rearrangements during Stationary Growth. J Bacteriol 187, 7176-7184. https://doi.org/10.1128/JB.187.21.7176-7184.2005). Our well-controlled conditions of amplification and low medium oxygenation may favor the decrease of reactive oxygen species and participate in the reduction of oxidative stress via production of magnetosomes which are known to be able to decrease and eliminate reactive oxygen species (Guo et al., 2012, A novel rapid and continuous procedure for large-scale purification of magnetosomes from Magnetospirillum gryphiswaldense. Appl Microbiol Biotechnol 90, 1277-1283. https://doi.org/10.1007/s00253-011-3189-3).

Once bacterial amplification conditions were established, as described above, it was ensured that the MSR1 strain prepared under these conditions could be cryo-preserved at a bacterial concentration that is sufficiently large to allow the launch of batches of magnetosome production (Frahm, 2014, Animal Cell Biotechnology: Methods and Protocols, Methods in Molecular Biology. Humana Press, Totowa, NJ, pp. 355-367. https://doi.org/10.1007/978-1-62703-733-4_22; Sood et al., 2011, https://doi.org/10.1016/B978-0-08-088504-9.00090-8). For that, we have established successful cryo-preservation conditions of the MSR1 strain. First, bacteria were harvested by centrifugating before entering the lag phase to keep these bacteria in an optimal physiological state and to avoid any delay after inoculation in the production process (Hornbaek et al., 2004, The effect of inoculum age and solid versus liquid propagation on inoculum quality of an industrial Bacillus licheniformis strain. FEMS Microbiol Lett 236, 145-151. https://doi.org/10.1016/j.femsle.2004.05.035). Second, MSR1 bacteria were resuspended in pharmaceutical cell bank storage medium supplemented with DMSO 5 % to avoid inhibition and stress from a worn and depleted medium (Yamamoto et al., 1993, Reduction in the length of the lag phase of l-lactate fermentation by the use of inocula from electrodialysis seed cultures. Journal of Fermentation and Bioengineering 76, 151-152. https://doi.org/10.1016/0922-338X(93)90074-I). Third, the cell density of the inoculum was kept sufficiently large to enable bacterial growth, i.e. typically 3 and 10 % of the culture volume (Mulder et al., 2013, Critical aspects to be considered prior to large-scale production of peptides. Curr Protein Pept Sci 14, 556-567. https://doi.org/10.2174/13892037113149990071; Müller et al., 2022, Seed Train Intensification Using an Ultra-High Cell Density Cell Banking Process. Processes 10, 911. https://doi.org/10.3390/pr10050911 ) corresponding in our case to an $OD_{565}$ of 1 of the inoculum and to 9.108 MTB per mL, which corresponds to a 10 times larger bacterial concentration compared with the concentration of the commonly starting inoculum used in commercial cell banks (DSMZ 6361). Such high concentration enables to reduce the duration of the seed train before initiating fed-batch magnetosome production culture, the cost of production, and the number of bacteria generations during growth. It therefore strengthens the stability of the strain and contributes to prevent the apparition of contamination (Whitford, 2020, Bioprocess intensification: aspirations and achievements. Biotechniques 69, 84-87. https://doi.org/10.2144/btn-2020-0072). Furthermore, we have developed a PCB according to the recommendations of the ICH-Q5D guidelines. On the one hand, it contains a sufficiently large number of bacteria to initiate several batches of bacteria amplification and magnetosome production. On the other hand, it is preserved at -80 °C in the presence of a cryo-protectant (5 % of DMSO). The quality of the PCB is highlighted by measuring the MTB mortality rate 4 months following its cryo-preservation, which is below 25%. We have prevented MTB death by controlling the parameters of the steps of bacterial launch with the PCB. Bacteria thawing which can cause oxidative and hyperosmotic stress while dehydrating the bacteria and forming intracellular ice crystals (Sleight et al., 2006), was carried out over 30 min by letting the temperature slowly increase from -80°C to room temperature to avoid as much as possible a thermal chock resulting from a too fast temperature gradient applied on bacteria. Bacteria centrifugation, which is known to cause shear stress, damage cells as shown in populations of Staphylococcus aureus (Peterson et al., 2012, Bacterial cell surface damage due to centrifugal compaction. Appl Environ Microbiol 78, 120-125. https://doi.org/10.1128/AEM.06780-11) and E. coli (Pembrey et al., 1999, Cell Surface Analysis Techniques: What Do Cell Preparation Protocols Do to Cell Surface Properties? Appl Environ Microbiol 65, 2877-2894.), was carried out at a relatively low speed of 4000 rpm to prevent as much as possible cellular death. The cell load which can amplify the effects of thawing and centrifugation on cell viability when it is too high, as reported with erythrocytes (Mazur and Cole, 1985, Influence of cell concentration on the contribution of unfrozen fraction and salt concentration to the survival of slowly frozen human erythrocytes. Cryobiology 22, 509-536. https://doi.org/10.1016/0011-2240(85)90029-x) was purposedly kept below a too high value of an $OD_{565}$ of 5.

Finally, while the properties of MTBs and their magnetosomes can change depending on the used culture conditions, in particular by varying oxygenation or composition of the culture media (Berny et al., 2020, ; https://doi.org/10.3389/fbioe.2020.00016, Nguyen et al., 2023, https://doi.org/10.1007/s00253-022-12247-9, Pembrey et al., 1999, Cell Surface Analysis Techniques: What Do Cell Preparation Protocols Do to Cell Surface Properties? Appl Environ Microbiol 65, 2877-2894), we determined that the PCB preserves MSR1 purity, identity, characterized by a magnetosome cuboctahedra geometry, a sufficiently magnetosome large size (> 20 nm) to yield magnetosome ferrimagnetic properties, and a magnetosome arrangement in chain . In addition, the stability of the strain issued from the PCB was studied by amplifying MTB over 100 generations, inducing several cycles of cytokinesis that are believed to be responsible for magnetosome dissemination (Katzmann et al., 2011, https://doi.org/10.1111/j.1365-2958.2011.07874.x; Staniland et al., 2010, https://doi.org/10.1002/jobm.200900408) not resulting in any major changes of the magnetosome properties mentioned above. Other studies have shown that MTB bacterial amplification undertaken under different conditions than ours could yield genetic modifications of the MSR1 strain characterized by the deletion of mms and mam genes involved in crystal maturation in the MAI (Popa et al., 2009, Effect of oxidative stress on the growth of magnetic particles in Magnetospirillum magneticum. Int Microbiol 12, 49-57; Uebe and Schüler, 2016, Magnetosome biogenesis in magnetotactic bacteria. Nat Rev Microbiol 14, 621-637. https://doi.org/10.1038/nrmicro.2016.99; Ullrich et al., 2005, A Hypervariable 130-Kilobase Genomic Region of Magnetospirillum gryphiswaldense Comprises a Magnetosome Island Which Undergoes Frequent Rearrangements during Stationary Growth. J Bacteriol 187, 7176-7184. https://doi.org/10.1128/JB.187.21.7176-7184.2005). Interestingly, our conditions lead to much less drastic genetic changes, the only genetic variations that we observed occurring at a low occurrence rate of 94 and 85 %, first as a single nucleotide polymorphism in the gene ptsI 2 encoding a phosphoenolpyruvate protein phosphotransferase (Teplyakov et al., 2006, Structure of phosphorylated enzyme I, the phosphoenolpyruvate: sugar phosphotransferase system sugar translocation signal protein. Proc Natl Acad Sci USA 103, 16218-16223), and second as an insertion in the gene flhF which encodes the key protein FlhF required for complete flagellar synthesis (Li et al., 2020, Investigating the Role of FlhF Identifies Novel Interactions With Genes Involved in Flagellar Synthesis in Campylobacter jejuni. Frontiers in Microbiology 11.; Zhang and Wu, 2020, Flagella and Swimming Behavior of Marine Magnetotactic Bacteria. Biomolecules 10, 460. https://doi.org/10.3390/biom10030460). Furthermore, none of these modifications affected the genes responsible for magnetosome production in the MAI genetic region, therefore resulting in no apparent impact on magnetosome fabrication.

We attribute the successful magnetosome yield improvement during fed-batch production cultivation to the adaptation of MTB issued from PCB to the minimal growth medium. MTB originating from the PCB were first amplified in the minimal growth medium during production. The strain adapted itself to the minimal growth medium presumably by following the adaptative laboratory evolution (ALE) (Mavrommati et al., 2022, Adaptive laboratory evolution principles and applications in industrial biotechnology. Biotechnol Adv 54, 107795. https://doi.org/10.1016/j.biotechadv.2021.107795), in a similar manner as observed for other strains such as Geobacter sulfurreducens or E. Coli that resulted, after serial cultivations in a minimal medium, in an increased production of biomass and bacterial products of interest (Royce et al., 2015, Evolution for exogenous octanoic acid tolerance improves carboxylic acid production and membrane integrity. Metab Eng 29, 180-188. https://doi.org/10.1016/j.ymben.2015.03.014; Summers et al., 2012, Laboratory evolution of Geobacter sulfurreducens for enhanced growth on lactate via a single-base-pair substitution in a transcriptional regulator. ISME J 6, 975-983. https://doi.org/10.1038/ismej.2011.166). In addition, storage at -80°C over 16 months did not affect the increased production performances with PCB and successfully remained stable according to the high cell-load cryopreservation strategy that was developed.

In our study, we haven't only reduced the growth medium, but we have also shown a direct impact of the composition of such a medium in the magnetosome composition. In other words, the PCB that we have developed has the double advantage of being prepared in a minimal, non-toxic medium and of producing highly pure magnetosomes in iron. We drew our inspiration from a well-known property of MTB concerning the incorporation of other metals than iron such as Mn, Zn, Cu and Co inside magnetosomes, which is achieved by enriching MTB growth medium in these compounds (Muñoz et al., 2020, Magnetosomes could be protective shields against metal stress in magnetotactic bacteria. Sci Rep 10, 11430. https://doi.org/10.1038/s41598-020-68183-z; Tanaka et al., 2012, Highest levels of Cu, Mn and Co doped into nanomagnetic magnetosomes through optimized biomineralisation. J. Mater. Chem. 22, 11919-11921. https://doi.org/10.1039/C2JM31520C). Here we have carried out the experiment in reverse direction, i.e. we have reduced the composition of the MTB growth medium in other metals other than iron, to eliminate as much as possible their presence inside magnetosomes. The use of the PCB as starting inoculum allows the generation of highly pure magnetosomes in iron relatively to other metals, i.e. magnetosomes with a purity in iron of 99.935 %Fe. The level of purity is much higher than that obtained with the standard medium from Zhang et al. (2011) leading to ~ 93.6%Fe of iron per magnetosome or with a reduced minimal medium starting from the CCB resulting in ~ 99.8 %Fe of iron per magnetosome (Berny et al., 2020). While in terms of metallic composition, magnetosomes are characterized by the presence of an iron oxide core, in the absence of a specific treatment, some other non-metallic and non-toxic elements have been observed in small quantities (< 1.5 10-4 %Fe per element in average) such as Na, Ca, Mg and K which could under their cationic form surround the negatively charged magnetosome surface (Mickoleit et al., 2023, Highest levels of Cu, Mn and Co doped into nanomagnetic magnetosomes through optimized biomineralisation. J. Mater.

Chem. 22, 11919-11921. https://doi.org/10.1039/C2JM31520C).

**CONCLUSION.** Our study demonstrates the successful development of a Magnetospirillum gryphiswaldense MSR1 pharmaceutical cell bank, characterized by the same identity as the original MTB, a purity highlighted by an absence of contaminants, and a stability over 100 generations of MTB amplification, or following cryo-preservation after 16 months of storage. In addition, the realization of the PBC in a minimal growth medium enables to acclimate MTB to such medium, which in turn results in the production of highly pure magnetosomes containing an average of 99.935% of iron per magnetosome. Furthermore, the PCB is achieved under high cell load conditions of 9.108 cells/mL, which do not appear to be detrimental to bacterial survival, and enables the direct launch of a large-scale magnetosome production. Overall, our study presents the conditions for developing a dedicated cell bank of MSR1 strain adapted for the production of pharmaceutically compatible magnetosome dedicated to biomedical applications in the context of magnetosome industrial production and commercial sales.

**Claims**

1. A composition comprising at least one chain of at least two nanoparticles, wherein each nanoparticle in the chain comprises an iron oxide mineral core surrounded by a coating,

   wherein the composition further comprises a cryo-protectant,
   wherein the volume occupied by the cryo-protectant in the composition is larger than the volume occupied by at least one chain in the composition, by a factor of at least 1, 2, 5, 10 or $10^3$, wherein the percentage in mass of cryo-protectant in the composition is comprised between 0.5 and 50%,
   wherein the composition is isotonic.

2. The composition according to claim 1, wherein the composition comprises an organic part and an inorganic part, wherein the inorganic part preferentially comprises the core of the nanoparticle, wherein the organic part preferentially comprises the coating of the nanoparticle and/or the cryo-protectant, and wherein the percentage in mass of the inorganic part is larger than the percentage in mass of the organic part.

3. The composition according to claim 1, comprising i) and/or ii):

   i) the at least one nanoparticle comprising a) and b)::

   a) a core with at least one property selected in the group consisting of: i) a composition of iron oxide, preferentially essentially maghemite, preferentially with a percentage in mass of more than 0, 1, 50, 90, 99.6 % of iron in terms of metallic composition, ii) a size larger than 0, 1, 2, 5, 10, 20, 30 or 35 nm, and iii) a percentage in mass of organic material, preferentially of bacterial origin, preferentially non-denatured, that is lower than 100, 75, 50, 20, 5, 2 or 0%, and
   b) a surrounding coating with a thickness lower than the diameter of the core of the nanoparticle, and

   ii) a cryo-protectant, preferentially sorbitol, surrounding the at least one nanoparticle, where the percentages in mass of the nanoparticle core, nanoparticle coating and cryo-protectant, preferentially in the composition, most preferentially in the dried composition, have at least one property selected in the group consisting of:

   a) The percentage in mass of the nanoparticle core is larger than the percentages in mass of the nanoparticle coating and/or cryo-protectant,
   b) The percentage in mass of carbon or carbonaceous material of the nanoparticle core is lower than the percentage(s) in mass of carbon or carbonaceous material of the nanoparticle coating and/or cryo-protectant,
   c) The percentage in iron or metal mass of the nanoparticle core is larger than the percentage(s) in iron or metal mass of the nanoparticle coating and/or cryo-protectant,

   wherein preferentially the strength of the bond or interaction between the coating and core of the nanoparticle is stronger than the strength of the bond or interaction between the cryo-protectant and the coating and/or core of the nanoparticle,
   and/or wherein preferentially the coating and core form a complex,
   and/or wherein preferentially the cryo-protectant and nanoparticle don't form a complex.

4. The composition according to claim **1**, further comprising a compound, preferentially designated as the other compound, which has at least one property selected in the group consisting of:

   A) It is a chemical element, preferentially as listed in the periodic table of Mendeleev,
   B) It is selected in the group consisting of: i) Manganese, ii) Magnesium, iii), Potassium, iv) Calcium, v) Zinc, and vi) Sodium,

C) It originates from or is comprised in at least one chemical compound used to fabricate the at least one nanoparticle,

D) It originates from or is comprised in at least one medium used to amplify or grow the living organism, preferentially a magnetotactic bacterium, which synthetizes the at least one nanoparticle,

E) It is in ionic or charged form, preferentially in such a form that the interaction, preferentially electrostatic one, between the compound and the core or coating of the nanoparticles is favored,

F) It can be removed from the composition, for example by using a chelating agent or a solution containing a chelating agent that preferentially binds to such compound,

G) It has a concentration or percentage in mass in the composition that is lower than the concentration or percentage in mass of at least one of the following substances comprised in the composition: i) the nanoparticle, ii) the nanoparticle core, iii) the nanoparticle coating, and iv) the cryo-protectant,

I) It is comprised either outside of the nanoparticle core or at the surface of the nanoparticle core,

J) It is not in a crystalline form or does not predominantly contribute to the crystalline form or type of the nanoparticle.

5. The composition according to claim **1,** wherein the at least one chain of at least two nanoparticles has at least one property selected in the group consisting of:

A) the at least one chain exists during at least one step of fabrication or use of the at least two nanoparticle, preferentially selected among: i) amplification of a nanoparticle-producing cell, ii) purification or isolation of nanoparticles preferentially from some material or chemical, organic or not, preferentially originating from the at least one nanoparticle-cell, iii) coating or formulation or mixture of the nanoparticle preferentially with or between at least one or two constituent(s) of the composition, and iv) administration or presence of the at least one chain in or with a body part or cell or matrix or medium or water or gel or material or polymer,

B) the at least one chain comprises at least two nanoparticles, wherein the at least one first direction such as a crystallographic first direction or a first direction perpendicular to a nanoparticle facet or edge or surface or crystallographic plane or a first direction parallel to a nanoparticle diameter of a first nanoparticle is aligned with at least one second direction such as a crystallographic second direction or a second direction perpendicular to a nanoparticle facet or edge or surface or crystallographic plane or a second direction parallel to a nanoparticle diameter of a second nanoparticle, wherein the alignment of the first and second directions is preferentially **characterized by** an angle between the first and second directions that is smaller than 180, 90, 45, 30, 20, 10, 5, 2, 1, 0.1 or 0 °, wherein such angle is preferentially measured or preferentially exists in at least one moment in time and/or one location in space preferentially during the life time of the chain,

C) the at least one chain comprises at least two nanoparticles, which are separated by a distance larger than $10^{-3}$, 0, 1, 5, 10, $10^2$, $10^3$, $10^5$ or $10^9$ nm, preferentially when the at least two nanoparticle are dis-assembled or are not linked with each other by some binding material or interaction forces preferentially belonging to the composition but can preferentially be re-assembled preferentially by adding to or mixing with the at least two nanoparticles some binding material that preferentially re-assemble the at least two nanoparticles,

D) the at least one chain comprises at least two nanoparticles, which are separated by a distance smaller than $10^9$, $10^6$, $10^3$, 100, 50, 10, 5, 2, 1 or 0 nm, preferentially when the at least two nanoparticles are assembled or are linked with each other by some binding material or interaction forces preferentially belonging to the composition,

and

E) the at least one chain is in the form of: i) a powder, ii) a liquid, iii) a liquid suspension, iv) a solid, and/or v) one or a mixture of liquid, solid and/or gaseous state(s).

6. The composition according to claim **1**, wherein the at least one chain is in a liquid suspension and the composition has at least one of the following properties:

i) it is isotonic to animal plasma,
ii) the volume occupied by water in the composition is larger than the volume occupied by the at least one chain in the composition, and
iii) the percentage in mass of water in the composition is larger than the percentage in mass of the at least one chain in the composition,

7. The composition according to claim **1**, wherein the cryoprotectant is selected in the group consisting of: 1) Acetamide, 2) Acetate, 3) Albumin, 4) Amino acids, 5) Ammonium acetate, 6) Arginine, 7) Alcohols containing at least one or two hydroxyl groups, 8) Bridger, 9) Choline magnesium chloride sodium bromide, 10) Diethyl glycol, 11) Dimethylacetamide, 12) Dimethyl sulfoxide (DMSO), 13) Disaccharide, 14) Erythritol, 15) Ethanol, 16) Ethylene glycol, 17) Formamide, 18) Fructose, 19) Glucose, 20) Glycerol, 21) Glycerol 3-phosphate, 22) Glycol such as Diethyl glycol or Triethylene glycol, 23) Glycine, 24) Lactose, 25) L-tyrosine, 26) lysine hydrochloride, 27) Mannitol, 28) MDP (2-Methyl-2,4-pentanediol), 29) Phenylalanine, 30) Planic, 31) Polymers, 32) Polyethylene glycol such as PEG4000, polyethylene glycol succinate, folate modified distearoylphosphatidyl ethanolamine-polyethylene glycol, 33) Polyethyleneimine (PEI), 34) Polyvinylpyrrolidone (PVP), 35) Proline, 36) Propylene glycol, 37) Protein, 38) Pyridine (Pyridine-N-Oxide), 39) Ribose, 40) Sarcosine, 41) Serine, 42) Serum albumin, 43) Sodium bromide, 44) Sodium chloride, 45) Sodium dodecyl sulfonate, 46) Sodium glutamate, 47) Sodium iodide, 48) Sodium sulfate, 49) Sorbitol, 50) Starch (hydroxyethyl starch), 51) Sugar, 52) Sucrose, 53) The cell bank series, 54) Trehalose, 55) Triethylene glycol, 56) Trimethylamine, 57) Tween 80, 58) Tryptophan, 59) Valine, and 60) Xylose, and 61) a combination or derivative of any of these compounds.

8. The composition according to claim **1**, wherein the composition comprises an inert part and an active part, wherein the inner part does not comprise at least one center of activity or the active part comprises at least one center of activity or the active part comprises more or a larger number of centers of activity than the inner part, wherein the inner part preferentially ensures the cohesion of the composition or preferentially comprises links or bonds or forces or atoms or ions or nanoparticles that maintain the at least one or two constituent(s) of the composition assembled together within one volume, wherein the center of activity is comprised in the nanoparticle or at least one constituent of the composition, wherein the center of activity increases or decreases or amplifies or attenuates the production of heat or cold by the nanoparticle or at least one constituent of the composition, the medical activity of the nanoparticle or at least one constituent of the composition, and/or the radiation or strength or power or wavelength or intensity or frequency of the radiation applied on the nanoparticle or at least one constituent of the composition,

   where the center of activity is preferentially selected in the group consisting of:

   A) a thermal center that increases the thermal activity of the nanoparticle or at least one constituent of the composition, preferentially heat or the cold, preferentially by at least 0.1 °C, preferentially locally around the nanoparticle or at least one constituent of the composition or at a distance from the nanoparticle or at least one constituent of the composition of less than 1 μm,

   B) a thermal center that decreases the thermal activity of the nanoparticle or at least one constituent of the composition, preferentially heat or the cold, preferentially by at least 0.1 °C, preferentially locally around the nanoparticle or at least one constituent of the composition or at a distance from the nanoparticle or at least one constituent of the composition of less than 1 μm,

   C) a medical center that increases or enhances the effect or activity or strength of a medical compound, preferentially selected in the group consisting of: an enhancer of an immunotherapy, chemotherapy, hormonotherapy, radiotherapy or radio-enhancer, contrast agent, sonosensitizer, and surgical medical compound,

   D) a medical center that decreases the effect or activity or strength of a medical compound, preferentially selected in the group consisting of: an attenuator of an immunotherapy, chemotherapy, hormonotherapy, radiotherapy or radio-enhancer, contrast agent, sonosensitizer, and surgical medical compound,

   E) a center of radiation amplification that increases the strength of a radiation applied on the nanoparticle or at least one constituent of the composition, preferentially by at least $10^{-5}$ or 0.1 Gray or Watt or Candela, preferentially locally around the nanoparticle or at least one constituent of the composition or at a distance from the nanoparticle or at least one constituent of the composition of less than 1 μm,

   F) a center of radiation attenuation that decreases the strength of a radiation applied on the nanoparticle or at least one constituent of the composition, preferentially by at least $10^{-5}$ or 0.1 Gray or Watt or Candela, preferentially locally around the nanoparticle or at least one constituent of the composition or at a distance from the nanoparticle or at least one constituent of the composition of less than 1 μm, and

   G) a center of free radical production or capture,

   wherein the at least one center of activity is preferentially **characterized by** at least one property selected in the group consisting of:

      I) the nanoparticle core or at least a first constituent of the composition comprises a first center of activity, $C_{A1}$, preferentially being a first center of free radical production or capture $C_{1FRPC}$, wherein $C_{A1}$ or $C_{1FRPC}$ is preferentially selected in the group consisting of:

i) another metal than iron such as Zinc or Aluminum,
ii) another metal oxide than iron oxide such as Zinc Oxide or Aluminum Oxide,
iii) a compound that is essentially or in majority or at least partly inorganic or metallic,
and

II) the nanoparticle coating or second constituent of the composition comprises a second center of activity, $C_{A2}$, preferentially being a second center of free radical production or capture $C_{2FRPC}$, preferentially being a compound that is essentially or in majority or at least partly organic or non-metallic,
III) the cryo-protectant or third constituent of the composition comprises a third center of activity, $C_{A3}$, preferentially being a center of activity that protects or maintains or prevents the diminution or increases the activity of $C_{A1}$ and/or $C_{A2}$,
wherein $C_{A1}$, $C_{A2}$, and/or $C_{A3}$ has(have) at least one property selected in the group consisting of:

i) It(they) is(are) preferentially over time or under storage or under use of the composition or under administration of the composition to the body part.
ii) $C_{A1}$, $C_{A2}$, and/or $C_{A3}$ is(are) preferentially different compounds,
iii) $C_{A1}$, $C_{A2}$, and/or $C_{A3}$ activity can be measured by comparing the activity of a body part or medium comprising the composition with that of a body part or medium not comprising the composition, where the body part or medium comprising and not comprising the composition are exposed to similar or the same radiation or thermal variation,
iv) $C_{A1}$, $C_{A2}$, and/or $C_{A3}$ is(are) separated by a distance of at least 0.1, 1, 5, 10, 100 or $10^3$ nm,
v) $C_{A1}$, $C_{A2}$, and/or $C_{A3}$ is(are) preferentially different from at least one whole constituent of the composition, i.e. preferentially the at least one whole constituent preferentially comprises at least one other substance that is different from a center of activity,
vi) $C_{A1}$, $C_{A2}$, and/or $C_{A3}$ occupies(y) less than 100 or 99 or 50% of the volume or location of the at least one constituent,
and
vii) $C_{A1}$, $C_{A2}$, and/or $C_{A3}$ occupies(y) has(have) a percentage in mass of less than 100 or 99 or 50% relatively to the mass of the at least one constituent,
viii) $C_{A1}$, $C_{A2}$, and/or $C_{A3}$ release or diffuse, preferentially in an outward direction relatively to at least one constituent of the composition, or expel or activate at least one atom, electron, free radical, ion, metal, DNA, RNA, protein, lipid, enzyme, biological or non-biological material, organic or non-organic material, an immune entity from or of or in or at the surface or outside the nanoparticle or at least one constituent of the composition, preferentially against or to fight against a disease or preferentially against or to deactivate or kill at least one pathological cell or preferentially to activate a first type of immune entity that deactivates or kills at least one pathological cell or disease or preferentially to deactivate a second type of immune entity that protects at least one pathological cell or disease or tumor or tumor environment,
ix) $C_{A1}$, $C_{A2}$, and/or $C_{A3}$ capture or diffuse, preferentially in an inward direction relatively to at least one constituent of the composition, at least one atom, electron, free radical, ion, metal, DNA, RNA, protein, lipid, enzyme, biological or non-biological material, organic or non-organic material, an immune entity from or of or in or at the surface or inside the nanoparticle or at least one constituent of the composition, preferentially against or to fight against a disease or preferentially against or to deactivate or kill at least one pathological cell or preferentially to activate a first type of immune entity that deactivates or kills at least one pathological cell or disease or preferentially to deactivate a second type of immune entity that protects at least one pathological cell or disease or tumor or tumor environment,

wherein the at least one constituent of the composition is selected in the group consisting of: i) the nanoparticle coating, ii) the nanoparticle core, iii) the cryo-protectant, and iv) the other compound,

wherein the immune entity, preferentially the first and/or second immune entity(ies), is preferentially selected in the group consisting of: i) DNA preferentially different types of DNA, ii) RNA preferentially different types of RNA, iii) an antigen, ii) an antibody, iii) an immune cell, preferentially of or belonging to the innate and/or adaptative immune system(s), iv) an antigen presenting cell (APC), v) a basophil, vi) a dendritic cell, vii) an eosinophil, viii) a granulocyte, ix) a killing cell, x) a natural killer, xi) a leukocyte, xii) a lymphocyte, xiii) a macrophage, preferentially of M1 and/or M2 type(s), xiii) a mast cell, xiv) a neutrophil, xv) a phagocyte, xvi) a B cell, xvii) a T cell, xviii) a CD8 or CD8$^+$ or CD4 or CD4$^+$ or Treg or MAIT or T$\gamma\delta$ T lymphocyte or cell, xix) a helper cell preferentially of Th1 or Th2 type, and xx) a gamma delta T cell.

9. The composition according to any one of claims **1** to **9**, wherein the nanoparticle, preferentially the nanoparticle core, is synthesized by a living organism or nanoparticle producing cell, preferentially a magnetotactic bacterium.

10. The composition according to any one of claims **8**, wherein the center of activity is selected in the group consisting of:

A) a radio-sensitizer or amplificator of radiation, a radio-photosensitizer or amplificator of light radiation, an acoustic sensitizer or amplificator of acoustic radiation or wave, a sonosensitizer or amplificatory of acoustic wave, a particle radiation sensitizer or amplificator of particle radiation, where the particle comprises (or not) a mass, it is a thermal-sensitizer or amplificator of heat or cold or thermal treatment, an amplificator of the medical effect of a compound,

B) an attenuator of radiation, of light radiation, of acoustic radiation or wave, of particle radiation, where the particle comprises (or not) a mass, of heat or cold, of thermal treatment, and/or of the medical effect of a compound,

and

C) a compound, preferentially of meter or centimeter or millimeter or micrometer or nanometer or sub-nanometer or atomic size, selected in the group consisting of: 1) Acridine, such as Acridine Orange, acridine yellow, 2) ALA (5-Aminolevulinic acid), 3) Aluminum phthalocyanine tetrasulfonate (AlPcS4), 4) Aminolevulinic acid, delta- Aminolevulinic acid, 5) Antihistamines, 6) Azulene, 7) Bavteriochlorin, 8) TOOKAD or TOOKAD Soluble, 9) WST-11, 10) LUZ11, 11) BC19, 12) BC21, 13) porphyrin such as Benzoporphyrin derivative monoacid ring A (BPD-MA), 14) Chlorin such as Chlorin e6, m-tetrahydroxyphenylchlorin 15) Foscan, 16) Verteporfin, 17) benzoporphyrin derivative mono acid ring A, 18) Monoaspartyl chlorin(e6), 19) talaporfin sodium, 20) HPPH, 21) Transition metal compounds, 22) Chlorine e6 green porphrin, 23) Chlorine e6 porphrin, 24) Coal Tar and Derivatives, 25) Contraceptives, Oral and Estrogens, 26) Curcumin, 27) Cyanine, 28) Cysview, 29) Dyes such as synthetic dyes, 30) Phenothiazinium salts, 31) Rose Bengal, 32) Squaraines, 33) BODIPY dyes, 34) Phenalenones, 35) benzophenoxazinium dyes, 36) Erythrosine, 37) Flavins, 38) Foscan, 39) Fotoscan, 40) Fullerenes such as cationic fullerenes, 41) Furocoumarins, 42) HAL (Hexaminolevulinate), 43) Hemoporfin, 44) 2-(1-Hexyloxyethyl)-2-devinyl pyropheophorbide (HPPH), 45) Hypericin, 46) Hypocrellin, 47) ICG (Indocyanine Green), 48) Levulan, 49) MAL -methyl aminolevulinate), 50) Meta-tetra(hydroxyphenyl)chlorin (m-THPC), 51) Metvix, 52) Methylene Blue, 53) Monoterpene, 54) Motexafin lutetium (Lu-Tex), 54) N-aspartyl chlorin e6 (NPe6), 55) Nanoparticle or nanomaterial, 56) Natural products or compounds, 57) NonSteroidal Anti-Inflammatory Drugs, 58) Palladium bacteriopheophorbide (WST09), 59) Phatalocyanin dyes, 60) Phenothiazines, 61) Photochlor, 62) Photofrin, 63) Photosens, 64) Phthalocyanine such as Liposomal ZnPC, 65) Chloroaluminium sulfonated phthalocyanine (CASP), 66) Silicon phthalocyanine (PC4), 67) RLP068, 68) Porfimer sodium, 69) Porfins, 69) Porphyrins, such as 5, 10, 15,20-Tetrakis(1-methylpyridinium-4-yl) porphyrin tosylate, 70) XF70, 71) Protoporphyrin, 72) ALA-induced protoporphyrin IX, 73) Psoralens, 74) Quantum dots, 75) Quinones, 76) Riboflavin, 77) Rose Bengal, 78) silicon or Silicon phthalocyanine (Pc4), 79) Sulfonamides, 80) Sulfonylureas, 81) Talaporfin or Talaporfin soudium, 82) Temoporfin, 82) Tetrahydropyrroles, 83) Tin ethyl etiopurpurin, 84) Titanium dioxide, 85) Toldudine blue O, 86) Transition metal compounds such as Ruthenium(II), polypyridyl complexes, ruthenium, rhodium, cyclometalated, Rh(II)-Rh(II) bridged dimer compounds, platinum(II), gold(III), 87) Verteporfin, 88) Vulnic based compound such as Aminovulinic, aminovulinic acid, 89) WST11, and 90) Xanthene, 91) ABS-FA, 92) Acrylonitrile Butadiene Styrene, 93) Styrene, 94) Folic acid, 95) AIMP NP, aminoacyl tRNA synthetase complex-interacting multifunctional protein, 96) Au Nanomaterial, 97) gold, 98) Au-MnO nanomaterial, 99) manganese oxide, 100) Antineoplastic drugs, 101) NSAIDs, 102) nonsteroidal anti-inflammatory drug, 103) Artemether, 104) 5-ALA (5-aminolevulinic acid), 105) Acridine, Acridine Orange, 106) Au-doped TiO2, 107) Carbon based nanomaterial, 108) carbon nanotube, 109) Chlorine, 110) Ce6, 111) PTX, Paclitaxel, 112) chemotherapeutic drug or compound, 113) infrared dye or IR783, 114) Curcumin, 115) Cyanine or Cu-Cyanine, 116) DHMS, 117) dimethylsulfure, 118) Docetaxel, 119) chemotherapeutic drug or compound, 119) DOX/Mn-TPPS@RBCS, 120) doxorubicin, 121) manganese, 122) blood cell, 123) red blood cell, cell, 124) polymer, 125) elastomer, 126) Erythosin or Erythosin B, 127) FA or FA-OI or FA-OI NP or folic acid, 128) F3-PLGA@MB/Gd NPs, 129) poly(lactic-co-glycolic acid), 130) gadolinium, 131) Fe-TiO2 or titanium oxide, 132) Fe-VS$_2$, 133) iron, 134) vanadium disulfide, 135) FMSNs-DOX, 136) silica, 137) HCQ, 138) hydrochloroquine, 139) HP, 140) hematoporphyrin, 141) HMME, 142) hematoporphyrin monomethyl ether, 143) HSYA or Hydroxysafflor yellow A, 144) Hypocrellin, Hypocrellin B, 145) IR780, 146) Levofloxacin, 147) LIP3 or Lithium phosphide, 148) Lithium, 149) Liposome or Liposomal nanomaterial, 150) Lomefoxacin, 151) MG@P NPs, 152) MnP or Manganese peroxidase, 153) MnTTP-HSAs, 154) HSA-wrapped metal-porphyrin complex, 155) albumin, 156) MnWOx, 157) MnWOx-PEG, 158), PEG, 159) metallic or bimetallic or multi-metallic compound preferentially oxide, 160) Mn (III)-HFs, 161) managense, 162) hemoporfin, 163) nano-compound or nanoroad or nanoflower or nanowire or quantum dot, 164) Noble or halogen or Hydrogen or alkali metals or Alkaline earth metals or Triels or Tetrels or Pnicto-gen or Chal-cogens or metal or gas or liquid or solid preferentially nanomaterial, 165) oxygen indyocyanine preferentially nanoparticle, 166) Phthalocyanines, 167) PIO or Pioglitazone, 168) Polymeric nanomaterial, 169) Porphyrin, 170) Pt-doped TiO$_2$, 171) R837, 172) Rose Bengal, 173) Sparfloxacin, 174) TAPP or 5,10,15,20-tetrakis (4-aminophenyl) porphyrin, 175) TiO$_2$ or titanium dioxide nanomaterial, 176) TCPP, isomer, or Tris(1-chloro-2-propyl) phosphate 177) TPI or Thermoplastic Polyimide or thermoplastic polymer, 178) TPZ or Tirapazamine, 179) Transition metal oxide, 180) nanoparticle or Janus nanoparticle, and 181) Xanthones, 182) AQ4N, 183) Apaziquone (E09), 184) Bromodeoxyuridine, 185) Carbogen, 186) Cetuximab, 187) Chemotherapeutic drug or compound, 188) Chlorpromazine, 189) C-reactive peptide, 190) Curcumin, 191) Diamide, 192) Diethylmaeate, 193) Dihydroartemisinin, 194) Docetaxel, 195) ECI301, 196) Etanidazole, 197) Fludarabine, 198) 5-Fluorouracil, 199) Fluorodeoxyuridine, 200) Gadolynium, 201) Gemcitabine, 202) HER-3 ADC, 203) HSP, 204) Hydrogen peroxide, 205) Hydroxyurea, 206) Hyperbaric oxygen, 207) Hyperthermia, 208) Hypoxic cell cytotoxic agent, 209) Irinotecan, 210) lanthanide-doped radiosensitizer-based metal-phenolic network, 211) Lidocaine, 212) Lododeoxyuridine, 213) Metronidazole, 214) misonidazole, 215) etanidazole, 216) nimorazole, 217) N-Ethylmalemide, 218) malmeide, 219) ethylmalmeide, 220) Nanomaterial such as those consisting of or composed of at least partly or fully gold, silver, bismuth, gadolinium, polysiloxane matrix and gadolinium chelates, hafnium, Tantalum, Zinc, Gadolinium, Germanium, Chromium, Praseodymium, Silicon, iron, platinum, cobalt, manganese, magnesium, iron, Titanium, carbon nanotube, quantum dot, nanoroad, Triflate, or metal oxide, 221) Nelfinavir, 222) Nicotinamide, 223) Nimotuzumab, 224) RNA, or miRNA, or miR-201, or miR-205, or miR-144-5p, or miR-146a-5p, or miR-150, or miR-99a, or miR-139-5p, or miR-320a, 225) Membrane active agent, 226) Mitomycin-C or Mitomycin, 227) Motexafin, 228) NBTXR3, 229) Oligonucleotide, 230) Paclitaxel, 231) Papaverine or Papaverine hydrochloride, 232) Paraxonase-2, 233) Pocaine, 234) Porfiromycin (POR), 235) Protein, 236) Peptide, 237) Radiosensitizing nucleosides or compounds, 238) Resveratrol, 239) RRx-001, 240) SiRNa, 241) Suppressors of sulfhydral groups, 242) SYM004, 243) Texaphyrins, 244) TH-302, and 245) Tirapazamine.

11. The composition according to any of the claims **1** to **10** combined with at least one nanoparticle-producing cell, preferably a magnetotactic bacterium,

wherein the composition comprises a first cryo-protectant,
wherein the nanoparticle-producing cell comprises a second cryo-protectant,
wherein the first and second cryo-protectants are different compounds.

12. A method of fabrication or cryo-preservation of the composition optionally of the at least one cell producing the at least one nanoparticle comprised in the composition according to the invention or nanoparticle-producing cell, which comprises at least one of the following steps:

- **Step 1** of storing or cryo-preserving the at least one cell producing the at least one nanoparticle preferentially in a medium comprising a first cryo-protectant,
- **Step 2** of amplifying magnetotactic bacteria or nanoparticle-producing cells in at least one medium, comprising:

    1) the compounds necessary for the growth of magnetotactic bacteria or nanoparticle-producing cells and the production of magnetosomes or nanoparticles, which are preferentially selected in the group consisting of:

        - a source of carbon preferentially selected from the group consisting of: at least one compound comprising at least one atom of carbon, lactic acid, Na lactate, lactic acid, acetate, glycolate, glucose, pyruvate, succinate, carbon dioxide, glycerol and combinations thereof, at a concentration preferentially comprised between 1 nM and 2 Mol/L;
        - a source of iron preferentially selected from the group consisting of: at least one compound comprising at least one atom of iron, iron citrate, iron quinate, iron chloride, iron sulfate, $FeCl_3$, and combinations thereof, at a concentration preferentially comprised between 1 nM and $2.10^3$ Mol/L;
        - a source of nitrogen preferentially selected from the group consisting of: at least one compound comprising at least one atom of nitrogen, nitrate salt, nitrogen gas, ammonium, ammonia, ammonium salt, urea, an amino acid, ammonia gas, and combinations thereof, at a concentration preferentially comprised between 1 nM and 4 Mol/L;
        - a source of oxygen preferentially selected from the group consisting of: at least one compound comprising at least one atom of oxygen, oxygen or air or compressed air, preferentially in the form of a gas, the source of oxygen being in some cases bubbled or introduced to the growth medium, at a gas rate that is preferentially comprised between 5 mL of gas per minute and 50000 mL of gas per minute;
        - a source of phosphate preferentially consisting of at least one compound comprising at least one atom of phosphate, at a concentration preferentially comprised between 1 nM and $2.10^{-1}$ Mol/L;
        - a source of potassium preferentially consisting of at least one compound comprising at least one atom of potassium, at a concentration preferentially comprised between 1 nM and $2.10^{-1}$ Mol/L;
        - a source of sulfur or sulfate preferentially consisting of at least one compound comprising at least one atom of sulfur or sulfate, at a concentration preferentially comprised between 1 nM and $4.10^{-1}$ Mol/L;
        - a source of manganese preferentially consisting of at least one compound comprising at least one atom of manganese, at a concentration preferentially comprised between 1 nM and $4.10^{-1}$ Mol/L;
        - a source of vitamin preferentially selected from the group consisting of: at least one compound comprising at least one vitamin, Biotin, Calcium, pantothenate, Folic acid, Inositol, Nicotinic acid, p-Aminobenzoic acid, Pyridoxine HCl, Riboflavin, Thiamine, Thiamine HCL and derivatives thereof and combinations thereof, at a concentration preferentially comprised between 1 nM and $10^{-4}$ Mol/L, and
        - a source of calcium preferentially consisting of at least one compound comprising at least one atom of calcium, at a concentration preferentially comprised between 1 nM and $10^{-1}$ Mol/L.

    2) at least one compound preferentially necessary for doping the magnetosomes with $C_{A1}$ or $C_{1FRPC}$ or a center of activity or another metal than iron, preferentially zinc or aluminum, for example a source of zinc, preferentially zinc sulfate or zinc citrate or zinc chlorate or zinc quinate.

- **Step 3** of extracting or isolating magnetosomes or nanoparticles from magnetotactic bacteria or nanoparticle-producing cells;
- **Step 4** of purifying the extracted or isolated magnetosomes or nanoparticles preferentially by heating them preferentially to yield magnetosome minerals or nanoparticles comprising a percentage in mass of organic material preferentially originating from magnetotactic bacteria or from the at least one nanoparticle-producing cell that is lower than 100, 50, 20, 10, 5, 2 or 1%,
- **Step 5** of coating the magnetosome minerals or nanoparticles preferentially with a coating material, preferentially comprising the compound $C_{A2}$ or $C_{2FRPC}$ or a center of activity, preferentially by mixing the magnetosome minerals or nanoparticles with the coating material, where the mixing is preferentially realized in at least one of the following conditions:

under sonication,
under the application of radiation,
under temperature variation,
under pH changes,
under oxidoreduction potential adjustment,
using a ratio between the quantity or mass of magnetosome minerals and the quantity or mas of coating material, preferentially of compound D, that is adjusted or varied or larger than 1,

- **Step 6** of adding at least one cryoprotectant, preferentially the second cryo-protectant, to the coated magnetosome minerals or nanoparticles preferentially obtained at the end of step 5,
- **Step 7** of lyophilizing or dehydrating or drying or desiccating the composition preferentially obtained at the end of step 6,
- **Step 8** of re-suspending the lyophilized or dehydrated composition preferentially obtained of step 7, preferentially in water,

wherein the first and second cryo-protectants when they are present are compounds that are either the same or different compounds.

13. A method for the fabrication, storage, preservation, preservation of the geometric arrangement or assembly, preferentially chain arrangement of the at least one nanoparticle, cryo-preservation, size-preservation, composition-preservation, cohesion-preservation, magnetic property-preservation, or preservation of the composition or of at least one property of the composition or at least one constituent of the composition according to any one of claims **1** to **12,** which comprises at least one of the following steps:

- **Step 1:** Choosing or preparing the composition, preferentially by mixing at least one nanoparticle with a cryoprotectant or protectant compound,
- **Step 2:** Lyophilizing or desiccating or dehydrating or removing water or liquid or ion or atom, preferentially totally or partly or essentially different from iron, from the composition, applying a temperature or pressure gradient or temperature decrease or oxidation or reduction or radiation inducing a change of state to the composition preferentially originating from step 1,
- **Step 3:** Storing or keeping the composition preferentially essentially or totally or partly in a powder or solid form, preferentially originating from step 2, preferentially for a lapse of time of more than 1 second, 1 day, 1 month or 1 year,
- **Step 4:** (re)suspending or (re)dispersing the composition preferentially originating from step 3, preferentially in a liquid such as water or in a solid or in a gas sate, preferentially by or under sonication or radiation application, preferentially under sterile conditions, or preferentially prior to sterilization of the composition, preferentially in such a way that the nanoparticle maintains at least one of its property such as its chain arrangement, preferentially in such a way that the at least one nanoparticle can be injected to a body part or that the composition is isotonic.

14. A method for removing at least one compound from the composition according to any one of claims **1** to **14**, preferentially the compound defined in claim **4** by following at least one of the following steps:

- **Step 1:** Mixing the composition with at least one chelating agent and/or introducing at least one chelating agent in the composition,
- **Step 2:** Positioning a magnet near the composition to attract the magnetic nanoparticle in the region where the magnet is located,
- **Step 3:** Removing the part of the composition that has not been attracted by the magnet or that is not magnetic,
- **Step 4:** Re-suspending the magnetic nanoparticle or the part of the composition that is magnetic in a liquid, solid or gas, preferentially in a presence of a cryo-protectant,
- **Step 5:** Lyophilizing or desiccating or dehydrating or applying a temperature or pressure gradient or temperature decrease or oxidation or reduction or radiation inducing a change of state to the composition, preferentially originating from step 4,
- **Step 6:** Storing or keeping the composition preferentially originating from step 5, preferentially for a lapse of time of more than 1 second, 1 day, 1 month or 1 year,
- **Step 7:** (re)suspending or (re)dispersing the composition preferentially originating from step 6, preferentially in a liquid such as water or in a solid or in a gas sate, preferentially by or under sonication or radiation application, preferentially under sterile conditions, ore preferentially prior to sterilization of the composition, preferentially in such a way that the nanoparticle maintains at least one of its property such as its chain ar-

rangement, preferably in such a way that the at least one nanoparticle can be injected to a body part,

wherein the composition preferably comprises a magnetic part and/or a non-magnetic part, wherein the magnetic part of the composition is preferably a part of the composition that can be attracted or isolated or moved or modified partly or fully by a magnet, preferably of strength larger than the strength of the earth magnetic field or $10^{-6}$, $10^{-3}$ or $10^{-1}$, T, more importantly than the non-magnetic part.

15. A method for activating the composition or at least one center of activity of the composition, according to claim 1 by applying a radiation or physico-chemical disturbance on the composition for a sufficiently long time, preferably for more than 10-3, 1, 0, 1, 10, 103 second(s),

wherein the activation of the composition or of at least one constituent of the composition comprises at least one at least one of the following event(s) or step(s):

i) releasing or diffusing or triggering the release or diffusion, preferably in an outward direction relatively to at least one constituent (of) the composition, or activate at least one atom, electron, free radical, ion, metal, DNA, RNA, protein, lipid, enzyme, biological or non-biological material, organic or non-organic material, an immune entity from or of or in or at the surface or outside the nanoparticle or at least one constituent of the composition, preferably against or to fight against a disease or preferably against or to deactivate or kill at least one pathological cell or preferably to activate a first type of immune entity that deactivates or kills at least one pathological cell or disease or preferably to deactivate a second type of immune entity that protects at least one pathological cell or disease or tumor or tumor microenvironment,

ii) capturing or diffusing or triggering the release or diffusion, preferably in an inward direction relatively to at least one constituent (of) the composition, at least one atom, electron, free radical, ion, metal, DNA, RNA, protein, lipid, enzyme, biological or non-biological material, organic or non-organic material, an immune entity from or of or in or at the surface or inside the nanoparticle or at least one constituent of the composition, preferably against or to fight against a disease or preferably against or to deactivate or kill at least one pathological cell or preferably to activate a first type of immune entity that deactivates or kills at least one pathological cell or disease or preferentially to deactivate a second type of immune entity that protects at least one pathological cell or disease or tumor or tumor environment,

and

iii) activating or triggering the activation (of) at least one atom, electron, free radical, ion, metal, DNA, RNA, protein, lipid, enzyme, biological or non-biological material, organic or non-organic material, an immune entity from or of or in or at the surface or inside the nanoparticle or at least one constituent of the composition, preferably against or to fight against a disease or preferably against or to deactivate or kill at least one pathological cell or preferably to activate a first type of immune entity that deactivates or kills at least one pathological cell or disease or preferably to deactivate a second type of immune entity that protects at least one pathological cell or disease or tumor or tumor environment,

wherein the at least one constituent of the composition is selected in the group consisting of: i) the nanoparticle coating, ii) the nanoparticle core, iii) the cryo-protectant, and iv) the other compound,

wherein the immune entity, preferably the first and/or second immune entity(ies), is preferentially selected in the group consisting of: i) DNA preferably different types of DNA, ii) RNA preferably different types of RNA, iii) an antigen, ii) an antibody, iii) an immune cell, preferentially of or belonging to the innate and/or adaptative immune system(s), iv) an antigen presenting cell (APC), v) a basophil, vi) a dendritic cell, vii) an eosinophil, viii) a granulocyte, ix) a killing cell, x) a natural killer, xi) a leukocyte, xii) a lymphocyte, xiii) a macrophage, preferentially of M1 and/or M2 type(s), xiii) a mast cell, xiv) a neutrophil, xv) a phagocyte, xvi) a B cell, xvii) a T cell, xviii) a CD8 or CD8$^+$ or CD4 or CD4$^+$ or Treg or MAIT or T$\gamma\delta$ T lymphocyte or cell, xix) a helper cell preferentially of Th1 or Th2 type, and xx) a gamma delta T cell,

wherein the radiation is selected from the group consisting of: i) a magnetic or electric or electromagnetic field or wave, a wave a particulate radiation, ii) laser light, iii) light produced by a lamp, iv) light emitted at a single wavelength, v) light emitted at multiple wavelengths, vi) a ionizing radiation, vii) microwave, viii) radiofrequencies, and ix) a sound, an ultrasound an infrasound, or an acoustic wave.

wherein the physico-chemical disturbance is or is caused by or induces at least one action selected from the group consisting of:

i) a variation of an environment of at least one constituent of the composition, where the environment of the at least one constituent of the composition is a liquid, solid, or gaseous medium or at least one substance surrounding or including the at least one constituent of the composition,

ii) a variation of the environment of the at least one constituent of the composition selected from the group consisting of: a pH variation of this environment that is between $10^{-3}$ and 10 pH units, a variation in temperature of this environment that is between $10^{-13}$ and $10^3$ °C, a variation in redox potential of this environment that is between 0.001 and 100 V, a variation in viscosity of this environment that is between $10^{-9}$ and $10^{20}$ Pa.s, and a variation in the concentration of at least one substance of the environment that is between $10^{-13}$ and $10^{10}$ mole per liter, micromole per liter, nanomole per liter, mole per milliliter, micromole per milliliter, nano-mole per milliliter, mole per cubic meter, mole per cubic decimeter, mole per cubic centimeter or mole per cubic millimeter,

iii) a modification of at least one condition of the at least one constituent of the composition selected from the group consisting of a pH variation of the at least one constituent of the composition between $10^{-3}$ and 10 pH units, a temperature variation between $10^{-13}$ and $10^3$ ° C, a variation in standard potential between 0.001 V and 100 V, an increase or decrease in charge of the at least one constituent of the composition between 0.001 and 100 Volt, a variation between 1 and $10^{10}$ atom(s) in the number of atoms comprised in the at least one constituent of the composition,

iv) a variation of the concentration of at least one substance of an environment of the at least one constituent of the composition larger than $10^{-13}$ mole per liter, micromole per liter, nano-mole per liter, mole per milliliter, micromole per milliliter, nan-mole per milliliter, mole per cubic meter, mole per cubic decimeter, mole per cubic centimeter, or mole per cubic millimeter,

v) a variation in chemical composition of at least one substance of an environment of the at least one constituent of the composition of less than $10^{10}$ mole per liter, micromole per liter, nano-mole per liter, mole per milliliter, micromole per milliliter, nanomole per milliliter, mole per cubic meter, mole per cubic decimeter, mole per cubic centimeter, or mole per cubic millimeter,

vi) a modification of at least one substance in an environment of the at least one constituent of the composition is selected from the group consisting of a chemical modification, a structural modification , an appearance of at least one substance in the environment, a disappearance of at least one substance from the environment, and combinations thereof,

and

vii) a variation of chemical composition of less than 1010 substances in an environment of the at least one constituent of the composition, where the variation is selected from the group consisting of a chemical modification, a structural modification, an appearance of at least one substance in the environment or disappearance of at least one substance from the environment, and combinations thereof.

## EUROPEAN SEARCH REPORT

Application Number

EP 23 02 0495

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ALPHANDÉRY EDOUARD ED – BLANCO-PRIETO MARIA J ET AL: "Bio-synthesized iron oxide nanoparticles for cancer treatment", INTERNATIONAL JOURNAL OF PHARMACEUTICS, ELSEVIER, NL, vol. 586, 23 June 2020 (2020-06-23), XP086242237, ISSN: 0378-5173, DOI: 10.1016/J.IJPHARM.2020.119472 [retrieved on 2020-06-23] *"Different methods of iron oxide nanoparticle bio-synthesis" on page 2, left-hand column* *paragraph bridging the two columns on page 3* * figures 1, 2 * *"6. Conclusion" on page 11* ----- -/-- | 1-15 | INV. A61K9/19 A61K9/50 A61K41/00 |

TECHNICAL FIELDS SEARCHED (IPC)

A61K

The present search report has been drawn up for all claims

| Place of search | Date of completion of the search | Examiner |
|---|---|---|
| Munich | 9 February 2024 | Gerber, Myriam |

EPO FORM 1503 03.82 (P04C01)

page 1 of 2

## EUROPEAN SEARCH REPORT

Application Number

EP 23 02 0495

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | ALPHANDÉRY ET AL: "Applications of magnetotactic bacteria and magnetosome for cancer treatment: A review emphasizing on practical and mechanistic aspects", DRUG DISCOVERY TODAY, ELSEVIER, AMSTERDAM, NL, vol. 25, no. 8, 1 August 2020 (2020-08-01), pages 1444-1452, XP009544695, ISSN: 1359-6446, DOI: 10.1016/J.DRUDIS.2020.06.010 [retrieved on 2020-06-16] *"Preparations of magnetosomes / MTB" on page 1447* * tables 1, 2 * * figure 1 * *"Results obtained with stabilized magnetosome minerals" on pages 1450-1451* *"Magnetosome mechanisms of action" on page 1451* *"Concluding remarks and future perspectives" on page 1451* | 1-15 | |
| Y | WO 2011/061259 A1 (NANOBACTERIE [FR]; ALPHANDERY EDOUARD [FR] ET AL.) 26 May 2011 (2011-05-26) * examples 1-7 * * claim 22 * * figure 1 * | 1-15 | |
| | | | TECHNICAL FIELDS SEARCHED (IPC) |
| Y | WO 2008/052766 A2 (MEDIGENE AG [DE]; HAAS HEINRICH [DE]; RICCIO PAOLO [IT]) 8 May 2008 (2008-05-08) * page 15, lines 1-2 * * page 24, line 23 – page 25, line 1 * | 1-15 | |

The present search report has been drawn up for all claims

| Place of search | Date of completion of the search | Examiner |
|---|---|---|
| Munich | 9 February 2024 | Gerber, Myriam |

CATEGORY OF CITED DOCUMENTS

X : particularly relevant if taken alone
Y : particularly relevant if combined with another
　document of the same category
A : technological background
O : non-written disclosure
P : intermediate document

T : theory or principle underlying the invention
E : earlier patent document, but published on, or
　after the filing date
D : document cited in the application
L : document cited for other reasons

& : member of the same patent family, corresponding
　document

EPO FORM 1503 03.82 (P04C01)

page 2 of 2

## ANNEX TO THE EUROPEAN SEARCH REPORT
## ON EUROPEAN PATENT APPLICATION NO.

EP 23 02 0495

This annex lists the patent family members relating to the patent documents cited in the above-mentioned European search report.
The members are as contained in the European Patent Office EDP file on
The European Patent Office is in no way liable for these particulars which are merely given for the purpose of information.

09-02-2024

| Patent document cited in search report | | Publication date | Patent family member(s) | | Publication date |
|---|---|---|---|---|---|
| WO 2011061259 | A1 | 26-05-2011 | AU 2010320918 | A1 | 07-06-2012 |
| | | | BR 112012011767 | A2 | 27-03-2018 |
| | | | CA 2780851 | A1 | 26-05-2011 |
| | | | CN 102811726 | A | 05-12-2012 |
| | | | EP 2370085 | A1 | 05-10-2011 |
| | | | ES 2539588 | T3 | 02-07-2015 |
| | | | IL 219745 | A | 21-04-2016 |
| | | | JP 6202358 | B2 | 27-09-2017 |
| | | | JP 6469603 | B2 | 13-02-2019 |
| | | | JP 2013511491 | A | 04-04-2013 |
| | | | JP 2016155821 | A | 01-09-2016 |
| | | | JP 2018162317 | A | 18-10-2018 |
| | | | RU 2012122639 | A | 27-12-2013 |
| | | | US 2012302819 | A1 | 29-11-2012 |
| | | | US 2017157253 | A1 | 08-06-2017 |
| | | | US 2019201704 | A1 | 04-07-2019 |
| | | | WO 2011061259 | A1 | 26-05-2011 |
| WO 2008052766 | A2 | 08-05-2008 | EP 2086508 | A2 | 12-08-2009 |
| | | | US 2011002851 | A1 | 06-01-2011 |
| | | | WO 2008052766 | A2 | 08-05-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **ALPHANDÉRY et al.** *Drug Discovery Today,* 2020, vol. 25, 1444
- **BLAKEMORE.** *Ann. Rev Microbiol,* 1981, vol. 36, 117-38
- **SCHÜLER.** *J. Molec. Microbiol. Biotechnol.,* 1999, vol. 1 (1), 79-86
- **LEFEVRE, C.T. ; MENGUY, N. ; ABREU, F. ; LINS, U. ; POSFAI, M. ; PROZOROV, T. ; PIGNOL, D. ; FRANKEL, R.B. ; BAZYLINSKI, D.A.** A Cultured Greigite-Producing Magnetotactic Bacterium in a Novel Group of Sulfate-Reducing Bacteria. *Science,* 2011, vol. 334, 1720-1723
- **LAURENT et al.** *Chem. Rev.,* 2008, vol. 108, 2064-2110
- **LISY et al.** *Investigative Radiology,* April 2007, vol. 42 (4
- **MATSUNAGA et al.** *IEEE transactions on magnetics,* September 1990, vol. 26 (5
- **ZHANG et al.** *Applied and environmental microbiology,* September 2011, 5851-5856
- **TANAKA et al.** *J. Mater. Chem.,* 2012, vol. 22, 11919
- **ALPHANDERY et al.** *ACS Nano,* 2011, vol. 5 (8), 6279-6296
- **MARCANO et al.** *J. Phys. Chem. C,* 2018, vol. 124, 22827-22838
- *J. Phys. Chem. C,* 2018, vol. 122, 7541-7550
- **RIESE et al.** *Microb Cell Fact,* 2020, vol. 19, 206
- **NGUYEN et al.** *Applied Microbiology and Biotechnology,* 2023, vol. 107, 1159-1176
- **HAREL ; HAREL.** *Cell & Tissue Transplantation & Therapy,* 2013, vol. 5, 1-7
- **SOOD et al.** Inoculum Preparation. Elsevier, 2011, 151-164
- **SETH.** Recent advances in optimal cell banking of mammalian cells for biopharmaceutical production. *Pharmaceutical Bioprocessing,* 2015, vol. 3, 35-43, https://doi.org/10.4155/pbp.14.46
- **SOBOLEWSKA-RUTA ; ZALESKI.** Sobolewska-Ruta. *Advancements of Microbiology,* 2019, vol. 58, 87-100, https://doi.org/10.21307/PM-2019.58.1.087
- **KIM et al.** Method for the Industrial Use of <small>D</small>-Amino Acid Oxidase-Overexpressing <I>Escherichia coli</I>. *Bioscience, Biotechnology, and Biochemistry,* 2009, vol. 73, 299-303, https://doi.org/10.1271/bbb.80507

- **STANBURY et al.** Principles of Fermentation Technology. Butterworth-Heinemann, 2017, 335-399
- **MÜLLER et al.** Seed Train Intensification Using an Ultra-High Cell Density Cell Banking Process. *Processes,* 2022, vol. 10, 911, https://doi.org/10.3390/pr10050911
- **HEYEN ; SCHÜLER.** Growth and magnetosome formation by microaerophilic Magnetospirillum strains in an oxygen-controlled fermentor. *Appl Microbiol Biotechnol,* 2003, vol. 61, 536-544, https://doi.org/10.1007/s00253-002-1219-x
- **SEIDEL et al.** Oxygen Mass Transfer in Biopharmaceutical Processes: Numerical and Experimental Approaches. *Chemie Ingenieur Technik,* 2021, vol. 93, 42-61, https://doi.org/10.1002/cite.202000179
- **ZHANG et al.** Semicontinuous Culture of Magnetospirillum gryphiswaldense MSR-1 Cells in an Autofermentor by Nutrient-Balanced and Isosmotic Feeding Strategies. *Appl Environ Microbiol,* 2011, vol. 77, 5851-5856, https://doi.org/10.1128/AEM.05962-11
- **BERNY et al.** A Method for Producing Highly Pure Magnetosomes in Large Quantity for Medical Applications Using Magnetospirillum gryphiswaldense MSR-1 Magnetotactic Bacteria Amplified in Minimal Growth Media. *Front. Bioeng. Biotechnol.,* 2020, vol. 8, 16, https://doi.org/10.3389/fbioe.2020.00016
- **NGUYEN et al.** Non-pyrogenic highly pure magnetosomes for efficient hyperthermia treatment of prostate cancer. *Appl Microbiol Biotechnol,* 2023, vol. 107, 1159-1176, https://doi.org/10.1007/s00253-022-12247-9
- **SCHÜLER ; BAEUERLEIN.** Iron-limited growth and kinetics of iron uptake in Magnetospirillum gryphiswaldense. *Arch Microbiol,* 1996, vol. 166, 301-307, https://doi.org/10.1007/s002030050387
- **UEBE ; SCHÜLER.** Magnetosome biogenesis in magnetotactic bacteria. *Nat Rev Microbiol,* 2016, vol. 14, 621-637, https://doi.org/10.1038/nrmicro.2016.99
- **POPA et al.** Effect of oxidative stress on the growth of magnetic particles in Magnetospirillum magneticum. *Int Microbiol,* 2009, vol. 12, 49-57

- **ULLRICH et al.** Genomic Region of Magnetospirillum gryphiswaldense Comprises a Magnetosome Island Which Undergoes Frequent Rearrangements during Stationary Growth. *J Bacteriol,* 2005, vol. 187, 7176-7184, https://doi.org/10.1128/JB.187.21.7176-7184.2005
- **GUO et al.** A novel rapid and continuous procedure for large-scale purification of magnetosomes from Magnetospirillum gryphiswaldense. *Appl Microbiol Biotechnol,* 2012, vol. 90, 1277-1283, https://doi.org/10.1007/s00253-011-3189-3
- **FRAHM.** Animal Cell Biotechnology: Methods and Protocols, Methods in Molecular Biology. Humana Press, 2014, 355-367
- **HORNBAEK et al.** The effect of inoculum age and solid versus liquid propagation on inoculum quality of an industrial Bacillus licheniformis strain. *FEMS Microbiol Lett,* 2004, vol. 236, 145-151, https://doi.org/10.1016/j.femsle.2004.05.035
- **YAMAMOTO et al.** Reduction in the length of the lag phase of l-lactate fermentation by the use of inocula from electrodialysis seed cultures. *Journal of Fermentation and Bioengineering,* 1993, vol. 76, 151-152, https://doi.org/10.1016/0922-338X(93)90074-I
- **MULDER et al.** Critical aspects to be considered prior to large-scale production of peptides. *Curr Protein Pept Sci,* 2013, vol. 14, 556-567, https://doi.org/10.2174/13892037113149990071
- **WHITFORD.** Bioprocess intensification: aspirations and achievements. *Biotechniques,* 2020, vol. 69, 84-87, https://doi.org/10.2144/btn-2020-0072
- **PETERSON et al.** Bacterial cell surface damage due to centrifugal compaction. *Appl Environ Microbiol,* 2012, vol. 78, 120-125, https://doi.org/10.1128/AEM.06780-11
- **PEMBREY et al.** Cell Surface Analysis Techniques: What Do Cell Preparation Protocols Do to Cell Surface Properties?. *Appl Environ Microbiol,* 1999, vol. 65, 2877-2894
- **MAZUR ; COLE.** Influence of cell concentration on the contribution of unfrozen fraction and salt concentration to the survival of slowly frozen human erythrocytes. *Cryobiology,* 1985, vol. 22, 509-536, https://doi.org/10.1016/0011-2240(85)90029-x
- **ULLRICH et al.** A Hypervariable 130-Kilobase Genomic Region of Magnetospirillum gryphiswaldense Comprises a Magnetosome Island Which Undergoes Frequent Rearrangements during Stationary Growth. *J Bacteriol,* 2005, vol. 187, 7176-7184, https://doi.org/10.1128/JB.187.21.7176-7184.2005
- **TEPLYAKOV et al.** Structure of phosphorylated enzyme I, the phosphoenolpyruvate: sugar phosphotransferase system sugar translocation signal protein. *Proc Natl Acad Sci USA,* 2006, vol. 103, 16218-16223
- **LI et al.** Investigating the Role of FlhF Identifies Novel Interactions With Genes Involved in Flagellar Synthesis in Campylobacter jejuni. *Frontiers in Microbiology,* 2020, vol. 11
- **ZHANG ; WU.** Flagella and Swimming Behavior of Marine Magnetotactic Bacteria. *Biomolecules,* 2020, vol. 10, 460, https://doi.org/10.3390/biom10030460
- **MAVROMMATI et al.** Adaptive laboratory evolution principles and applications in industrial biotechnology. *Biotechnol Adv,* 2022, vol. 54, 107795, https://doi.org/10.1016/j.biotechadv.2021.107795
- **ROYCE et al.** Evolution for exogenous octanoic acid tolerance improves carboxylic acid production and membrane integrity. *Metab Eng,* 2015, vol. 29, 180-188, https://doi.org/10.1016/j.ymben.2015.03.014
- **SUMMERS et al.** Laboratory evolution of Geobacter sulfurreducens for enhanced growth on lactate via a single-base-pair substitution in a transcriptional regulator. *ISME J,* 2012, vol. 6, 975-983, https://doi.org/10.1038/ismej.2011.166
- **MUÑOZ et al.** Magnetosomes could be protective shields against metal stress in magnetotactic bacteria. *Sci Rep,* 2020, vol. 10, 11430, https://doi.org/10.1038/s41598-020-68183-z
- **TANAKA et al.** Highest levels of Cu, Mn and Co doped into nanomagnetic magnetosomes through optimized biomineralisation. *J. Mater. Chem.,* 2012, vol. 22, 11919-11921, https://doi.org/10.1039/C2JM31520C
- **MICKOLEIT et al.** Highest levels of Cu, Mn and Co doped into nanomagnetic magnetosomes through optimized biomineralisation. *J. Mater. Chem.,* 2023, vol. 22, 11919-11921, https://doi.org/10.1039/C2JM31520C